(19) Europäisches Patentamt
European Patent Office
Office européen des brevets

(11) EP 4 001 418 A1

(12) EUROPEAN PATENT APPLICATION

(43) Date of publication:
**25.05.2022 Bulletin 2022/21**

(21) Application number: **20207634.5**

(22) Date of filing: **13.11.2020**

(51) International Patent Classification (IPC):
***C12N 15/82*** *(2006.01)* ***C07K 14/325*** *(2006.01)*

(52) Cooperative Patent Classification (CPC):
**C12N 15/8286; C07K 14/325**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Centro de Tecnologia Canavieira S.A**
**01452-000 Jardim Paulistano São Paulo (BR)**

(72) Inventors:
- **YANAGUI, Karina**
**13.400-970 Piracicaba São Paulo (BR)**
- **KAUPERT NETO, Antonio Adalberto**
**13.400-970 Piracicaba São Paulo (BR)**
- **ZAKIR PEREIRA, Ana Carolina Vieira**
**13.400-970 Piracicaba São Paulo (BR)**
- **LORENA SERENO, Maria**
**13.400-970 Piracicaba São Paulo (BR)**
- **CHEAVEGATTI GIANOTTO, Adriana**
**13.400-970 Piracicaba São Paulo (BR)**
- **GENTILE, Agustina**
**13.400-970 Piracicaba São Paulo (BR)**
- **CUTRI, Lucas**
**13.400-970 Piracicaba São Paulo (BR)**
- **SALLES DE OLIVEIRA, Wladecir**
**13.400-970 Piracicaba São Paulo (BR)**

(74) Representative: **Williams, Andrea**
**Marks & Clerk LLP**
**62-68 Hills Road**
**Cambridge CB2 1LA (GB)**

# (54) POLYNUCLEOTIDES, PRIMERS, AND METHODS FOR DETECTION OF TRANSGENIC EVENT, GENETIC CONSTRUCT, KIT FOR DETECTION MATERIAL FROM A PLANT SAMPLE, EVENT CTC79005-2, INSECT-RESISTANT SUGARCANE PLANT, AND METHOD FOR PRODUCING AN INSECT-RESISTANT SUGARCANE PLANT, PLANT CELL, PLANT PART OR SEED

(57) The present invention relates to the field of biotechnology. More precisely, a genetic construct and method for producing a transgenic plant event, especially a sugarcane event (*Saccharum* spp.), which is resistant to infestation by the *Diatraea saccharalis* pest, popularly known as a pest, is described ordinary borer, reed borer or just borer. The invention describes the event, the methods for event identification as well as the insertion detection method based on the unique region of intersection between the insert and the host genome and the flanking regions that characterize it.

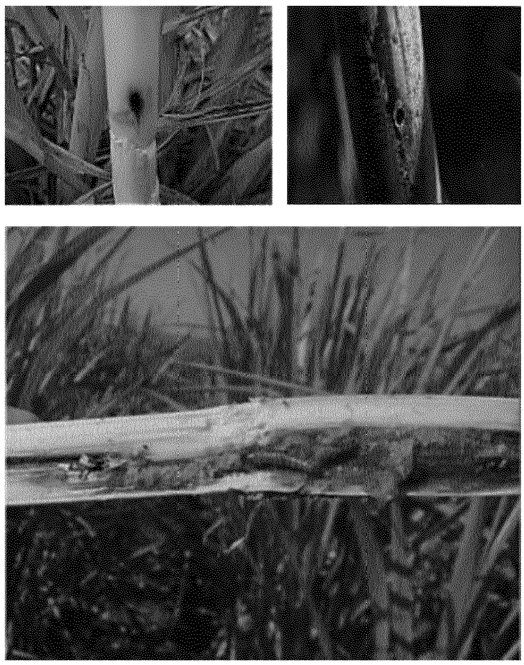

Figure 1

EP 4 001 418 A1

SEQUENCE LISTING

<110> CTC - Centro de Tecnologia Canavieira S.A.

<120> POLYNUCLEOTIDES, PRIMERS, AND METHODS FOR DETECTION OF TRANSGENIC EVENT, GENETIC CONSTRUCT, KIT FOR DETECTION MATERIAL FROM A PLANT SAMPLE, EVENT CTC93209-4, INSECT-RESISTANT SUGARCANE PLANT, AND METHOD FOR PRODUCING AN INSECT-RESISTANT SUGARCANE PLANT, PLANT CELL, PLANT PART OR SEED

<130> P249050-CN

<160> 94

<170> PatentIn version 3.5

<210> 1
<211> 7479
<212> DNA
<213> Artificial sequence

<220>
<223> Genetic construct comprising Cry1Ac and nptII genes

<400> 1
tggcaggata tattgtggtg taaacaaatt gacgcttaga caacttaata acacattgcg 60

gacgttttta atgtactgaa ttagtactga tatcggtacc cggggcggcc gcgagctcga 120

tccactagta acggccgcca gtgtgctgga attcgccctt ggcgcgccga tctagtaaca 180

tagatgacac cgcgcgcgat aatttatcct agtttgcgcg ctatattttg ttttctatcg 240

cgtattaaat gtataattgc gggactctaa tcataaaaac ccatctcata aataacgtca 300

tgcattacat gttaattatt acatgcttaa cgtaattcaa cagaaattat atgataatca 360

tcgcaagacc ggcaacagga ttcaatctta agaaacttta ttgccaaatg tttgaacgat 420

ctcagaagaa ctcgtcaaga aggcgataga aggcgatgcg ctgcgaatcg ggagcggcga 480

taccgtaaag cacgaggaag cggtcagccc attcgccgcc aagctcttca gcaatatcac 540

gggtagccaa cgctatgtcc tgatagcggt ccgccacacc cagccggcca cagtcgatga 600

atccagaaaa gcggccattt tccaccatga tattcggcaa gcaggcatcg ccatgggtca 660

cgacgagatc ctcgccgtcg ggcatgcgcg ccttgagcct ggcgaacagt tcggctggcg 720

cgagcccctg atgctcttcg tccagatcat cctgatcgac aagaccggct tccatccgag 780

tacgtgctcg ctcgatgcga tgtttcgctt ggtggtcgaa tgggcaggta gccggatcaa 840

gcgtatgcag ccgccgcatt gcatcagcca tgatggatac tttctcggca ggagcaaggt 900

gagatgacag gagatcctgc cccggcactt cgcccaatag cagccagtcc cttcccgctt 960

cagtgacaac gtcgagcaca gctgcgcaag gaacgcccgt cgtggccagc cacgatagcc 1020

gcgctgcctc gtcctgcagt tcattcaggg caccggacag gtcggtcttg acaaaaagaa 1080

ccgggcgccc ctgcgctgac agccggaaca cggcggcatc agagcagccg attgtctgtt 1140

gtgcccagtc atagccgaat agcctctcca cccaagcggc cggagaacct gcgtgcaatc 1200
catcttgttc aatccacatt ctagagtcga cctgcagaag taacaccaaa caacagggtg 1260
agcatcgaca aaagaaacag taccaagcaa ataaatagcg tatgaaggca gggctaaaaa 1320
aatccacata tagctgctgc atatgccatc atccaagtat atcaagatca aaataattat 1380
aaaacatact tgtttattat aatagatagg tactcaaggt tagagcatat gaatagatgc 1440
tgcatatgcc atcatgtata tgcatcagta aaacccacat caacatgtat acctatccta 1500
gatcgatatt tccatccatc ttaaactcgt aactatgaag atgtatgaca cacacataca 1560
gttccaaaat taataaatac accaggtagt ttgaaacagt attctactcc gatctagaac 1620
gaatgaacga ccgcccaacc acaccacatc atcacaacca agcgaacaaa aagcatctct 1680
gtatatgcat cagtaaaacc cgcatcaaca tgtataccta tcctagatcg atatttccat 1740
ccatcatctt caattcgtaa ctatgaatat gtatggcaca cacatacaga tccaaaatta 1800
ataaatccac caggtagttt gaaacagaat tctactccga tctagaacga ccgcccaacc 1860
agaccacatc atcacaacca agacaaaaaa aagcatgaaa agatgacccg acaaacaagt 1920
gcacggcata tattgaaata aaggaaaagg gcaaaccaaa ccctatgcaa cgaaacaaaa 1980
aaaatcatga aatcgatccc gtctgcggaa cggctagagc catcccagga ttccccaaag 2040
agaaacactg gcaagttagc aatcagaacg tgtctgacgt acaggtcgca tccgtgtacg 2100
aacgctagca gcacggatct aacacaaaca cggatctaac acaaacatga acagaagtag 2160
aactaccggg ccctaaccat ggaccggaac gccgatctag agaaggtaga gagggggggg 2220
gggggaggac gagcggcgta ccttgaagcg gaggtgccga cgggtggatt tgggggagat 2280
ctggttgtgt gtgtgtgcgc tccgaacaac acgaggttgg ggaaagaggg tgtggagggg 2340
gtgtctattt attacggcgg gcgaggaagg gaaagcgaag gagcggtggg aaaggaatcc 2400
cccgtagctg ccgtgccgtg agaggaggag gaggccgcct gccgtgccgg ctcacgtctg 2460
ccgctccgcc acgcaatttc tggatgccga cagcggagca agtccaacgg tggagcggaa 2520
ctctcgagag gggtccagag gcagcgacag agatgccgtg ccgtctgctt cgcttggccc 2580
gacgcgacgc tgctggttcg ctggttggtg tccgttagac tcgtcgacgg cgtttaacag 2640
gctggcatta tctactcgaa acaagaaaaa tgtttcctta gtttttttaa tttcttaaag 2700
ggtatttgtt taatttttag tcactttatt ttattctatt ttatatctaa attattaaat 2760
aaaaaaacta aaatagagtt ttagttttct taatttagag gctaaaatag aataaaatag 2820
atgtactaaa aaaattagtc tataaaaacc attaacccta aaccctaaat ggatgtacta 2880
ataaaatgga tgaagtatta tataggtgaa gctatttgca aaaaaaaagg agaacacatg 2940
cacactaaaa agataaaact gtagagtcct gttgtcaaaa tactcaattg tcctttagac 3000
catgtctaac tgttcattta tatgattctc taaaacactg atattattgt agtactatag 3060

attatattat tcgtagagta aagtttaaat atatgtataa agatagataa actgcacttc 3120
aaacaagtgt gacaaaaaaa atatgtggta atttttttata acttagacat gcaatgctca 3180
ttatctctag agaggggcac gaccgggtca cgctgcactg caggcatacg cgtgatccac 3240
tagtaacggc cgccagtgtg ctggaattcg cccttggcgc gccgatctag taacatagat 3300
gacaccgcgc gcgataattt atcctagttt gcgcgctata ttttgttttc tatcgcgtat 3360
taaatgtata attgcgggac tctaatcata aaaacccatc tcataaataa cgtcatgcat 3420
tacatgttaa ttattacatg cttaacgtaa ttcaacagaa attatatgat aatcatcgca 3480
agaccggcaa caggattcaa tcttaagaaa ctttattgcc aaatgtttga acgatctcac 3540
tcagcctcga gggtggcggt cactgggatg aactcgaacc tgtcgatgat cacgccggcg 3600
gtgccgctga agttcctcac gcccacgatg ttaccgaggg aggaggtgaa agcgttggcg 3660
ctctcgaagt aaccgaagtc gctggattgg aggttgtcca gggaggtagc ggtagctggc 3720
acggtgttgg agaagatgga ggagttaccc cagttcacgt tgaggtggat cggggtcacg 3780
gaagcgtacc tcacgcgcac cctgtacctg gtggaggtgg atgggaagtg gattggcacc 3840
tcgatgtagc ccctgttctg gatgttgttg ccgctgctgt tgagcctcac gaggtcgcca 3900
ccggtgaagc ctgggccgga aatgacggaa ccgttgaaga ggaagttgcc cttcacggcc 3960
gggatttggg tgatgctgtc ggaggcgatg atgttgttga actcagcgct gcggtggatc 4020
caggagaaca tcggagccct gatgatgctc acggagctgt tgctgaagcc ggagcggaac 4080
atggacacgt ggctcagcct gtgggagaag ccttgcctgg gtggcacgtt gttgttctgt 4140
ggtgggatct cgtccaggga gtccacggtg ccgctcttcc tgtagacagc ggatggcagg 4200
ttggaggagg tgccgtaggc gaactcggtg ccgtcgagca cggacagttg ctggttgttg 4260
ataccgatgt tgaagggcct cctgtacagg gtggaggaca gggtcctgta gacaccctga 4320
cccagttgag ccacgatgcg ttgctgtgga gcggcgttgc ccatggtgcc gtagagcggg 4380
aaggtgaact cggggccgct gaagcccact ggggaggcca tgatctggtg gccggaccag 4440
tagtactcgc ccctgtgagc gtcggtgtag atggtgatgc tgttcaggat gtccatcagg 4500
tgtgggctcc tgatggagcc ctcgataccc tgggcggaac cgcggaagct accgtcgaag 4560
ttctccagca ctgggttggt gtagatctcc ctggtgagtt gggacacggt gcggatcggg 4620
taggtcctgg agtcgtagtt cgggaagagg gacacaatgt ccagcacggt gagggtcaac 4680
tccctcctga actggttgta cctgatccag tccctggagt ccggacccca gacgcgctcc 4740
aggccggtgt tgtaccagcg cacagcgtgg tcggtgtagt tgccaatcag cctggtcagg 4800
tcgttgtagc ggctgttgat ggtggcagca tcgaagcccc acctttggcc gaacacgctc 4860
acgtcgcgca gcacgctgag gtgcaggtta gcggcttgca cgtacacgga caggagcggc 4920

acttggtagt tctggacggc gaacagtggg atagcggtgg tcagggcgct gttcatgtcg 4980
ttgaattgaa tgcgcatttc ctcgcggaga gctgggttgg tcgggtcggc ctcccactcc 5040
ctgaagctct cggcgtagat ttggtagagg ttgctcaggc cctccagcct ggagatggcc 5100
tggttcctgg cgaactcttc gatcctctgg ttgatcagct gctcgatttg caccaggaag 5160
gcgtcccatt gggatggacc gaagataccc cagatgatgt ccaccaggcc gagcacgaag 5220
ccagcacctg gcacgaactc gctgagcagg aactgggtca aggacaggga gatgtcgatg 5280
ggggtgtaac cggtctcgat gcgctcgcca cccagcacct ccacctctgg gttgctcagg 5340
cagttgtatg ggatgcactc gttgatgttt gggttgttgt ccattctaga gtcgacctgc 5400
agaagtaaca ccaaacaaca gggtgagcat cgacaaaaga aacagtacca agcaaataaa 5460
tagcgtatga aggcagggct aaaaaaatcc acatatagct gctgcatatg ccatcatcca 5520
agtatatcaa gatcaaaata attataaaac atacttgttt attataatag ataggtactc 5580
aaggttagag catatgaata gatgctgcat atgccatcat gtatatgcat cagtaaaacc 5640
cacatcaaca tgtataccta tcctagatcg atatttccat ccatcttaaa ctcgtaacta 5700
tgaagatgta tgacacacac atacagttcc aaaattaata aatacaccag gtagtttgaa 5760
acagtattct actccgatct agaacgaatg aacgaccgcc caaccacacc acatcatcac 5820
aaccaagcga acaaaaagca tctctgtata tgcatcagta aaacccgcat caacatgtat 5880
acctatccta gatcgatatt tccatccatc atcttcaatt cgtaactatg aatatgtatg 5940
gcacacacat acagatccaa aattaataaa tccaccaggt agtttgaaac agaattctac 6000
tccgatctag aacgaccgcc caaccagacc acatcatcac aaccaagaca aaaaaaagca 6060
tgaaaagatg acccgacaaa caagtgcacg gcatatattg aaataaagga aaagggcaaa 6120
ccaaacccta tgcaacgaaa caaaaaaaat catgaaatcg atcccgtctg cggaacggct 6180
agagccatcc caggattccc caaagagaaa cactggcaag ttagcaatca gaacgtgtct 6240
gacgtacagg tcgcatccgt gtacgaacgc tagcagcacg gatctaacac aaacacggat 6300
ctaacacaaa catgaacaga agtagaacta ccgggcccta accatggacc ggaacgccga 6360
tctagagaag gtagagaggg gggggggggg aggacgagcg gcgtaccttg aagcggaggt 6420
gccgacgggt ggatttgggg gagatctggt tgtgtgtgtg tgcgctccga acaacacgag 6480
gttggggaaa gagggtgtgg agggggtgtc tatttattac ggcgggcgag gaagggaaag 6540
cgaaggagcg gtgggaaagg aatccccgt agctgccgtg ccgtgagagg aggaggaggc 6600
cgcctgccgt gccggctcac gtctgccgct ccgccacgca atttctggat gccgacagcg 6660
gagcaagtcc aacggtggag cggaactctc gagaggggtc cagaggcagc gacagagatg 6720
ccgtgccgtc tgcttcgctt ggcccgacgc gacgctgctg gttcgctggt tggtgtccgt 6780
tagactcgtc gacggcgttt aacaggctgg cattatctac tcgaaacaag aaaaatgttt 6840

ccttagtttt tttaatttct taaagggtat ttgtttaatt tttagtcact ttattttatt 6900

ctattttata tctaaattat taaataaaaa aactaaaata gagttttagt tttcttaatt 6960

tagaggctaa aatagaataa aatagatgta ctaaaaaaat tagtctataa aaaccattaa 7020

ccctaaaccc taaatggatg tactaataaa atggatgaag tattatatag gtgaagctat 7080

ttgcaaaaaa aaaggagaac acatgcacac taaaaagata aaactgtaga gtcctgttgt 7140

caaaatactc aattgtcctt tagaccatgt ctaactgttc atttatatga ttctctaaaa 7200

cactgatatt attgtagtac tatagattat attattcgta gagtaaagtt taaatatatg 7260

tataaagata gataaactgc acttcaaaca agtgtgacaa aaaaaatatg tggtaatttt 7320

ttataactta gacatgcaat gctcattatc tctagagagg ggcacgaccg ggtcacgctg 7380

cactgcaggc atcgatcgaa gcttacgcgt ccatggctgc aggagctcgt cgactctaga 7440

ctcgagggcg cgccgacagg atatattggc gggtaaacc 7479

<210> 2
<211> 7382
<212> DNA
<213> Artificial sequence

<220>
<223> T-DNA fragment event CTC79005

<400> 2
ttgtggtgta aacaaattga cgcttagaca acttaataac acattgcgga cgtttttaat 60

gtactgaatt agtactgata tcggtacccg ggcggccgc gagctcgatc cactagtaac 120

ggccgccagt gtgctggaat tcgcccttgg cgcgccgatc tagtaacata gatgacaccg 180

cgcgcgataa tttatcctag tttgcgcgct atattttgtt ttctatcgcg tattaaatgt 240

ataattgcgg gactctaatc ataaaaaccc atctcataaa taacgtcatg cattacatgt 300

taattattac atgcttaacg taattcaaca gaaattatat gataatcatc gcaagaccgg 360

caacaggatt caatcttaag aaactttatt gccaaatgtt tgaacgatct cagaagaact 420

cgtcaagaag gcgatagaag gcgatgcgct gcgaatcggg agcggcgata ccgtaaagca 480

cgaggaagcg gtcagcccat tcgccgccaa gctcttcagc aatatcacgg gtagccaacg 540

ctatgtcctg atagcggtcc gccacaccca gccggccaca gtcgatgaat ccagaaaagc 600

ggccattttc caccatgata ttcggcaagc aggcatcgcc atgggtcacg acgagatcct 660

cgccgtcggg catgcgcgcc ttgagcctgg cgaacagttc ggctggcgcg agcccctgat 720

gctcttcgtc cagatcatcc tgatcgacaa gaccggcttc catccgagta cgtgctcgct 780

cgatgcgatg tttcgcttgg tggtcgaatg ggcaggtagc cggatcaagc gtatgcagcc 840

gccgcattgc atcagccatg atggatactt tctcggcagg agcaaggtga gatgacagga 900

gatcctgccc cggcacttcg cccaatagca gccagtccct tcccgcttca gtgacaacgt 960
cgagcacagc tgcgcaagga acgcccgtcg tggccagcca cgatagccgc gctgcctcgt 1020
cctgcagttc attcagggca ccggacaggt cggtcttgac aaaaagaacc gggcgcccct 1080
gcgctgacag ccggaacacg gcggcatcag agcagccgat tgtctgttgt gcccagtcat 1140
agccgaatag cctctccacc caagcggccg gagaacctgc gtgcaatcca tcttgttcaa 1200
tccacattct agagtcgacc tgcagaagta acaccaaaca acagggtgag catcgacaaa 1260
agaaacagta ccaagcaaat aaatagcgta tgaaggcagg gctaaaaaaa tccacatata 1320
gctgctgcat atgccatcat ccaagtatat caagatcaaa ataattataa aacatacttg 1380
tttattataa tagataggta ctcaaggtta gagcatatga atagatgctg catatgccat 1440
catgtatatg catcagtaaa acccacatca acatgtatac ctatcctaga tcgatatttc 1500
catccatctt aaactcgtaa ctatgaagat gtatgacaca cacatacagt tccaaaatta 1560
ataaatacac caggtagttt gaaacagtat tctactccga tctagaacga atgaacgacc 1620
gcccaaccac accacatcat cacaaccaag cgaacaaaaa gcatctctgt atatgcatca 1680
gtaaaacccg catcaacatg tatacctatc ctagatcgat atttccatcc atcatcttca 1740
attcgtaact atgaatatgt atggcacaca catacagatc caaaattaat aaatccacca 1800
ggtagtttga aacagaattc tactccgatc tagaacgacc gcccaaccag accacatcat 1860
cacaaccaag acaaaaaaaa gcatgaaaag atgacccgac aaacaagtgc acggcatata 1920
ttgaaataaa ggaaaagggc aaaccaaacc ctatgcaacg aaacaaaaaa aatcatgaaa 1980
tcgatcccgt ctgcggaacg gctagagcca tcccaggatt ccccaaagag aaacactggc 2040
aagttagcaa tcagaacgtg tctgacgtac aggtcgcatc cgtgtacgaa cgctagcagc 2100
acggatctaa cacaaacacg gatctaacac aaacatgaac agaagtagaa ctaccgggcc 2160
ctaaccatgg accggaacgc cgatctagag aaggtagaga gggggggggg gggaggacga 2220
gcggcgtacc ttgaagcgga ggtgccgacg ggtggatttg gggagatct ggttgtgtgt 2280
gtgtgcgctc cgaacaacac gaggttgggg aaagagggtg tggaggggt gtctatttat 2340
tacggcgggc gaggaaggga aagcgaagga gcggtgggaa aggaatcccc cgtagctgcc 2400
gtgccgtgag aggaggagga ggccgcctgc cgtgccggct cacgtctgcc gctccgccac 2460
gcaatttctg gatgccgaca gcggagcaag tccaacggtg gagcggaact ctcgagaggg 2520
gtccagaggc agcgacagag atgccgtgcc gtctgcttcg cttggcccga cgcgacgctg 2580
ctggttcgct ggttggtgtc cgttagactc gtcgacggcg tttaacaggc tggcattatc 2640
tactcgaaac aagaaaaatg tttccttagt ttttttaatt tcttaaaggg tatttgttta 2700
atttttagtc actttatttt attctatttt atatctaaat tattaaataa aaaaactaaa 2760
atagagtttt agttttctta atttagaggc taaaatagaa taaaatagat gtactaaaaa 2820

aattagtcta taaaaaccat taaccctaaa ccctaaatgg atgtactaat aaaatggatg 2880

aagtattata taggtgaagc tatttgcaaa aaaaaaggag aacacatgca cactaaaaag 2940

ataaaactgt agagtcctgt tgtcaaaata ctcaattgtc ctttagacca tgtctaactg 3000

ttcatttata tgattctcta aaacactgat attattgtag tactatagat tatattattc 3060

gtagagtaaa gtttaaatat atgtataaag atagataaac tgcacttcaa acaagtgtga 3120

caaaaaaaat atgtggtaat tttttataac ttagacatgc aatgctcatt atctctagag 3180

aggggcacga ccgggtcacg ctgcactgca ggcatacgcg tgatccacta gtaacggccg 3240

ccagtgtgct ggaattcgcc cttggcgcgc cgatctagta acatagatga caccgcgcgc 3300

gataatttat cctagtttgc gcgctatatt ttgttttcta tcgcgtatta aatgtataat 3360

tgcgggactc taatcataaa aacccatctc ataaataacg tcatgcatta catgttaatt 3420

attacatgct taacgtaatt caacagaaat tatatgataa tcatcgcaag accggcaaca 3480

ggattcaatc ttaagaaact ttattgccaa atgtttgaac gatctcactc agcctcgagg 3540

gtggcggtca ctgggatgaa ctcgaacctg tcgatgatca cgccggcggt gccgctgaag 3600

ttcctcacgc ccacgatgtt accgagggag gaggtgaaag cgttggcgct ctcgaagtaa 3660

ccgaagtcgc tggattggag gttgtccagg gaggtagcgg tagctggcac ggtgttggag 3720

aagatggagg agttacccca gttcacgttg aggtggatcg gggtcacgga agcgtacctc 3780

acgcgcaccc tgtacctggt ggaggtggat gggaagtgga ttggcacctc gatgtagccc 3840

ctgttctgga tgttgttgcc gctgctgttg agcctcacga ggtcgccacc ggtgaagcct 3900

gggccggaaa tgacggaacc gttgaagagg aagttgccct tcacggccgg gatttgggtg 3960

atgctgtcgg aggcgatgat gttgttgaac tcagcgctgc ggtggatcca ggagaacatc 4020

ggagccctga tgatgctcac ggagctgttg ctgaagccgg agcggaacat ggacacgtgg 4080

ctcagcctgt gggagaagcc ttgcctgggt ggcacgttgt tgttctgtgg tgggatctcg 4140

tccagggagt ccacggtgcc gctcttcctg tagacagcgg atggcaggtt ggaggaggtg 4200

ccgtaggcga actcggtgcc gtcgagcacg gacagttgct ggttgttgat accgatgttg 4260

aagggcctcc tgtacagggt ggaggacagg gtcctgtaga caccctgacc cagttgagcc 4320

acgatgcgtt gctgtggagc ggcgttgccc atggtgccgt agagcgggaa ggtgaactcg 4380

gggccgctga agcccactgg ggaggccatg atctggtggc cggaccagta gtactcgccc 4440

ctgtgagcgt cggtgtagat ggtgatgctg ttcaggatgt ccatcaggtg tgggctcctg 4500

atggagccct cgataccctg ggcggaaccg cggaagctac cgtcgaagtt ctccagcact 4560

gggttggtgt agatctccct ggtgagttgg gacacggtgc ggatcgggta ggtcctggag 4620

tcgtagttcg ggaagaggga cacaatgtcc agcacggtga gggtcaactc cctcctgaac 4680

tggttgtacc tgatccagtc cctggagtcc ggaccccaga cgcgctccag gccggtgttg 4740
taccagcgca cagcgtggtc ggtgtagttg ccaatcagcc tggtcaggtc gttgtagcgg 4800
ctgttgatgg tggcagcatc gaagccccac ctttggccga acacgctcac gtcgcgcagc 4860
acgctgaggt gcaggttagc ggcttgcacg tacacggaca ggagcggcac ttggtagttc 4920
tggacggcga acagtgggat agcggtggtc agggcgctgt tcatgtcgtt gaattgaatg 4980
cgcatttcct cgcggagagc tgggttggtc gggtcggcct cccactccct gaagctctcg 5040
gcgtagattt ggtagaggtt gctcaggccc tccagcctgg agatggcctg gttcctggcg 5100
aactcttcga tcctctggtt gatcagctgc tcgatttgca ccaggaaggc gtcccattgg 5160
gatggaccga agatacccca gatgatgtcc accaggccga gcacgaagcc agcacctggc 5220
acgaactcgc tgagcaggaa ctgggtcaag gacagggaga tgtcgatggg ggtgtaaccg 5280
gtctcgatgc gctcgccacc cagcacctcc acctctgggt tgctcaggca gttgtatggg 5340
atgcactcgt tgatgtttgg gttgttgtcc attctagagt cgacctgcag aagtaacacc 5400
aaacaacagg gtgagcatcg acaaaagtgc tgcatatgcc atcatccaag tatatcaaga 5460
tcaaaataat tataaaacat acttgtttat tataatagat aggtactcaa ggttagagca 5520
tatgaataga tgctgcatat gccatcatgt atatgcatca gtaaaaccca catcaacatg 5580
tatacctatc ctagatcgat atttccatcc atcttaaact cgtaactatg aagatgtatg 5640
acacacacat acagttccaa aattaataaa tacaccaggt agtttgaaac agtattctac 5700
tccgatctag aacgaatgaa cgaccgccca accacaccac atcatcacaa ccaagcgaac 5760
aaaaagcatc tctgtatatg catcagtaaa acccgcatca acatgtatac ctatcctaga 5820
tcgatatttc catccatcat cttcaattcg taactatgaa tatgtatggc acacacatac 5880
agatccaaaa ttaataaatc caccaggtag tttgaaacag aattctactc cgatctagaa 5940
cgaccgccca accagaccac atcatcacaa ccaagacaaa aaaaagcatg aaaagatgac 6000
ccgacaaaca agtgcacggc atatattgaa ataaaggaaa agggcaaacc aaaccctatg 6060
caacgaaaca aaaaaaatca tgaaatcgat cccgtctgcg gaacggctag agccatccca 6120
ggattcccca aagagaaaca ctggcaagtt agcaatcaga acgtgtctga cgtacaggtc 6180
gcatccgtgt acgaacgcta gcagcacgga tctaacacaa acacggatct aacacaaaca 6240
tgaacagaag tagaactacc gggccctaac catggaccgg aacgccgatc tagagaaggt 6300
agagaggggg gggggggag gacgagcggc gtaccttgaa gcggaggtgc cgacgggtgg 6360
atttggggga gatctggttg tgtgtgtgtg cgctccgaac aacacgaggt tggggaaaga 6420
gggtgtggag ggggtgtcta tttattacgg cgggcgagga agggaaagcg aaggagcggt 6480
gggaaaggaa tcccccgtag ctgccgtgcc gtgagaggag gaggaggccg cctgccgtgc 6540
cggctcacgt ctgccgctcc gccacgcaat ttctggatgc cgacagcgga gcaagtccaa 6600

cggtggagcg gaactctcga gaggggtcca gaggcagcga cagagatgcc gtgccgtctg 6660

cttcgcttgg cccgacgcga cgctgctggt tcgctggttg gtgtccgtta gactcgtcga 6720

cggcgtttaa caggctggca ttatctactc gaaacaagaa aaatgtttcc ttagtttttt 6780

taatttctta aagggtattt gtttaatttt tagtcacttt attttattct attttatatc 6840

taaattatta aataaaaaaa ctaaaataga gttttagttt tcttaattta gaggctaaaa 6900

tagaataaaa tagatgtact aaaaaaatta gtctataaaa accattaacc ctaaacccta 6960

aatggatgta ctaataaaat ggatgaagta ttatataggt gaagctattt gcaaaaaaaa 7020

aggagaacac atgcacacta aaaagataaa actgtagagt cctgttgtca aaatactcaa 7080

ttgtccttta gaccatgtct aactgttcat ttatatgatt ctctaaaaca ctgatattat 7140

tgtagtacta tagattatat tattcgtaga gtaaagttta aatatatgta taaagataga 7200

taaactgcac ttcaaacaag tgtgacaaaa aaaatatgtg gtaatttttt ataacttaga 7260

catgcaatgc tcattatctc tagagagggg cacgaccggg tcacgctgca ctgcaggcat 7320

cgatcgaagc ttacgcgtcc atggctgcag gagctcgtcg actctagact cgagggcgag 7380

cc 7382

<210> 3
<211> 206
<212> DNA
<213> Artificial sequence

<220>
<223> Junction nucleotide sequence between 5′ region of insert and sugarcane genome of event CTC79005

<400> 3
ggagaggcga agccctaggc cggcgctgtc gccacgccag gatcggtcgc ccaggttgac 60

gtgactgggt tgcctgcgct gaacctaata acacattgct gggttgtggt gtaaacaaat 120

tgacgcttag acaacttaat aacacattgc ggacgttttt aatgtactga attagtactg 180

atatcggtac ccggggcggc cgcgag 206

<210> 4
<211> 206
<212> DNA
<213> Artificial sequence

<220>
<223> Junction nucleotide sequence between 3′ region of insert and sugarcane genome of event CTC79005

<400> 4
ctagagaggg gcacgaccgg gtcacgctgc actgcaggca tcgatcgaag cttacgcgtc 60

catggctgca ggagctcgtc gactctagac tcgagggcga gccccgagcg gggcagctg 120

```
aggcgccatt gggaggagga agagggcagc gctccggggc ggaagtcgtg gggcggcacg      180

agggaagggc acacgacgga cggacg                                           206
```

```
<210>  5
<211>  7588
<212>  DNA
<213>  Artificial sequence

<220>
<223>  Event CTC79005: flanking sequences and T-DNA

<400>  5
ggagaggcga agccctaggc cggcgctgtc gccacgccag gatcggtcgc ccaggttgac       60

gtgactgggt tgcctgcgct gaacctaata acacattgct gggttgtggt gtaaacaaat      120

tgacgcttag acaacttaat aacacattgc ggacgttttt aatgtactga attagtactg      180

atatcggtac ccggggcggc cgcgagctcg atccactagt aacggccgcc agtgtgctgg      240

aattcgccct tggcgcgccg atctagtaac atagatgaca ccgcgcgcga taatttatcc      300

tagtttgcgc gctatatttt gttttctatc gcgtattaaa tgtataattg cgggactcta      360

atcataaaaa cccatctcat aaataacgtc atgcattaca tgttaattat tacatgctta      420

acgtaattca acagaaatta tatgataatc atcgcaagac cggcaacagg attcaatctt      480

aagaaacttt attgccaaat gtttgaacga tctcagaaga actcgtcaag aaggcgatag      540

aaggcgatgc gctgcgaatc gggagcggcg ataccgtaaa gcacgaggaa gcggtcagcc      600

cattcgccgc caagctcttc agcaatatca cgggtagcca acgctatgtc ctgatagcgg      660

tccgccacac ccagccggcc acagtcgatg aatccagaaa agcggccatt ttccaccatg      720

atattcggca agcaggcatc gccatgggtc acgacgagat cctcgccgtc gggcatgcgc      780

gccttgagcc tggcgaacag ttcggctggc gcgagcccct gatgctcttc gtccagatca      840

tcctgatcga caagaccggc ttccatccga gtacgtgctc gctcgatgcg atgtttcgct      900

tggtggtcga atgggcaggt agccggatca agcgtatgca gccgccgcat tgcatcagcc      960

atgatggata ctttctcggc aggagcaagg tgagatgaca ggagatcctg ccccggcact     1020

tcgcccaata gcagccagtc ccttcccgct tcagtgacaa cgtcgagcac agctgcgcaa     1080

ggaacgcccg tcgtggccag ccacgatagc cgcgctgcct cgtcctgcag ttcattcagg     1140

gcaccggaca ggtcggtctt gacaaaaaga accgggcgcc cctgcgctga cagccggaac     1200

acggcggcat cagagcagcc gattgtctgt tgtgcccagt catagccgaa tagcctctcc     1260

acccaagcgg ccggagaacc tgcgtgcaat ccatcttgtt caatccacat tctagagtcg     1320

acctgcagaa gtaacaccaa acaacagggt gagcatcgac aaaagaaaca gtaccaagca     1380

aataaatagc gtatgaaggc agggctaaaa aaatccacat atagctgctg catatgccat     1440

catccaagta tatcaagatc aaaataatta taaaacatac ttgtttatta taatagatag     1500
```

gtactcaagg ttagagcata tgaatagatg ctgcatatgc catcatgtat atgcatcagt 1560

aaaacccaca tcaacatgta tacctatcct agatcgatat ttccatccat cttaaactcg 1620

taactatgaa gatgtatgac acacacatac agttccaaaa ttaataaata caccaggtag 1680

tttgaaacag tattctactc cgatctagaa cgaatgaacg accgcccaac cacaccacat 1740

catcacaacc aagcgaacaa aaagcatctc tgtatatgca tcagtaaaac ccgcatcaac 1800

atgtatacct atcctagatc gatatttcca tccatcatct tcaattcgta actatgaata 1860

tgtatggcac acacatacag atccaaaatt aataaatcca ccaggtagtt tgaaacagaa 1920

ttctactccg atctagaacg accgcccaac cagaccacat catcacaacc aagacaaaaa 1980

aaagcatgaa aagatgaccc gacaaacaag tgcacggcat atattgaaat aaaggaaaag 2040

ggcaaaccaa accctatgca acgaaacaaa aaaaatcatg aaatcgatcc cgtctgcgga 2100

acggctagag ccatcccagg attccccaaa gagaaacact ggcaagttag caatcagaac 2160

gtgtctgacg tacaggtcgc atccgtgtac gaacgctagc agcacggatc taacacaaac 2220

acggatctaa cacaaacatg aacagaagta gaactaccgg gccctaacca tggaccggaa 2280

cgccgatcta gagaaggtag agaggggggg gggggagga cgagcggcgt accttgaagc 2340

ggaggtgccg acgggtggat ttgggggaga tctggttgtg tgtgtgtgcg ctccgaacaa 2400

cacgaggttg gggaaagagg gtgtggaggg ggtgtctatt tattacggcg ggcgaggaag 2460

ggaaagcgaa ggagcggtgg gaaaggaatc ccccgtagct gccgtgccgt gagaggagga 2520

ggaggccgcc tgccgtgccg gctcacgtct gccgctccgc cacgcaattt ctggatgccg 2580

acagcggagc aagtccaacg gtggagcgga actctcgaga ggggtccaga ggcagcgaca 2640

gagatgccgt gccgtctgct tcgcttggcc cgacgcgacg ctgctggttc gctggttggt 2700

gtccgttaga ctcgtcgacg gcgtttaaca ggctggcatt atctactcga aacaagaaaa 2760

atgtttcctt agttttttta atttcttaaa gggtatttgt ttaattttta gtcactttat 2820

tttattctat tttatatcta aattattaaa taaaaaaact aaaatagagt tttagttttc 2880

ttaatttaga ggctaaaata gaataaaata gatgtactaa aaaaattagt ctataaaaac 2940

cattaaccct aaaccctaaa tggatgtact aataaaatgg atgaagtatt atataggtga 3000

agctatttgc aaaaaaaaag gagaacacat gcacactaaa aagataaaac tgtagagtcc 3060

tgttgtcaaa atactcaatt gtcctttaga ccatgtctaa ctgttcattt atatgattct 3120

ctaaaacact gatattattg tagtactata gattatatta ttcgtagagt aaagtttaaa 3180

tatatgtata aagatagata aactgcactt caaacaagtg tgacaaaaaa aatatgtggt 3240

aatttttat aacttagaca tgcaatgctc attatctcta gagaggggca cgaccgggtc 3300

acgctgcact gcaggcatac gcgtgatcca ctagtaacgg ccgccagtgt gctggaattc 3360

gcccttggcg cgccgatcta gtaacataga tgacaccgcg cgcgataatt tatcctagtt 3420

tgcgcgctat attttgtttt ctatcgcgta ttaaatgtat aattgcggga ctctaatcat 3480

aaaaacccat ctcataaata acgtcatgca ttacatgtta attattacat gcttaacgta 3540

attcaacaga aattatatga taatcatcgc aagaccggca acaggattca atcttaagaa 3600

actttattgc caaatgtttg aacgatctca ctcagcctcg agggtggcgg tcactgggat 3660

gaactcgaac ctgtcgatga tcacgccggc ggtgccgctg aagttcctca cgcccacgat 3720

gttaccgagg gaggaggtga aagcgttggc gctctcgaag taaccgaagt cgctggattg 3780

gaggttgtcc agggaggtag cggtagctgg cacggtgttg gagaagatgg aggagttacc 3840

ccagttcacg ttgaggtgga tcggggtcac ggaagcgtac ctcacgcgca ccctgtacct 3900

ggtggaggtg gatgggaagt ggattggcac ctcgatgtag cccctgttct ggatgttgtt 3960

gccgctgctg ttgagcctca cgaggtcgcc accggtgaag cctgggccgg aaatgacgga 4020

accgttgaag aggaagttgc ccttcacggc cgggatttgg gtgatgctgt cggaggcgat 4080

gatgttgttg aactcagcgc tgcggtggat ccaggagaac atcggagccc tgatgatgct 4140

cacggagctg ttgctgaagc cggagcggaa catggacacg tggctcagcc tgtgggagaa 4200

gccttgcctg ggtggcacgt tgttgttctg tggtgggatc tcgtccaggg agtccacggt 4260

gccgctcttc ctgtagacag cggatggcag gttggaggag gtgccgtagg cgaactcggt 4320

gccgtcgagc acggacagtt gctggttgtt gataccgatg ttgaagggcc tcctgtacag 4380

ggtggaggac agggtcctgt agacaccctg acccagttga gccacgatgc gttgctgtgg 4440

agcggcgttg cccatggtgc cgtagagcgg gaaggtgaac tcggggccgc tgaagcccac 4500

tggggaggcc atgatctggt ggccggacca gtagtactcg cccctgtgag cgtcggtgta 4560

gatggtgatg ctgttcagga tgtccatcag gtgtgggctc ctgatggagc cctcgatacc 4620

ctgggcggaa ccgcggaagc taccgtcgaa gttctccagc actgggttgg tgtagatctc 4680

cctggtgagt tgggacacgg tgcggatcgg gtaggtcctg gagtcgtagt tcgggaagag 4740

ggacacaatg tccagcacgg tgagggtcaa ctccctcctg aactggttgt acctgatcca 4800

gtccctggag tccggacccc agacgcgctc caggccggtg ttgtaccagc gcacagcgtg 4860

gtcggtgtag ttgccaatca gcctggtcag gtcgttgtag cggctgttga tggtggcagc 4920

atcgaagccc cacctttggc cgaacacgct cacgtcgcgc agcacgctga ggtgcaggtt 4980

agcggcttgc acgtacacgg acaggagcgg cacttggtag ttctggacgg cgaacagtgg 5040

gatagcggtg gtcagggcgc tgttcatgtc gttgaattga atgcgcattt cctcgcggag 5100

agctgggttg gtcgggtcgg cctcccactc cctgaagctc tcggcgtaga tttggtagag 5160

gttgctcagg ccctccagcc tggagatggc ctggttcctg gcgaactctt cgatcctctg 5220

gttgatcagc tgctcgattt gcaccaggaa ggcgtcccat tgggatggac cgaagatacc 5280

ccagatgatg tccaccaggc cgagcacgaa gccagcacct ggcacgaact cgctgagcag 5340
gaactgggtc aaggacaggg agatgtcgat gggggtgtaa ccggtctcga tgcgctcgcc 5400
acccagcacc tccacctctg ggttgctcag gcagttgtat gggatgcact cgttgatgtt 5460
tgggttgttg tccattctag agtcgacctg cagaagtaac accaaacaac agggtgagca 5520
tcgacaaaag tgctgcatat gccatcatcc aagtatatca agatcaaaat aattataaaa 5580
catacttgtt tattataata gataggtact caaggttaga gcatatgaat agatgctgca 5640
tatgccatca tgtatatgca tcagtaaaac ccacatcaac atgtatacct atcctagatc 5700
gatatttcca tccatcttaa actcgtaact atgaagatgt atgacacaca catacagttc 5760
caaaattaat aaatacacca ggtagtttga aacagtattc tactccgatc tagaacgaat 5820
gaacgaccgc ccaaccacac cacatcatca caaccaagcg aacaaaaagc atctctgtat 5880
atgcatcagt aaaacccgca tcaacatgta tacctatcct agatcgatat ttccatccat 5940
catcttcaat tcgtaactat gaatatgtat ggcacacaca tacagatcca aaattaataa 6000
atccaccagg tagtttgaaa cagaattcta ctccgatcta gaacgaccgc ccaaccagac 6060
cacatcatca caaccaagac aaaaaaaagc atgaaaagat gacccgacaa acaagtgcac 6120
ggcatatatt gaaataaagg aaaagggcaa accaaaccct atgcaacgaa acaaaaaaaa 6180
tcatgaaatc gatcccgtct gcggaacggc tagagccatc ccaggattcc ccaaagagaa 6240
acactggcaa gttagcaatc agaacgtgtc tgacgtacag gtcgcatccg tgtacgaacg 6300
ctagcagcac ggatctaaca caaacacgga tctaacacaa acatgaacag aagtagaact 6360
accgggccct aaccatggac cggaacgccg atctagagaa ggtagagagg gggggggggg 6420
gaggacgagc ggcgtacctt gaagcggagg tgccgacggg tggatttggg ggagatctgg 6480
ttgtgtgtgt gtgcgctccg aacaacacga ggttggggaa agagggtgtg gagggggtgt 6540
ctatttatta cggcgggcga ggaagggaaa gcgaaggagc ggtgggaaag gaatcccccg 6600
tagctgccgt gccgtgagag gaggaggagg ccgcctgccg tgccggctca cgtctgccgc 6660
tccgccacgc aatttctgga tgccgacagc ggagcaagtc caacggtgga gcggaactct 6720
cgagaggggt ccagaggcag cgacagagat gccgtgccgt ctgcttcgct tggcccgacg 6780
cgacgctgct ggttcgctgg ttggtgtccg ttagactcgt cgacggcgtt taacaggctg 6840
gcattatcta ctcgaaacaa gaaaaatgtt tccttagttt ttttaatttc ttaaagggta 6900
tttgtttaat ttttagtcac tttattttat tctattttat atctaaatta ttaaataaaa 6960
aaactaaaat agagttttag ttttcttaat ttagaggcta aaatagaata aaatagatgt 7020
actaaaaaaa ttagtctata aaaaccatta accctaaacc ctaaatggat gtactaataa 7080
aatggatgaa gtattatata ggtgaagcta tttgcaaaaa aaaaggagaa cacatgcaca 7140

ctaaaaagat aaaactgtag agtcctgttg tcaaaatact caattgtcct ttagaccatg 7200

tctaactgtt catttatatg attctctaaa acactgatat tattgtagta ctatagatta 7260

tattattcgt agagtaaagt ttaaatatat gtataaagat agataaactg cacttcaaac 7320

aagtgtgaca aaaaaaatat gtggtaattt tttataactt agacatgcaa tgctcattat 7380

ctctagagag gggcacgacc gggtcacgct gcactgcagg catcgatcga agcttacgcg 7440

tccatggctg caggagctcg tcgactctag actcgagggc gagccccgag cgggggcagc 7500

tgaggcgcca ttgggaggag gaagagggca gcgctccggg gcggaagtcg tggggcggca 7560

cgagggaagg gcacacgacg gacggacg 7588

<210> 6
<211> 23
<212> DNA
<213> Artificial sequence

<220>
<223> primer forward (Right Border)

<400> 6
caggagctcg tcgactctag act 23

<210> 7
<211> 18
<212> DNA
<213> Artificial sequence

<220>
<223> primer reverse (Right border)

<400> 7
gtgtgccctt ccctcgtg 18

<210> 8
<211> 21
<212> DNA
<213> Artificial sequence

<220>
<223> primer forward (Left Border)

<400> 8
caggttgacg tgactgggtt g 21

<210> 9
<211> 24
<212> DNA
<213> Artificial sequence

<220>
<223> primer reverse (Left Border)

<400> 9
cgtccgcaat gtgttattaa gttg 24

<210> 10
<211> 17
<212> DNA
<213> Artificial sequence

<220>
<223> probe (Right border)

<400> 10
cattgggagg aggaaga 17

<210> 11
<211> 20
<212> DNA
<213> Artificial sequence

<220>
<223> probe (Left border)

<400> 11
tggtgtaaac aaattgacgc 20

<210> 12
<211> 126
<212> DNA
<213> Artificial sequence

<220>
<223> Amplicon Event Event CTC79005 (Right Border)

<400> 12
caggagctcg tcgactctag actcgagggc gagccccgag cgggggcagc tgaggcgcca 60

ttgggaggag gaagagggca gcgctccggg gcggaagtcg tggggcggca cgagggaagg 120

gcacac 126

<210> 13
<211> 104
<212> DNA
<213> Artificial sequence

<220>
<223> Amplicon Event Event CTC79005 (Left Border- Line ID)

<400> 13
caggttgacg tgactgggtt gcctgcgctg aacctaataa cacattgctg ggttgtggtg 60

taaacaaatt gacgcttaga caacttaata acacattgcg gacg 104

<210> 14
<211> 13723
<212> DNA
<213> Artificial sequence

<220>
<223> Binary plasmid comprising cry1Ac and nptII genes

<400> 14

```
tggcaggata tattgtggtg taaacaaatt gacgcttaga caacttaata acacattgcg      60
gacgttttta atgtactgaa ttagtactga tatcggtacc cggggcggcc gcgagctcga     120
tccactagta acggccgcca gtgtgctgga attcgccctt ggcgcgccga tctagtaaca     180
tagatgacac cgcgcgcgat aatttatcct agtttgcgcg ctatattttg ttttctatcg     240
cgtattaaat gtataattgc gggactctaa tcataaaaac ccatctcata aataacgtca     300
tgcattacat gttaattatt acatgcttaa cgtaattcaa cagaaattat atgataatca     360
tcgcaagacc ggcaacagga ttcaatctta agaaacttta ttgccaaatg tttgaacgat     420
ctcagaagaa ctcgtcaaga aggcgataga aggcgatgcg ctgcgaatcg ggagcggcga     480
taccgtaaac acattgctgg gaagcacgag gaagcggtca gcccattcgc cgccaagctc     540
ttcagcaata tcacgggtag ccaacgctat gtcctgatag cggtccgcca cacccagccg     600
gccacagtcg atgaatccag aaaagcggcc attttccacc atgatattcg gcaagcaggc     660
atcgccatgg gtcacgacga gatcctcgcc gtcgggcatg cgcgccttga gcctggcgaa     720
cagttcggct ggcgcgagcc cctgatgctc ttcgtccaga tcatcctgat cgacaagacc     780
ggcttccatc cgagtacgtg ctcgctcgat gcgatgtttc gcttggtggt cgaatgggca     840
ggtagccgga tcaagcgtat gcagccgccg cattgcatca gccatgatgg atactttctc     900
ggcaggagca aggtgagatg acaggagatc ctgccccggc acttcgccca atagcagcca     960
gtcccttccc gcttcagtga caacgtcgag cacagctgcg caaggaacgc ccgtcgtggc    1020
cagccacgat agccgcgctg cctcgtcctg cagttcattc agggcaccgg acaggtcggt    1080
cttgacaaaa agaaccgggc gcccctgcgc tgacagccgg aacacggcgg catcagagca    1140
gccgattgtc tgttgtgccc agtcatagcc gaatagcctc tccacccaag cggccggaga    1200
acctgcgtgc aatccatctt gttcaatcca cattctagag tcgacctgca gaagtaacac    1260
caaacaacag ggtgagcatc gacaaaagaa acagtaccaa gcaaataaat agcgtatgaa    1320
ggcagggcta aaaaaatcca catatagctg ctgcatatgc catcatccaa gtatatcaag    1380
atcaaaataa ttataaaaca tacttgttta ttataataga taggtactca aggttagagc    1440
atatgaatag atgctgcata tgccatcatg tatatgcatc agtaaaaccc acatcaacat    1500
gtatacctat cctagatcga tatttccatc catcttaaac tcgtaactat gaagatgtat    1560
gacacacaca tacagttcca aaattaataa atacaccagg tagtttgaaa cagtattcta    1620
ctccgatcta gaacgaatga acgaccgccc aaccacacca catcatcaca accaagcgaa    1680
caaaaagcat ctctgtatat gcatcagtaa aacccgcatc aacatgtata cctatcctag    1740
atcgatattt ccatccatca tcttcaattc gtaactatga atatgtatgg cacacacata    1800
cagatccaaa attaataaat ccaccaggta gtttgaaaca gaattctact ccgatctaga    1860
```

acgaccgccc aaccagacca catcatcaca accaagacaa aaaaaagcat gaaaagatga 1920
cccgacaaac aagtgcacgg catatattga aataaaggaa aagggcaaac caaaccctat 1980
gcaacgaaac aaaaaaaatc atgaaatcga tcccgtctgc ggaacggcta gagccatccc 2040
aggattcccc aaagagaaac actggcaagt tagcaatcag aacgtgtctg acgtacaggt 2100
cgcatccgtg tacgaacgct agcagcacgg atctaacaca aacacggatc taacacaaac 2160
atgaacagaa gtagaactac cgggccctaa ccatggaccg gaacgccgat ctagagaagg 2220
tagagagggg gggggggga ggacgagcgg cgtaccttga agcggaggtg ccgacgggtg 2280
gatttggggg agatctggtt gtgtgtgtgt gcgctccgaa caacacgagg ttggggaaag 2340
agggtgtgga gggggtgtct atttattacg gcgggcgagg aagggaaagc gaaggagcgg 2400
tgggaaagga atccccgta gctgccgtgc cgtgagagga ggaggaggcc gcctgccgtg 2460
ccggctcacg tctgccgctc cgccacgcaa tttctggatg ccgacagcgg agcaagtcca 2520
acggtggagc ggaactctcg agaggggtcc agaggcagcg acagagatgc cgtgccgtct 2580
gcttcgcttg gcccgacgcg acgctgctgg ttcgctggtt ggtgtccgtt agactcgtcg 2640
acggcgttta acaggctggc attatctact cgaaacaaga aaaatgtttc cttagttttt 2700
ttaatttctt aaagggtatt tgtttaattt ttagtcactt tattttattc tattttatat 2760
ctaaattatt aaataaaaaa actaaaatag agttttagtt ttcttaattt agaggctaaa 2820
atagaataaa atagatgtac taaaaaaatt agtctataaa aaccattaac cctaaaccct 2880
aaatggatgt actaataaaa tggatgaagt attatatagg tgaagctatt tgcaaaaaaa 2940
aaggagaaca catgcacact aaaaagataa aactgtagag tcctgttgtc aaaatactca 3000
attgtccttt agaccatgtc taactgttca tttatatgat tctctaaaac actgatatta 3060
ttgtagtact atagattata ttattcgtag agtaaagttt aaatatatgt ataaagatag 3120
ataaactgca cttcaaacaa gtgtgacaaa aaaaatatgt ggtaattttt tataacttag 3180
acatgcaatg ctcattatct ctagagaggg gcacgaccgg gtcacgctgc actgcaggca 3240
tacgcgtgat ccactagtaa cggccgccag tgtgctggaa ttcgcccttg gcgcgccgat 3300
ctagtaacat agatgacacc gcgcgcgata atttatccta gtttgcgcgc tatattttgt 3360
tttctatcgc gtattaaatg tataattgcg ggactctaat cataaaaacc catctcataa 3420
ataacgtcat gcattacatg ttaattatta catgcttaac gtaattcaac agaaattata 3480
tgataatcat cgcaagaccg gcaacaggat tcaatcttaa gaaactttat tgccaaatgt 3540
ttgaacgatc tcactcagcc tcgagggtgg cggtcactgg gatgaactcg aacctgtcga 3600
tgatcacgcc ggcggtgccg ctgaagttcc tcacgcccac gatgttaccg agggaggagg 3660
tgaaagcgtt ggcgctctcg aagtaaccga agtcgctgga ttggaggttg tccagggagg 3720

tagcggtagc tggcacggtg ttggagaaga tggaggagtt accccagttc acgttgaggt 3780

ggatcggggt cacggaagcg tacctcacgc gcaccctgta cctggtggag gtggatggga 3840

agtggattgg cacctcgatg tagcccctgt tctggatgtt gttgccgctg ctgttgagcc 3900

tcacgaggtc gccaccggtg aagcctgggc cggaaatgac ggaaccgttg aagaggaagt 3960

tgcccttcac ggccgggatt tgggtgatgc tgtcggaggc gatgatgttg ttgaactcag 4020

cgctgcggtg gatccaggag aacatcggag ccctgatgat gctcacggag ctgttgctga 4080

agccggagcg gaacatggac acgtggctca gcctgtggga gaagccttgc ctgggtggca 4140

cgttgttgtt ctgtggtggg atctcgtcca gggagtccac ggtgccgctc ttcctgtaga 4200

cagcggatgg caggttggag gaggtgccgt aggcgaactc ggtgccgtcg agcacggaca 4260

gttgctggtt gttgataccg atgttgaagg gcctcctgta cagggtggag gacagggtcc 4320

tgtagacacc ctgacccagt tgagccacga tgcgttgctg tggagcggcg ttgcccatgg 4380

tgccgtagag cgggaaggtg aactcggggc cgctgaagcc cactggggag gccatgatct 4440

ggtggccgga ccagtagtac tcgcccctgt gagcgtcggt gtagatggtg atgctgttca 4500

ggatgtccat caggtgtggg ctcctgatgg agccctcgat accctgggcg gaaccgcgga 4560

agctaccgtc gaagttctcc agcactgggt tggtgtagat ctccctggtg agttgggaca 4620

cggtgcggat cgggtaggtc ctggagtcgt agttcgggaa gagggacaca atgtccagca 4680

cggtgagggt caactccctc ctgaactggt tgtacctgat ccagtccctg gagtccggac 4740

cccagacgcg ctccaggccg gtgttgtacc agcgcacagc gtggtcggtg tagttgccaa 4800

tcagcctggt caggtcgttg tagcggctgt tgatggtggc agcatcgaag ccccaccttt 4860

ggccgaacac gctcacgtcg cgcagcacgc tgaggtgcag gttagcggct tgcacgtaca 4920

cggacaggag cggcacttgg tagttctgga cggcgaacag tgggatagcg gtggtcaggg 4980

cgctgttcat gtcgttgaat tgaatgcgca tttcctcgcg gagagctggg ttggtcgggt 5040

cggcctccca ctccctgaag ctctcggcgt agatttggta gaggttgctc aggccctcca 5100

gcctggagat ggcctggttc ctggcgaact cttcgatcct ctggttgatc agctgctcga 5160

tttgcaccag gaaggcgtcc cattgggatg gaccgaagat accccagatg atgtccacca 5220

ggccgagcac gaagccagca cctggcacga actcgctgag caggaactgg gtcaaggaca 5280

gggagatgtc gatgggggtg taaccggtct cgatgcgctc gccacccagc acctccacct 5340

ctgggttgct caggcagttg tatgggatgc actcgttgat gtttgggttg ttgtccattc 5400

tagagtcgac ctgcagaagt aacaccaaac aacagggtga gcatcgacaa aagaaacagt 5460

accaagcaaa taaatagcgt atgaaggcag ggctaaaaaa atccacatat agctgctgca 5520

tatgccatca tccaagtata tcaagatcaa aataattata aaacatactt gtttattata 5580

atagataggt actcaaggtt agagcatatg aatagatgct gcatatgcca tcatgtatat 5640

gcatcagtaa aacccacatc aacatgtata cctatcctag atcgatattt ccatccatct 5700
taaactcgta actatgaaga tgtatgacac acacatacag ttccaaaatt aataaataca 5760
ccaggtagtt tgaaacagta ttctactccg atctagaacg aatgaacgac cgcccaacca 5820
caccacatca tcacaaccaa gcgaacaaaa agcatctctg tatatgcatc agtaaaaccc 5880
gcatcaacat gtatacctat cctagatcga tatttccatc catcatcttc aattcgtaac 5940
tatgaatatg tatggcacac acatacagat ccaaaattaa taaatccacc aggtagtttg 6000
aaacagaatt ctactccgat ctagaacgac cgcccaacca gaccacatca tcacaaccaa 6060
gacaaaaaaa agcatgaaaa gatgacccga caaacaagtg cacggcatat attgaaataa 6120
aggaaaaggg caaaccaaac cctatgcaac gaaacaaaaa aaatcatgaa atcgatcccg 6180
tctgcggaac ggctagagcc atcccaggat tccccaaaga gaaacactgg caagttagca 6240
atcagaacgt gtctgacgta caggtcgcat ccgtgtacga acgctagcag cacggatcta 6300
acacaaacac ggatctaaca caaacatgaa cagaagtaga actaccgggc cctaaccatg 6360
gaccggaacg ccgatctaga gaaggtagag aggggggggg ggggaggacg agcggcgtac 6420
cttgaagcgg aggtgccgac gggtggattt gggggagatc tggttgtgtg tgtgtgcgct 6480
ccgaacaaca cgaggttggg gaaagagggt gtggaggggg tgtctattta ttacggcggg 6540
cgaggaaggg aaagcgaagg agcggtggga aaggaatccc ccgtagctgc cgtgccgtga 6600
gaggaggagg aggccgcctg ccgtgccggc tcacgtctgc cgctccgcca cgcaatttct 6660
ggatgccgac agcggagcaa gtccaacggt ggagcggaac tctcgagagg ggtccagagg 6720
cagcgacaga gatgccgtgc cgtctgcttc gcttggcccg acgcgacgct gctggttcgc 6780
tggttggtgt ccgttagact cgtcgacggc gtttaacagg ctggcattat ctactcgaaa 6840
caagaaaaat gtttccttag ttttttaat ttcttaaagg gtatttgttt aatttttagt 6900
cactttattt tattctattt tatatctaaa ttattaaata aaaaaactaa aatagagttt 6960
tagttttctt aatttagagg ctaaaataga ataaaataga tgtactaaaa aaattagtct 7020
ataaaaacca ttaaccctaa accctaaatg gatgtactaa taaaatggat gaagtattat 7080
ataggtgaag ctatttgcaa aaaaaaagga gaacacatgc acactaaaaa gataaaactg 7140
tagagtcctg ttgtcaaaat actcaattgt cctttagacc atgtctaact gttcatttat 7200
atgattctct aaaacactga tattattgta gtactataga ttatattatt cgtagagtaa 7260
agtttaaata tatgtataaa gatagataaa ctgcacttca aacaagtgtg acaaaaaaaa 7320
tatgtggtaa ttttttataa cttagacatg caatgctcat tatctctaga gaggggcacg 7380
accgggtcac gctgcactgc aggcatcgat cgaagcttac gcgtccatgg ctgcaggagc 7440
tcgtcgactc tagactcgag ggcgcgccga caggatatat tggcgggtaa accttagaat 7500

aacggatatt taaaagggcg tgaaaaggtt tatccgttcg tccatttgta tgtgcatgcc 7560
aaccacaggg ttcccctcgg gatcaaagta ctttgatcca acccctccgc tgctatagtg 7620
cagtcggctt ctgacgttca gtgcagccgt cttctgaaaa cgacatgtcg cacaagtcct 7680
aagttacgcg acaggctgcc gccctgccct tttcctggcg ttttcttgtc gcgtgtttta 7740
gtcgcataaa gtagaatact tgcgactaga accggagaca ttacgccatg aacaagagcg 7800
ccgccgctgg cctgctgggc tatgcccgcg tcagcaccga cgaccaggac ttgaccaacc 7860
aacgggccga actgcacgcg gccggctgca ccaagctgtt ttccgagaag atcaccggca 7920
ccaggcgcga ccgcccggag ctggccagga tgcttgacca cctacgccct ggcgacgttg 7980
tgacagtgac caggctagac cgcctggccc gcagcacccg cgacctactg gacattgccg 8040
agcgcatcca ggaggccggc gcgggcctgc gtagcctggc agagccgtgg gccgacacca 8100
ccacgccggc cggccgcatg gtgttgaccg tgttcgccgg cattgccgag ttcgagcgtt 8160
ccctaatcat cgaccgcacc cggagcgggc gcgaggccgc caaggcccga ggcgtgaagt 8220
ttggcccccg ccctaccctc accccggcac agatcgcgca cgcccgcgag ctgatcgacc 8280
aggaaggccg caccgtgaaa gaggcggctg cactgcttgg cgtgcatcgc tcgaccctgt 8340
accgcgcact tgagcgcagc gaggaagtga cgcccaccga ggccaggcgg cgcggtgcct 8400
tccgtgagga cgcattgacc gaggccgacg ccctggcggc cgccgagaat gaacgccaag 8460
aggaacaagc atgaaaccgc accaggacgg ccaggacgaa ccgtttttca ttaccgaaga 8520
gatcgaggcg gagatgatcg cggccgggta cgtgttcgag ccgcccgcgc acgtctcaac 8580
cgtgcggctg catgaaatcc tggccggttt gtctgatgcc aagctggcgg cctggccggc 8640
cagcttggcc gctgaagaaa ccgagcgccg ccgtctaaaa aggtgatgtg tatttgagta 8700
aaacagcttg cgtcatgcgg tcgctgcgta tatgatgcga tgagtaaata aacaaatacg 8760
caaggggaac gcatgaaggt tatcgctgta cttaaccaga aaggcgggtc aggcaagacg 8820
accatcgcaa cccatctagc ccgcgccctg caactcgccg gggccgatgt tctgttagtc 8880
gattccgatc cccagggcag tgcccgcgat tgggcggccg tgcgggaaga tcaaccgcta 8940
accgttgtcg gcatcgaccg cccgacgatt gaccgcgacg tgaaggccat cggccggcgc 9000
gacttcgtag tgatcgacgg agcgccccag gcggcggact tggctgtgtc cgcgatcaag 9060
gcagccgact tcgtgctgat tccggtgcag ccaagccctt acgacatatg ggccaccgcc 9120
gacctggtgg agctggttaa gcagcgcatt gaggtcacgg atggaaggct acaagcggcc 9180
tttgtcgtgt cgcgggcgat caaaggcacg cgcatcggcg gtgaggttgc cgaggcgctg 9240
gccgggtacg agctgcccat tcttgagtcc cgtatcacgc agcgcgtgag ctacccaggc 9300
actgccgccg ccggcacaac cgttcttgaa tcagaacccg agggcgacgc tgcccgcgag 9360
gtccaggcgc tggccgctga aattaaatca aaactcattt gagttaatga ggtaaagaga 9420

aaatgagcaa aagcacaaac acgctaagtg ccggccgtcc gagcgcacgc agcagcaagg 9480
ctgcaacgtt ggccagcctg gcagacacgc cagccatgaa gcgggtcaac tttcagttgc 9540
cggcggagga tcacaccaag ctgaagatgt acgcggtacg ccaaggcaag accattaccg 9600
agctgctatc tgaatacatc gcgcagctac cagagtaaat gagcaaatga ataaatgagt 9660
agatgaattt tagcggctaa aggaggcggc atggaaaatc aagaacaacc aggcaccgac 9720
gccgtggaat gccccatgtg tggaggaacg ggcggttggc caggcgtaag cggctgggtt 9780
gtctgccggc cctgcaatgg cactggaacc cccaagcccg aggaatcggc gtgacggtcg 9840
caaaccatcc ggcccggtac aaatcggcgc ggcgctgggt gatgacctgg tggagaagtt 9900
gaaggccgcg caggccgccc agcggcaacg catcgaggca gaagcacgcc ccggtgaatc 9960
gtggcaagcg gccgctgatc gaatccgcaa agaatcccgg caaccgccgg cagccggtgc 10020
gccgtcgatt aggaagccgc ccaagggcga cgagcaacca gatttttcg ttccgatgct 10080
ctatgacgtg ggcacccgcg atagtcgcag catcatggac gtggccgttt tccgtctgtc 10140
gaagcgtgac cgacgagctg gcgaggtgat ccgctacgag cttccagacg ggcacgtaga 10200
ggtttccgca gggccggccg gcatggccag tgtgtgggat tacgacctgg tactgatggc 10260
ggtttcccat ctaaccgaat ccatgaaccg ataccgggaa gggaagggag acaagcccgg 10320
ccgcgtgttc cgtccacacg ttgcggacgt actcaagttc tgccggcgag ccgatggcgg 10380
aaagcagaaa gacgacctgg tagaaacctg cattcggtta aacaccacgc acgttgccat 10440
gcagcgtacg aagaaggcca agaacggccg cctggtgacg gtatccgagg gtgaagcctt 10500
gattagccgc tacaagatcg taaagagcga aaccgggcgg ccggagtaca tcgagatcga 10560
gctagctgat tggatgtacc gcgagatcac agaaggcaag aacccggacg tgctgacggt 10620
tcaccccgat tactttttga tcgatcccgg catcggccgt tttctctacc gcctggcacg 10680
ccgcgccgca ggcaaggcag aagccagatg gttgttcaag acgatctacg aacgcagtgg 10740
cagcgccgga gagttcaaga agttctgttt caccgtgcgc aagctgatcg ggtcaaatga 10800
cctgccggag tacgatttga aggaggaggc ggggcaggct ggcccgatcc tagtcatgcg 10860
ctaccgcaac ctgatcgagg gcgaagcatc cgccggttcc taatgtacgg agcagatgct 10920
agggcaaatt gccctagcag gggaaaaagg tcgaaaaggt ctctttcctg tggatagcac 10980
gtacattggg aacccaaagc cgtacattgg gaaccggaac ccgtacattg ggaacccaaa 11040
gccgtacatt gggaaccggt cacacatgta agtgactgat ataaaagaga aaaaaggcga 11100
tttttccgcc taaaactctt taaaacttat taaaactctt aaaacccgcc tggcctgtgc 11160
ataactgtct ggccagcgca cagccgaaga gctgcaaaaa gcgcctaccc ttcggtcgct 11220
gcgctcccta cgccccgccg cttcgcgtcg gcctatcgcg gccgctggcc gctcaaaaat 11280

ggctggccta cggccaggca atctaccagg gcgcggacaa gccgcgccgt cgccactcga 11340
ccgccggcgc ccacatcaag gcaccctgcc tcgcgcgttt cggtgatgac ggtgaaaacc 11400
tctgacacat gcagctcccg gagacggtca cagcttgtct gtaagcggat gccgggagca 11460
gacaagcccg tcagggcgcg tcagcgggtg ttggcgggtg tcggggcgca gccatgaccc 11520
agtcacgtag cgatagcgga gtgtatactg gcttaactat gcggcatcag agcagattgt 11580
actgagagtg caccatatgc ggtgtgaaat accgcacaga tgcgtaagga gaaaataccg 11640
catcaggcgc tcttccgctt cctcgctcac tgactcgctg cgctcggtcg ttcggctgcg 11700
gcgagcggta tcagctcact caaaggcggt aatacggtta tccacagaat caggggataa 11760
cgcaggaaag aacatgtgag caaaaggcca gcaaaaggcc aggaaccgta aaaaggccgc 11820
gttgctggcg tttttccata ggctccgccc ccctgacgag catcacaaaa atcgacgctc 11880
aagtcagagg tggcgaaacc cgacaggact ataaagatac caggcgtttc cccctggaag 11940
ctccctcgtg cgctctcctg ttccgaccct gccgcttacc ggatacctgt ccgcctttct 12000
cccttcggga agcgtggcgc tttctcatag ctcacgctgt aggtatctca gttcggtgta 12060
ggtcgttcgc tccaagctgg gctgtgtgca cgaacccccc gttcagcccg accgctgcgc 12120
cttatccggt aactatcgtc ttgagtccaa cccggtaaga cacgacttat cgccactggc 12180
agcagccact ggtaacagga ttagcagagc gaggtatgta ggcggtgcta cagagttctt 12240
gaagtggtgg cctaactacg gctacactag aaggacagta tttggtatct gcgctctgct 12300
gaagccagtt accttcggaa aaagagttgg tagctcttga tccggcaaac aaaccaccgc 12360
tggtagcggt ggtttttttg tttgcaagca gcagattacg cgcagaaaaa aaggatctca 12420
agaagatcct ttgatctttt ctacggggtc tgacgctcag tggaacgaaa actcacgtta 12480
agggattttg gtcatgcatt ctaggtatta tttgccaacg accttcgtga tctcgccctt 12540
gacatagtgg acaaattctt cgagctggtc ggcccgggac gcgagacggt cttcttcttg 12600
gcccagatag gcttggcgcg cttcgaggat cacgggctgg tattgcgccg gaaggcgctc 12660
catcgcccag tcggcggcga catccttcgg cgcgatcttg ccggtaaccg ccgagtacca 12720
aatccggctc agcgtaagga ccacattgcg ctcatcgccc gcccaatccg gcggggagtt 12780
ccacagggtc agcgtctcgt tcagtgcttc gaacagatcc tgttccggca ccgggtcgaa 12840
aagttcctcg gccgcggggc cgacgagggc cacgctatgc tcccgggcct tggtgagcag 12900
gatcgccaga tcaatgtcga tggtggccgg ttcaaagata cccgccagaa tatcattacg 12960
ctgccattcg ccgaactgga gttcgcgttt ggccggatag cgccagggga tgatgtcatc 13020
gtgcaccaca atcgtcacct caaccgcgcg caggatttcg ctctcgccgg gggaggcgga 13080
cgtttccaga aggtcgttga taagcgcgcg gcgcgtggtc tcgtcgagac ggacggtaac 13140
ggtgacaagc aggtcgatgt ccgaatgggg cttaaggccg ccgtcaacgg cgctaccata 13200

cagatgcacg gcgaggaggg tcggttcgag gtggcgctcg atgacaccca cgacttccga 13260

cagctgggtg gacacctcgg cgatgaccgc ttcacccatt tattatttcc ttcctctttt 13320

ctacagtatt taaagatacc ccaagaagct aattataaca agacgaactc caattcactg 13380

ttccttgcat tctaaaacct taaataccag aaaacagctt tttcaaagtt gttttcaaag 13440

ttggcgtata acatagtatc gacggagccg attttgaaac cgcggtgatc acaggcagca 13500

acgctctgtc atcgttacaa tcaacatgct accctccgcg agatcatccg tgtttcaaac 13560

ccggcagctt agttgccgtt cttccgaata gcatcggtaa catgagcaaa gtctgccgcc 13620

ttacaacggc tctcccgctg acgccgtccc ggactgatgg gctgcctgta tcgagtggtg 13680

attttgtgcc gagctgccgg tcggggagct gttggctggc tgg 13723

<210> 15
<211> 22
<212> DNA
<213> Artificial sequence

<220>
<223> primer forward: sugarcane poly-ubiquitin gene

<400> 15
accattaccc tggaggttga ga 22

<210> 16
<211> 20
<212> DNA
<213> Artificial sequence

<220>
<223> primer reverse: sugarcane poly-ubiquitin gene

<400> 16
gtcctggatc ttcgccttca 20

<210> 17
<211> 16
<212> DNA
<213> Artificial sequence

<220>
<223> probe sugarcane poly-ubiquitin gene

<400> 17
ctctgacacc atcgac 16

<210> 18
<211> 26
<212> DNA
<213> Artificial sequence

<220>
<223> Junction sequence between 5’region of insert and sugarcane genome

of event CTC79005

<400> 18
acacattgct gggttgtggt gtaaac 26

<210> 19
<211> 26
<212> DNA
<213> Artificial sequence

<220>
<223> Junction sequence between 3'region of insert and sugarcane genome of event CTC79005

<400> 19
tcgagggcga gccccgagcg ggggca 26

<210> 20
<211> 1848
<212> DNA
<213> Artificial sequence

<220>
<223> Sugarcane optimized truncate cry1Ac gene (B. thurigiensis).

<400> 20
atggacaaca acccaaacat caacgagtgc atcccataca actgcctgag caacccagag 60

gtggaggtgc tgggtggcga gcgcatcgag accggttaca cccccatcga catctccctg 120

tccttgaccc agttcctgct cagcgagttc gtgccaggtg ctggcttcgt gctcggcctg 180

gtggacatca tctggggtat cttcggtcca tcccaatggg acgccttcct ggtgcaaatc 240

gagcagctga tcaaccagag gatcgaagag ttcgccagga accaggccat ctccaggctg 300

gagggcctga gcaacctcta ccaaatctac gccgagagct tcagggagtg ggaggccgac 360

ccgaccaacc cagctctccg cgaggaaatg cgcattcaat tcaacgacat gaacagcgcc 420

ctgaccaccg ctatcccact gttcgccgtc cagaactacc aagtgccgct cctgtccgtg 480

tacgtgcaag ccgctaacct gcacctcagc gtgctgcgcg acgtgagcgt gttcggccaa 540

aggtggggct tcgatgctgc caccatcaac agccgctaca acgacctgac caggctgatt 600

ggcaactaca ccgaccacgc tgtgcgctgg tacaacaccg gcctggagcg cgtctggggt 660

ccggactcca gggactggat caggtacaac cagttcagga gggagttgac cctcaccgtg 720

ctggacattg tgtccctctt cccgaactac gactccagga cctacccgat ccgcaccgtg 780

tcccaactca ccagggagat ctacaccaac ccagtgctgg agaacttcga cggtagcttc 840

cgcggttccg cccagggtat cgagggctcc atcaggagcc cacacctgat ggacatcctg 900

aacagcatca ccatctacac cgacgctcac aggggcgagt actactggtc cggccaccag 960

atcatggcct ccccagtggg cttcagcggc cccgagttca ccttcccgct ctacggcacc 1020

atgggcaacg ccgctccaca gcaacgcatc gtggctcaac tgggtcaggg tgtctacagg 1080

accctgtcct ccaccctgta caggaggccc ttcaacatcg gtatcaacaa ccagcaactg 1140

tccgtgctcg acggcaccga gttcgcctac ggcacctcct ccaacctgcc atccgctgtc 1200

tacaggaaga gcggcaccgt ggactccctg gacgagatcc caccacagaa caacaacgtg 1260

ccacccaggc aaggcttctc ccacaggctg agccacgtgt ccatgttccg ctccggcttc 1320

agcaacagct ccgtgagcat catcagggct ccgatgttct cctggatcca ccgcagcgct 1380

gagttcaaca acatcatcgc ctccgacagc atcacccaaa tcccggccgt gaagggcaac 1440

ttcctcttca acggttccgt catttccggc ccaggcttca ccggtggcga cctcgtgagg 1500

ctcaacagca gcggcaacaa catccagaac aggggctaca tcgaggtgcc aatccacttc 1560

ccatccacct ccaccaggta cagggtgcgc gtgaggtacg cttccgtgac cccgatccac 1620

ctcaacgtga actggggtaa ctcctccatc ttctccaaca ccgtgccagc taccgctacc 1680

tccctggaca acctccaatc cagcgacttc ggttacttcg agagcgccaa cgctttcacc 1740

tcctccctcg gtaacatcgt gggcgtgagg aacttcagcg gcaccgccgg cgtgatcatc 1800

gacaggttcg agttcatccc agtgaccgcc accctcgagg ctgagtga 1848

<210> 21
<211> 798
<212> DNA
<213> Artificial sequence

<220>
<223> NptII gene

<400> 21
tcagaagaac tcgtcaagaa ggcgatagaa ggcgatgcgc tgcgaatcgg gagcggcgat 60

accgtaaagc acgaggaagc ggtcagccca ttcgccgcca agctcttcag caatatcacg 120

ggtagccaac gctatgtcct gatagcggtc cgccacaccc agccggccac agtcgatgaa 180

tccagaaaag cggccatttt ccaccatgat attcggcaag caggcatcgc catgggtcac 240

gacgagatcc tcgccgtcgg gcatgcgcgc cttgagcctg gcgaacagtt cggctggcgc 300

gagcccctga tgctcttcgt ccagatcatc ctgatcgaca agaccggctt ccatccgagt 360

acgtgctcgc tcgatgcgat gtttcgcttg gtggtcgaat gggcaggtag ccggatcaag 420

cgtatgcagc cgccgcattg catcagccat gatggatact ttctcggcag gagcaaggtg 480

agatgacagg agatcctgcc ccggcacttc gcccaatagc agccagtccc ttcccgcttc 540

agtgacaacg tcgagcacag ctgcgcaagg aacgcccgtc gtggccagcc acgatagccg 600

cgctgcctcg tcctgcagtt cattcagggc accggacagg tcggtcttga caaaaagaac 660

cgggcgcccc tgcgctgaca gccggaacac ggcggcatca gagcagccga ttgtctgttg 720

tgcccagtca tagccgaata gcctctccac ccaagcggcc ggagaacctg cgtgcaatcc 780

atcttgttca atccacat 798

<210> 22
<211> 8686
<212> DNA
<213> Artificial sequence

<220>
<223> CTC79005 event: Flanking sequences and T DNA fragment

<400> 22
ggtaatcctt caaaggattt taaaaaatac taaatctgag ctaggatctg cagatgagga 60

agaaagacga agacgagata gagagcggga aattgagatg taaccctagc tgatggaacg 120

ggcacaggca caaatacggg catgggccga gatcgagggg cggcgcatca gcagcagtgc 180

agtaggcgag cagggccgcc gcattggtgg gccgcccatg ccggtgcacc gccgccgctc 240

tcgcgggggc cgccgtgcca tggctcgtcc gatgcaggat caccggcgac ggagaggcga 300

agccctaggc cggcgctgtc gccacgccag gatcggtcgc ccaggttgac gtgactgggt 360

tgcctgcgct gaacctaata acacattgct gggttgtggt gtaaacaaat tgacgcttag 420

acaacttaat aacacattgc ggacgttttt aatgtactga attagtactg atatcggtac 480

ccggggcggc cgcgagctcg atccactagt aacggccgcc agtgtgctgg aattcgccct 540

tggcgcgccg atctagtaac atagatgaca ccgcgcgcga taatttatcc tagtttgcgc 600

gctatatttt gttttctatc gcgtattaaa tgtataattg cgggactcta atcataaaaa 660

cccatctcat aaataacgtc atgcattaca tgttaattat tacatgctta acgtaattca 720

acagaaatta tatgataatc atcgcaagac cggcaacagg attcaatctt aagaaacttt 780

attgccaaat gtttgaacga tctcagaaga actcgtcaag aaggcgatag aaggcgatgc 840

gctgcgaatc gggagcggcg ataccgtaaa gcacgaggaa gcggtcagcc cattcgccgc 900

caagctcttc agcaatatca cgggtagcca acgctatgtc ctgatagcgg tccgccacac 960

ccagccggcc acagtcgatg aatccagaaa agcggccatt ttccaccatg atattcggca 1020

agcaggcatc gccatgggtc acgacgagat cctcgccgtc gggcatgcgc gccttgagcc 1080

tggcgaacag ttcggctggc gcgagcccct gatgctcttc gtccagatca tcctgatcga 1140

caagaccggc ttccatccga gtacgtgctc gctcgatgcg atgtttcgct tggtggtcga 1200

atgggcaggt agccggatca agcgtatgca gccgccgcat tgcatcagcc atgatggata 1260

ctttctcggc aggagcaagg tgagatgaca ggagatcctg ccccggcact tcgcccaata 1320

gcagccagtc ccttcccgct tcagtgacaa cgtcgagcac agctgcgcaa ggaacgcccg 1380

tcgtggccag ccacgatagc cgcgctgcct cgtcctgcag ttcattcagg gcaccggaca 1440

ggtcggtctt gacaaaaaga accgggcgcc cctgcgctga cagccggaac acggcggcat 1500

cagagcagcc gattgtctgt tgtgcccagt catagccgaa tagcctctcc acccaagcgg 1560

ccggagaacc tgcgtgcaat ccatcttgtt caatccacat tctagagtcg acctgcagaa 1620

gtaacaccaa acaacagggt gagcatcgac aaaagaaaca gtaccaagca aataaatagc 1680

gtatgaaggc agggctaaaa aaatccacat atagctgctg catatgccat catccaagta 1740

tatcaagatc aaaataatta taaaacatac ttgtttatta taatagatag gtactcaagg 1800

ttagagcata tgaatagatg ctgcatatgc catcatgtat atgcatcagt aaaacccaca 1860

tcaacatgta tacctatcct agatcgatat ttccatccat cttaaactcg taactatgaa 1920

gatgtatgac acacacatac agttccaaaa ttaataaata caccaggtag tttgaaacag 1980

tattctactc cgatctagaa cgaatgaacg accgcccaac cacaccacat catcacaacc 2040

aagcgaacaa aaagcatctc tgtatatgca tcagtaaaac ccgcatcaac atgtatacct 2100

atcctagatc gatatttcca tccatcatct tcaattcgta actatgaata tgtatggcac 2160

acacatacag atccaaaatt aataaatcca ccaggtagtt tgaaacagaa ttctactccg 2220

atctagaacg accgcccaac cagaccacat catcacaacc aagacaaaaa aaagcatgaa 2280

aagatgaccc gacaaacaag tgcacggcat atattgaaat aaaggaaaag ggcaaaccaa 2340

accctatgca acgaaacaaa aaaaatcatg aaatcgatcc cgtctgcgga acggctagag 2400

ccatcccagg attccccaaa gagaaacact ggcaagttag caatcagaac gtgtctgacg 2460

tacaggtcgc atccgtgtac gaacgctagc agcacggatc taacacaaac acggatctaa 2520

cacaaacatg aacagaagta gaactaccgg gccctaacca tggaccggaa cgccgatcta 2580

gagaaggtag agaggggggg gggggaggga cgagcggcgt accttgaagc ggaggtgccg 2640

acgggtggat ttgggggaga tctggttgtg tgtgtgtgcg ctccgaacaa cacgaggttg 2700

gggaaagagg gtgtggaggg ggtgtctatt tattacggcg ggcgaggaag ggaaagcgaa 2760

ggagcggtgg gaaaggaatc ccccgtagct gccgtgccgt gagaggagga ggaggccgcc 2820

tgccgtgccg gctcacgtct gccgctccgc cacgcaattt ctggatgccg acagcggagc 2880

aagtccaacg gtggagcgga actctcgaga ggggtccaga ggcagcgaca gagatgccgt 2940

gccgtctgct tcgcttggcc cgacgcgacg ctgctggttc gctggttggt gtccgttaga 3000

ctcgtcgacg gcgtttaaca ggctggcatt atctactcga aacaagaaaa atgtttcctt 3060

agttttttta atttcttaaa gggtatttgt ttaattttta gtcactttat tttattctat 3120

tttatatcta aattattaaa taaaaaaact aaaatagagt tttagttttc ttaatttaga 3180

ggctaaaata gaataaaata gatgtactaa aaaaattagt ctataaaaac cattaaccct 3240

aaaccctaaa tggatgtact aataaaatgg atgaagtatt atataggtga agctatttgc 3300

aaaaaaaaag gagaacacat gcacactaaa aagataaaac tgtagagtcc tgttgtcaaa 3360

atactcaatt gtcctttaga ccatgtctaa ctgttcattt atatgattct ctaaaacact 3420

gatattattg tagtactata gattatatta ttcgtagagt aaagtttaaa tatatgtata 3480
aagatagata aactgcactt caaacaagtg tgacaaaaaa aatatgtggt aattttttat 3540
aacttagaca tgcaatgctc attatctcta gagaggggca cgaccgggtc acgctgcact 3600
gcaggcatac gcgtgatcca ctagtaacgg ccgccagtgt gctggaattc gcccttggcg 3660
cgccgatcta gtaacataga tgacaccgcg cgcgataatt tatcctagtt tgcgcgctat 3720
attttgtttt ctatcgcgta ttaaatgtat aattgcggga ctctaatcat aaaaacccat 3780
ctcataaata acgtcatgca ttacatgtta attattacat gcttaacgta attcaacaga 3840
aattatatga taatcatcgc aagaccggca acaggattca atcttaagaa actttattgc 3900
caaatgtttg aacgatctca ctcagcctcg agggtggcgg tcactgggat gaactcgaac 3960
ctgtcgatga tcacgccggc ggtgccgctg aagttcctca cgcccacgat gttaccgagg 4020
gaggaggtga aagcgttggc gctctcgaag taaccgaagt cgctggattg gaggttgtcc 4080
agggaggtag cggtagctgg cacggtgttg gagaagatgg aggagttacc ccagttcacg 4140
ttgaggtgga tcggggtcac ggaagcgtac ctcacgcgca ccctgtacct ggtggaggtg 4200
gatgggaagt ggattggcac ctcgatgtag cccctgttct ggatgttgtt gccgctgctg 4260
ttgagcctca cgaggtcgcc accggtgaag cctgggccgg aaatgacgga accgttgaag 4320
aggaagttgc ccttcacggc cgggatttgg gtgatgctgt cggaggcgat gatgttgttg 4380
aactcagcgc tgcggtggat ccaggagaac atcggagccc tgatgatgct cacggagctg 4440
ttgctgaagc cggagcggaa catggacacg tggctcagcc tgtgggagaa gccttgcctg 4500
ggtggcacgt tgttgttctg tggtgggatc tcgtccaggg agtccacggt gccgctcttc 4560
ctgtagacag cggatggcag gttggaggag gtgccgtagg cgaactcggt gccgtcgagc 4620
acggacagtt gctggttgtt gataccgatg ttgaagggcc tcctgtacag ggtggaggac 4680
agggtcctgt agacaccctg acccagttga gccacgatgc gttgctgtgg agcggcgttg 4740
cccatggtgc cgtagagcgg gaaggtgaac tcggggccgc tgaagcccac tggggaggcc 4800
atgatctggt ggccggacca gtagtactcg cccctgtgag cgtcggtgta gatggtgatg 4860
ctgttcagga tgtccatcag gtgtgggctc ctgatggagc cctcgatacc ctgggcggaa 4920
ccgcggaagc taccgtcgaa gttctccagc actgggttgg tgtagatctc cctggtgagt 4980
tgggacacgg tgcggatcgg gtaggtcctg gagtcgtagt tcgggaagag ggacacaatg 5040
tccagcacgg tgagggtcaa ctccctcctg aactggttgt acctgatcca gtccctggag 5100
tccggacccc agacgcgctc caggccggtg ttgtaccagc gcacagcgtg gtcggtgtag 5160
ttgccaatca gcctggtcag gtcgttgtag cggctgttga tggtggcagc atcgaagccc 5220
cacctttggc cgaacacgct cacgtcgcgc agcacgctga ggtgcaggtt agcggcttgc 5280
acgtacacgg acaggagcgg cacttggtag ttctggacgg cgaacagtgg gatagcggtg 5340

gtcagggcgc tgttcatgtc gttgaattga atgcgcattt cctcgcggag agctgggttg 5400
gtcgggtcgg cctcccactc cctgaagctc tcggcgtaga tttggtagag gttgctcagg 5460
ccctccagcc tggagatggc ctggttcctg gcgaactctt cgatcctctg gttgatcagc 5520
tgctcgattt gcaccaggaa ggcgtcccat tgggatggac cgaagatacc ccagatgatg 5580
tccaccaggc cgagcacgaa gccagcacct ggcacgaact cgctgagcag gaactgggtc 5640
aaggacaggg agatgtcgat gggggtgtaa ccggtctcga tgcgctcgcc acccagcacc 5700
tccacctctg ggttgctcag gcagttgtat gggatgcact cgttgatgtt tgggttgttg 5760
tccattctag agtcgacctg cagaagtaac accaaacaac agggtgagca tcgacaaaag 5820
tgctgcatat gccatcatcc aagtatatca agatcaaaat aattataaaa catacttgtt 5880
tattataata gataggtact caaggttaga gcatatgaat agatgctgca tatgccatca 5940
tgtatatgca tcagtaaaac ccacatcaac atgtatacct atcctagatc gatatttcca 6000
tccatcttaa actcgtaact atgaagatgt atgacacaca catacagttc caaaattaat 6060
aaatacacca ggtagtttga aacagtattc tactccgatc tagaacgaat gaacgaccgc 6120
ccaaccacac cacatcatca caaccaagcg aacaaaaagc atctctgtat atgcatcagt 6180
aaaacccgca tcaacatgta tacctatcct agatcgatat ttccatccat catcttcaat 6240
tcgtaactat gaatatgtat ggcacacaca tacagatcca aaattaataa atccaccagg 6300
tagtttgaaa cagaattcta ctccgatcta gaacgaccgc ccaaccagac cacatcatca 6360
caaccaagac aaaaaaaagc atgaaaagat gacccgacaa acaagtgcac ggcatatatt 6420
gaaataaagg aaaagggcaa accaaaccct atgcaacgaa acaaaaaaaa tcatgaaatc 6480
gatcccgtct gcggaacggc tagagccatc ccaggattcc ccaaagagaa acactggcaa 6540
gttagcaatc agaacgtgtc tgacgtacag gtcgcatccg tgtacgaacg ctagcagcac 6600
ggatctaaca caaacacgga tctaacacaa acatgaacag aagtagaact accgggccct 6660
aaccatggac cggaacgccg atctagagaa ggtagagagg gggggggggg gaggacgagc 6720
ggcgtacctt gaagcggagg tgccgacggg tggatttggg ggagatctgg ttgtgtgtgt 6780
gtgcgctccg aacaacacga ggttggggaa agagggtgtg gagggggtgt ctatttatta 6840
cggcgggcga ggaagggaaa gcgaaggagc ggtgggaaag gaatcccccg tagctgccgt 6900
gccgtgagag gaggaggagg ccgcctgccg tgccggctca cgtctgccgc tccgccacgc 6960
aatttctgga tgccgacagc ggagcaagtc caacggtgga gcggaactct cgagaggggt 7020
ccagaggcag cgacagagat gccgtgccgt ctgcttcgct tggcccgacg cgacgctgct 7080
ggttcgctgg ttggtgtccg ttagactcgt cgacggcgtt taacaggctg gcattatcta 7140
ctcgaaacaa gaaaaatgtt tccttagttt ttttaatttc ttaaagggta tttgtttaat 7200

ttttagtcac tttattttat tctattttat atctaaatta ttaaataaaa aaactaaaat 7260

agagttttag ttttcttaat ttagaggcta aaatagaata aaatagatgt actaaaaaaa 7320

ttagtctata aaaaccatta accctaaacc ctaaatggat gtactaataa aatggatgaa 7380

gtattatata ggtgaagcta tttgcaaaaa aaaaggagaa cacatgcaca ctaaaaagat 7440

aaaactgtag agtcctgttg tcaaaatact caattgtcct ttagaccatg tctaactgtt 7500

catttatatg attctctaaa acactgatat tattgtagta ctatagatta tattattcgt 7560

agagtaaagt ttaaatatat gtataaagat agataaactg cacttcaaac aagtgtgaca 7620

aaaaaaatat gtggtaattt tttataactt agacatgcaa tgctcattat ctctagagag 7680

gggcacgacc gggtcacgct gcactgcagg catcgatcga agcttacgcg tccatggctg 7740

caggagctcg tcgactctag actcgagggc gagccccgag cgggggcagc tgaggcgcca 7800

ttgggaggag gaagagggca gcgctccggg gcggaagtcg tggggcggca cgagggaagg 7860

gcacacgacg gacggacgga ggaagaagaa gagcgcggag gtagctggcc gagcgtgacc 7920

tgcatgtcaa gtcaatggtc gcgggtgcga gtagaaaaga gggaggacga ccggacacac 7980

cgccgcgtca ggtcaccgat gacctaacgt gtctggtcac tattcacgcg ctaaaaacac 8040

ttactagact cgatctaacg cgggcagccc tctgagtccg atcatttatt ctgtgcgtcc 8100

ggtcatcgac gtgtgcggga aagcgttggc tgttaacggc tagttttgaa tcgtgtgacg 8160

tggcacatcg tggttgctcg actgttacga ccggacacgg ggctgagtcc ggtcgatgag 8220

tccgatcacc cccgagctgc gtaacggttg gtttttcaga gggggtcttt aaatagacct 8280

tggtcggctc caagcctttt ctcttggtat tttgatcatc ttgacatcct cttaagctaa 8340

gcatacttcc tcccactcat ctctcttgct tggttgtgaa tccaaagtga gattgagtga 8400

ttccaagtgt atttgcttgt gagtttgcat atagtggcac ttggggatcg tttgagctgc 8460

ggaattcttg ttactcttgg tggctaccgc cacctagaca gcttggagca acagatgagt 8520

tgtggcaaga gttggtgatt atttttggtc acctcccggt gattgtgagg ggtttttgtg 8580

cctttcccgg cagagcgtta aaggcaactc tagtggattg cgcatgtcat tgagctacct 8640

cacttatggg tcggttcttg cggtttccaa cgtgtggacg aggttc 8686

<210> 23
<211> 393
<212> DNA
<213> Artificial sequence

<220>
<223> CTC79005 event: Flanking sequence - Left border (5′)

<400> 23
ggtaatcctt caaaggattt taaaaaatac taaatctgag ctaggatctg cagatgagga 60

agaaagacga agacgagata gagagcggga aattgagatg taaccctagc tgatggaacg 120

ggcacaggca caaatacggg catgggccga gatcgagggg cggcgcatca gcagcagtgc 180

agtaggcgag cagggccgcc gcattggtgg gccgcccatg ccggtgcacc gccgccgctc 240

tcgcgggggc cgccgtgcca tggctcgtcc gatgcaggat caccggcgac ggagaggcga 300

agccctaggc cggcgctgtc gccacgccag gatcggtcgc ccaggttgac gtgactgggt 360

tgcctgcgct gaacctaata acacattgct ggg 393

<210> 24
<211> 911
<212> DNA
<213> Artificial sequence

<220>
<223> CTC79005 event: Flanking sequence - Right border (3′)

<400> 24
ccgagcgggg gcagctgagg cgccattggg aggaggaaga gggcagcgct ccggggcgga 60

agtcgtgggg cggcacgagg gaagggcaca cgacggacgg acggaggaag aagaagagcg 120

cggaggtagc tggccgagcg tgacctgcat gtcaagtcaa tggtcgcggg tgcgagtaga 180

aaagagggag gacgaccgga cacaccgccg cgtcaggtca ccgatgacct aacgtgtctg 240

gtcactattc acgcgctaaa aacacttact agactcgatc taacgcgggc agccctctga 300

gtccgatcat ttattctgtg cgtccggtca tcgacgtgtg cgggaaagcg ttggctgtta 360

acggctagtt ttgaatcgtg tgacgtggca catcgtggtt gctcgactgt tacgaccgga 420

cacggggctg agtccggtcg atgagtccga tcacccccga gctgcgtaac ggttggtttt 480

tcagaggggg tctttaaata gaccttggtc ggctccaagc cttttctctt ggtattttga 540

tcatcttgac atcctcttaa gctaagcata cttcctccca ctcatctctc ttgcttggtt 600

gtgaatccaa agtgagattg agtgattcca agtgtatttg cttgtgagtt tgcatatagt 660

ggcacttggg gatcgtttga gctgcggaat tcttgttact cttggtggct accgccacct 720

agacagcttg gagcaacaga tgagttgtgg caagagttgg tgattatttt tggtcacctc 780

ccggtgattg tgaggggttt ttgtgccttt cccggcagag cgttaaaggc aactctagtg 840

gattgcgcat gtcattgagc tacctcactt atgggtcggt tcttgcggtt tccaacgtgt 900

ggacgaggtt c 911

<210> 25
<211> 26920
<212> DNA
<213> Artificial sequence

<220>
<223> Cas9/crRNA/HR template construction

<400> 25

cttagaataa cggatattta aaagggcgtg aaaaggttta tccgttcgtc catttgtatg 60
tgcatgccaa ccacagggtt cccctcggga tcaaagtact ttgatccaac ccctccgctg 120
ctatagtgca gtcggcttct gacgttcagt gcagccgtct tctgaaaacg acatgtcgca 180
caagtcctaa gttacgcgac aggctgccgc cctgcccttt tcctggcgtt ttcttgtcgc 240
gtgttttagt cgcataaagt agaatacttg cgactagaac cggagacatt acgccatgaa 300
caagagcgcc gccgctggcc tgctgggcta tgcccgcgtc agcaccgacg accaggactt 360
gaccaaccaa cgggccgaac tgcacgcggc cggctgcacc aagctgtttt ccgagaagat 420
caccggcacc aggcgcgacc gcccggagct ggccaggatg cttgaccacc tacgccctgg 480
cgacgttgtg acagtgacca ggctagaccg cctggcccgc agcacccgcg acctactgga 540
cattgccgag cgcatccagg aggccggcgc gggcctgcgt agcctggcag agccgtgggc 600
cgacaccacc acgccggccg gccgcatggt gttgaccgtg ttcgccggca ttgccgagtt 660
cgagcgttcc ctaatcatcg accgcacccg gagcgggcgc gaggccgcca aggcccgagg 720
cgtgaagttt ggcccccgcc ctaccctcac cccggcacag atcgcgcacg cccgcgagct 780
gatcgaccag gaaggccgca ccgtgaaaga ggcggctgca ctgcttggcg tgcatcgctc 840
gaccctgtac cgcgcacttg agcgcagcga ggaagtgacg cccaccgagg ccaggcggcg 900
cggtgccttc cgtgaggacg cattgaccga ggccgacgcc ctggcggccg ccgagaatga 960
acgccaagag gaacaagcat gaaaccgcac caggacggcc aggacgaacc gtttttcatt 1020
accgaagaga tcgaggcgga gatgatcgcg gccgggtacg tgttcgagcc gcccgcgcac 1080
gtctcaaccg tgcggctgca tgaaatcctg gccggtttgt ctgatgccaa gctggcggcc 1140
tggccggcca gcttggccgc tgaagaaacc gagcgccgcc gtctaaaaag gtgatgtgta 1200
tttgagtaaa acagcttgcg tcatgcggtc gctgcgtata tgatgcgatg agtaaataaa 1260
caaatacgca aggggaacgc atgaaggtta tcgctgtact taaccagaaa ggcgggtcag 1320
gcaagacgac catcgcaacc catctagccc gcgccctgca actcgccggg gccgatgttc 1380
tgttagtcga ttccgatccc cagggcagtg cccgcgattg ggcggccgtg cgggaagatc 1440
aaccgctaac cgttgtcggc atcgaccgcc cgacgattga ccgcgacgtg aaggccatcg 1500
gccggcgcga cttcgtagtg atcgacggag cgccccaggc ggcggacttg gctgtgtccg 1560
cgatcaaggc agccgacttc gtgctgattc cggtgcagcc aagcccttac gacatatggg 1620
ccaccgccga cctggtggag ctggttaagc agcgcattga ggtcacggat ggaaggctac 1680
aagcggcctt tgtcgtgtcg cgggcgatca aaggcacgcg catcggcggt gaggttgccg 1740
aggcgctggc cgggtacgag ctgcccattc ttgagtcccg tatcacgcag cgcgtgagct 1800
acccaggcac tgccgccgcc ggcacaaccg ttcttgaatc agaacccgag ggcgacgctg 1860
cccgcgaggt ccaggcgctg gccgctgaaa ttaaatcaaa actcatttga gttaatgagg 1920

taaagagaaa atgagcaaaa gcacaaacac gctaagtgcc ggccgtccga gcgcacgcag 1980

cagcaaggct gcaacgttgg ccagcctggc agacacgcca gccatgaagc gggtcaactt 2040

tcagttgccg gcggaggatc acaccaagct gaagatgtac gcggtacgcc aaggcaagac 2100

cattaccgag ctgctatctg aatacatcgc gcagctacca gagtaaatga gcaaatgaat 2160

aaatgagtag atgaatttta gcggctaaag gaggcggcat ggaaaatcaa gaacaaccag 2220

gcaccgacgc cgtggaatgc cccatgtgtg gaggaacggg cggttggcca ggcgtaagcg 2280

gctgggttgt ctgccggccc tgcaatggca ctggaacccc caagcccgag gaatcggcgt 2340

gacggtcgca aaccatccgg cccggtacaa atcggcgcgg cgctgggtga tgacctggtg 2400

gagaagttga aggccgcgca ggccgcccag cggcaacgca tcgaggcaga agcacgcccc 2460

ggtgaatcgt ggcaagcggc cgctgatcga atccgcaaag aatcccggca accgccggca 2520

gccggtgcgc cgtcgattag gaagccgccc aagggcgacg agcaaccaga tttttcgtt 2580

ccgatgctct atgacgtggg cacccgcgat agtcgcagca tcatggacgt ggccgttttc 2640

cgtctgtcga agcgtgaccg acgagctggc gaggtgatcc gctacgagct tccagacggg 2700

cacgtagagg tttccgcagg gccggccggc atggccagtg tgtgggatta cgacctggta 2760

ctgatggcgg tttcccatct aaccgaatcc atgaaccgat accgggaagg gaagggagac 2820

aagcccggcc gcgtgttccg tccacacgtt gcggacgtac tcaagttctg ccggcgagcc 2880

gatggcggaa agcagaaaga cgacctggta gaaacctgca ttcggttaaa caccacgcac 2940

gttgccatgc agcgtacgaa gaaggccaag aacggccgcc tggtgacggt atccgagggt 3000

gaagccttga ttagccgcta caagatcgta aagagcgaaa ccgggcggcc ggagtacatc 3060

gagatcgagc tagctgattg gatgtaccgc gagatcacag aaggcaagaa cccggacgtg 3120

ctgacggttc accccgatta ctttttgatc gatcccggca tcggccgttt tctctaccgc 3180

ctggcacgcc gcgccgcagg caaggcagaa gccagatggt tgttcaagac gatctacgaa 3240

cgcagtggca gcgccggaga gttcaagaag ttctgtttca ccgtgcgcaa gctgatcggg 3300

tcaaatgacc tgccggagta cgatttgaag gaggaggcgg ggcaggctgg cccgatccta 3360

gtcatgcgct accgcaacct gatcgagggc gaagcatccg ccggttccta atgtacggag 3420

cagatgctag ggcaaattgc cctagcaggg gaaaaaggtc gaaaaggtct ctttcctgtg 3480

gatagcacgt acattgggaa cccaaagccg tacattggga accggaaccc gtacattggg 3540

aacccaaagc cgtacattgg gaaccggtca cacatgtaag tgactgatat aaaagagaaa 3600

aaaggcgatt tttccgccta aaactcttta aaacttatta aaactcttaa aacccgcctg 3660

gcctgtgcat aactgtctgg ccagcgcaca gccgaagagc tgcaaaaagc gcctaccctt 3720

cggtcgctgc gctccctacg ccccgccgct tcgcgtcggc ctatcgcggc cgctggccgc 3780

tcaaaaatgg ctggcctacg gccaggcaat ctaccagggc gcggacaagc cgcgccgtcg 3840
ccactcgacc gccggcgccc acatcaaggc accctgcctc gcgcgtttcg gtgatgacgg 3900
tgaaaacctc tgacacatgc agctcccgga gacggtcaca gcttgtctgt aagcggatgc 3960
cgggagcaga caagcccgtc agggcgcgtc agcgggtgtt ggcgggtgtc ggggcgcagc 4020
catgacccag tcacgtagcg atagcggagt gtatactggc ttaactatgc ggcatcagag 4080
cagattgtac tgagagtgca ccatatgcgg tgtgaaatac cgcacagatg cgtaaggaga 4140
aaataccgca tcaggcgctc ttccgcttcc tcgctcactg actcgctgcg ctcggtcgtt 4200
cggctgcggc gagcggtatc agctcactca aaggcggtaa tacggttatc cacagaatca 4260
ggggataacg caggaaagaa catgtgagca aaaggccagc aaaaggccag gaaccgtaaa 4320
aaggccgcgt tgctggcgtt tttccatagg ctccgccccc ctgacgagca tcacaaaaat 4380
cgacgctcaa gtcagaggtg gcgaaacccg acaggactat aaagatacca ggcgtttccc 4440
cctggaagct ccctcgtgcg ctctcctgtt ccgaccctgc cgcttaccgg atacctgtcc 4500
gcctttctcc cttcgggaag cgtggcgctt tctcatagct cacgctgtag gtatctcagt 4560
tcggtgtagg tcgttcgctc caagctgggc tgtgtgcacg aaccccccgt tcagcccgac 4620
cgctgcgcct tatccggtaa ctatcgtctt gagtccaacc cggtaagaca cgacttatcg 4680
ccactggcag cagccactgg taacaggatt agcagagcga ggtatgtagg cggtgctaca 4740
gagttcttga agtggtggcc taactacggc tacactagaa ggacagtatt tggtatctgc 4800
gctctgctga agccagttac cttcggaaaa agagttggta gctcttgatc cggcaaacaa 4860
accaccgctg gtagcggtgg tttttttgtt tgcaagcagc agattacgcg cagaaaaaaa 4920
ggatctcaag aagatccttt gatcttttct acggggtctg acgctcagtg gaacgaaaac 4980
tcacgttaag ggattttggt catgcattct aggtattatt tgccaacgac cttcgtgatc 5040
tcgcccttga catagtggac aaattcttcg agctggtcgg cccgggacgc gagacggtct 5100
tcttcttggc ccagataggc ttggcgcgct tcgaggatca cgggctggta ttgcgccgga 5160
aggcgctcca tcgcccagtc ggcggcgaca tccttcggcg cgatcttgcc ggtaaccgcc 5220
gagtaccaaa tccggctcag cgtaaggacc acattgcgct catcgcccgc ccaatccggc 5280
ggggagttcc acagggtcag cgtctcgttc agtgcttcga acagatcctg ttccggcacc 5340
gggtcgaaaa gttcctcggc cgcggggccg acgagggcca cgctatgctc ccgggccttg 5400
gtgagcagga tcgccagatc aatgtcgatg gtggccggtt caaagatacc cgccagaata 5460
tcattacgct gccattcgcc gaactggagt tcgcgtttgg ccggatagcg ccaggggatg 5520
atgtcatcgt gcaccacaat cgtcacctca accgcgcgca ggatttcgct ctcgccgggg 5580
gaggcggacg tttccagaag gtcgttgata agcgcgcggc gcgtggtctc gtcgagacgg 5640
acggtaacgg tgacaagcag gtcgatgtcc gaatggggct taaggccgcc gtcaacggcg 5700

ctaccataca gatgcacggc gaggagggtc ggttcgaggt ggcgctcgat gacacccacg 5760

acttccgaca gctgggtgga cacctcggcg atgaccgctt cacccattta ttatttcctt 5820

cctcttttct acagtattta aagatacccc aagaagctaa ttataacaag acgaactcca 5880

attcactgtt ccttgcattc taaaacctta aataccagaa aacagctttt tcaaagttgt 5940

tttcaaagtt ggcgtataac atagtatcga cggagccgat tttgaaaccg cggtgatcac 6000

aggcagcaac gctctgtcat cgttacaatc aacatgctac cctccgcgag atcatccgtg 6060

tttcaaaccc ggcagcttag ttgccgttct tccgaatagc atcggtaaca tgagcaaagt 6120

ctgccgcctt acaacggctc tcccgctgac gccgtcccgg actgatgggc tgcctgtatc 6180

gagtggtgat tttgtgccga gctgccggtc ggggagctgt tggctggctg gtggcaggat 6240

atattgtggt gtaaacaaat tgacgcttag acaacttaat aacacattgc ggacgttttt 6300

aatgtactga attagtactg ataaatggcg cgccaagctt tggcaaacag ctattatggg 6360

tattatgggt ggtaccacgc gtcgatccac tagtaacggc cgccagtgtg ctggaattcg 6420

cccttggcgc gccgatctag taacatagat gacaccgcgc gcgataattt atcctagttt 6480

gcgcgctata ttttgttttc tatcgcgtat taaatgtata attgcgggac tctaatcata 6540

aaaacccatc tcataaataa cgtcatgcat tacatgttaa ttattacatg cttaacgtaa 6600

ttcaacagaa attatatgat aatcatcgca agaccggcaa caggattcaa tcttaagaaa 6660

ctttattgcc aaatgtttga acgatctcag aagaactcgt caagaaggcg atagaaggcg 6720

atgcgctgcg aatcgggagc ggcgataccg taaagcacga ggaagcggtc agcccattcg 6780

ccgccaagct cttcagcaat atcacgggta gccaacgcta tgtcctgata gcggtccgcc 6840

acacccagcc ggccacagtc gatgaatcca gaaaagcggc cattttccac catgatattc 6900

ggcaagcagg catcgccatg ggtcacgacg agatcctcgc cgtcgggcat gcgcgccttg 6960

agcctggcga acagttcggc tggcgcgagc ccctgatgct cttcgtccag atcatcctga 7020

tcgacaagac cggcttccat ccgagtacgt gctcgctcga tgcgatgttt cgcttggtgg 7080

tcgaatgggc aggtagccgg atcaagcgta tgcagccgcc gcattgcatc agccatgatg 7140

gatactttct cggcaggagc aaggtgagat gacaggagat cctgccccgg cacttcgccc 7200

aatagcagcc agtcccttcc cgcttcagtg acaacgtcga gcacagctgc gcaaggaacg 7260

cccgtcgtgg ccagccacga tagccgcgct gcctcgtcct gcagttcatt cagggcaccg 7320

gacaggtcgg tcttgacaaa aagaaccggg cgcccctgcg ctgacagccg gaacacggcg 7380

gcatcagagc agccgattgt ctgttgtgcc cagtcatagc cgaatagcct ctccacccaa 7440

gcggccggag aacctgcgtg caatccatct tgttcaatcc acatggtggt gtgacctgca 7500

gaagtaacac caaacaacag ggtgagcatc gacaaaagaa acagtaccaa gcaaataaat 7560

agcgtatgaa ggcagggcta aaaaaatcca catatagctg ctgcatatgc catcatccaa 7620
gtatatcaag atcaaaataa ttataaaaca tacttgttta ttataataga taggtactca 7680
aggttagagc atatgaatag atgctgcata tgccatcatg tatatgcatc agtaaaaccc 7740
acatcaacat gtatacctat cctagatcga tatttccatc catcttaaac tcgtaactat 7800
gaagatgtat gacacacaca tacagttcca aaattaataa atacaccagg tagtttgaaa 7860
cagtattcta ctccgatcta gaacgaatga acgaccgccc aaccacacca catcatcaca 7920
accaagcgaa caaaaagcat ctctgtatat gcatcagtaa aacccgcatc aacatgtata 7980
cctatcctag atcgatattt ccatccatca tcttcaattc gtaactatga atatgtatgg 8040
cacacacata cagatccaaa attaataaat ccaccaggta gtttgaaaca gaattctact 8100
ccgatctaga acgaccgccc aaccagacca catcatcaca accaagacaa aaaaaagcat 8160
gaaaagatga cccgacaaac aagtgcacgg catatattga aataaaggaa aagggcaaac 8220
caaaccctat gcaacgaaac aaaaaaaatc atgaaatcga tcccgtctgc ggaacggcta 8280
gagccatccc aggattcccc aaagagaaac actggcaagt tagcaatcag aacgtgtctg 8340
acgtacaggt cgcatccgtg tacgaacgct agcagcacgg atctaacaca aacacggatc 8400
taacacaaac atgaacagaa gtagaactac cgggccctaa ccatggaccg gaacgccgat 8460
ctagagaagg tagagagggg gggggggga ggacgagcgg cgtaccttga agcggaggtg 8520
ccgacgggtg gatttggggg agatctggtt gtgtgtgtgt gcgctccgaa caacacgagg 8580
ttggggaaag agggtgtgga gggggtgtct atttattacg gcgggcgagg aagggaaagc 8640
gaaggagcgg tgggaaagga atcccccgta gctgccgtgc cgtgagagga ggaggaggcc 8700
gcctgccgtg ccggctcacg tctgccgctc cgccacgcaa tttctggatg ccgacagcgg 8760
agcaagtcca acggtggagc ggaactctcg agaggggtcc agaggcagcg acagagatgc 8820
cgtgccgtct gcttcgcttg gcccgacgcg acgctgctgg ttcgctggtt ggtgtccgtt 8880
agactcgtcg acggcgttta acaggctggc attatctact cgaaacaaga aaaatgtttc 8940
cttagttttt ttaatttctt aaagggtatt tgtttaattt ttagtcactt tattttattc 9000
tattttatat ctaaattatt aaataaaaaa actaaaatag agttttagtt ttcttaattt 9060
agaggctaaa atagaataaa atagatgtac taaaaaaatt agtctataaa aaccattaac 9120
cctaaaccct aaatggatgt actaataaaa tggatgaagt attatatagg tgaagctatt 9180
tgcaaaaaaa aaggagaaca catgcacact aaaaagataa aactgtagag tcctgttgtc 9240
aaaatactca attgtccttt agaccatgtc taactgttca tttatatgat tctctaaaac 9300
actgatatta ttgtagtact atagattata ttattcgtag agtaaagttt aaatatatgt 9360
ataaagatag ataaactgca cttcaaacaa gtgtgacaaa aaaaatatgt ggtaattttt 9420
tataacttag acatgcaatg ctcattatct ctagagaggg gcacgaccgg gtcacgctgc 9480

actgcaggga tccgatctag taacatagat gacaccgcgc gcgataattt atcctagttt 9540
gcgcgctata ttttgttttc tatcgcgtat taaatgtata attgcgggac tctaatcata 9600
aaaacccatc tcataaataa cgtcatgcat tacatgttaa ttattacatg cttaacgtaa 9660
ttcaacagaa attatatgat aatcatcgca agaccggcaa caggattcaa tcttaagaaa 9720
ctttattgcc aaatgtttga acgatcccta ggacgatctc acttgtacag ctcgtccatg 9780
ccgtgggtga tgccagctgc ggtgacgaac tccagcagga ccatgtggtc gcgcttctcg 9840
ttggggtcct tgctcagagc ggactgggtg ctcaggtagt ggttgtcggg cagcagcacg 9900
ggaccgtcgc cgatgggcgt gttctgctgg tagtggtcgg cgagctggac gctgccgtcc 9960
tcgatgttgt ggcggatctt gaagttgacc ttgatgccgt tcttctgctt gtcagccatg 10020
atgtagacgt tgtggctgtt gtagttgtac tccagcttgt gccccaggat gttgccgtcc 10080
tccttgaagt cgatgccctt cagctcgatg cggttcacca gggtgtcgcc ctcgaacttc 10140
acctcggctc gggtcttgta gttgccgtcg tccttgaaga agatggtgcg ctcctggacg 10200
tagccttcgg gcatggcgga cttgaagaag tcgtgctgct tcatgtggtc ggggtagcgg 10260
ctgaagcact gcacgccgta ggtgaaggtg gtcacgaggg tgggccaggg cacgggcagc 10320
ttgccggtgg tgcagatgaa cttcagggtc agcttgccgt aggtggcgtc gccctcgccc 10380
tcgccgctga cgctgaactt gtggccgttc acgtcgccgt ccagctcgac caggatgggc 10440
accaccccag tgaacagctc ctcgcccttg ctcactacaa aaaagctccg cacgaggctg 10500
catttgtcac aaatcatgaa aagaaaaact accgatgaac aatgctgagg gattcaaatt 10560
ctacccacaa aaagaagaaa gaaagatcta gcacatctaa gcctgacgaa gcagcagaaa 10620
tatataaaaa tataaaccat agtgcccttt tcccctcttc ctgatcttgt ttagcacggc 10680
ggaaatttta aacccccccat catctccccc aacaacggcg gatcgcagat ctacatccga 10740
gagccccatt ccccgcgaga tccgggccgg atccacgccg gcgagagccc cagccgcgag 10800
atcccgcccc tcccgcgcac cgatctgggc gcgcacgaag ccgcctctcg cccacccaaa 10860
ctaccaaggc caaagatcga gaccgagacg gaaaaaaaaa cggagaaaga aagaggagag 10920
gggcggggtg gttaccggcg gcggcggagg cctcccttgg atcttatggt gtgttgtccc 10980
tgtgtgttct ccaatagtgt ggcttgagtg tgtggaagat ggttctagag gatctgctag 11040
agtcagcttg tcagcgtgtc ctctccaaat gaaatgaact tccttatata gaggaagggt 11100
cttgcgaagg atagtgggat tgtgcgtcat cccttacgtc agtggagata tcacatcaat 11160
ccacttgctt tgaagacgtg gttggaacgt cttctttttc cacgatgctc ctcgtgggtg 11220
gggtccatc tttgggacca ctgtcggcag aggcatcttc aacgatggcc tttcctttat 11280
cgcaatgatg gcatttgtag gagccacctt ccttttccac tatcttcaca ataaagtgac 11340

agatagctgg gcaatggaat ccgaggaggt ttccggatat taccctttgt tgaaaagtct 11400
caatcggacc atcacatcaa tccacttgct ttgaagacgt ggttggaacg tcttcttttt 11460
ccacgatgct cctcgtgggt gggggtccat ctttgggacc actgtcggca gaggcatctt 11520
caacgatggc ctttccttta tcgcaatgat ggcatttgta ggagccacct tccttttcca 11580
ctatcttcac aataaagtga cagatagctg ggcaatggaa tccgaggagg tttccggata 11640
ttaccctttg ttgaaaagtc tcaatcggac cccctcagcc tgcagtgcag cgtgacccgg 11700
tcgtgcccct ctctagagat aatgagcatt gcatgtctaa gttataaaaa attaccacat 11760
attttttttg tcacacttgt ttgaagtgca gtttatctat ctttatacat atatttaaac 11820
tttactctac gaataatata atctatagta ctacaataat atcagtgttt tagagaatca 11880
tataaatgaa cagttagaca tggtctaaag gacaattgag tattttgaca acaggactct 11940
acagttttat ctttttagtg tgcatgtgtt ctcctttttt tttgcaaata gcttcaccta 12000
tataatactt catccatttt attagtacat ccatttaggg tttagggtta atggttttta 12060
tagactaatt tttttagtac atctatttta ttctatttta gcctctaaat taagaaaact 12120
aaaactctat tttagttttt ttatttaata atttagatat aaaatagaat aaaataaagt 12180
gactaaaaat taaacaaata ccctttaaga aattaaaaaa actaaggaaa catttttctt 12240
gtttcgagta gataatgcca gcctgttaaa cgccgtcgac gagtctaacg gacaccaacc 12300
agcgaaccag cagcgtcgcg tcgggccaag cgaagcagac ggcacggcat ctctgtcgct 12360
gcctctggac ccctctcgag agttccgctc caccgttgga cttgctccgc tgtcggcatc 12420
cagaaattgc gtggcggagc ggcagacgtg agccggcacg gcaggcggcc tcctcctcct 12480
ctcacggcac ggcagctacg gggattcct ttcccaccgc tccttcgctt tcccttcctc 12540
gcccgccgta ataaatagac accccctcca caccctcttt ccccaacctc gtgttgttcg 12600
gagcgcacac acacacaacc agatctcccc caaatccacc cgtcggcacc tccgcttcaa 12660
ggtacgccgc tcgtcctccc cccccccccc tctctacctt ctctagatcg gcgttccggt 12720
ccatggttag ggcccggtag ttctacttct gttcatgttt gtgttagatc cgtgtttgtg 12780
ttagatccgt gctgctagcg ttcgtacacg gatgcgacct gtacgtcaga cacgttctga 12840
ttgctaactt gccagtgttt ctctttgggg aatcctggga tggctctagc cgttccgcag 12900
acgggatcga tttcatgatt ttttttgttt cgttgcatag ggtttggttt gccctttcc 12960
tttatttcaa tatatgccgt gcacttgttt gtcgggtcat cttttcatgc tttttttgt 13020
cttggttgtg atgatgtggt ctggttgggc ggtcgttcta gatcggagta gaattctgtt 13080
tcaaactacc tggtggattt attaatttg gatctgtatg tgtgtgccat acatattcat 13140
agttacgaat tgaagatgat ggatggaaat atcgatctag gataggtata catgttgatg 13200
cgggttttac tgatgcatat acagagatgc tttttgttcg cttggttgtg atgatgtggt 13260

gtggttgggc ggtcgttcat tcgttctaga tcggagtaga atactgtttc aaactacctg 13320

gtgtatttat taattttgga actgtatgtg tgtgtcatac atcttcatag ttacgagttt 13380

aagatggatg gaaatatcga tctaggatag gtatacatgt tgatgtgggt tttactgatg 13440

catatacatg atggcatatg cagcatctat tcatatgctc taaccttgag tacctatcta 13500

ttataataaa caagtatgtt ttataattat tttgatcttg atatacttgg atgatggcat 13560

atgcagcagc tatatgtgga tttttttagc cctgccttca tacgctattt atttgcttgg 13620

tactgtttct tttgtcgatg ctcaccctgt tgtttggtgt tacttctgca ggcgatcgcc 13680

acaccaccat gccgaagaag aagcgcaagg tcatggacaa gaagtactcc atcggcctgg 13740

acatcggcac caacagcgtg ggctgggccg tcatcaccga cgagtacaag gtgccctcca 13800

agaagttcaa ggtcctcggc aacaccgaca ggcacagcat caagaagaac ctgatcggcg 13860

ccctgctgtt cgactccggc gagactgcgg aggctaccag gctgaagcgc actgctcgca 13920

ggcgctacac caggcgcaag aaccgcatct gctacctcca ggagattttc tccaacgaga 13980

tggccaaggt ggacgactcc ttcttccacc gcctggagga gagcttcctg gtcgaggaag 14040

acaagaagca cgagcgccac cctatcttcg gcaacatcgt ggacgaggtc gcctaccacg 14100

agaagtaccc aaccatctac cacctccgca agaagctggt ggactccacc gacaaggccg 14160

acctgaggct catctacctg gccctcgccc acatgatcaa gttccgcggc cacttcctca 14220

tcgagggcga cctgaacccg gacaacagcg acgtggacaa gctcttcatc cagctggtcc 14280

agacctacaa ccagctgttc gaggagaacc ccatcaacgc ctccggcgtg gacgctaagg 14340

ctatcctcag cgctaggctg tccaagagca ggcgcctgga gaacctcatc gcccagctcc 14400

cgggcgagaa gaagaacggc ctcttcggca acctgatcgc tctgtccctc ggcctgaccc 14460

ccaacttcaa gagcaacttc gacctggccg aggacgccaa gctccagctg tccaaggaca 14520

cctacgacga cgacctcgac aacctgctcg cccagatcgg cgaccagtac gccgacctct 14580

tcctggccgc caagaacctc tccgacgcca tcctgctcag cgacatcctg agggtgaaca 14640

ccgagatcac caaggccccg ctgtccgcca gcatgatcaa gcgctacgac gagcaccacc 14700

aggacctcac tctcctgaag gccctcgtcc gccagcagct gcccgagaag tacaaggaga 14760

ttttcttcga ccagagcaag aacggctacg cgggctacat cgatggcggc gcctcccagg 14820

aagagttcta caagttcatc aagcctatcc tggagaagat ggacggcacc gaggagctgc 14880

tcgtgaagct gaaccgcgag gacctgctcc gcaagcagag gaccttcgac aacggcagca 14940

tccctcacca gatccacctg ggcgagctgc acgctatcct ccgccgccag gaagacttct 15000

acccattcct gaaggacaac cgcgagaaga tcgagaagat cctcaccttc cgcatcccgt 15060

actacgtggg ccccctggcc cgcggcaact ccaggttcgc ctggatgacc aggaagagcg 15120

aggagaccat caccccgtgg aacttcgagg aagtggtgga caagggcgcc tccgctcaga 15180

gcttcatcga gcgcatgacc aacttcgaca agaacctccc taacgagaag gtgctgccaa 15240

agcactccct gctctacgag tacttcaccg tctacaacga gctgaccaag gtgaagtatg 15300

tgaccgaggg catgaggaag cccgccttcc tcagcggcga gcagaagaag gccatcgtgg 15360

acctgctctt caagaccaac cgcaaggtga ccgtcaagca gctgaaggaa gactacttca 15420

agaagatcga gtgcttcgac tccgtggaga tcagcggcgt ggaggaccgc ttcaacgcct 15480

ccctcggcac ctaccacgac ctgctcaaga tcatcaagga caaggacttc ctcgacaacg 15540

aggagaacga ggacatcctg gaggacatcg tgctcaccct gaccctcttc gaggaccgcg 15600

agatgatcga ggagaggctc aagacctacg cccacctgtt cgacgacaag gtcatgaagc 15660

agctgaagag gcgcaggtac actggctggg gccgcctcag caggaagctg atcaacggca 15720

tcagggacaa gcagtccggc aagaccatcc tggacttcct caagagcgac ggcttcgcca 15780

accgcaactt catgcagctc atccacgacg actccctgac cttcaaggaa gacatccaga 15840

aggctcaggt gtccggccag ggcgacagcc tccacgagca catcgctaac ctggcgggca 15900

gccctgccat caagaagggc atcctccaga ccgtgaaggt ggtggacgag ctggtgaagg 15960

tcatgggccg ccacaagcca gagaacatcg tcatcgagat ggccagggag aaccagacca 16020

cccagaaggg tcagaagaac tcccgcgaga ggatgaagag gatcgaggaa ggcatcaagg 16080

agctgggcag ccagatcctg aaggagcacc cggtggagaa cacccagctc cagaacgaga 16140

agctgtacct ctactacctg cagaacggcc gcgacatgta tgtggaccag gagctggaca 16200

tcaacaggct gtccgactac gacgtggacc acatcgtccc tcagtccttc ctcaaggacg 16260

acagcatcga caacaaggtg ctgacccgca gcgacaagaa caggggcaag tccgacaacg 16320

tcccaagcga ggaagtggtc aagaagatga agaactactg gcgccagctg ctcaacgcca 16380

agctcatcac ccagcgcaag ttcgacaacc tgactaaggc ggagaggggc ggcctgtccg 16440

agctggacaa ggctggcttc atcaagcgcc agctcgtgga gaccaggcag atcaccaagc 16500

acgtcgccca gatcctggac agcaggatga acaccaagta cgacgagaac gacaagctca 16560

tccgcgaggt gaaggtcatc accctcaagt ccaagctggt gagcgacttc cgcaaggact 16620

tccagttcta caaggtcagg gagatcaaca actaccacca cgcccacgat gcttacctca 16680

acgcggtggt gggcaccgcc ctcatcaaga agtaccctaa gctggagagc gagttcgtgt 16740

acggcgacta caaggtgtac gacgtccgca agatgatcgc caagtccgag caggagatcg 16800

gcaaggccac cgccaagtac ttcttctaca gcaacatcat gaacttcttc aagaccgaga 16860

tcaccctcgc caacggcgag atccgcaaga ggccactgat cgagaccaac ggcgagactg 16920

gcgagatcgt gtgggacaag ggcagggact tcgccaccgt gaggaaggtc ctgtccatgc 16980

ctcaggtgaa catcgtcaag aagaccgagg tccagaccgg cggcttctcc aaggagagca 17040

tcctcccaaa gcgcaacagc gacaagctga tcgccaggaa gaaggactgg gacccgaaga 17100
agtacggtgg cttcgactcc cctactgtgg cttacagcgt cctggtggtc gccaaggtgg 17160
agaagggcaa gtccaagaag ctgaagagcg tcaaggagct gctcggcatc accatcatgg 17220
agaggtccag cttcgagaag aacccgatcg acttcctgga ggccaagggc tacaaggaag 17280
tgaagaagga cctgatcatc aagctgccca agtacagcct gttcgagctg gagaacggcc 17340
gcaagaggat gctcgcctcc gctggcgagc tgcagaaggg caacgagctg gccctcccgt 17400
ccaagtatgt gaacttcctg tacctcgcct cccactacga gaagctgaag ggcagccccg 17460
aggacaacga gcagaagcag ctcttcgtcg agcagcacaa gcactacctg gacgagatca 17520
tcgagcagat cagcgagttc agcaagcgcg tgatcctcgc cgacgccaac ctcgacaagg 17580
tcctgtccgc ctacaacaag caccgcgaca agcctatcag ggagcaggcc gagaacatca 17640
tccacctgtt caccctcacc aacctgggcg ccccagctgc cttcaagtac ttcgacacca 17700
ccatcgaccg caagaggtac accagcacca aggaagtgct ggacgccacc ctgatccacc 17760
agtccatcac cggcctgtac gagactcgca tcgacctcag ccagctgggc ggcgacccga 17820
agaagaagcg caaagtctga gggaccctcg atcgacaagc tcgagtttct ccataataat 17880
gtgtgagtag ttcccagata agggaattag ggttcctata gggtttcgct catgtgttga 17940
gcatataaga aacccttagt atgtatttgt atttgtaaaa tacttctatc aataaaattt 18000
ctaattccta aaaccaaaat ccagtactaa aatccagatc ccccgaatta acctgcaggg 18060
gcggcaggga gagttttaac attgactagc gtgctgataa tttgtgagaa ataataattg 18120
acaagtagat actgacattt gagaagagct tctgaactgt tattagtaac aaaaatggaa 18180
agctgatgca cggaaaaagg aaagaaaaag ccatactttt ttttaggtag gaaaagaaaa 18240
agccatacga gactgatgtc tctcagatgg gccgggatct gtctatctag caggcagcag 18300
ccctaccaac ctcacgggcc agcaattacg agtccttcta aaacgtcccg ccgagggcgc 18360
gtggccgtgc tgtgcagcag cacgtctaac attagtccca cctcgccagt ttacagggag 18420
cagaaccagc ttataagcgg aggcgcggca ccaagaagca gcgctccggg gcggaagtcg 18480
gttttagagc tagaaatagc aagttaaaat aaggctagtc cgttatcaac ttgaaaaagt 18540
ggcaccgagt cggtgctttt ttttcctcga gggcgctccg gggcggaagt cgtgggtaat 18600
ccttcaaagg attttaaaaa atactaaatc tgagctagga tctgcagatg aggaagaaag 18660
acgaagacga gatagagagc gggaaattga gatgtaaccc tagctgatgg aacgggcaca 18720
ggcacaaata cgggcatggg ccgagatcga ggggcggcgc atcagcagca gtgcagtagg 18780
cgagcagggc cgccgcattg gtgggccgcc catgccggtg caccgccgcc gctctcgcgg 18840
gggccgccgt gccatggctc gtccgatgca ggatcaccgg cgacggagag gcgaagccct 18900

aggccggcgc tgtcgccacg ccaggatcgg tcgcccaggt tgacgtgact gggttgcctg 18960
cgctgaacct aataacacat tgctgggttg tggtgtaaac aaattgacgc ttagacaact 19020
taataacaca ttgcggacgt ttttaatgta ctgaattagt actgatatcg gtacccgggg 19080
cggccgcgag ctcgatccac tagtaacggc cgccagtgtg ctggaattcg cccttggcgc 19140
gccgatctag taacatagat gacaccgcgc gcgataattt atcctagttt gcgcgctata 19200
ttttgttttc tatcgcgtat taaatgtata attgcgggac tctaatcata aaaacccatc 19260
tcataaataa cgtcatgcat tacatgttaa ttattacatg cttaacgtaa ttcaacagaa 19320
attatatgat aatcatcgca agaccggcaa caggattcaa tcttaagaaa ctttattgcc 19380
aaatgtttga acgatctcag aagaactcgt caagaaggcg atagaaggcg atgcgctgcg 19440
aatcgggagc ggcgataccg taaagcacga ggaagcggtc agcccattcg ccgccaagct 19500
cttcagcaat atcacgggta gccaacgcta tgtcctgata gcggtccgcc acacccagcc 19560
ggccacagtc gatgaatcca gaaaagcggc cattttccac catgatattc ggcaagcagg 19620
catcgccatg ggtcacgacg agatcctcgc cgtcgggcat gcgcgccttg agcctggcga 19680
acagttcggc tggcgcgagc ccctgatgct cttcgtccag atcatcctga tcgacaagac 19740
cggcttccat ccgagtacgt gctcgctcga tgcgatgttt cgcttggtgg tcgaatgggc 19800
aggtagccgg atcaagcgta tgcagccgcc gcattgcatc agccatgatg gatactttct 19860
cggcaggagc aaggtgagat gacaggagat cctgccccgg cacttcgccc aatagcagcc 19920
agtcccttcc cgcttcagtg acaacgtcga gcacagctgc gcaaggaacg cccgtcgtgg 19980
ccagccacga tagccgcgct gcctcgtcct gcagttcatt cagggcaccg gacaggtcgg 20040
tcttgacaaa aagaaccggg cgcccctgcg ctgacagccg gaacacggcg gcatcagagc 20100
agccgattgt ctgttgtgcc cagtcatagc cgaatagcct ctccacccaa gcggccggag 20160
aacctgcgtg caatccatct tgttcaatcc acattctaga gtcgacctgc agaagtaaca 20220
ccaaacaaca gggtgagcat cgacaaaaga aacagtacca agcaaataaa tagcgtatga 20280
aggcagggct aaaaaaatcc acatatagct gctgcatatg ccatcatcca agtatatcaa 20340
gatcaaaata attataaaac atacttgttt attataatag ataggtactc aaggttagag 20400
catatgaata gatgctgcat atgccatcat gtatatgcat cagtaaaacc cacatcaaca 20460
tgtataccta tcctagatcg atatttccat ccatcttaaa ctcgtaacta tgaagatgta 20520
tgacacacac atacagttcc aaaattaata aatacaccag gtagtttgaa acagtattct 20580
actccgatct agaacgaatg aacgaccgcc caaccacacc acatcatcac aaccaagcga 20640
acaaaaagca tctctgtata tgcatcagta aaacccgcat caacatgtat acctatccta 20700
gatcgatatt tccatccatc atcttcaatt cgtaactatg aatatgtatg gcacacacat 20760
acagatccaa aattaataaa tccaccaggt agtttgaaac agaattctac tccgatctag 20820

aacgaccgcc caaccagacc acatcatcac aaccaagaca aaaaaaagca tgaaaagatg 20880

acccgacaaa caagtgcacg gcatatattg aaataaagga aaagggcaaa ccaaacccta 20940

tgcaacgaaa caaaaaaaat catgaaatcg atcccgtctg cggaacggct agagccatcc 21000

caggattccc caaagagaaa cactggcaag ttagcaatca gaacgtgtct gacgtacagg 21060

tcgcatccgt gtacgaacgc tagcagcacg gatctaacac aaacacggat ctaacacaaa 21120

catgaacaga agtagaacta ccgggcccta accatggacc ggaacgccga tctagagaag 21180

gtagagaggg gggggggggg aggacgagcg gcgtaccttg aagcggaggt gccgacgggt 21240

ggatttgggg gagatctggt tgtgtgtgtg tgcgctccga acaacacgag gttggggaaa 21300

gagggtgtgg agggggtgtc tatttattac ggcgggcgag gaagggaaag cgaaggagcg 21360

gtgggaaagg aatcccccgt agctgccgtg ccgtgagagg aggaggaggc cgcctgccgt 21420

gccggctcac gtctgccgct ccgccacgca atttctggat gccgacagcg gagcaagtcc 21480

aacggtggag cggaactctc gagaggggtc cagaggcagc gacagagatg ccgtgccgtc 21540

tgcttcgctt ggcccgacgc gacgctgctg gttcgctggt tggtgtccgt tagactcgtc 21600

gacggcgttt aacaggctgg cattatctac tcgaaacaag aaaaatgttt ccttagtttt 21660

tttaatttct taaagggtat ttgtttaatt tttagtcact ttattttatt ctattttata 21720

tctaaattat taaataaaaa aactaaaata gagttttagt tttcttaatt tagaggctaa 21780

aatagaataa aatagatgta ctaaaaaaat tagtctataa aaaccattaa ccctaaaccc 21840

taaatggatg tactaataaa atggatgaag tattatatag gtgaagctat ttgcaaaaaa 21900

aaaggagaac acatgcacac taaaaagata aaactgtaga gtcctgttgt caaaatactc 21960

aattgtcctt tagaccatgt ctaactgttc atttatatga ttctctaaaa cactgatatt 22020

attgtagtac tatagattat attattcgta gagtaaagtt taaatatatg tataaagata 22080

gataaactgc acttcaaaca agtgtgacaa aaaaaatatg tggtaatttt ttataactta 22140

gacatgcaat gctcattatc tctagagagg ggcacgaccg ggtcacgctg cactgcaggc 22200

atacgcgtga tccactagta acggccgcca gtgtgctgga attcgccctt ggcgcgccga 22260

tctagtaaca tagatgacac cgcgcgcgat aatttatcct agtttgcgcg ctatattttg 22320

ttttctatcg cgtattaaat gtataattgc gggactctaa tcataaaaac ccatctcata 22380

aataacgtca tgcattacat gttaattatt acatgcttaa cgtaattcaa cagaaattat 22440

atgataatca tcgcaagacc ggcaacagga ttcaatctta agaaacttta ttgccaaatg 22500

tttgaacgat ctcactcagc ctcgagggtg gcggtcactg ggatgaactc gaacctgtcg 22560

atgatcacgc cggcggtgcc gctgaagttc ctcacgccca cgatgttacc gagggaggag 22620

gtgaaagcgt tggcgctctc gaagtaaccg aagtcgctgg attggaggtt gtccagggag 22680

gtagcggtag ctggcacggt gttggagaag atggaggagt taccccagtt cacgttgagg 22740
tggatcgggg tcacggaagc gtacctcacg cgcaccctgt acctggtgga ggtggatggg 22800
aagtggattg gcacctcgat gtagcccctg ttctggatgt tgttgccgct gctgttgagc 22860
ctcacgaggt cgccaccggt gaagcctggg ccggaaatga cggaaccgtt gaagaggaag 22920
ttgcccttca cggccgggat ttgggtgatg ctgtcggagg cgatgatgtt gttgaactca 22980
gcgctgcggt ggatccagga gaacatcgga gccctgatga tgctcacgga gctgttgctg 23040
aagccggagc ggaacatgga cacgtggctc agcctgtggg agaagccttg cctgggtggc 23100
acgttgttgt tctgtggtgg gatctcgtcc agggagtcca cggtgccgct cttcctgtag 23160
acagcggatg gcaggttgga ggaggtgccg taggcgaact cggtgccgtc gagcacggac 23220
agttgctggt tgttgatacc gatgttgaag ggcctcctgt acagggtgga ggacagggtc 23280
ctgtagacac cctgacccag ttgagccacg atgcgttgct gtggagcggc gttgcccatg 23340
gtgccgtaga gcgggaaggt gaactcgggg ccgctgaagc ccactgggga ggccatgatc 23400
tggtggccgg accagtagta ctcgcccctg tgagcgtcgg tgtagatggt gatgctgttc 23460
aggatgtcca tcaggtgtgg gctcctgatg gagccctcga taccctgggc ggaaccgcgg 23520
aagctaccgt cgaagttctc cagcactggg ttggtgtaga tctccctggt gagttgggac 23580
acggtgcgga tcgggtaggt cctggagtcg tagttcggga agagggacac aatgtccagc 23640
acggtgaggg tcaactccct cctgaactgg ttgtacctga tccagtccct ggagtccgga 23700
ccccagacgc gctccaggcc ggtgttgtac cagcgcacag cgtggtcggt gtagttgcca 23760
atcagcctgg tcaggtcgtt gtagcggctg ttgatggtgg cagcatcgaa gccccacctt 23820
tggccgaaca cgctcacgtc gcgcagcacg ctgaggtgca ggttagcggc ttgcacgtac 23880
acggacagga gcggcacttg gtagttctgg acggcgaaca gtgggatagc ggtggtcagg 23940
gcgctgttca tgtcgttgaa ttgaatgcgc atttcctcgc ggagagctgg gttggtcggg 24000
tcggcctccc actccctgaa gctctcggcg tagatttggt agaggttgct caggccctcc 24060
agcctggaga tggcctggtt cctggcgaac tcttcgatcc tctggttgat cagctgctcg 24120
atttgcacca ggaaggcgtc ccattgggat ggaccgaaga taccccagat gatgtccacc 24180
aggccgagca cgaagccagc acctggcacg aactcgctga gcaggaactg ggtcaaggac 24240
agggagatgt cgatgggggt gtaaccggtc tcgatgcgct cgccacccag cacctccacc 24300
tctgggttgc tcaggcagtt gtatgggatg cactcgttga tgtttgggtt gttgtccatt 24360
ctagagtcga cctgcagaag taacaccaaa caacagggtg agcatcgaca aaagtgctgc 24420
atatgccatc atccaagtat atcaagatca aaataattat aaaacatact tgtttattat 24480
aatagatagg tactcaaggt tagagcatat gaatagatgc tgcatatgcc atcatgtata 24540
tgcatcagta aaacccacat caacatgtat acctatccta gatcgatatt tccatccatc 24600

ttaaactcgt aactatgaag atgtatgaca cacacataca gttccaaaat taataaatac 24660
accaggtagt ttgaaacagt attctactcc gatctagaac gaatgaacga ccgcccaacc 24720
acaccacatc atcacaacca agcgaacaaa aagcatctct gtatatgcat cagtaaaacc 24780
cgcatcaaca tgtataccta tcctagatcg atatttccat ccatcatctt caattcgtaa 24840
ctatgaatat gtatggcaca cacatacaga tccaaaatta ataaatccac caggtagttt 24900
gaaacagaat tctactccga tctagaacga ccgcccaacc agaccacatc atcacaacca 24960
agacaaaaaa aagcatgaaa agatgacccg acaaacaagt gcacggcata tattgaaata 25020
aaggaaaagg gcaaaccaaa ccctatgcaa cgaaacaaaa aaaatcatga aatcgatccc 25080
gtctgcggaa cggctagagc catcccagga ttccccaaag agaaacactg gcaagttagc 25140
aatcagaacg tgtctgacgt acaggtcgca tccgtgtacg aacgctagca gcacggatct 25200
aacacaaaca cggatctaac acaaacatga acagaagtag aactaccggg ccctaaccat 25260
ggaccggaac gccgatctag agaaggtaga gagggggggg gggggaggac gagcggcgta 25320
ccttgaagcg gaggtgccga cgggtggatt tgggggagat ctggttgtgt gtgtgtgcgc 25380
tccgaacaac acgaggttgg ggaaagaggg tgtggagggg gtgtctattt attacggcgg 25440
gcgaggaagg gaaagcgaag gagcggtggg aaaggaatcc cccgtagctg ccgtgccgtg 25500
agaggaggag gaggccgcct gccgtgccgg ctcacgtctg ccgctccgcc acgcaatttc 25560
tggatgccga cagcggagca agtccaacgg tggagcggaa ctctcgagag gggtccagag 25620
gcagcgacag agatgccgtg ccgtctgctt cgcttggccc gacgcgacgc tgctggttcg 25680
ctggttggtg tccgttagac tcgtcgacgg cgtttaacag gctggcatta tctactcgaa 25740
acaagaaaaa tgtttcctta gtttttttaa tttcttaaag ggtatttgtt taatttttag 25800
tcactttatt ttattctatt ttatatctaa attattaaat aaaaaaacta aaatagagtt 25860
ttagttttct taatttagag gctaaaatag aataaaatag atgtactaaa aaaattagtc 25920
tataaaaacc attaacccta aaccctaaat ggatgtacta ataaaatgga tgaagtatta 25980
tataggtgaa gctatttgca aaaaaaaagg agaacacatg cacactaaaa agataaaact 26040
gtagagtcct gttgtcaaaa tactcaattg tcctttagac catgtctaac tgttcattta 26100
tatgattctc taaaacactg atattattgt agtactatag attatattat tcgtagagta 26160
aagtttaaat atatgtataa agatagataa actgcacttc aaacaagtgt gacaaaaaaa 26220
atatgtggta attttttata acttagacat gcaatgctca ttatctctag agaggggcac 26280
gaccgggtca cgctgcactg caggcatcga tcgaagctta cgcgtccatg gctgcaggag 26340
ctcgtcgact ctagactcga gggcgagccc cgagcggggg cagctgaggc gccattggga 26400
ggaggaagag ggcagcgctc cggggcggaa gtcgtgcggc ggcacgaggg aagggcacac 26460

gacggacgga cggaggaaga agaagagcgc ggaggtagct ggccgagcgt gacctgcatg 26520

tcaagtcaat ggtcgcgggt gcgagtagaa aagagggagg acgaccggac acaccgccgc 26580

gtcaggtcac cgatgaccta acgtgtctgg tcactattca cgcgctaaaa acacttacta 26640

gactcgatct aacgcgggca gccctctgag tccgatcatt tattctgtgc gtccggtcat 26700

cgacgtgtgc gggaaagcgt tggctgttaa cggctagttt tgaatcgtgt gacgtggcac 26760

accacgactt ccgccccgga gcgcggtacc actagtatta attaagttta aacggcgcgc 26820

caagggcgaa ttccagcaca ctggcggccg ttactagtgg atcgagctcg tcgactctag 26880

actcgagggc gcgcctgaca ggatatattg gcgggtaaac 26920

<210> 26
<211> 17650
<212> DNA
<213> Artificial sequence

<220>
<223> HR template construction

<400> 26
cttagaataa cggatattta aaagggcgtg aaaaggttta tccgttcgtc catttgtatg 60

tgcatgccaa ccacagggtt cccctcggga tcaaagtact ttgatccaac ccctccgctg 120

ctatagtgca gtcggcttct gacgttcagt gcagccgtct tctgaaaacg acatgtcgca 180

caagtcctaa gttacgcgac aggctgccgc cctgcccttt tcctggcgtt ttcttgtcgc 240

gtgttttagt cgcataaagt agaatacttg cgactagaac cggagacatt acgccatgaa 300

caagagcgcc gccgctggcc tgctgggcta tgcccgcgtc agcaccgacg accaggactt 360

gaccaaccaa cgggccgaac tgcacgcggc cggctgcacc aagctgtttt ccgagaagat 420

caccggcacc aggcgcgacc gcccggagct ggccaggatg cttgaccacc tacgccctgg 480

cgacgttgtg acagtgacca ggctagaccg cctggcccgc agcacccgcg acctactgga 540

cattgccgag cgcatccagg aggccggcgc gggcctgcgt agcctggcag agccgtgggc 600

cgacaccacc acgccggccg gccgcatggt gttgaccgtg ttcgccggca ttgccgagtt 660

cgagcgttcc ctaatcatcg accgcacccg gagcgggcgc gaggccgcca aggcccgagg 720

cgtgaagttt ggcccccgcc ctaccctcac cccggcacag atcgcgcacg cccgcgagct 780

gatcgaccag gaaggccgca ccgtgaaaga ggcggctgca ctgcttggcg tgcatcgctc 840

gaccctgtac cgcgcacttg agcgcagcga ggaagtgacg cccaccgagg ccaggcggcg 900

cggtgccttc cgtgaggacg cattgaccga ggccgacgcc ctggcggccg ccgagaatga 960

acgccaagag gaacaagcat gaaaccgcac caggacggcc aggacgaacc gtttttcatt 1020

accgaagaga tcgaggcgga gatgatcgcg gccgggtacg tgttcgagcc gcccgcgcac 1080

gtctcaaccg tgcggctgca tgaaatcctg gccggtttgt ctgatgccaa gctggcggcc 1140

tggccggcca gcttggccgc tgaagaaacc gagcgccgcc gtctaaaaag gtgatgtgta 1200
tttgagtaaa acagcttgcg tcatgcggtc gctgcgtata tgatgcgatg agtaaataaa 1260
caaatacgca aggggaacgc atgaaggtta tcgctgtact taaccagaaa ggcgggtcag 1320
gcaagacgac catcgcaacc catctagccc gcgccctgca actcgccggg gccgatgttc 1380
tgttagtcga ttccgatccc cagggcagtg cccgcgattg ggcggccgtg cgggaagatc 1440
aaccgctaac cgttgtcggc atcgaccgcc cgacgattga ccgcgacgtg aaggccatcg 1500
gccggcgcga cttcgtagtg atcgacggag cgccccaggc ggcggacttg gctgtgtccg 1560
cgatcaaggc agccgacttc gtgctgattc cggtgcagcc aagcccttac gacatatggg 1620
ccaccgccga cctggtggag ctggttaagc agcgcattga ggtcacggat ggaaggctac 1680
aagcggcctt tgtcgtgtcg cgggcgatca aaggcacgcg catcggcggt gaggttgccg 1740
aggcgctggc cgggtacgag ctgcccattc ttgagtcccg tatcacgcag cgcgtgagct 1800
acccaggcac tgccgccgcc ggcacaaccg ttcttgaatc agaacccgag ggcgacgctg 1860
cccgcgaggt ccaggcgctg gccgctgaaa ttaaatcaaa actcatttga gttaatgagg 1920
taaagagaaa atgagcaaaa gcacaaacac gctaagtgcc ggccgtccga gcgcacgcag 1980
cagcaaggct gcaacgttgg ccagcctggc agacacgcca gccatgaagc gggtcaactt 2040
tcagttgccg gcggaggatc acaccaagct gaagatgtac gcggtacgcc aaggcaagac 2100
cattaccgag ctgctatctg aatacatcgc gcagctacca gagtaaatga gcaaatgaat 2160
aaatgagtag atgaatttta gcggctaaag gaggcggcat ggaaaatcaa gaacaaccag 2220
gcaccgacgc cgtggaatgc cccatgtgtg gaggaacggg cggttggcca ggcgtaagcg 2280
gctgggttgt ctgccggccc tgcaatggca ctggaacccc caagcccgag gaatcggcgt 2340
gacggtcgca aaccatccgg cccggtacaa atcggcgcgg cgctgggtga tgacctggtg 2400
gagaagttga aggccgcgca ggccgcccag cggcaacgca tcgaggcaga agcacgcccc 2460
ggtgaatcgt ggcaagcggc cgctgatcga atccgcaaag aatcccggca accgccggca 2520
gccggtgcgc cgtcgattag gaagccgccc aagggcgacg agcaaccaga tttttcgtt 2580
ccgatgctct atgacgtggg cacccgcgat agtcgcagca tcatggacgt ggccgttttc 2640
cgtctgtcga agcgtgaccg acgagctggc gaggtgatcc gctacgagct tccagacggg 2700
cacgtagagg tttccgcagg gccggccggc atggccagtg tgtgggatta cgacctggta 2760
ctgatggcgg tttcccatct aaccgaatcc atgaaccgat accgggaagg gaagggagac 2820
aagcccggcc gcgtgttccg tccacacgtt gcggacgtac tcaagttctg ccggcgagcc 2880
gatggcggaa agcagaaaga cgacctggta gaaacctgca ttcggttaaa caccacgcac 2940
gttgccatgc agcgtacgaa gaaggccaag aacggccgcc tggtgacggt atccgagggt 3000

gaagccttga ttagccgcta caagatcgta aagagcgaaa ccgggcggcc ggagtacatc 3060

gagatcgagc tagctgattg gatgtaccgc gagatcacag aaggcaagaa cccggacgtg 3120

ctgacggttc accccgatta ctttttgatc gatcccggca tcggccgttt tctctaccgc 3180

ctggcacgcc gcgccgcagg caaggcagaa gccagatggt tgttcaagac gatctacgaa 3240

cgcagtggca gcgccggaga gttcaagaag ttctgtttca ccgtgcgcaa gctgatcggg 3300

tcaaatgacc tgccggagta cgatttgaag gaggaggcgg ggcaggctgg cccgatccta 3360

gtcatgcgct accgcaacct gatcgagggc gaagcatccg ccggttccta atgtacggag 3420

cagatgctag ggcaaattgc cctagcaggg gaaaaaggtc gaaaaggtct ctttcctgtg 3480

gatagcacgt acattgggaa cccaaagccg tacattggga accggaaccc gtacattggg 3540

aacccaaagc cgtacattgg gaaccggtca cacatgtaag tgactgatat aaaagagaaa 3600

aaaggcgatt tttccgccta aaactcttta aaacttatta aaactcttaa aacccgcctg 3660

gcctgtgcat aactgtctgg ccagcgcaca gccgaagagc tgcaaaaagc gcctaccctt 3720

cggtcgctgc gctccctacg ccccgccgct tcgcgtcggc ctatcgcggc cgctggccgc 3780

tcaaaaatgg ctggcctacg gccaggcaat ctaccagggc gcggacaagc cgcgccgtcg 3840

ccactcgacc gccggcgccc acatcaaggc accctgcctc gcgcgtttcg gtgatgacgg 3900

tgaaaacctc tgacacatgc agctcccgga gacggtcaca gcttgtctgt aagcggatgc 3960

cgggagcaga caagcccgtc agggcgcgtc agcgggtgtt ggcgggtgtc ggggcgcagc 4020

catgacccag tcacgtagcg atagcggagt gtatactggc ttaactatgc ggcatcagag 4080

cagattgtac tgagagtgca ccatatgcgg tgtgaaatac cgcacagatg cgtaaggaga 4140

aaataccgca tcaggcgctc ttccgcttcc tcgctcactg actcgctgcg ctcggtcgtt 4200

cggctgcggc gagcggtatc agctcactca aaggcggtaa tacggttatc cacagaatca 4260

ggggataacg caggaaagaa catgtgagca aaaggccagc aaaaggccag gaaccgtaaa 4320

aaggccgcgt tgctggcgtt tttccatagg ctccgccccc ctgacgagca tcacaaaaat 4380

cgacgctcaa gtcagaggtg gcgaaacccg acaggactat aaagatacca ggcgtttccc 4440

cctggaagct ccctcgtgcg ctctcctgtt ccgaccctgc cgcttaccgg atacctgtcc 4500

gcctttctcc cttcgggaag cgtggcgctt tctcatagct cacgctgtag gtatctcagt 4560

tcggtgtagg tcgttcgctc caagctgggc tgtgtgcacg aaccccccgt tcagcccgac 4620

cgctgcgcct tatccggtaa ctatcgtctt gagtccaacc cggtaagaca cgacttatcg 4680

ccactggcag cagccactgg taacaggatt agcagagcga ggtatgtagg cggtgctaca 4740

gagttcttga agtggtggcc taactacggc tacactagaa ggacagtatt tggtatctgc 4800

gctctgctga agccagttac cttcggaaaa agagttggta gctcttgatc cggcaaacaa 4860

accaccgctg gtagcggtgg tttttttgtt tgcaagcagc agattacgcg cagaaaaaaa 4920

ggatctcaag aagatccttt gatcttttct acggggtctg acgctcagtg gaacgaaaac 4980
tcacgttaag ggattttggt catgcattct aggtattatt tgccaacgac cttcgtgatc 5040
tcgcccttga catagtggac aaattcttcg agctggtcgg cccgggacgc gagacggtct 5100
tcttcttggc ccagataggc ttggcgcgct tcgaggatca cgggctggta ttgcgccgga 5160
aggcgctcca tcgcccagtc ggcggcgaca tccttcggcg cgatcttgcc ggtaaccgcc 5220
gagtaccaaa tccggctcag cgtaaggacc acattgcgct catcgcccgc ccaatccggc 5280
ggggagttcc acagggtcag cgtctcgttc agtgcttcga acagatcctg ttccggcacc 5340
gggtcgaaaa gttcctcggc cgcggggccg acgagggcca cgctatgctc ccgggccttg 5400
gtgagcagga tcgccagatc aatgtcgatg gtggccggtt caaagatacc cgccagaata 5460
tcattacgct gccattcgcc gaactggagt tcgcgtttgg ccggatagcg ccaggggatg 5520
atgtcatcgt gcaccacaat cgtcacctca accgcgcgca ggatttcgct ctcgccgggg 5580
gaggcggacg tttccagaag gtcgttgata agcgcgcggc gcgtggtctc gtcgagacgg 5640
acggtaacgg tgacaagcag gtcgatgtcc gaatggggct taaggccgcc gtcaacggcg 5700
ctaccataca gatgcacggc gaggagggtc ggttcgaggt ggcgctcgat gacacccacg 5760
acttccgaca gctgggtgga cacctcggcg atgaccgctt cacccattta ttatttcctt 5820
cctcttttct acagtattta aagatacccc aagaagctaa ttataacaag acgaactcca 5880
attcactgtt ccttgcattc taaaacctta aataccagaa aacagctttt tcaaagttgt 5940
tttcaaagtt ggcgtataac atagtatcga cggagccgat tttgaaaccg cggtgatcac 6000
aggcagcaac gctctgtcat cgttacaatc aacatgctac cctccgcgag atcatccgtg 6060
tttcaaaccc ggcagcttag ttgccgttct tccgaatagc atcggtaaca tgagcaaagt 6120
ctgccgcctt acaacggctc tcccgctgac gccgtcccgg actgatgggc tgcctgtatc 6180
gagtggtgat tttgtgccga gctgccggtc ggggagctgt tggctggctg gtggcaggat 6240
atattgtggt gtaaacaaat tgacgcttag acaacttaat aacacattgc ggacgttttt 6300
aatgtactga attagtactg ataaatggcg cgccaagctt tggcaaacag ctattatggg 6360
tattatgggt ggtaccacgc gtcggatccg atctagtaac atagatgaca ccgcgcgcga 6420
taatttatcc tagtttgcgc gctatatttt gttttctatc gcgtattaaa tgtataattg 6480
cgggactcta atcataaaaa cccatctcat aaataacgtc atgcattaca tgttaattat 6540
tacatgctta acgtaattca acagaaatta tatgataatc atcgcaagac cggcaacagg 6600
attcaatctt aagaaacttt attgccaaat gtttgaacga tccctaggac gatctcactt 6660
gtagagctcg tccatgccgt agaggaacag gtgatggcgg ccctcggacc tctcgtactg 6720
ctcaacaata gtgtaatcct cgttatggct agtaatatcc agcttagtat ccacgtagta 6780

gtagccaggg agctgaaccg gcttcttggc catgtagata gtcttgaact ccaccaggta 6840
atggccacca tccttcagct tgagagcctg atgaatctcg cccttcagaa cgccatccct 6900
agggtagagc ctctcagtgg aggcctccca ccccatggtc ttcttctgca tgacagggcc 6960
gtctggaggg aagttggtgc cgcgcatctt caccttgtag atgagggtgc catcttggag 7020
ggagctatcc tgagtcacag taaccaggcc accatcttca aagttcatga cgcgttccca 7080
cttgaagcct tctgggaagg agagcttctt gtaatcagga atatcagcag ggtgcttcac 7140
gtaggctttg gagccgtaca tgaactgagg ggagaggata tcccaggcga aagggagagg 7200
gccgccctta gtcactttga gcttagcagt ctgggtgcct tcataagggc ggccctcgcc 7260
ctcaccttca atctcgaact catggccgtt catggagccc tccatcctga ccttgaagcg 7320
catgaactcc ttgatcacgg ccatgttgtt gtcctcgctg gaggcggtgc cgctggagcc 7380
gctgccagtg gagccagtgc cgtggccgag gaacaggtgg tgcctgccct cgctgcgctc 7440
gtactgctcc acgatggtgt agtcctcgtt gtgggaggtg atgtcgagct tggtgtcgac 7500
gtagtagtag cctggcagct gcacaggctt cttggccatg tagatggtct tgaactcgac 7560
caggtagtgg ccaccgtcct tgagcttcag ggcctggtgg atctcgccct tgagcacgcc 7620
gtccctgggg tacagcctct cagtgctagc ctcccagccc atggtcttct tctgcatgac 7680
cgggccatcg ggcgggaagt tagtgcccct catcttcacc ttgtagatga gggtgccatc 7740
ctggaggctg gagtcctgag tgacggtcac gaggccaccg tcctcgaagt tcatgacgcg 7800
ctcccacttg aagccctcgg ggaaggacag cttcttgtag tcggggatgt cggccgggtg 7860
cttcacgtag gccttgctgc cgtacatgaa ctgcggggag aggatgtccc aagcgaatgg 7920
cagcgggccg cccttagtga ccttgagctt ggcggtctgg gtgccctcgt aaggcctgcc 7980
ctcgccctcg ccctcgatct cgaactcgtg gccgttcatg ctgccctcca tcctcacctt 8040
gaagcgcatg aactccttga tcacttcctc gcccttggac accactacaa aaaagctccg 8100
cacgaggctg catttgtcac aaatcatgaa aagaaaaact accgatgaac aatgctgagg 8160
gattcaaatt ctacccacaa aaagaagaaa gaaagatcta gcacatctaa gcctgacgaa 8220
gcagcagaaa tatataaaaa tataaaccat agtgcccttt tcccctcttc ctgatcttgt 8280
ttagcacggc ggaaatttta aacccccat catctccccc aacaacggcg gatcgcagat 8340
ctacatccga gagccccatt ccccgcgaga tccgggccgg atccacgccg gcgagagccc 8400
cagccgcgag atcccgcccc tcccgcgcac cgatctgggc gcgcacgaag ccgcctctcg 8460
cccacccaaa ctaccaaggc caaagatcga gaccgagacg gaaaaaaaa cggagaaaga 8520
aagaggagag gggcggggtg gttaccggcg gcggcggagg cctcccttgg atcttatggt 8580
gtgttgtccc tgtgtgttct ccaatagtgt ggcttgagtg tgtggaagat ggttctagag 8640
gatctgctag agtcagcttg tcagcgtgtc ctctccaaat gaaatgaact tccttatata 8700

gaggaagggt cttgcgaagg atagtgggat tgtgcgtcat cccttacgtc agtggagata 8760
tcacatcaat ccacttgctt tgaagacgtg gttggaacgt cttctttttc cacgatgctc 8820
ctcgtgggtg ggggtccatc tttgggacca ctgtcggcag aggcatcttc aacgatggcc 8880
tttcctttat cgcaatgatg gcatttgtag gagccacctt ccttttccac tatcttcaca 8940
ataaagtgac agatagctgg gcaatggaat ccgaggaggt ttccggatat taccctttgt 9000
tgaaaagtct caatcggacc atcacatcaa tccacttgct ttgaagacgt ggttggaacg 9060
tcttcttttt ccacgatgct cctcgtgggt gggggtccat ctttgggacc actgtcggca 9120
gaggcatctt caacgatggc ctttccttta tcgcaatgat ggcatttgta ggagccacct 9180
tccttttcca ctatcttcac aataaagtga cagatagctg ggcaatggaa tccgaggagg 9240
tttccggata ttaccctttg ttgaaaagtc tcaatcggac cccctcagcc tgcacctcga 9300
gggcgctccg ggcggaagt cgtgggtaat ccttcaaagg attttaaaaa atactaaatc 9360
tgagctagga tctgcagatg aggaagaaag acgaagacga gatagagagc gggaaattga 9420
gatgtaaccc tagctgatgg aacggcaca ggcacaaata cgggcatggg ccgagatcga 9480
ggggcggcgc atcagcagca gtgcagtagg cgagcagggc cgccgcattg gtgggccgcc 9540
catgccggtg caccgccgcc gctctcgcgg gggccgccgt gccatggctc gtccgatgca 9600
ggatcaccgg cgacggagag gcgaagccct aggccggcgc tgtcgccacg ccaggatcgg 9660
tcgcccaggt tgacgtgact gggttgcctg cgctgaacct aataacacat tgctgggttg 9720
tggtgtaaac aaattgacgc ttagacaact taataacaca ttgcggacgt ttttaatgta 9780
ctgaattagt actgatatcg gtacccgggg cggccgcgag ctcgatccac tagtaacggc 9840
cgccagtgtg ctggaattcg cccttggcgc gccgatctag taacatagat gacaccgcgc 9900
gcgataattt atcctagttt gcgcgctata ttttgttttc tatcgcgtat taaatgtata 9960
attgcgggac tctaatcata aaaacccatc tcataaataa cgtcatgcat tacatgttaa 10020
ttattacatg cttaacgtaa ttcaacagaa attatatgat aatcatcgca agaccggcaa 10080
caggattcaa tcttaagaaa ctttattgcc aaatgtttga acgatctcag aagaactcgt 10140
caagaaggcg atagaaggcg atgcgctgcg aatcgggagc ggcgataccg taaagcacga 10200
ggaagcggtc agcccattcg ccgccaagct cttcagcaat atcacgggta gccaacgcta 10260
tgtcctgata gcggtccgcc acacccagcc ggccacagtc gatgaatcca gaaaagcggc 10320
cattttccac catgatattc ggcaagcagg catcgccatg ggtcacgacg agatcctcgc 10380
cgtcgggcat gcgcgccttg agcctggcga acagttcggc tggcgcgagc ccctgatgct 10440
cttcgtccag atcatcctga tcgacaagac cggcttccat ccgagtacgt gctcgctcga 10500
tgcgatgttt cgcttggtgg tcgaatgggc aggtagccgg atcaagcgta tgcagccgcc 10560

gcattgcatc agccatgatg gatactttct cggcaggagc aaggtgagat gacaggagat 10620
cctgccccgg cacttcgccc aatagcagcc agtcccttcc cgcttcagtg acaacgtcga 10680
gcacagctgc gcaaggaacg cccgtcgtgg ccagccacga tagccgcgct gcctcgtcct 10740
gcagttcatt cagggcaccg gacaggtcgg tcttgacaaa aagaaccggg cgcccctgcg 10800
ctgacagccg gaacacggcg gcatcagagc agccgattgt ctgttgtgcc cagtcatagc 10860
cgaatagcct ctccacccaa gcggccggag aacctgcgtg caatccatct tgttcaatcc 10920
acattctaga gtcgacctgc agaagtaaca ccaaacaaca gggtgagcat cgacaaaaga 10980
aacagtacca agcaaataaa tagcgtatga aggcagggct aaaaaaatcc acatatagct 11040
gctgcatatg ccatcatcca agtatatcaa gatcaaaata attataaaac atacttgttt 11100
attataatag ataggtactc aaggttagag catatgaata gatgctgcat atgccatcat 11160
gtatatgcat cagtaaaacc cacatcaaca tgtataccta tcctagatcg atatttccat 11220
ccatcttaaa ctcgtaacta tgaagatgta tgacacacac atacagttcc aaaattaata 11280
aatacaccag gtagtttgaa acagtattct actccgatct agaacgaatg aacgaccgcc 11340
caaccacacc acatcatcac aaccaagcga acaaaaagca tctctgtata tgcatcagta 11400
aaacccgcat caacatgtat acctatccta gatcgatatt tccatccatc atcttcaatt 11460
cgtaactatg aatatgtatg gcacacacat acagatccaa aattaataaa tccaccaggt 11520
agtttgaaac agaattctac tccgatctag aacgaccgcc caaccagacc acatcatcac 11580
aaccaagaca aaaaaaagca tgaaaagatg acccgacaaa caagtgcacg gcatatattg 11640
aaataaagga aaagggcaaa ccaaacccta tgcaacgaaa caaaaaaaat catgaaatcg 11700
atcccgtctg cggaacggct agagccatcc caggattccc caaagagaaa cactggcaag 11760
ttagcaatca gaacgtgtct gacgtacagg tcgcatccgt gtacgaacgc tagcagcacg 11820
gatctaacac aaacacggat ctaacacaaa catgaacaga agtagaacta ccgggcccta 11880
accatggacc ggaacgccga tctagagaag gtagagaggg gggggggggg aggacgagcg 11940
gcgtaccttg aagcggaggt gccgacgggt ggatttgggg gagatctggt tgtgtgtgtg 12000
tgcgctccga acaacacgag gttggggaaa gagggtgtgg agggggtgtc tatttattac 12060
ggcgggcgag gaagggaaag cgaaggagcg gtgggaaagg aatcccccgt agctgccgtg 12120
ccgtgagagg aggaggaggc cgcctgccgt gccggctcac gtctgccgct ccgccacgca 12180
atttctggat gccgacagcg gagcaagtcc aacggtggag cggaactctc gagaggggtc 12240
cagaggcagc gacagagatg ccgtgccgtc tgcttcgctt ggcccgacgc gacgctgctg 12300
gttcgctggt tggtgtccgt tagactcgtc gacggcgttt aacaggctgg cattatctac 12360
tcgaaacaag aaaaatgttt ccttagtttt tttaatttct taaagggtat ttgtttaatt 12420
tttagtcact ttattttatt ctattttata tctaaattat taaataaaaa aactaaaata 12480

gagttttagt tttcttaatt tagaggctaa aatagaataa aatagatgta ctaaaaaaat 12540

tagtctataa aaaccattaa ccctaaaccc taaatggatg tactaataaa atggatgaag 12600

tattatatag gtgaagctat ttgcaaaaaa aaaggagaac acatgcacac taaaaagata 12660

aaactgtaga gtcctgttgt caaaatactc aattgtcctt tagaccatgt ctaactgttc 12720

atttatatga ttctctaaaa cactgatatt attgtagtac tatagattat attattcgta 12780

gagtaaagtt taaatatatg tataaagata gataaactgc acttcaaaca agtgtgacaa 12840

aaaaaatatg tggtaatttt ttataactta gacatgcaat gctcattatc tctagagagg 12900

ggcacgaccg ggtcacgctg cactgcaggc atacgcgtga tccactagta acggccgcca 12960

gtgtgctgga attcgccctt ggcgcgccga tctagtaaca tagatgacac cgcgcgcgat 13020

aatttatcct agtttgcgcg ctatattttg ttttctatcg cgtattaaat gtataattgc 13080

gggactctaa tcataaaaac ccatctcata aataacgtca tgcattacat gttaattatt 13140

acatgcttaa cgtaattcaa cagaaattat atgataatca tcgcaagacc ggcaacagga 13200

ttcaatctta agaaacttta ttgccaaatg tttgaacgat ctcactcagc ctcgagggtg 13260

gcggtcactg ggatgaactc gaacctgtcg atgatcacgc cggcggtgcc gctgaagttc 13320

ctcacgccca cgatgttacc gagggaggag gtgaaagcgt tggcgctctc gaagtaaccg 13380

aagtcgctgg attggaggtt gtccagggag gtagcggtag ctggcacggt gttggagaag 13440

atggaggagt taccccagtt cacgttgagg tggatcgggg tcacggaagc gtacctcacg 13500

cgcaccctgt acctggtgga ggtggatggg aagtggattg gcacctcgat gtagcccctg 13560

ttctggatgt tgttgccgct gctgttgagc ctcacgaggt cgccaccggt gaagcctggg 13620

ccggaaatga cggaaccgtt gaagaggaag ttgcccttca cggccgggat ttgggtgatg 13680

ctgtcggagg cgatgatgtt gttgaactca gcgctgcggt ggatccagga gaacatcgga 13740

gccctgatga tgctcacgga gctgttgctg aagccggagc ggaacatgga cacgtggctc 13800

agcctgtggg agaagccttg cctgggtggc acgttgttgt tctgtggtgg gatctcgtcc 13860

agggagtcca cggtgccgct cttcctgtag acagcggatg gcaggttgga ggaggtgccg 13920

taggcgaact cggtgccgtc gagcacggac agttgctggt tgttgatacc gatgttgaag 13980

ggcctcctgt acagggtgga ggacagggtc ctgtagacac cctgacccag ttgagccacg 14040

atgcgttgct gtggagcggc gttgcccatg gtgccgtaga gcgggaaggt gaactcgggg 14100

ccgctgaagc ccactgggga ggccatgatc tggtggccgg accagtagta ctcgcccctg 14160

tgagcgtcgg tgtagatggt gatgctgttc aggatgtcca tcaggtgtgg gctcctgatg 14220

gagccctcga taccctgggc ggaaccgcgg aagctaccgt cgaagttctc cagcactggg 14280

ttggtgtaga tctccctggt gagttgggac acggtgcgga tcgggtaggt cctggagtcg 14340

tagttcggga agagggacac aatgtccagc acggtgaggg tcaactccct cctgaactgg 14400
ttgtacctga tccagtccct ggagtccgga ccccagacgc gctccaggcc ggtgttgtac 14460
cagcgcacag cgtggtcggt gtagttgcca atcagcctgg tcaggtcgtt gtagcggctg 14520
ttgatggtgg cagcatcgaa gccccacctt tggccgaaca cgctcacgtc gcgcagcacg 14580
ctgaggtgca ggttagcggc ttgcacgtac acggacagga gcggcacttg gtagttctgg 14640
acggcgaaca gtgggatagc ggtggtcagg gcgctgttca tgtcgttgaa ttgaatgcgc 14700
atttcctcgc ggagagctgg gttggtcggg tcggcctccc actccctgaa gctctcggcg 14760
tagatttggt agaggttgct caggccctcc agcctggaga tggcctggtt cctggcgaac 14820
tcttcgatcc tctggttgat cagctgctcg atttgcacca ggaaggcgtc ccattgggat 14880
ggaccgaaga taccccagat gatgtccacc aggccgagca cgaagccagc acctggcacg 14940
aactcgctga gcaggaactg ggtcaaggac agggagatgt cgatgggggt gtaaccggtc 15000
tcgatgcgct cgccacccag cacctccacc tctgggttgc tcaggcagtt gtatgggatg 15060
cactcgttga tgtttgggtt gttgtccatt ctagagtcga cctgcagaag taacaccaaa 15120
caacagggtg agcatcgaca aaagtgctgc atatgccatc atccaagtat atcaagatca 15180
aaataattat aaaacatact tgtttattat aatagatagg tactcaaggt tagagcatat 15240
gaatagatgc tgcatatgcc atcatgtata tgcatcagta aaacccacat caacatgtat 15300
acctatccta gatcgatatt tccatccatc ttaaactcgt aactatgaag atgtatgaca 15360
cacacataca gttccaaaat taataaatac accaggtagt ttgaaacagt attctactcc 15420
gatctagaac gaatgaacga ccgcccaacc acaccacatc atcacaacca agcgaacaaa 15480
aagcatctct gtatatgcat cagtaaaacc cgcatcaaca tgtataccta tcctagatcg 15540
atatttccat ccatcatctt caattcgtaa ctatgaatat gtatggcaca cacatacaga 15600
tccaaaatta ataaatccac caggtagttt gaaacagaat tctactccga tctagaacga 15660
ccgcccaacc agaccacatc atcacaacca agacaaaaaa aagcatgaaa agatgacccg 15720
acaaacaagt gcacggcata tattgaaata aaggaaaagg gcaaaccaaa ccctatgcaa 15780
cgaaacaaaa aaaatcatga aatcgatccc gtctgcggaa cggctagagc catcccagga 15840
ttccccaaag agaaacactg gcaagttagc aatcagaacg tgtctgacgt acaggtcgca 15900
tccgtgtacg aacgctagca gcacggatct aacacaaaca cggatctaac acaaacatga 15960
acagaagtag aactaccggg ccctaaccat ggaccggaac gccgatctag agaaggtaga 16020
gaggggggggg gggggaggac gagcggcgta ccttgaagcg gaggtgccga cgggtggatt 16080
tgggggagat ctggttgtgt gtgtgtgcgc tccgaacaac acgaggttgg ggaaagaggg 16140
tgtggagggg gtgtctattt attacggcgg gcgaggaagg gaaagcgaag gagcggtggg 16200
aaaggaatcc cccgtagctg ccgtgccgtg agaggaggag gaggccgcct gccgtgccgg 16260

ctcacgtctg ccgctccgcc acgcaatttc tggatgccga cagcggagca agtccaacgg 16320

tggagcggaa ctctcgagag gggtccagag gcagcgacag agatgccgtg ccgtctgctt 16380

cgcttggccc gacgcgacgc tgctggttcg ctggttggtg tccgttagac tcgtcgacgg 16440

cgtttaacag gctggcatta tctactcgaa acaagaaaaa tgtttcctta gtttttttaa 16500

tttcttaaag ggtatttgtt taatttttag tcactttatt ttattctatt ttatatctaa 16560

attattaaat aaaaaaacta aaatagagtt ttagttttct taatttagag gctaaaatag 16620

aataaaatag atgtactaaa aaaattagtc tataaaaacc attaacccta aaccctaaat 16680

ggatgtacta ataaaatgga tgaagtatta tataggtgaa gctatttgca aaaaaaaagg 16740

agaacacatg cacactaaaa agataaaact gtagagtcct gttgtcaaaa tactcaattg 16800

tcctttagac catgtctaac tgttcattta tatgattctc taaaacactg atattattgt 16860

agtactatag attatattat tcgtagagta aagtttaaat atatgtataa agatagataa 16920

actgcacttc aaacaagtgt gacaaaaaaa atatgtggta atttttttata acttagacat 16980

gcaatgctca ttatctctag agaggggcac gaccgggtca cgctgcactg caggcatcga 17040

tcgaagctta cgcgtccatg gctgcaggag ctcgtcgact ctagactcga gggcgagccc 17100

cgagcggggg cagctgaggc gccattggga ggaggaagag ggcagcgctc cggggcggaa 17160

gtcgtgcggc ggcacgaggg aagggcacac gacggacgga cggaggaaga agaagagcgc 17220

ggaggtagct ggccgagcgt gacctgcatg tcaagtcaat ggtcgcgggt gcgagtagaa 17280

aagagggagg acgaccggac acaccgccgc gtcaggtcac cgatgaccta acgtgtctgg 17340

tcactattca cgcgctaaaa acacttacta gactcgatct aacgcgggca gccctctgag 17400

tccgatcatt tattctgtgc gtccggtcat cgacgtgtgc gggaaagcgt tggctgttaa 17460

cggctagttt tgaatcgtgt gacgtggcac accacgactt ccgccccgga gcgcggtacc 17520

actagtatta attaagttta aacggcgcgc caagggcgaa ttccagcaca ctggcggccg 17580

ttactagtgg atcgagctcg tcgactctag actcgagggc gcgcctgaca ggatatattg 17640

gcgggtaaac 17650

<210> 27
<211> 18754
<212> DNA
<213> Artificial sequence

<220>
<223> Cas9/crRNA template construction

<400> 27
cttagaataa cggatattta aaagggcgtg aaaaggttta tccgttcgtc catttgtatg 60

tgcatgccaa ccacagggtt cccctcggga tcaaagtact ttgatccaac cctccgctg 120

ctatagtgca gtcggcttct gacgttcagt gcagccgtct tctgaaaacg acatgtcgca 180
caagtcctaa gttacgcgac aggctgccgc cctgcccttt tcctggcgtt ttcttgtcgc 240
gtgttttagt cgcataaagt agaatacttg cgactagaac cggagacatt acgccatgaa 300
caagagcgcc gccgctggcc tgctgggcta tgcccgcgtc agcaccgacg accaggactt 360
gaccaaccaa cgggccgaac tgcacgcggc cggctgcacc aagctgtttt ccgagaagat 420
caccggcacc aggcgcgacc gcccggagct ggccaggatg cttgaccacc tacgccctgg 480
cgacgttgtg acagtgacca ggctagaccg cctggcccgc agcacccgcg acctactgga 540
cattgccgag cgcatccagg aggccggcgc gggcctgcgt agcctggcag agccgtgggc 600
cgacaccacc acgccggccg gccgcatggt gttgaccgtg ttcgccggca ttgccgagtt 660
cgagcgttcc ctaatcatcg accgcacccg gagcgggcgc gaggccgcca aggcccgagg 720
cgtgaagttt ggcccccgcc ctaccctcac cccggcacag atcgcgcacg cccgcgagct 780
gatcgaccag gaaggccgca ccgtgaaaga ggcggctgca ctgcttggcg tgcatcgctc 840
gaccctgtac cgcgcacttg agcgcagcga ggaagtgacg cccaccgagg ccaggcggcg 900
cggtgccttc cgtgaggacg cattgaccga ggccgacgcc ctggcggccg ccgagaatga 960
acgccaagag gaacaagcat gaaaccgcac caggacggcc aggacgaacc gtttttcatt 1020
accgaagaga tcgaggcgga gatgatcgcg gccgggtacg tgttcgagcc gcccgcgcac 1080
gtctcaaccg tgcggctgca tgaaatcctg gccggtttgt ctgatgccaa gctggcggcc 1140
tggccggcca gcttggccgc tgaagaaacc gagcgccgcc gtctaaaaag gtgatgtgta 1200
tttgagtaaa acagcttgcg tcatgcggtc gctgcgtata tgatgcgatg agtaaataaa 1260
caaatacgca aggggaacgc atgaaggtta tcgctgtact taaccagaaa ggcgggtcag 1320
gcaagacgac catcgcaacc catctagccc gcgccctgca actcgccggg gccgatgttc 1380
tgttagtcga ttccgatccc cagggcagtg cccgcgattg ggcggccgtg cgggaagatc 1440
aaccgctaac cgttgtcggc atcgaccgcc cgacgattga ccgcgacgtg aaggccatcg 1500
gccggcgcga cttcgtagtg atcgacggag cgccccaggc ggcggacttg gctgtgtccg 1560
cgatcaaggc agccgacttc gtgctgattc cggtgcagcc aagcccttac gacatatggg 1620
ccaccgccga cctggtggag ctggttaagc agcgcattga ggtcacggat ggaaggctac 1680
aagcggcctt tgtcgtgtcg cgggcgatca aaggcacgcg catcggcggt gaggttgccg 1740
aggcgctggc cgggtacgag ctgcccattc ttgagtcccg tatcacgcag cgcgtgagct 1800
acccaggcac tgccgccgcc ggcacaaccg ttcttgaatc agaacccgag ggcgacgctg 1860
cccgcgaggt ccaggcgctg gccgctgaaa ttaaatcaaa actcatttga gttaatgagg 1920
taaagagaaa atgagcaaaa gcacaaacac gctaagtgcc ggccgtccga gcgcacgcag 1980
cagcaaggct gcaacgttgg ccagcctggc agacacgcca gccatgaagc gggtcaactt 2040

tcagttgccg gcggaggatc acaccaagct gaagatgtac gcggtacgcc aaggcaagac 2100
cattaccgag ctgctatctg aatacatcgc gcagctacca gagtaaatga gcaaatgaat 2160
aaatgagtag atgaatttta gcggctaaag gaggcggcat ggaaaatcaa gaacaaccag 2220
gcaccgacgc cgtggaatgc cccatgtgtg gaggaacggg cggttggcca ggcgtaagcg 2280
gctgggttgt ctgccggccc tgcaatggca ctggaacccc caagcccgag gaatcggcgt 2340
gacggtcgca aaccatccgg cccggtacaa atcggcgcgg cgctgggtga tgacctggtg 2400
gagaagttga aggccgcgca ggccgcccag cggcaacgca tcgaggcaga agcacgcccc 2460
ggtgaatcgt ggcaagcggc cgctgatcga atccgcaaag aatcccggca accgccggca 2520
gccggtgcgc cgtcgattag gaagccgccc aagggcgacg agcaaccaga tttttcgtt 2580
ccgatgctct atgacgtggg cacccgcgat agtcgcagca tcatggacgt ggccgttttc 2640
cgtctgtcga agcgtgaccg acgagctggc gaggtgatcc gctacgagct tccagacggg 2700
cacgtagagg tttccgcagg gccggccggc atggccagtg tgtgggatta cgacctggta 2760
ctgatggcgg tttcccatct aaccgaatcc atgaaccgat accgggaagg gaagggagac 2820
aagcccggcc gcgtgttccg tccacacgtt gcggacgtac tcaagttctg ccggcgagcc 2880
gatggcggaa agcagaaaga cgacctggta gaaacctgca ttcggttaaa caccacgcac 2940
gttgccatgc agcgtacgaa gaaggccaag aacggccgcc tggtgacggt atccgagggt 3000
gaagccttga ttagccgcta caagatcgta aagagcgaaa ccgggcggcc ggagtacatc 3060
gagatcgagc tagctgattg gatgtaccgc gagatcacag aaggcaagaa cccggacgtg 3120
ctgacggttc accccgatta ctttttgatc gatcccggca tcggccgttt tctctaccgc 3180
ctggcacgcc gcgccgcagg caaggcagaa gccagatggt tgttcaagac gatctacgaa 3240
cgcagtggca gcgccggaga gttcaagaag ttctgtttca ccgtgcgcaa gctgatcggg 3300
tcaaatgacc tgccggagta cgatttgaag gaggaggcgg ggcaggctgg cccgatccta 3360
gtcatgcgct accgcaacct gatcgagggc gaagcatccg ccggttccta atgtacggag 3420
cagatgctag ggcaaattgc cctagcaggg gaaaaaggtc gaaaaggtct ctttcctgtg 3480
gatagcacgt acattgggaa cccaaagccg tacattggga accggaaccc gtacattggg 3540
aacccaaagc cgtacattgg gaaccggtca cacatgtaag tgactgatat aaaagagaaa 3600
aaaggcgatt tttccgccta aaactcttta aaacttatta aaactcttaa aacccgcctg 3660
gcctgtgcat aactgtctgg ccagcgcaca gccgaagagc tgcaaaaagc gcctaccctt 3720
cggtcgctgc gctccctacg ccccgccgct tcgcgtcggc ctatcgcggc cgctggccgc 3780
tcaaaaatgg ctggcctacg gccaggcaat ctaccagggc gcggacaagc cgcgccgtcg 3840
ccactcgacc gccggcgccc acatcaaggc accctgcctc gcgcgtttcg gtgatgacgg 3900

tgaaaacctc tgacacatgc agctcccgga gacggtcaca gcttgtctgt aagcggatgc 3960
cgggagcaga caagcccgtc agggcgcgtc agcgggtgtt ggcgggtgtc ggggcgcagc 4020
catgacccag tcacgtagcg atagcggagt gtatactggc ttaactatgc ggcatcagag 4080
cagattgtac tgagagtgca ccatatgcgg tgtgaaatac cgcacagatg cgtaaggaga 4140
aaataccgca tcaggcgctc ttccgcttcc tcgctcactg actcgctgcg ctcggtcgtt 4200
cggctgcggc gagcggtatc agctcactca aaggcggtaa tacggttatc cacagaatca 4260
ggggataacg caggaaagaa catgtgagca aaaggccagc aaaaggccag gaaccgtaaa 4320
aaggccgcgt tgctggcgtt tttccatagg ctccgccccc ctgacgagca tcacaaaaat 4380
cgacgctcaa gtcagaggtg gcgaaacccg acaggactat aaagatacca ggcgtttccc 4440
cctggaagct ccctcgtgcg ctctcctgtt ccgaccctgc cgcttaccgg atacctgtcc 4500
gcctttctcc cttcgggaag cgtggcgctt tctcatagct cacgctgtag gtatctcagt 4560
tcggtgtagg tcgttcgctc caagctgggc tgtgtgcacg aaccccccgt tcagcccgac 4620
cgctgcgcct tatccggtaa ctatcgtctt gagtccaacc cggtaagaca cgacttatcg 4680
ccactggcag cagccactgg taacaggatt agcagagcga ggtatgtagg cggtgctaca 4740
gagttcttga agtggtggcc taactacggc tacactagaa ggacagtatt tggtatctgc 4800
gctctgctga agccagttac cttcggaaaa agagttggta gctcttgatc cggcaaacaa 4860
accaccgctg gtagcggtgg tttttttgtt tgcaagcagc agattacgcg cagaaaaaaa 4920
ggatctcaag aagatccttt gatcttttct acggggtctg acgctcagtg gaacgaaaac 4980
tcacgttaag ggattttggt catgcattct aggtattatt tgccaacgac cttcgtgatc 5040
tcgcccttga catagtggac aaattcttcg agctggtcgg cccgggacgc gagacggtct 5100
tcttcttggc ccagataggc ttggcgcgct tcgaggatca cgggctggta ttgcgccgga 5160
aggcgctcca tcgcccagtc ggcggcgaca tccttcggcg cgatcttgcc ggtaaccgcc 5220
gagtaccaaa tccggctcag cgtaaggacc acattgcgct catcgcccgc ccaatccggc 5280
ggggagttcc acagggtcag cgtctcgttc agtgcttcga acagatcctg ttccggcacc 5340
gggtcgaaaa gttcctcggc cgcggggccg acgagggcca cgctatgctc ccgggccttg 5400
gtgagcagga tcgccagatc aatgtcgatg gtggccggtt caaagatacc cgccagaata 5460
tcattacgct gccattcgcc gaactggagt tcgcgtttgg ccggatagcg ccaggggatg 5520
atgtcatcgt gcaccacaat cgtcacctca accgcgcgca ggatttcgct ctcgccgggg 5580
gaggcggacg tttccagaag gtcgttgata agcgcgcggc gcgtggtctc gtcgagacgg 5640
acggtaacgg tgacaagcag gtcgatgtcc gaatggggct taaggccgcc gtcaacggcg 5700
ctaccataca gatgcacggc gaggagggtc ggttcgaggt ggcgctcgat gacacccacg 5760
acttccgaca gctgggtgga cacctcggcg atgaccgctt cacccattta ttatttcctt 5820

cctcttttct acagtattta aagatacccc aagaagctaa ttataacaag acgaactcca 5880

attcactgtt ccttgcattc taaaacctta aataccagaa aacagctttt tcaaagttgt 5940

tttcaaagtt ggcgtataac atagtatcga cggagccgat tttgaaaccg cggtgatcac 6000

aggcagcaac gctctgtcat cgttacaatc aacatgctac cctccgcgag atcatccgtg 6060

tttcaaaccc ggcagcttag ttgccgttct tccgaatagc atcggtaaca tgagcaaagt 6120

ctgccgcctt acaacggctc tcccgctgac gccgtcccgg actgatgggc tgcctgtatc 6180

gagtggtgat tttgtgccga gctgccggtc ggggagctgt tggctggctg gtggcaggat 6240

atattgtggt gtaaacaaat tgacgcttag acaacttaat aacacattgc ggacgttttt 6300

aatgtactga attagtactg ataaatggcg cgccaagctt tggcaaacag ctattatggg 6360

tattatgggt ggtaccacgc gtcgatccac tagtaacggc cgccagtgtg ctggaattcg 6420

cccttggcgc gccgatctag taacatagat gacaccgcgc gcgataattt atcctagttt 6480

gcgcgctata ttttgttttc tatcgcgtat taaatgtata attgcgggac tctaatcata 6540

aaaacccatc tcataaataa cgtcatgcat tacatgttaa ttattacatg cttaacgtaa 6600

ttcaacagaa attatatgat aatcatcgca agaccggcaa caggattcaa tcttaagaaa 6660

ctttattgcc aaatgtttga acgatctcag aagaactcgt caagaaggcg atagaaggcg 6720

atgcgctgcg aatcgggagc ggcgataccg taaagcacga ggaagcggtc agcccattcg 6780

ccgccaagct cttcagcaat atcacgggta gccaacgcta tgtcctgata gcggtccgcc 6840

acacccagcc ggccacagtc gatgaatcca gaaaagcggc cattttccac catgatattc 6900

ggcaagcagg catcgccatg ggtcacgacg agatcctcgc cgtcgggcat gcgcgccttg 6960

agcctggcga acagttcggc tggcgcgagc ccctgatgct cttcgtccag atcatcctga 7020

tcgacaagac cggcttccat ccgagtacgt gctcgctcga tgcgatgttt cgcttggtgg 7080

tcgaatgggc aggtagccgg atcaagcgta tgcagccgcc gcattgcatc agccatgatg 7140

gatactttct cggcaggagc aaggtgagat gacaggagat cctgccccgg cacttcgccc 7200

aatagcagcc agtcccttcc cgcttcagtg acaacgtcga gcacagctgc gcaaggaacg 7260

cccgtcgtgg ccagccacga tagccgcgct gcctcgtcct gcagttcatt cagggcaccg 7320

gacaggtcgg tcttgacaaa aagaaccggg cgcccctgcg ctgacagccg gaacacggcg 7380

gcatcagagc agccgattgt ctgttgtgcc cagtcatagc cgaatagcct ctccacccaa 7440

gcggccggag aacctgcgtg caatccatct tgttcaatcc acatggtggt gtgacctgca 7500

gaagtaacac caaacaacag ggtgagcatc gacaaaagaa acagtaccaa gcaaataaat 7560

agcgtatgaa ggcagggcta aaaaaatcca catatagctg ctgcatatgc catcatccaa 7620

gtatatcaag atcaaaataa ttataaaaca tacttgttta ttataataga taggtactca 7680

aggttagagc atatgaatag atgctgcata tgccatcatg tatatgcatc agtaaaaccc 7740
acatcaacat gtatacctat cctagatcga tatttccatc catcttaaac tcgtaactat 7800
gaagatgtat gacacacaca tacagttcca aaattaataa atacaccagg tagtttgaaa 7860
cagtattcta ctccgatcta gaacgaatga acgaccgccc aaccacacca catcatcaca 7920
accaagcgaa caaaaagcat ctctgtatat gcatcagtaa aacccgcatc aacatgtata 7980
cctatcctag atcgatattt ccatccatca tcttcaattc gtaactatga atatgtatgg 8040
cacacacata cagatccaaa attaataaat ccaccaggta gtttgaaaca gaattctact 8100
ccgatctaga acgaccgccc aaccagacca catcatcaca accaagacaa aaaaaagcat 8160
gaaaagatga cccgacaaac aagtgcacgg catatattga aataaaggaa aagggcaaac 8220
caaaccctat gcaacgaaac aaaaaaaatc atgaaatcga tcccgtctgc ggaacggcta 8280
gagccatccc aggattcccc aaagagaaac actggcaagt tagcaatcag aacgtgtctg 8340
acgtacaggt cgcatccgtg tacgaacgct agcagcacgg atctaacaca aacacggatc 8400
taacacaaac atgaacagaa gtagaactac cgggccctaa ccatggaccg gaacgccgat 8460
ctagagaagg tagagagggg gggggggga ggacgagcgg cgtaccttga agcggaggtg 8520
ccgacgggtg gatttggggg agatctggtt gtgtgtgtgt gcgctccgaa caacacgagg 8580
ttggggaaag agggtgtgga gggggtgtct atttattacg gcgggcgagg aagggaaagc 8640
gaaggagcgg tgggaaagga atcccccgta gctgccgtgc cgtgagagga ggaggaggcc 8700
gcctgccgtg ccggctcacg tctgccgctc cgccacgcaa tttctggatg ccgacagcgg 8760
agcaagtcca acggtggagc ggaactctcg agaggggtcc agaggcagcg acagagatgc 8820
cgtgccgtct gcttcgcttg gcccgacgcg acgctgctgg ttcgctggtt ggtgtccgtt 8880
agactcgtcg acggcgttta acaggctggc attatctact cgaaacaaga aaaatgtttc 8940
cttagttttt ttaatttctt aaagggtatt tgtttaattt ttagtcactt tattttattc 9000
tattttatat ctaaattatt aaataaaaaa actaaaatag agttttagtt ttcttaattt 9060
agaggctaaa atagaataaa atagatgtac taaaaaaatt agtctataaa aaccattaac 9120
cctaaaccct aaatggatgt actaataaaa tggatgaagt attatatagg tgaagctatt 9180
tgcaaaaaaa aaggagaaca catgcacact aaaaagataa aactgtagag tcctgttgtc 9240
aaaatactca attgtccttt agaccatgtc taactgttca tttatatgat tctctaaaac 9300
actgatatta ttgtagtact atagattata ttattcgtag agtaaagttt aaatatatgt 9360
ataaagatag ataaactgca cttcaaacaa gtgtgacaaa aaaaatatgt ggtaattttt 9420
tataacttag acatgcaatg ctcattatct ctagagaggg gcacgaccgg gtcacgctgc 9480
actgcaggga tccgatctag taacatagat gacaccgcgc gcgataattt atcctagttt 9540
gcgcgctata ttttgttttc tatcgcgtat taaatgtata attgcgggac tctaatcata 9600

aaaacccatc tcataaataa cgtcatgcat tacatgttaa ttattacatg cttaacgtaa 9660
ttcaacagaa attatatgat aatcatcgca agaccggcaa caggattcaa tcttaagaaa 9720
ctttattgcc aaatgtttga acgatcccta ggacgatctc acttgtacag ctcgtccatg 9780
ccgtgggtga tgccagctgc ggtgacgaac tccagcagga ccatgtggtc gcgcttctcg 9840
ttggggtcct tgctcagagc ggactgggtg ctcaggtagt ggttgtcggg cagcagcacg 9900
ggaccgtcgc cgatgggcgt gttctgctgg tagtggtcgg cgagctggac gctgccgtcc 9960
tcgatgttgt ggcggatctt gaagttgacc ttgatgccgt tcttctgctt gtcagccatg 10020
atgtagacgt tgtggctgtt gtagttgtac tccagcttgt gccccaggat gttgccgtcc 10080
tccttgaagt cgatgccctt cagctcgatg cggttcacca gggtgtcgcc ctcgaacttc 10140
acctcggctc gggtcttgta gttgccgtcg tccttgaaga agatggtgcg ctcctggacg 10200
tagccttcgg gcatggcgga cttgaagaag tcgtgctgct tcatgtggtc ggggtagcgg 10260
ctgaagcact gcacgccgta ggtgaaggtg gtcacgaggg tgggccaggg cacgggcagc 10320
ttgccggtgg tgcagatgaa cttcagggtc agcttgccgt aggtggcgtc gccctcgccc 10380
tcgccgctga cgctgaactt gtggccgttc acgtcgccgt ccagctcgac caggatgggc 10440
accaccccag tgaacagctc ctcgcccttg ctcactacaa aaaagctccg cacgaggctg 10500
catttgtcac aaatcatgaa aagaaaaact accgatgaac aatgctgagg gattcaaatt 10560
ctacccacaa aaagaagaaa gaaagatcta gcacatctaa gcctgacgaa gcagcagaaa 10620
tatataaaaa tataaaccat agtgcccttt tcccctcttc ctgatcttgt ttagcacggc 10680
ggaaatttta aacccccccat catctccccc aacaacggcg gatcgcagat ctacatccga 10740
gagccccatt ccccgcgaga tccgggccgg atccacgccg gcgagagccc cagccgcgag 10800
atcccgcccc tcccgcgcac cgatctgggc gcgcacgaag ccgcctctcg cccacccaaa 10860
ctaccaaggc caaagatcga gaccgagacg gaaaaaaaaa cggagaaaga aagaggagag 10920
gggcggggtg gttaccggcg gcggcggagg cctcccttgg atcttatggt gtgttgtccc 10980
tgtgtgttct ccaatagtgt ggcttgagtg tgtggaagat ggttctagag gatctgctag 11040
agtcagcttg tcagcgtgtc ctctccaaat gaaatgaact tccttatata gaggaagggt 11100
cttgcgaagg atagtgggat tgtgcgtcat cccttacgtc agtggagata tcacatcaat 11160
ccacttgctt tgaagacgtg gttggaacgt cttctttttc cacgatgctc ctcgtgggtg 11220
ggggtccatc tttgggacca ctgtcggcag aggcatcttc aacgatggcc tttcctttat 11280
cgcaatgatg gcatttgtag gagccacctt ccttttccac tatcttcaca ataaagtgac 11340
agatagctgg gcaatggaat ccgaggaggt ttccggatat taccctttgt tgaaaagtct 11400
caatcggacc atcacatcaa tccacttgct ttgaagacgt ggttggaacg tcttcttttt 11460

ccacgatgct cctcgtgggt gggggtccat ctttgggacc actgtcggca gaggcatctt 11520
caacgatggc ctttccttta tcgcaatgat ggcatttgta ggagccacct tccttttcca 11580
ctatcttcac aataaagtga cagatagctg ggcaatggaa tccgaggagg tttccggata 11640
ttaccctttg ttgaaaagtc tcaatcggac cccctcagcc tgcagtgcag cgtgacccgg 11700
tcgtgcccct ctctagagat aatgagcatt gcatgtctaa gttataaaaa attaccacat 11760
atttttttg tcacacttgt ttgaagtgca gtttatctat ctttatacat atatttaaac 11820
tttactctac gaataatata atctatagta ctacaataat atcagtgttt tagagaatca 11880
tataaatgaa cagttagaca tggtctaaag gacaattgag tattttgaca acaggactct 11940
acagttttat ctttttagtg tgcatgtgtt ctcctttttt tttgcaaata gcttcaccta 12000
tataatactt catccatttt attagtacat ccatttaggg tttagggtta atggttttta 12060
tagactaatt tttttagtac atctatttta ttctatttta gcctctaaat taagaaaact 12120
aaaactctat tttagttttt ttatttaata atttagatat aaaatagaat aaaataaagt 12180
gactaaaaat taaacaaata ccctttaaga aattaaaaaa actaaggaaa catttttctt 12240
gtttcgagta gataatgcca gcctgttaaa cgccgtcgac gagtctaacg gacaccaacc 12300
agcgaaccag cagcgtcgcg tcgggccaag cgaagcagac ggcacggcat ctctgtcgct 12360
gcctctggac ccctctcgag agttccgctc caccgttgga cttgctccgc tgtcggcatc 12420
cagaaattgc gtggcggagc ggcagacgtg agccggcacg gcaggcggcc tcctcctcct 12480
ctcacggcac ggcagctacg ggggattcct ttcccaccgc tccttcgctt tcccttcctc 12540
gcccgccgta ataaatagac acccccctcca caccctcttt ccccaacctc gtgttgttcg 12600
gagcgcacac acacacaacc agatctcccc caaatccacc cgtcggcacc tccgcttcaa 12660
ggtacgccgc tcgtcctccc cccccccccc tctctacctt ctctagatcg gcgttccggt 12720
ccatggttag ggcccggtag ttctacttct gttcatgttt gtgttagatc cgtgtttgtg 12780
ttagatccgt gctgctagcg ttcgtacacg gatgcgacct gtacgtcaga cacgttctga 12840
ttgctaactt gccagtgttt ctctttgggg aatcctggga tggctctagc cgttccgcag 12900
acgggatcga tttcatgatt ttttttgttt cgttgcatag ggtttggttt gccctttcc 12960
tttatttcaa tatatgccgt gcacttgttt gtcgggtcat cttttcatgc tttttttgt 13020
cttggttgtg atgatgtggt ctggttgggc ggtcgttcta gatcggagta gaattctgtt 13080
tcaaactacc tggtggattt attaattttg gatctgtatg tgtgtgccat acatattcat 13140
agttacgaat tgaagatgat ggatggaaat atcgatctag gataggtata catgttgatg 13200
cgggttttac tgatgcatat acagagatgc tttttgttcg cttggttgtg atgatgtggt 13260
gtggttgggc ggtcgttcat tcgttctaga tcggagtaga atactgtttc aaactacctg 13320
gtgtatttat taattttgga actgtatgtg tgtgtcatac atcttcatag ttacgagttt 13380

aagatggatg gaaatatcga tctaggatag gtatacatgt tgatgtgggt tttactgatg 13440
catatacatg atggcatatg cagcatctat tcatatgctc taaccttgag tacctatcta 13500
ttataataaa caagtatgtt ttataattat tttgatcttg atatacttgg atgatggcat 13560
atgcagcagc tatatgtgga tttttttagc cctgccttca tacgctattt atttgcttgg 13620
tactgtttct tttgtcgatg ctcaccctgt tgtttggtgt tacttctgca ggcgatcgcc 13680
acaccaccat gccgaagaag aagcgcaagg tcatggacaa gaagtactcc atcggcctgg 13740
acatcggcac caacagcgtg ggctgggccg tcatcaccga cgagtacaag gtgccctcca 13800
agaagttcaa ggtcctcggc aacaccgaca ggcacagcat caagaagaac ctgatcggcg 13860
ccctgctgtt cgactccggc gagactgcgg aggctaccag gctgaagcgc actgctcgca 13920
ggcgctacac caggcgcaag aaccgcatct gctacctcca ggagattttc tccaacgaga 13980
tggccaaggt ggacgactcc ttcttccacc gcctggagga gagcttcctg gtcgaggaag 14040
acaagaagca cgagcgccac cctatcttcg gcaacatcgt ggacgaggtc gcctaccacg 14100
agaagtaccc aaccatctac cacctccgca agaagctggt ggactccacc gacaaggccg 14160
acctgaggct catctacctg gccctcgccc acatgatcaa gttccgcggc cacttcctca 14220
tcgagggcga cctgaacccg gacaacagcg acgtggacaa gctcttcatc cagctggtcc 14280
agacctacaa ccagctgttc gaggagaacc ccatcaacgc ctccggcgtg gacgctaagg 14340
ctatcctcag cgctaggctg tccaagagca ggcgcctgga gaacctcatc gcccagctcc 14400
cgggcgagaa gaagaacggc ctcttcggca acctgatcgc tctgtccctc ggcctgaccc 14460
ccaacttcaa gagcaacttc gacctggccg aggacgccaa gctccagctg tccaaggaca 14520
cctacgacga cgacctcgac aacctgctcg cccagatcgg cgaccagtac gccgacctct 14580
tcctggccgc caagaacctc tccgacgcca tcctgctcag cgacatcctg agggtgaaca 14640
ccgagatcac caaggccccg ctgtccgcca gcatgatcaa gcgctacgac gagcaccacc 14700
aggacctcac tctcctgaag gccctcgtcc gccagcagct gcccgagaag tacaaggaga 14760
ttttcttcga ccagagcaag aacggctacg cgggctacat cgatggcggc gcctcccagg 14820
aagagttcta caagttcatc aagcctatcc tggagaagat ggacggcacc gaggagctgc 14880
tcgtgaagct gaaccgcgag gacctgctcc gcaagcagag gaccttcgac aacggcagca 14940
tccctcacca gatccacctg ggcgagctgc acgctatcct ccgccgccag gaagacttct 15000
acccattcct gaaggacaac cgcgagaaga tcgagaagat cctcaccttc cgcatcccgt 15060
actacgtggg ccccctggcc cgcggcaact ccaggttcgc ctggatgacc aggaagagcg 15120
aggagaccat caccccgtgg aacttcgagg aagtggtgga caagggcgcc tccgctcaga 15180
gcttcatcga gcgcatgacc aacttcgaca agaacctccc taacgagaag gtgctgccaa 15240

agcactccct gctctacgag tacttcaccg tctacaacga gctgaccaag gtgaagtatg 15300

tgaccgaggg catgaggaag cccgccttcc tcagcggcga gcagaagaag gccatcgtgg 15360

acctgctctt caagaccaac cgcaaggtga ccgtcaagca gctgaaggaa gactacttca 15420

agaagatcga gtgcttcgac tccgtggaga tcagcggcgt ggaggaccgc ttcaacgcct 15480

ccctcggcac ctaccacgac ctgctcaaga tcatcaagga caaggacttc ctcgacaacg 15540

aggagaacga ggacatcctg gaggacatcg tgctcaccct gaccctcttc gaggaccgcg 15600

agatgatcga ggagaggctc aagacctacg cccacctgtt cgacgacaag gtcatgaagc 15660

agctgaagag gcgcaggtac actggctggg gccgcctcag caggaagctg atcaacggca 15720

tcagggacaa gcagtccggc aagaccatcc tggacttcct caagagcgac ggcttcgcca 15780

accgcaactt catgcagctc atccacgacg actccctgac cttcaaggaa gacatccaga 15840

aggctcaggt gtccggccag ggcgacagcc tccacgagca catcgctaac ctggcgggca 15900

gccctgccat caagaagggc atcctccaga ccgtgaaggt ggtggacgag ctggtgaagg 15960

tcatgggccg ccacaagcca gagaacatcg tcatcgagat ggccagggag aaccagacca 16020

cccagaaggg tcagaagaac tcccgcgaga ggatgaagag gatcgaggaa ggcatcaagg 16080

agctgggcag ccagatcctg aaggagcacc cggtggagaa cacccagctc cagaacgaga 16140

agctgtacct ctactacctg cagaacggcc gcgacatgta tgtggaccag gagctggaca 16200

tcaacaggct gtccgactac gacgtggacc acatcgtccc tcagtccttc ctcaaggacg 16260

acagcatcga caacaaggtg ctgacccgca gcgacaagaa caggggcaag tccgacaacg 16320

tcccaagcga ggaagtggtc aagaagatga agaactactg gcgccagctg ctcaacgcca 16380

agctcatcac ccagcgcaag ttcgacaacc tgactaaggc ggagaggggc ggcctgtccg 16440

agctggacaa ggctggcttc atcaagcgcc agctcgtgga gaccaggcag atcaccaagc 16500

acgtcgccca gatcctggac agcaggatga acaccaagta cgacgagaac gacaagctca 16560

tccgcgaggt gaaggtcatc accctcaagt ccaagctggt gagcgacttc cgcaaggact 16620

tccagttcta caaggtcagg gagatcaaca actaccacca cgcccacgat gcttacctca 16680

acgcggtggt gggcaccgcc ctcatcaaga agtaccctaa gctggagagc gagttcgtgt 16740

acggcgacta caaggtgtac gacgtccgca agatgatcgc caagtccgag caggagatcg 16800

gcaaggccac cgccaagtac ttcttctaca gcaacatcat gaacttcttc aagaccgaga 16860

tcaccctcgc caacggcgag atccgcaaga ggccactgat cgagaccaac ggcgagactg 16920

gcgagatcgt gtgggacaag ggcagggact tcgccaccgt gaggaaggtc ctgtccatgc 16980

ctcaggtgaa catcgtcaag aagaccgagg tccagaccgg cggcttctcc aaggagagca 17040

tcctcccaaa gcgcaacagc gacaagctga tcgccaggaa gaaggactgg gacccgaaga 17100

agtacggtgg cttcgactcc cctactgtgg cttacagcgt cctggtggtc gccaaggtgg 17160

agaagggcaa gtccaagaag ctgaagagcg tcaaggagct gctcggcatc accatcatgg 17220

agaggtccag cttcgagaag aacccgatcg acttcctgga ggccaagggc tacaaggaag 17280

tgaagaagga cctgatcatc aagctgccca agtacagcct gttcgagctg gagaacggcc 17340

gcaagaggat gctcgcctcc gctggcgagc tgcagaaggg caacgagctg gccctcccgt 17400

ccaagtatgt gaacttcctg tacctcgcct cccactacga gaagctgaag ggcagccccg 17460

aggacaacga gcagaagcag ctcttcgtcg agcagcacaa gcactacctg gacgagatca 17520

tcgagcagat cagcgagttc agcaagcgcg tgatcctcgc cgacgccaac ctcgacaagg 17580

tcctgtccgc ctacaacaag caccgcgaca agcctatcag ggagcaggcc gagaacatca 17640

tccacctgtt caccctcacc aacctgggcg ccccagctgc cttcaagtac ttcgacacca 17700

ccatcgaccg caagaggtac accagcacca aggaagtgct ggacgccacc ctgatccacc 17760

agtccatcac cggcctgtac gagactcgca tcgacctcag ccagctgggc ggcgacccga 17820

agaagaagcg caaagtctga gggaccctcg atcgacaagc tcgagtttct ccataataat 17880

gtgtgagtag ttcccagata agggaattag ggttcctata gggtttcgct catgtgttga 17940

gcatataaga aacccttagt atgtatttgt atttgtaaaa tacttctatc aataaaattt 18000

ctaattccta aaaccaaaat ccagtactaa aatccagatc ccccgaatta acctgcaggg 18060

gcggcaggga gagttttaac attgactagc gtgctgataa tttgtgagaa ataataattg 18120

acaagtagat actgacattt gagaagagct tctgaactgt tattagtaac aaaaatggaa 18180

agctgatgca cggaaaaagg aaagaaaaag ccatactttt ttttaggtag gaaaagaaaa 18240

agccatacga gactgatgtc tctcagatgg gccgggatct gtctatctag caggcagcag 18300

ccctaccaac ctcacgggcc agcaattacg agtccttcta aaacgtcccg ccgagggcgc 18360

gtggccgtgc tgtgcagcag cacgtctaac attagtccca cctcgccagt ttacagggag 18420

cagaaccagc ttataagcgg aggcgcggca ccaagaagca gcgctccggg gcggaagtcg 18480

gttttagagc tagaaatagc aagttaaaat aaggctagtc cgttatcaac ttgaaaaagt 18540

ggcaccgagt cggtgctttt ttttcctcga ggggcgatag gaacacgtac aacggccgtt 18600

gtacgtgttc ctatcgccgg taccactagt attaattaag tttaaacggc gcgccaaggg 18660

cgaattccag cacactggcg gccgttacta gtggatcgag ctcgtcgact ctagactcga 18720

gggcgcgcct gacaggatat attggcgggt aaac 18754

<210> 28
<211> 20
<212> DNA
<213> Artificial sequence

<220>
<223> Cas9 - crRNA

<400> 28
gcgctccggg gcggaagtcg 20

<210> 29
<211> 8686
<212> DNA
<213> Artificial sequence

<220>
<223> Complementary sequence of SEQ ID NO 22 (reverse-complement)

<400> 29
gaacctcgtc cacacgttgg aaaccgcaag aaccgaccca taagtgaggt agctcaatga 60

catgcgcaat ccactagagt tgcctttaac gctctgccgg gaaaggcaca aaaacccctc 120

acaatcaccg ggaggtgacc aaaaataatc accaactctt gccacaactc atctgttgct 180

ccaagctgtc taggtggcgg tagccaccaa gagtaacaag aattccgcag ctcaaacgat 240

ccccaagtgc cactatatgc aaactcacaa gcaaatacac ttggaatcac tcaatctcac 300

tttggattca caaccaagca agagagatga gtgggaggaa gtatgcttag cttaagagga 360

tgtcaagatg atcaaaatac caagagaaaa ggcttggagc cgaccaaggt ctatttaaag 420

accccctctg aaaaaccaac cgttacgcag ctcgggggtg atcggactca tcgaccggac 480

tcagccccgt gtccggtcgt aacagtcgag caaccacgat gtgccacgtc acacgattca 540

aaactagccg ttaacagcca acgctttccc gcacacgtcg atgaccggac gcacagaata 600

aatgatcgga ctcagagggc tgcccgcgtt agatcgagtc tagtaagtgt ttttagcgcg 660

tgaatagtga ccagacacgt taggtcatcg gtgacctgac gcggcggtgt gtccggtcgt 720

cctccctctt ttctactcgc acccgcgacc attgacttga catgcaggtc acgctcggcc 780

agctacctcc gcgctcttct tcttcctccg tccgtccgtc gtgtgccctt ccctcgtgcc 840

gccccacgac ttccgccccg gagcgctgcc ctcttcctcc tcccaatggc gcctcagctg 900

cccccgctcg ggctcgccc tcgagtctag agtcgacgag ctcctgcagc catggacgcg 960

taagcttcga tcgatgcctg cagtgcagcg tgacccggtc gtgcccctct ctagagataa 1020

tgagcattgc atgtctaagt tataaaaaat taccacatat ttttttgtc acacttgttt 1080

gaagtgcagt ttatctatct ttatacatat atttaaactt tactctacga ataatataat 1140

ctatagtact acaataatat cagtgtttta gagaatcata taaatgaaca gttagacatg 1200

gtctaaagga caattgagta ttttgacaac aggactctac agttttatct ttttagtgtg 1260

catgtgttct cctttttttt tgcaaatagc ttcacctata taatacttca tccattttat 1320

tagtacatcc atttagggtt tagggttaat ggtttttata gactaatttt tttagtacat 1380

ctattttatt ctattttagc ctctaaatta agaaaactaa aactctattt tagttttttt 1440

atttaataat ttagatataa aatagaataa aataaagtga ctaaaaatta aacaaatacc 1500

ctttaagaaa ttaaaaaaac taaggaaaca tttttcttgt ttcgagtaga taatgccagc 1560

ctgttaaacg ccgtcgacga gtctaacgga caccaaccag cgaaccagca gcgtcgcgtc 1620

gggccaagcg aagcagacgg cacggcatct ctgtcgctgc ctctggaccc ctctcgagag 1680

ttccgctcca ccgttggact tgctccgctg tcggcatcca gaaattgcgt ggcggagcgg 1740

cagacgtgag ccggcacggc aggcggcctc ctcctcctct cacggcacgg cagctacggg 1800

ggattccttt cccaccgctc cttcgctttc ccttcctcgc ccgccgtaat aaatagacac 1860

cccctccaca ccctctttcc ccaacctcgt gttgttcgga gcgcacacac acacaaccag 1920

atctccccca aatccacccg tcggcacctc cgcttcaagg tacgccgctc gtcctccccc 1980

cccccccctc tctaccttct ctagatcggc gttccggtcc atggttaggg cccggtagtt 2040

ctacttctgt tcatgtttgt gttagatccg tgtttgtgtt agatccgtgc tgctagcgtt 2100

cgtacacgga tgcgacctgt acgtcagaca cgttctgatt gctaacttgc cagtgtttct 2160

ctttggggaa tcctgggatg gctctagccg ttccgcagac gggatcgatt tcatgatttt 2220

ttttgtttcg ttgcataggg tttggtttgc cctttccctt tatttcaata tatgccgtgc 2280

acttgtttgt cgggtcatct tttcatgctt ttttttgtct tggttgtgat gatgtggtct 2340

ggttgggcgg tcgttctaga tcggagtaga attctgtttc aaactacctg gtggatttat 2400

taattttgga tctgtatgtg tgtgccatac atattcatag ttacgaattg aagatgatgg 2460

atggaaatat cgatctagga taggtataca tgttgatgcg ggttttactg atgcatatac 2520

agagatgctt tttgttcgct tggttgtgat gatgtggtgt ggttgggcgg tcgttcattc 2580

gttctagatc ggagtagaat actgtttcaa actacctggt gtatttatta attttggaac 2640

tgtatgtgtg tgtcatacat cttcatagtt acgagtttaa gatggatgga aatatcgatc 2700

taggataggt atacatgttg atgtgggttt tactgatgca tatacatgat ggcatatgca 2760

gcatctattc atatgctcta accttgagta cctatctatt ataataaaca agtatgtttt 2820

ataattattt tgatcttgat atacttggat gatggcatat gcagcacttt tgtcgatgct 2880

caccctgttg tttggtgtta cttctgcagg tcgactctag aatggacaac aacccaaaca 2940

tcaacgagtg catcccatac aactgcctga gcaacccaga ggtggaggtg ctgggtggcg 3000

agcgcatcga gaccggttac acccccatcg acatctccct gtccttgacc cagttcctgc 3060

tcagcgagtt cgtgccaggt gctggcttcg tgctcggcct ggtggacatc atctggggta 3120

tcttcggtcc atcccaatgg gacgccttcc tggtgcaaat cgagcagctg atcaaccaga 3180

ggatcgaaga gttcgccagg aaccaggcca tctccaggct ggagggcctg agcaacctct 3240

accaaatcta cgccgagagc ttcagggagt gggaggccga cccgaccaac ccagctctcc 3300

gcgaggaaat gcgcattcaa ttcaacgaca tgaacagcgc cctgaccacc gctatcccac 3360

tgttcgccgt ccagaactac caagtgccgc tcctgtccgt gtacgtgcaa gccgctaacc 3420
tgcacctcag cgtgctgcgc gacgtgagcg tgttcggcca aaggtggggc ttcgatgctg 3480
ccaccatcaa cagccgctac aacgacctga ccaggctgat tggcaactac accgaccacg 3540
ctgtgcgctg gtacaacacc ggcctggagc gcgtctgggg tccggactcc agggactgga 3600
tcaggtacaa ccagttcagg agggagttga ccctcaccgt gctggacatt gtgtccctct 3660
tcccgaacta cgactccagg acctacccga tccgcaccgt gtcccaactc accagggaga 3720
tctacaccaa cccagtgctg gagaacttcg acggtagctt ccgcggttcc gcccagggta 3780
tcgagggctc catcaggagc ccacacctga tggacatcct gaacagcatc accatctaca 3840
ccgacgctca caggggcgag tactactggt ccggccacca gatcatggcc tccccagtgg 3900
gcttcagcgg ccccgagttc accttcccgc tctacggcac catgggcaac gccgctccac 3960
agcaacgcat cgtggctcaa ctgggtcagg gtgtctacag gaccctgtcc tccaccctgt 4020
acaggaggcc cttcaacatc ggtatcaaca accagcaact gtccgtgctc gacggcaccg 4080
agttcgccta cggcacctcc tccaacctgc catccgctgt ctacaggaag agcggcaccg 4140
tggactccct ggacgagatc ccaccacaga acaacaacgt gccacccagg caaggcttct 4200
cccacaggct gagccacgtg tccatgttcc gctccggctt cagcaacagc tccgtgagca 4260
tcatcagggc tccgatgttc tcctggatcc accgcagcgc tgagttcaac aacatcatcg 4320
cctccgacag catcacccaa atcccggccg tgaagggcaa cttcctcttc aacggttccg 4380
tcatttccgg cccaggcttc accggtggcg acctcgtgag gctcaacagc agcggcaaca 4440
acatccagaa caggggctac atcgaggtgc caatccactt cccatccacc tccaccaggt 4500
acagggtgcg cgtgaggtac gcttccgtga ccccgatcca cctcaacgtg aactggggta 4560
actcctccat cttctccaac accgtgccag ctaccgctac ctccctggac aacctccaat 4620
ccagcgactt cggttacttc gagagcgcca acgctttcac ctcctccctc ggtaacatcg 4680
tgggcgtgag gaacttcagc ggcaccgccg gcgtgatcat cgacaggttc gagttcatcc 4740
cagtgaccgc caccctcgag gctgagtgag atcgttcaaa catttggcaa taaagtttct 4800
taagattgaa tcctgttgcc ggtcttgcga tgattatcat ataatttctg ttgaattacg 4860
ttaagcatgt aataattaac atgtaatgca tgacgttatt tatgagatgg gtttttatga 4920
ttagagtccc gcaattatac atttaatacg cgatagaaaa caaaatatag cgcgcaaact 4980
aggataaatt atcgcgcgcg gtgtcatcta tgttactaga tcggcgcgcc aagggcgaat 5040
tccagcacac tggcggccgt tactagtgga tcacgcgtat gcctgcagtg cagcgtgacc 5100
cggtcgtgcc cctctctaga gataatgagc attgcatgtc taagttataa aaaattacca 5160
catatttttt ttgtcacact tgtttgaagt gcagtttatc tatctttata catatattta 5220
aactttactc tacgaataat ataatctata gtactacaat aatatcagtg ttttagagaa 5280

tcatataaat gaacagttag acatggtcta aaggacaatt gagtattttg acaacaggac 5340
tctacagttt tatcttttta gtgtgcatgt gttctccttt ttttttgcaa atagcttcac 5400
ctatataata cttcatccat tttattagta catccattta gggtttaggg ttaatggttt 5460
ttatagacta atttttttag tacatctatt ttattctatt ttagcctcta aattaagaaa 5520
actaaaactc tattttagtt tttttattta ataatttaga tataaaatag aataaaataa 5580
agtgactaaa aattaaacaa ataccottta agaaattaaa aaaactaagg aaacattttt 5640
cttgtttcga gtagataatg ccagcctgtt aaacgccgtc gacgagtcta acggacacca 5700
accagcgaac cagcagcgtc gcgtcgggcc aagcgaagca gacggcacgg catctctgtc 5760
gctgcctctg gacccctctc gagagttccg ctccaccgtt ggacttgctc cgctgtcggc 5820
atccagaaat tgcgtggcgg agcggcagac gtgagccggc acggcaggcg gcctcctcct 5880
cctctcacgg cacggcagct acgggggatt cctttcccac cgctccttcg ctttcccttc 5940
ctcgcccgcc gtaataaata gacacccct ccacaccctc tttccccaac ctcgtgttgt 6000
tcggagcgca cacacacaca accagatctc ccccaaatcc acccgtcggc acctccgctt 6060
caaggtacgc cgctcgtcct cccccccccc ccctctctac cttctctaga tcggcgttcc 6120
ggtccatggt tagggcccgg tagttctact tctgttcatg tttgtgttag atccgtgttt 6180
gtgttagatc cgtgctgcta gcgttcgtac acggatgcga cctgtacgtc agacacgttc 6240
tgattgctaa cttgccagtg tttctctttg gggaatcctg ggatggctct agccgttccg 6300
cagacgggat cgatttcatg attttttttg tttcgttgca tagggtttgg tttgcccttt 6360
tcctttattt caatatatgc cgtgcacttg tttgtcgggt catcttttca tgcttttttt 6420
tgtcttggtt gtgatgatgt ggtctggttg ggcggtcgtt ctagatcgga gtagaattct 6480
gtttcaaact acctggtgga tttattaatt ttggatctgt atgtgtgtgc catacatatt 6540
catagttacg aattgaagat gatggatgga aatatcgatc taggataggt atacatgttg 6600
atgcgggttt tactgatgca tatacagaga tgctttttgt tcgcttggtt gtgatgatgt 6660
ggtgtggttg ggcggtcgtt cattcgttct agatcggagt agaatactgt ttcaaactac 6720
ctggtgtatt tattaatttt ggaactgtat gtgtgtgtca tacatcttca tagttacgag 6780
tttaagatgg atggaaatat cgatctagga taggtataca tgttgatgtg ggttttactg 6840
atgcatatac atgatggcat atgcagcatc tattcatatg ctctaacctt gagtacctat 6900
ctattataat aaacaagtat gttttataat tattttgatc ttgatatact tggatgatgg 6960
catatgcagc agctatatgt ggattttttt agccctgcct tcatacgcta tttatttgct 7020
tggtactgtt tcttttgtcg atgctcaccc tgttgtttgg tgttacttct gcaggtcgac 7080
tctagaatgt ggattgaaca agatggattg cacgcaggtt ctccggccgc ttgggtggag 7140

aggctattcg gctatgactg ggcacaacag acaatcggct gctctgatgc cgccgtgttc 7200

cggctgtcag cgcaggggcg cccggttctt tttgtcaaga ccgacctgtc cggtgccctg 7260

aatgaactgc aggacgaggc agcgcggcta tcgtggctgg ccacgacggg cgttccttgc 7320

gcagctgtgc tcgacgttgt cactgaagcg ggaagggact ggctgctatt gggcgaagtg 7380

ccggggcagg atctcctgtc atctcacctt gctcctgccg agaaagtatc catcatggct 7440

gatgcaatgc ggcggctgca tacgcttgat ccggctacct gcccattcga ccaccaagcg 7500

aaacatcgca tcgagcgagc acgtactcgg atggaagccg gtcttgtcga tcaggatgat 7560

ctggacgaag agcatcaggg gctcgcgcca gccgaactgt tcgccaggct caaggcgcgc 7620

atgcccgacg gcgaggatct cgtcgtgacc catggcgatg cctgcttgcc gaatatcatg 7680

gtggaaaatg gccgcttttc tggattcatc gactgtggcc ggctgggtgt ggcggaccgc 7740

tatcaggaca tagcgttggc tacccgtgat attgctgaag agcttggcgg cgaatgggct 7800

gaccgcttcc tcgtgcttta cggtatcgcc gctcccgatt cgcagcgcat cgccttctat 7860

cgccttcttg acgagttctt ctgagatcgt tcaaacattt ggcaataaag tttcttaaga 7920

ttgaatcctg ttgccggtct tgcgatgatt atcatataat ttctgttgaa ttacgttaag 7980

catgtaataa ttaacatgta atgcatgacg ttatttatga gatgggtttt tatgattaga 8040

gtcccgcaat tatacattta atacgcgata gaaaacaaaa tatagcgcgc aaactaggat 8100

aaattatcgc gcgcggtgtc atctatgtta ctagatcggc gcgccaaggg cgaattccag 8160

cacactggcg gccgttacta gtggatcgag ctcgcggccg ccccgggtac cgatatcagt 8220

actaattcag tacattaaaa acgtccgcaa tgtgttatta agttgtctaa gcgtcaattt 8280

gtttacacca caacccagca atgtgttatt aggttcagcg caggcaaccc agtcacgtca 8340

acctgggcga ccgatcctgg cgtggcgaca gcgccggcct agggcttcgc ctctccgtcg 8400

ccggtgatcc tgcatcggac gagccatggc acggcggccc ccgcgagagc ggcggcggtg 8460

caccggcatg ggcggcccac caatgcggcg gccctgctcg cctactgcac tgctgctgat 8520

gcgccgcccc tcgatctcgg cccatgcccg tatttgtgcc tgtgcccgtt ccatcagcta 8580

gggttacatc tcaatttccc gctctctatc tcgtcttcgt ctttcttcct catctgcaga 8640

tcctagctca gatttagtat tttttaaaat cctttgaagg attacc 8686

<210> 30
<211> 393
<212> DNA
<213> Artificial sequence

<220>
<223> Complementary sequence of SEQ ID NO 23 (reverse-complement)

<400> 30
cccagcaatg tgttattagg ttcagcgcag gcaacccagt cacgtcaacc tgggcgaccg 60

atcctggcgt ggcgacagcg ccggcctagg gcttcgcctc tccgtcgccg gtgatcctgc 120

atcggacgag ccatggcacg gcggccccccg cgagagcggc ggcggtgcac cggcatgggc 180

ggcccaccaa tgcggcggcc ctgctcgcct actgcactgc tgctgatgcg ccgcccctcg 240

atctcggccc atgcccgtat ttgtgcctgt gcccgttcca tcagctaggg ttacatctca 300

atttcccgct ctctatctcg tcttcgtctt tcttcctcat ctgcagatcc tagctcagat 360

ttagtatttt ttaaaatcct ttgaaggatt acc 393

<210> 31
<211> 911
<212> DNA
<213> Artificial sequence

<220>
<223> Complementary sequence of SEQ ID NO 24 (reverse-complement)

<400> 31
gaacctcgtc cacacgttgg aaaccgcaag aaccgaccca taagtgaggt agctcaatga 60

catgcgcaat ccactagagt tgcctttaac gctctgccgg gaaaggcaca aaaacccctc 120

acaatcaccg ggaggtgacc aaaaataatc accaactctt gccacaactc atctgttgct 180

ccaagctgtc taggtggcgg tagccaccaa gagtaacaag aattccgcag ctcaaacgat 240

ccccaagtgc cactatatgc aaactcacaa gcaaatacac ttggaatcac tcaatctcac 300

tttggattca caaccaagca agagagatga gtgggaggaa gtatgcttag cttaagagga 360

tgtcaagatg atcaaaatac caagagaaaa ggcttggagc cgaccaaggt ctatttaaag 420

accccctctg aaaaaccaac cgttacgcag ctcgggggtg atcggactca tcgaccggac 480

tcagccccgt gtccggtcgt aacagtcgag caaccacgat gtgccacgtc acacgattca 540

aaactagccg ttaacagcca acgctttccc gcacacgtcg atgaccggac gcacagaata 600

aatgatcgga ctcagagggc tgcccgcgtt agatcgagtc tagtaagtgt ttttagcgcg 660

tgaatagtga ccagacacgt taggtcatcg gtgacctgac gcggcggtgt gtccggtcgt 720

cctccctctt ttctactcgc acccgcgacc attgacttga catgcaggtc acgctcggcc 780

agctacctcc gcgctcttct tcttcctccg tccgtccgtc gtgtgccctt ccctcgtgcc 840

gccccacgac ttccgccccg gagcgctgcc ctcttcctcc tcccaatggc gcctcagctg 900

cccccgctcg g 911

<210> 32
<211> 26
<212> DNA
<213> Artificial sequence

<220>
<223> Complementary sequence of SEQ ID NO 18 (reverse-complement)

<400> 32
gtttacacca caacccagca atgtgt 26

<210> 33
<211> 26
<212> DNA
<213> Artificial sequence

<220>
<223> Complementary sequence of SEQ ID NO 19 (reverse-complement)

<400> 33
tgcccccgct cggggctcgc cctcga 26

<210> 34
<211> 615
<212> PRT
<213> Artificial sequence

<220>
<223> (Cry1Ac protein) - Truncate cry1Ac protein (B. thurigiensis)

<400> 34

Met Asp Asn Asn Pro Asn Ile Asn Glu Cys Ile Pro Tyr Asn Cys Leu
1 5 10 15

Ser Asn Pro Glu Val Glu Val Leu Gly Gly Glu Arg Ile Glu Thr Gly
20 25 30

Tyr Thr Pro Ile Asp Ile Ser Leu Ser Leu Thr Gln Phe Leu Leu Ser
35 40 45

Glu Phe Val Pro Gly Ala Gly Phe Val Leu Gly Leu Val Asp Ile Ile
50 55 60

Trp Gly Ile Phe Gly Pro Ser Gln Trp Asp Ala Phe Leu Val Gln Ile
65 70 75 80

Glu Gln Leu Ile Asn Gln Arg Ile Glu Glu Phe Ala Arg Asn Gln Ala
85 90 95

Ile Ser Arg Leu Glu Gly Leu Ser Asn Leu Tyr Gln Ile Tyr Ala Glu
100 105 110

Ser Phe Arg Glu Trp Glu Ala Asp Pro Thr Asn Pro Ala Leu Arg Glu
115 120 125

Glu Met Arg Ile Gln Phe Asn Asp Met Asn Ser Ala Leu Thr Thr Ala
130 135 140

```
Ile Pro Leu Phe Ala Val Gln Asn Tyr Gln Val Pro Leu Leu Ser Val
145                 150                 155                 160

Tyr Val Gln Ala Ala Asn Leu His Leu Ser Val Leu Arg Asp Val Ser
                165                 170                 175

Val Phe Gly Gln Arg Trp Gly Phe Asp Ala Ala Thr Ile Asn Ser Arg
            180                 185                 190

Tyr Asn Asp Leu Thr Arg Leu Ile Gly Asn Tyr Thr Asp His Ala Val
        195                 200                 205

Arg Trp Tyr Asn Thr Gly Leu Glu Arg Val Trp Gly Pro Asp Ser Arg
    210                 215                 220

Asp Trp Ile Arg Tyr Asn Gln Phe Arg Arg Glu Leu Thr Leu Thr Val
225                 230                 235                 240

Leu Asp Ile Val Ser Leu Phe Pro Asn Tyr Asp Ser Arg Thr Tyr Pro
                245                 250                 255

Ile Arg Thr Val Ser Gln Leu Thr Arg Glu Ile Tyr Thr Asn Pro Val
            260                 265                 270

Leu Glu Asn Phe Asp Gly Ser Phe Arg Gly Ser Ala Gln Gly Ile Glu
        275                 280                 285

Gly Ser Ile Arg Ser Pro His Leu Met Asp Ile Leu Asn Ser Ile Thr
    290                 295                 300

Ile Tyr Thr Asp Ala His Arg Gly Glu Tyr Tyr Trp Ser Gly His Gln
305                 310                 315                 320

Ile Met Ala Ser Pro Val Gly Phe Ser Gly Pro Glu Phe Thr Phe Pro
                325                 330                 335

Leu Tyr Gly Thr Met Gly Asn Ala Ala Pro Gln Gln Arg Ile Val Ala
            340                 345                 350

Gln Leu Gly Gln Gly Val Tyr Arg Thr Leu Ser Ser Thr Leu Tyr Arg
        355                 360                 365

Arg Pro Phe Asn Ile Gly Ile Asn Asn Gln Gln Leu Ser Val Leu Asp
    370                 375                 380

Gly Thr Glu Phe Ala Tyr Gly Thr Ser Ser Asn Leu Pro Ser Ala Val
385                 390                 395                 400
```

```
Tyr Arg Lys Ser Gly Thr Val Asp Ser Leu Asp Glu Ile Pro Pro Gln
                405                 410                 415

Asn Asn Asn Val Pro Pro Arg Gln Gly Phe Ser His Arg Leu Ser His
            420                 425                 430

Val Ser Met Phe Arg Ser Gly Phe Ser Asn Ser Ser Val Ser Ile Ile
        435                 440                 445

Arg Ala Pro Met Phe Ser Trp Ile His Arg Ser Ala Glu Phe Asn Asn
    450                 455                 460

Ile Ile Ala Ser Asp Ser Ile Thr Gln Ile Pro Ala Val Lys Gly Asn
465                 470                 475                 480

Phe Leu Phe Asn Gly Ser Val Ile Ser Gly Pro Gly Phe Thr Gly Gly
                485                 490                 495

Asp Leu Val Arg Leu Asn Ser Ser Gly Asn Asn Ile Gln Asn Arg Gly
            500                 505                 510

Tyr Ile Glu Val Pro Ile His Phe Pro Ser Thr Ser Thr Arg Tyr Arg
        515                 520                 525

Val Arg Val Arg Tyr Ala Ser Val Thr Pro Ile His Leu Asn Val Asn
    530                 535                 540

Trp Gly Asn Ser Ser Ile Phe Ser Asn Thr Val Pro Ala Thr Ala Thr
545                 550                 555                 560

Ser Leu Asp Asn Leu Gln Ser Ser Asp Phe Gly Tyr Phe Glu Ser Ala
                565                 570                 575

Asn Ala Phe Thr Ser Ser Leu Gly Asn Ile Val Gly Val Arg Asn Phe
            580                 585                 590

Ser Gly Thr Ala Gly Val Ile Ile Asp Arg Phe Glu Phe Ile Pro Val
        595                 600                 605

Thr Ala Thr Leu Glu Ala Glu
    610                 615
```

<210>  35
<211>  265
<212>  PRT
<213>  Artificial sequence

<220>

<223> nptII protein

<400> 35

Met Trp Ile Glu Gln Asp Gly Leu His Ala Gly Ser Pro Ala Ala Trp
1               5                   10                  15

Val Glu Arg Leu Phe Gly Tyr Asp Trp Ala Gln Gln Thr Ile Gly Cys
            20                  25                  30

Ser Asp Ala Ala Val Phe Arg Leu Ser Ala Gln Gly Arg Pro Val Leu
        35                  40                  45

Phe Val Lys Thr Asp Leu Ser Gly Ala Leu Asn Glu Leu Gln Asp Glu
    50                  55                  60

Ala Ala Arg Leu Ser Trp Leu Ala Thr Thr Gly Val Pro Cys Ala Ala
65                  70                  75                  80

Val Leu Asp Val Val Thr Glu Ala Gly Arg Asp Trp Leu Leu Leu Gly
                85                  90                  95

Glu Val Pro Gly Gln Asp Leu Leu Ser Ser His Leu Ala Pro Ala Glu
            100                 105                 110

Lys Val Ser Ile Met Ala Asp Ala Met Arg Arg Leu His Thr Leu Asp
        115                 120                 125

Pro Ala Thr Cys Pro Phe Asp His Gln Ala Lys His Arg Ile Glu Arg
    130                 135                 140

Ala Arg Thr Arg Met Glu Ala Gly Leu Val Asp Gln Asp Asp Leu Asp
145                 150                 155                 160

Glu Glu His Gln Gly Leu Ala Pro Ala Glu Leu Phe Ala Arg Leu Lys
                165                 170                 175

Ala Arg Met Pro Asp Gly Glu Asp Leu Val Val Thr His Gly Asp Ala
            180                 185                 190

Cys Leu Pro Asn Ile Met Val Glu Asn Gly Arg Phe Ser Gly Phe Ile
        195                 200                 205

Asp Cys Gly Arg Leu Gly Val Ala Asp Arg Tyr Gln Asp Ile Ala Leu
    210                 215                 220

Ala Thr Arg Asp Ile Ala Glu Glu Leu Gly Gly Glu Trp Ala Asp Arg
225                 230                 235                 240

Phe Leu Val Leu Tyr Gly Ile Ala Ala Pro Asp Ser Gln Arg Ile Ala
245 250 255

Phe Tyr Arg Leu Leu Asp Glu Phe Phe
260 265

<210> 36
<211> 7382
<212> DNA
<213> Artificial sequence

<220>
<223> Complementary sequence of SEQ ID NO 02 (reverse-complement)

<400> 36
ggctcgccct cgagtctaga gtcgacgagc tcctgcagcc atggacgcgt aagcttcgat 60

cgatgcctgc agtgcagcgt gacccggtcg tgcccctctc tagagataat gagcattgca 120

tgtctaagtt ataaaaaatt accacatatt ttttttgtca cacttgtttg aagtgcagtt 180

tatctatctt tatacatata tttaaacttt actctacgaa taatataatc tatagtacta 240

caataatatc agtgttttag agaatcatat aaatgaacag ttagacatgg tctaaaggac 300

aattgagtat tttgacaaca ggactctaca gttttatctt tttagtgtgc atgtgttctc 360

cttttttttt gcaaatagct tcacctatat aatacttcat ccattttatt agtacatcca 420

tttagggttt agggttaatg gtttttatag actaattttt ttagtacatc tattttattc 480

tattttagcc tctaaattaa gaaaactaaa actctatttt agttttttta tttaataatt 540

tagatataaa atagaataaa ataaagtgac taaaaattaa acaaataccc tttaagaaat 600

taaaaaaact aaggaaacat ttttcttgtt tcgagtagat aatgccagcc tgttaaacgc 660

cgtcgacgag tctaacggac accaaccagc gaaccagcag cgtcgcgtcg ggccaagcga 720

agcagacggc acggcatctc tgtcgctgcc tctggacccc tctcgagagt tccgctccac 780

cgttggactt gctccgctgt cggcatccag aaattgcgtg gcggagcggc agacgtgagc 840

cggcacggca ggcggcctcc tcctcctctc acggcacggc agctacgggg gattcctttc 900

ccaccgctcc ttcgctttcc cttcctcgcc cgccgtaata aatagacacc ccctccacac 960

cctctttccc caacctcgtg ttgttcggag cgcacacaca cacaaccaga tctcccccaa 1020

atccacccgt cggcacctcc gcttcaaggt acgccgctcg tcctcccccc cccccctct 1080

ctaccttctc tagatcggcg ttccggtcca tggttagggc ccggtagttc tacttctgtt 1140

catgtttgtg ttagatccgt gtttgtgtta gatccgtgct gctagcgttc gtacacggat 1200

gcgacctgta cgtcagacac gttctgattg ctaacttgcc agtgtttctc tttggggaat 1260

cctgggatgg ctctagccgt tccgcagacg ggatcgattt catgattttt tttgtttcgt 1320

tgcatagggt ttggtttgcc cttttccttt atttcaatat atgccgtgca cttgtttgtc 1380

gggtcatctt ttcatgcttt tttttgtctt ggttgtgatg atgtggtctg gttgggcggt 1440
cgttctagat cggagtagaa ttctgtttca aactacctgg tggatttatt aattttggat 1500
ctgtatgtgt gtgccataca tattcatagt tacgaattga agatgatgga tggaaatatc 1560
gatctaggat aggtatacat gttgatgcgg gttttactga tgcatataca gagatgcttt 1620
ttgttcgctt ggttgtgatg atgtggtgtg gttgggcggt cgttcattcg ttctagatcg 1680
gagtagaata ctgtttcaaa ctacctggtg tatttattaa ttttggaact gtatgtgtgt 1740
gtcatacatc ttcatagtta cgagtttaag atggatggaa atatcgatct aggataggta 1800
tacatgttga tgtgggtttt actgatgcat atacatgatg gcatatgcag catctattca 1860
tatgctctaa ccttgagtac ctatctatta taataaacaa gtatgtttta taattatttt 1920
gatcttgata tacttggatg atggcatatg cagcactttt gtcgatgctc accctgttgt 1980
ttggtgttac ttctgcaggt cgactctaga atggacaaca acccaaacat caacgagtgc 2040
atcccataca actgcctgag caacccagag gtggaggtgc tgggtggcga gcgcatcgag 2100
accggttaca cccccatcga catctccctg tccttgaccc agttcctgct cagcgagttc 2160
gtgccaggtg ctggcttcgt gctcggcctg gtggacatca tctggggtat cttcggtcca 2220
tcccaatggg acgccttcct ggtgcaaatc gagcagctga tcaaccagag gatcgaagag 2280
ttcgccagga accaggccat ctccaggctg gagggcctga gcaacctcta ccaaatctac 2340
gccgagagct tcagggagtg ggaggccgac ccgaccaacc cagctctccg cgaggaaatg 2400
cgcattcaat tcaacgacat gaacagcgcc ctgaccaccg ctatcccact gttcgccgtc 2460
cagaactacc aagtgccgct cctgtccgtg tacgtgcaag ccgctaacct gcacctcagc 2520
gtgctgcgcg acgtgagcgt gttcggccaa aggtggggct tcgatgctgc caccatcaac 2580
agccgctaca acgacctgac caggctgatt ggcaactaca ccgaccacgc tgtgcgctgg 2640
tacaacaccg gcctggagcg cgtctggggt ccggactcca gggactggat caggtacaac 2700
cagttcagga gggagttgac cctcaccgtg ctggacattg tgtccctctt cccgaactac 2760
gactccagga cctacccgat ccgcaccgtg tcccaactca ccagggagat ctacaccaac 2820
ccagtgctgg agaacttcga cggtagcttc cgcggttccg cccagggtat cgagggctcc 2880
atcaggagcc cacacctgat ggacatcctg aacagcatca ccatctacac cgacgctcac 2940
aggggcgagt actactggtc cggccaccag atcatggcct ccccagtggg cttcagcggc 3000
cccgagttca ccttcccgct ctacggcacc atgggcaacg ccgctccaca gcaacgcatc 3060
gtggctcaac tgggtcaggg tgtctacagg accctgtcct ccaccctgta caggaggccc 3120
ttcaacatcg gtatcaacaa ccagcaactg tccgtgctcg acggcaccga gttcgcctac 3180
ggcacctcct ccaacctgcc atccgctgtc tacaggaaga gcggcaccgt ggactccctg 3240

gacgagatcc caccacagaa caacaacgtg ccacccaggc aaggcttctc ccacaggctg 3300
agccacgtgt ccatgttccg ctccggcttc agcaacagct ccgtgagcat catcagggct 3360
ccgatgttct cctggatcca ccgcagcgct gagttcaaca acatcatcgc ctccgacagc 3420
atcacccaaa tcccggccgt gaagggcaac ttcctcttca acggttccgt catttccggc 3480
ccaggcttca ccggtggcga cctcgtgagg ctcaacagca gcggcaacaa catccagaac 3540
aggggctaca tcgaggtgcc aatccacttc ccatccacct ccaccaggta cagggtgcgc 3600
gtgaggtacg cttccgtgac cccgatccac ctcaacgtga actggggtaa ctcctccatc 3660
ttctccaaca ccgtgccagc taccgctacc tccctggaca acctccaatc cagcgacttc 3720
ggttacttcg agagcgccaa cgctttcacc tcctccctcg gtaacatcgt gggcgtgagg 3780
aacttcagcg gcaccgccgg cgtgatcatc gacaggttcg agttcatccc agtgaccgcc 3840
accctcgagg ctgagtgaga tcgttcaaac atttggcaat aaagtttctt aagattgaat 3900
cctgttgccg gtcttgcgat gattatcata taatttctgt tgaattacgt taagcatgta 3960
ataattaaca tgtaatgcat gacgttattt atgagatggg tttttatgat tagagtcccg 4020
caattataca tttaatacgc gatagaaaac aaaatatagc gcgcaaacta ggataaatta 4080
tcgcgcgcgg tgtcatctat gttactagat cggcgcgcca agggcgaatt ccagcacact 4140
ggcggccgtt actagtggat cacgcgtatg cctgcagtgc agcgtgaccc ggtcgtgccc 4200
ctctctagag ataatgagca ttgcatgtct aagttataaa aaattaccac atattttttt 4260
tgtcacactt gtttgaagtg cagtttatct atctttatac atatatttaa actttactct 4320
acgaataata taatctatag tactacaata atatcagtgt tttagagaat catataaatg 4380
aacagttaga catggtctaa aggacaattg agtattttga caacaggact ctacagtttt 4440
atctttttag tgtgcatgtg ttctcctttt tttttgcaaa tagcttcacc tatataatac 4500
ttcatccatt ttattagtac atccatttag ggtttagggt taatggtttt tatagactaa 4560
tttttttagt acatctattt tattctattt tagcctctaa attaagaaaa ctaaaactct 4620
attttagttt ttttatttaa taatttagat ataaaataga ataaaataaa gtgactaaaa 4680
attaaacaaa taccctttaa gaaattaaaa aaactaagga aacatttttc ttgtttcgag 4740
tagataatgc cagcctgtta aacgccgtcg acgagtctaa cggacaccaa ccagcgaacc 4800
agcagcgtcg cgtcgggcca agcgaagcag acggcacggc atctctgtcg ctgcctctgg 4860
acccctctcg agagttccgc tccaccgttg gacttgctcc gctgtcggca tccagaaatt 4920
gcgtggcgga gcggcagacg tgagccggca cggcaggcgg cctcctcctc ctctcacggc 4980
acggcagcta cgggggattc ctttcccacc gctccttcgc tttcccttcc tcgcccgccg 5040
taataaatag acaccccctc cacaccctct ttccccaacc tcgtgttgtt cggagcgcac 5100
acacacacaa ccagatctcc cccaaatcca cccgtcggca cctccgcttc aaggtacgcc 5160

gctcgtcctc cccccccccc cctctctacc ttctctagat cggcgttccg gtccatggtt 5220
agggcccggt agttctactt ctgttcatgt ttgtgttaga tccgtgtttg tgttagatcc 5280
gtgctgctag cgttcgtaca cggatgcgac ctgtacgtca gacacgttct gattgctaac 5340
ttgccagtgt ttctctttgg ggaatcctgg gatggctcta gccgttccgc agacgggatc 5400
gatttcatga ttttttttgt ttcgttgcat agggtttggt ttgccctttt cctttatttc 5460
aatatatgcc gtgcacttgt ttgtcgggtc atcttttcat gctttttttt gtcttggttg 5520
tgatgatgtg gtctggttgg gcggtcgttc tagatcggag tagaattctg tttcaaacta 5580
cctggtggat ttattaattt tggatctgta tgtgtgtgcc atacatattc atagttacga 5640
attgaagatg atggatggaa atatcgatct aggataggta tacatgttga tgcgggtttt 5700
actgatgcat atacagagat gctttttgtt cgcttggttg tgatgatgtg gtgtggttgg 5760
gcggtcgttc attcgttcta gatcggagta gaatactgtt tcaaactacc tggtgtattt 5820
attaattttg gaactgtatg tgtgtgtcat acatcttcat agttacgagt ttaagatgga 5880
tggaaatatc gatctaggat aggtatacat gttgatgtgg gttttactga tgcatataca 5940
tgatggcata tgcagcatct attcatatgc tctaaccttg agtacctatc tattataata 6000
aacaagtatg ttttataatt attttgatct tgatatactt ggatgatggc atatgcagca 6060
gctatatgtg gattttttta gccctgcctt catacgctat ttatttgctt ggtactgttt 6120
cttttgtcga tgctcaccct gttgtttggt gttacttctg caggtcgact ctagaatgtg 6180
gattgaacaa gatggattgc acgcaggttc tccggccgct tgggtggaga ggctattcgg 6240
ctatgactgg gcacaacaga caatcggctg ctctgatgcc gccgtgttcc ggctgtcagc 6300
gcaggggcgc ccggttcttt ttgtcaagac cgacctgtcc ggtgccctga atgaactgca 6360
ggacgaggca gcgcggctat cgtggctggc cacgacgggc gttccttgcg cagctgtgct 6420
cgacgttgtc actgaagcgg gaagggactg gctgctattg ggcgaagtgc cggggcagga 6480
tctcctgtca tctcaccttg ctcctgccga gaaagtatcc atcatggctg atgcaatgcg 6540
gcggctgcat acgcttgatc cggctacctg cccattcgac caccaagcga aacatcgcat 6600
cgagcgagca cgtactcgga tggaagccgg tcttgtcgat caggatgatc tggacgaaga 6660
gcatcagggg ctcgcgccag ccgaactgtt cgccaggctc aaggcgcgca tgcccgacgg 6720
cgaggatctc gtcgtgaccc atggcgatgc ctgcttgccg aatatcatgg tggaaaatgg 6780
ccgcttttct ggattcatcg actgtggccg gctgggtgtg gcggaccgct atcaggacat 6840
agcgttggct acccgtgata ttgctgaaga gcttggcggc gaatgggctg accgcttcct 6900
cgtgctttac ggtatcgccg ctcccgattc gcagcgcatc gccttctatc gccttcttga 6960
cgagttcttc tgagatcgtt caaacatttg gcaataaagt ttcttaagat tgaatcctgt 7020

tgccggtctt gcgatgatta tcatataatt tctgttgaat tacgttaagc atgtaataat 7080

taacatgtaa tgcatgacgt tatttatgag atgggttttt atgattagag tcccgcaatt 7140

atacatttaa tacgcgatag aaaacaaaat atagcgcgca aactaggata aattatcgcg 7200

cgcggtgtca tctatgttac tagatcggcg cgccaagggc gaattccagc acactggcgg 7260

ccgttactag tggatcgagc tcgcggccgc cccgggtacc gatatcagta ctaattcagt 7320

acattaaaaa cgtccgcaat gtgttattaa gttgtctaag cgtcaatttg tttacaccac 7380

aa 7382

<210> 37
<211> 7588
<212> DNA
<213> Artificial sequence

<220>
<223> Complementary sequence of SEQ ID NO 05 (reverse-complement)

<400> 37
cgtccgtccg tcgtgtgccc ttccctcgtg ccgccccacg acttccgccc cggagcgctg 60

ccctcttcct cctcccaatg gcgcctcagc tgccccgct cggggctcgc cctcgagtct 120

agagtcgacg agctcctgca gccatggacg cgtaagcttc gatcgatgcc tgcagtgcag 180

cgtgacccgg tcgtgcccct ctctagagat aatgagcatt gcatgtctaa gttataaaaa 240

attaccacat attttttttg tcacacttgt ttgaagtgca gtttatctat ctttatacat 300

atatttaaac tttactctac gaataatata atctatagta ctacaataat atcagtgttt 360

tagagaatca tataaatgaa cagttagaca tggtctaaag gacaattgag tattttgaca 420

acaggactct acagttttat ctttttagtg tgcatgtgtt ctcctttttt tttgcaaata 480

gcttcaccta tataatactt catccatttt attagtacat ccatttaggg tttagggtta 540

atggttttta tagactaatt tttttagtac atctatttta ttctatttta gcctctaaat 600

taagaaaact aaaactctat tttagttttt ttatttaata atttagatat aaaatagaat 660

aaaataaagt gactaaaaat taaacaaata ccctttaaga aattaaaaaa actaaggaaa 720

catttttctt gtttcgagta gataatgcca gcctgttaaa cgccgtcgac gagtctaacg 780

gacaccaacc agcgaaccag cagcgtcgcg tcgggccaag cgaagcagac ggcacggcat 840

ctctgtcgct gcctctggac ccctctcgag agttccgctc caccgttgga cttgctccgc 900

tgtcggcatc cagaaattgc gtggcggagc ggcagacgtg agccggcacg gcaggcggcc 960

tcctcctcct ctcacggcac ggcagctacg gggggattcct ttcccaccgc tccttcgctt 1020

tcccttcctc gcccgccgta ataaatagac accccctcca caccctcttt ccccaacctc 1080

gtgttgttcg gagcgcacac acacacaacc agatctcccc caaatccacc cgtcggcacc 1140

tccgcttcaa ggtacgccgc tcgtcctccc cccccccccc tctctacctt ctctagatcg 1200

gcgttccggt ccatggttag ggcccggtag ttctacttct gttcatgttt gtgttagatc 1260

cgtgtttgtg ttagatccgt gctgctagcg ttcgtacacg gatgcgacct gtacgtcaga 1320

cacgttctga ttgctaactt gccagtgttt ctctttgggg aatcctggga tggctctagc 1380

cgttccgcag acgggatcga tttcatgatt ttttttgttt cgttgcatag ggtttggttt 1440

gccctttcc tttatttcaa tatatgccgt gcacttgttt gtcgggtcat cttttcatgc 1500

tttttttgt cttggttgtg atgatgtggt ctggttgggc ggtcgttcta gatcggagta 1560

gaattctgtt tcaaactacc tggtggattt attaattttg gatctgtatg tgtgtgccat 1620

acatattcat agttacgaat tgaagatgat ggatggaaat atcgatctag gataggtata 1680

catgttgatg cgggttttac tgatgcatat acagagatgc tttttgttcg cttggttgtg 1740

atgatgtggt gtggttgggc ggtcgttcat tcgttctaga tcggagtaga atactgtttc 1800

aaactacctg gtgtatttat taattttgga actgtatgtg tgtgtcatac atcttcatag 1860

ttacgagttt aagatggatg gaaatatcga tctaggatag gtatacatgt tgatgtgggt 1920

tttactgatg catatacatg atggcatatg cagcatctat tcatatgctc taaccttgag 1980

tacctatcta ttataataaa caagtatgtt ttataattat tttgatcttg atatacttgg 2040

atgatggcat atgcagcact tttgtcgatg ctcaccctgt tgtttggtgt tacttctgca 2100

ggtcgactct agaatggaca acaacccaaa catcaacgag tgcatcccat acaactgcct 2160

gagcaaccca gaggtggagg tgctgggtgg cgagcgcatc gagaccggtt acacccccat 2220

cgacatctcc ctgtccttga cccagttcct gctcagcgag ttcgtgccag gtgctggctt 2280

cgtgctcggc ctggtggaca tcatctgggg tatcttcggt ccatcccaat gggacgcctt 2340

cctggtgcaa atcgagcagc tgatcaacca gaggatcgaa gagttcgcca ggaaccaggc 2400

catctccagg ctggagggcc tgagcaacct ctaccaaatc tacgccgaga gcttcaggga 2460

gtgggaggcc gacccgacca acccagctct ccgcgaggaa atgcgcattc aattcaacga 2520

catgaacagc gccctgacca ccgctatccc actgttcgcc gtccagaact accaagtgcc 2580

gctcctgtcc gtgtacgtgc aagccgctaa cctgcacctc agcgtgctgc gcgacgtgag 2640

cgtgttcggc caaaggtggg gcttcgatgc tgccaccatc aacagccgct acaacgacct 2700

gaccaggctg attggcaact acaccgacca cgctgtgcgc tggtacaaca ccggcctgga 2760

gcgcgtctgg ggtccggact ccagggactg gatcaggtac aaccagttca ggagggagtt 2820

gaccctcacc gtgctggaca ttgtgtccct cttcccgaac tacgactcca ggacctaccc 2880

gatccgcacc gtgtcccaac tcaccaggga gatctacacc aacccagtgc tggagaactt 2940

cgacggtagc ttccgcggtt ccgcccaggg tatcgagggc tccatcagga gcccacacct 3000

gatggacatc ctgaacagca tcaccatcta caccgacgct cacaggggcg agtactactg 3060

gtccggccac cagatcatgg cctccccagt gggcttcagc ggccccgagt tcaccttccc 3120
gctctacggc accatgggca acgccgctcc acagcaacgc atcgtggctc aactgggtca 3180
gggtgtctac aggaccctgt cctccaccct gtacaggagg cccttcaaca tcggtatcaa 3240
caaccagcaa ctgtccgtgc tcgacggcac cgagttcgcc tacggcacct cctccaacct 3300
gccatccgct gtctacagga agagcggcac cgtggactcc ctggacgaga tcccaccaca 3360
gaacaacaac gtgccaccca ggcaaggctt ctcccacagg ctgagccacg tgtccatgtt 3420
ccgctccggc ttcagcaaca gctccgtgag catcatcagg gctccgatgt tctcctggat 3480
ccaccgcagc gctgagttca acaacatcat cgcctccgac agcatcaccc aaatcccggc 3540
cgtgaagggc aacttcctct tcaacggttc cgtcatttcc ggcccaggct tcaccggtgg 3600
cgacctcgtg aggctcaaca gcagcggcaa caacatccag aacaggggct acatcgaggt 3660
gccaatccac ttcccatcca cctccaccag gtacagggtg cgcgtgaggt acgcttccgt 3720
gaccccgatc cacctcaacg tgaactgggg taactcctcc atcttctcca acaccgtgcc 3780
agctaccgct acctccctgg acaacctcca atccagcgac ttcggttact tcgagagcgc 3840
caacgctttc acctcctccc tcggtaacat cgtgggcgtg aggaacttca gcggcaccgc 3900
cggcgtgatc atcgacaggt tcgagttcat cccagtgacc gccaccctcg aggctgagtg 3960
agatcgttca aacatttggc aataaagttt cttaagattg aatcctgttg ccggtcttgc 4020
gatgattatc atataatttc tgttgaatta cgttaagcat gtaataatta acatgtaatg 4080
catgacgtta tttatgagat gggtttttat gattagagtc ccgcaattat acatttaata 4140
cgcgatagaa aacaaaatat agcgcgcaaa ctaggataaa ttatcgcgcg cggtgtcatc 4200
tatgttacta gatcggcgcg ccaagggcga attccagcac actggcggcc gttactagtg 4260
gatcacgcgt atgcctgcag tgcagcgtga cccggtcgtg cccctctcta gagataatga 4320
gcattgcatg tctaagttat aaaaaattac cacatatttt ttttgtcaca cttgtttgaa 4380
gtgcagttta tctatcttta tacatatatt taaactttac tctacgaata atataatcta 4440
tagtactaca ataatatcag tgttttagag aatcatataa atgaacagtt agacatggtc 4500
taaaggacaa ttgagtattt tgacaacagg actctacagt tttatctttt tagtgtgcat 4560
gtgttctcct ttttttttgc aaatagcttc acctatataa tacttcatcc attttattag 4620
tacatccatt tagggtttag ggttaatggt tttatagac taattttttt agtacatcta 4680
ttttattcta ttttagcctc taaattaaga aaactaaaac tctattttag tttttttatt 4740
taataattta gatataaaat agaataaaat aaagtgacta aaaattaaac aaataccctt 4800
taagaaatta aaaaaactaa ggaaacattt ttcttgtttc gagtagataa tgccagcctg 4860
ttaaacgccg tcgacgagtc taacggacac caaccagcga accagcagcg tcgcgtcggg 4920
ccaagcgaag cagacggcac ggcatctctg tcgctgcctc tggacccctc tcgagagttc 4980

cgctccaccg ttggacttgc tccgctgtcg gcatccagaa attgcgtggc ggagcggcag 5040
acgtgagccg gcacggcagg cggcctcctc ctcctctcac ggcacggcag ctacggggga 5100
ttcctttccc accgctcctt cgctttccct tcctcgcccg ccgtaataaa tagacacccc 5160
ctccacaccc tctttcccca acctcgtgtt gttcggagcg cacacacaca caaccagatc 5220
tcccccaaat ccacccgtcg gcacctccgc ttcaaggtac gccgctcgtc ctcccccccc 5280
ccccctctct accttctcta gatcggcgtt ccggtccatg gttagggccc ggtagttcta 5340
cttctgttca tgtttgtgtt agatccgtgt ttgtgttaga tccgtgctgc tagcgttcgt 5400
acacggatgc gacctgtacg tcagacacgt tctgattgct aacttgccag tgtttctctt 5460
tggggaatcc tgggatggct ctagccgttc cgcagacggg atcgatttca tgattttttt 5520
tgtttcgttg catagggttt ggtttgccct tttcctttat ttcaatatat gccgtgcact 5580
tgtttgtcgg gtcatctttt catgcttttt tttgtcttgg ttgtgatgat gtggtctggt 5640
tgggcggtcg ttctagatcg gagtagaatt ctgtttcaaa ctacctggtg gatttattaa 5700
ttttggatct gtatgtgtgt gccatacata ttcatagtta cgaattgaag atgatggatg 5760
gaaatatcga tctaggatag gtatacatgt tgatgcgggt tttactgatg catatacaga 5820
gatgcttttt gttcgcttgg ttgtgatgat gtggtgtggt tgggcggtcg ttcattcgtt 5880
ctagatcgga gtagaatact gtttcaaact acctggtgta tttattaatt ttggaactgt 5940
atgtgtgtgt catacatctt catagttacg agtttaagat ggatggaaat atcgatctag 6000
gataggtata catgttgatg tgggttttac tgatgcatat acatgatggc atatgcagca 6060
tctattcata tgctctaacc ttgagtacct atctattata ataaacaagt atgttttata 6120
attattttga tcttgatata cttggatgat ggcatatgca gcagctatat gtggattttt 6180
ttagccctgc cttcatacgc tatttatttg cttggtactg tttcttttgt cgatgctcac 6240
cctgttgttt ggtgttactt ctgcaggtcg actctagaat gtggattgaa caagatggat 6300
tgcacgcagg ttctccggcc gcttgggtgg agaggctatt cggctatgac tgggcacaac 6360
agacaatcgg ctgctctgat gccgccgtgt tccggctgtc agcgcagggg cgcccggttc 6420
tttttgtcaa gaccgacctg tccggtgccc tgaatgaact gcaggacgag gcagcgcggc 6480
tatcgtggct ggccacgacg ggcgttcctt gcgcagctgt gctcgacgtt gtcactgaag 6540
cgggaaggga ctggctgcta ttgggcgaag tgccggggca ggatctcctg tcatctcacc 6600
ttgctcctgc cgagaaagta tccatcatgg ctgatgcaat gcggcggctg catacgcttg 6660
atccggctac ctgcccattc gaccaccaag cgaaacatcg catcgagcga gcacgtactc 6720
ggatggaagc cggtcttgtc gatcaggatg atctggacga agagcatcag gggctcgcgc 6780
cagccgaact gttcgccagg ctcaaggcgc gcatgcccga cggcgaggat ctcgtcgtga 6840

cccatggcga tgcctgcttg ccgaatatca tggtggaaaa tggccgcttt tctggattca 6900

tcgactgtgg ccggctgggt gtggcggacc gctatcagga catagcgttg gctacccgtg 6960

atattgctga agagcttggc ggcgaatggg ctgaccgctt cctcgtgctt tacggtatcg 7020

ccgctcccga ttcgcagcgc atcgccttct atcgccttct tgacgagttc ttctgagatc 7080

gttcaaacat ttggcaataa agtttcttaa gattgaatcc tgttgccggt cttgcgatga 7140

ttatcatata atttctgttg aattacgtta agcatgtaat aattaacatg taatgcatga 7200

cgttatttat gagatgggtt tttatgatta gagtcccgca attatacatt taatacgcga 7260

tagaaaacaa aatatagcgc gcaaactagg ataaattatc gcgcgcggtg tcatctatgt 7320

tactagatcg gcgcgccaag ggcgaattcc agcacactgg cggccgttac tagtggatcg 7380

agctcgcggc cgccccgggt accgatatca gtactaattc agtacattaa aaacgtccgc 7440

aatgtgttat taagttgtct aagcgtcaat ttgtttacac cacaacccag caatgtgtta 7500

ttaggttcag cgcaggcaac ccagtcacgt caacctgggc gaccgatcct ggcgtggcga 7560

cagcgccggc ctagggcttc gcctctcc 7588

<210> 38
<211> 206
<212> DNA
<213> Artificial sequence

<220>
<223> Complementary sequence of SEQ ID NO 03 (reverse-complement)

<400> 38
ctcgcggccg ccccgggtac cgatatcagt actaattcag tacattaaaa acgtccgcaa 60

tgtgttatta agttgtctaa gcgtcaattt gtttacacca caacccagca atgtgttatt 120

aggttcagcg caggcaaccc agtcacgtca acctgggcga ccgatcctgg cgtggcgaca 180

gcgccggcct agggcttcgc ctctcc 206

<210> 39
<211> 206
<212> DNA
<213> Artificial sequence

<220>
<223> Complementary sequence of SEQ ID NO 04 (reverse-complement)

<400> 39
cgtccgtccg tcgtgtgccc ttccctcgtg ccgccccacg acttccgccc cggagcgctg 60

ccctcttcct cctcccaatg gcgcctcagc tgccccccgct cggggctcgc cctcgagtct 120

agagtcgacg agctcctgca gccatggacg cgtaagcttc gatcgatgcc tgcagtgcag 180

cgtgacccgg tcgtgcccct ctctag 206

<210> 40
<211> 20
<212> DNA
<213> Artificial sequence

<220>
<223> Artificial sequence

<400> 40
gaggtgccaa tccacttccc 20

<210> 41
<211> 23
<212> DNA
<213> Artificial sequence

<220>
<223> Artificial sequence

<400> 41
gagttacccc agttcacgtt gag 23

<210> 42
<211> 17
<212> DNA
<213> Artificial sequence

<220>
<223> Artificial sequence

<400> 42
acctccacca ggtacag 17

<210> 43
<211> 20
<212> DNA
<213> Artificial sequence

<220>
<223> Artificial sequence

<400> 43
tgccgaatat catggtggaa 20

<210> 44
<211> 19
<212> DNA
<213> Artificial sequence

<220>
<223> Artificial sequence

<400> 44
cggccacagt cgatgaatc 19

<210> 45
<211> 13
<212> DNA

<213> Artificial sequence

<220>
<223> Artificial sequence

<400> 45
tggccgcttt tct 13

<210> 46
<211> 18
<212> DNA
<213> Artificial sequence

<220>
<223> Artificial sequence

<400> 46
gccgcttgta gccttcca 18

<210> 47
<211> 16
<212> DNA
<213> Artificial sequence

<220>
<223> Artificial sequence

<400> 47
ccgccgacct ggtgga 16

<210> 48
<211> 15
<212> DNA
<213> Artificial sequence

<220>
<223> Artificial sequence

<400> 48
cgtgacctca atgcg 15

<210> 49
<211> 20
<212> DNA
<213> Artificial sequence

<220>
<223> Artificial sequence

<400> 49
cacaatcgtc acctcaaccg 20

<210> 50
<211> 21
<212> DNA
<213> Artificial sequence

<220>

<223> Artificial sequence

<400> 50
atcaacgacc ttctggaaac g 21

<210> 51
<211> 18
<212> DNA
<213> Artificial sequence

<220>
<223> Artificial sequence

<400> 51
cgcaggattt cgctctcg 18

<210> 52
<211> 19
<212> DNA
<213> Artificial sequence

<220>
<223> Artificial sequence

<400> 52
tttgacaaca ggactctac 19

<210> 53
<211> 21
<212> DNA
<213> Artificial sequence

<220>
<223> Artificial sequence

<400> 53
tgcaatgctc attatctcta g 21

<210> 54
<211> 23
<212> DNA
<213> Artificial sequence

<220>
<223> Artificial sequence

<400> 54
tagcttcacc tatataatac ttc 23

<210> 55
<211> 19
<212> DNA
<213> Artificial sequence

<220>
<223> Artificial sequence

<400> 55

tgcactgcag gcatcgatc 19

<210> 56
<211> 20
<212> DNA
<213> Artificial sequence

<220>
<223> Artificial sequence

<400> 56
tcgtcgactc tagactcgag 20

<210> 57
<211> 21
<212> DNA
<213> Artificial sequence

<220>
<223> Artificial sequence

<400> 57
gataatgcca gcctgttaaa c 21

<210> 58
<211> 30
<212> DNA
<213> Artificial sequence

<220>
<223> Artificial sequence

<400> 58
ggtaatcctt caaaggattt taaaaaatac 30

<210> 59
<211> 29
<212> DNA
<213> Artificial sequence

<220>
<223> Artificial sequence

<400> 59
agatagagag cgggaaattg agatgtaac 29

<210> 60
<211> 19
<212> DNA
<213> Artificial sequence

<220>
<223> Artificial sequence

<400> 60
tcgtccgatg caggatcac 19

<210> 61
<211> 19
<212> DNA
<213> Artificial sequence

<220>
<223> Artificial sequence

<400> 61
ggttgacgtg actgggttg 19

<210> 62
<211> 19
<212> DNA
<213> Artificial sequence

<220>
<223> Artificial sequence

<400> 62
gttgacgtga ctgggttgc 19

<210> 63
<211> 19
<212> DNA
<213> Artificial sequence

<220>
<223> Artificial sequence

<400> 63
ggccgttact agtggatcg 19

<210> 64
<211> 20
<212> DNA
<213> Artificial sequence

<220>
<223> Artificial sequence

<400> 64
gccgttacta gtggatcgag 20

<210> 65
<211> 20
<212> DNA
<213> Artificial sequence

<220>
<223> Artificial sequence

<400> 65
caacaaccca aacatcaacg 20

<210> 66
<211> 26
<212> DNA

<213> Artificial sequence

<220>
<223> Artificial sequence

<400> 66
atggctgcag gagctcgtcg actcta 26

<210> 67
<211> 20
<212> DNA
<213> Artificial sequence

<220>
<223> Artificial sequence

<400> 67
cgtcggtgta gatggtgatg 20

<210> 68
<211> 25
<212> DNA
<213> Artificial sequence

<220>
<223> Artificial sequence

<400> 68
tgatgcatat acagagatgc ttttt 25

<210> 69
<211> 25
<212> DNA
<213> Artificial sequence

<220>
<223> Artificial sequence

<400> 69
ttcatccatt ttattagtac atcca 25

<210> 70
<211> 18
<212> DNA
<213> Artificial sequence

<220>
<223> Artificial sequence

<400> 70
acggatgcga cctgtacg 18

<210> 71
<211> 20
<212> DNA
<213> Artificial sequence

<220>

<223> Artificial sequence

<400> 71
tcagtaaaac ccacatcaac 20

<210> 72
<211> 21
<212> DNA
<213> Artificial sequence

<220>
<223> Artificial sequence

<400> 72
gaccacatca tcacaaccaa g 21

<210> 73
<211> 21
<212> DNA
<213> Artificial sequence

<220>
<223> Artificial sequence

<400> 73
gctccgaaca acacgaggtt g 21

<210> 74
<211> 22
<212> DNA
<213> Artificial sequence

<220>
<223> Artificial sequence

<400> 74
atgaagtatt atataggtga ag 22

<210> 75
<211> 20
<212> DNA
<213> Artificial sequence

<220>
<223> Artificial sequence

<400> 75
agaggctatt cggctatgac 20

<210> 76
<211> 20
<212> DNA
<213> Artificial sequence

<220>
<223> Artificial sequence

<400> 76

cagccgaact gttcgccagg 20

<210> 77
<211> 20
<212> DNA
<213> Artificial sequence

<220>
<223> Artificial sequence

<400> 77
agcacgagga agcggtcagc 20

<210> 78
<211> 20
<212> DNA
<213> Artificial sequence

<220>
<223> Artificial sequence

<400> 78
tgagatgaca ggagatcctg 20

<210> 79
<211> 22
<212> DNA
<213> Artificial sequence

<220>
<223> Artificial sequence

<400> 79
caacagctcc gtgagcatca tc 22

<210> 80
<211> 20
<212> DNA
<213> Artificial sequence

<220>
<223> Artificial sequence

<400> 80
ccagcgactt cggttacttc 20

<210> 81
<211> 21
<212> DNA
<213> Artificial sequence

<220>
<223> Artificial sequence

<400> 81
ctctcggcgt agatttggta g 21

<210> 82
<211> 20
<212> DNA
<213> Artificial sequence

<220>
<223> Artificial sequence

<400> 82
cagaacaaca acgtgccacc 20

<210> 83
<211> 21
<212> DNA
<213> Artificial sequence

<220>
<223> Artificial sequence

<400> 83
agcatcacca tctacaccga c 21

<210> 84
<211> 21
<212> DNA
<213> Artificial sequence

<220>
<223> Artificial sequence

<400> 84
gatccaggag aacatcggag c 21

<210> 85
<211> 21
<212> DNA
<213> Artificial sequence

<220>
<223> Artificial sequence

<400> 85
gtcactggga tgaactcgaa c 21

<210> 86
<211> 20
<212> DNA
<213> Artificial sequence

<220>
<223> Artificial sequence

<400> 86
gggtcctgta gacaccctga 20

<210> 87
<211> 22
<212> DNA

<213> Artificial sequence

<220>
<223> Artificial sequence

<400> 87
gctcaccctg ttgtttggtg tt 22

<210> 88
<211> 20
<212> DNA
<213> Artificial sequence

<220>
<223> Artificial sequence

<400> 88
ctgtcggcat ccagaaattg 20

<210> 89
<211> 19
<212> DNA
<213> Artificial sequence

<220>
<223> Artificial sequence

<400> 89
tcccctcggg atcaaagta 19

<210> 90
<211> 20
<212> DNA
<213> Artificial sequence

<220>
<223> Artificial sequence

<400> 90
tagcgtgttt gtgcttttgc 20

<210> 91
<211> 20
<212> DNA
<213> Artificial sequence

<220>
<223> Artificial sequence

<400> 91
ggccgctgaa attaaatcaa 20

<210> 92
<211> 20
<212> DNA
<213> Artificial sequence

<220>

```
<223>  Artificial sequence

<400>  92
gagtcagtga gcgaggaagc                                                    20


<210>  93
<211>  20
<212>  DNA
<213>  Artificial sequence

<220>
<223>  Artificial sequence

<400>  93
cggtgaaaac ctctgacaca                                                    20


<210>  94
<211>  20
<212>  DNA
<213>  Artificial sequence

<220>
<223>  Artificial sequence

<400>  94
atacaggcag cccatcagtc                                                    20
```

## Description

### Technical Field

The present invention relates to the field of biotechnology. More specifically, it is described a genetic construct and a method for producing a transgenic plant event, especially a sugarcane (*Saccharum* spp.) event, which expresses the Cry1Ac toxin conferring resistance to infestation by the pest *Diatraea saccharalis,* popularly known as common borer, cane borer or just borer. The invention describes a method for detecting the event and material derived from the event resistant to cane borer infestation, as well as polynucleotides, primers, probes and the flanking regions identifying such an event.

## Background

Sugarcane (*Saccharum* spp.) is a grass belonging to the botanical family Poaceae, originating from Southeast Asia, more precisely from the large central region of New Guinea and Indonesia. It is one of the most important plant species grown in the tropical and subtropical regions, with an area of over 23 million hectares spread over 121 countries (FAO Statistical Yearbook 2012 p. 233).

Sugarcane is a source of raw material for the production of sugar, wine, molasses, rum, cachaça (Brazil's national distillate) and ethanol for fuel. The bagasse that is produced after the sugarcane milling can be used for baling, supplying heat energy, processing at mills, producing electricity (that is typically sold to the consumer's electric grid), or as raw material for the production of sugarcane second-generation ethanol (BR 11 2014 02385-1). Thus, the sugarcane agro-industry has great economic and social importance by generating millions of jobs in the area and fostering foreign exchange through the commercialization of sugar and ethanol and sustainable and optimal use of plant biomass.

More recently, with the advent of global warming and the subsequent desire for alternatives to fossil fuels (biofuels), worldwide interest in sugarcane has increased significantly. The use of sugarcane-based ethanol as a renewable energy source has been considered extremely important for reducing greenhouse gases and dependence on fossil fuels, thus making it a key element in efforts to control global climate change (Savage, 2011).

Due to the economic and social importance of sugarcane, an increasing amount of research has been directed toward defining best agricultural practices for its cultivation and improving the quality of cultivated varieties. Efforts to improve sugarcane agronomic characteristics have focused on increasing sugar production and accumulation, increasing tolerance to biotic and abiotic stresses, resistance and tolerance to pests and pathogens, and developing alternative technologies for the production of sugarcane ethanol from lignocellulosic biomass (PI 0802153-8; PI 0904538-4; PI 1101295-1).

The complexity of the polyploid and aneuploid genome of modern sugarcane varieties, coupled with their relatively restricted genetic base and low fertility, impose great difficulties and numerous limitations on the selection of plants with desirable agronomic characteristics using conventional breeding (Souza et al, 2011; D'Hont & Glaszmann, 2005, Basel, v. 109, no. 1-3, p. 27-33; Cheavegatti-Gianotto et al., 2011). Therefore, high production costs, the necessity of manual labor, and long product-to-market timelines may prevent the sugarcane industry from meeting the growing demands of the global market.

The sugar cane production has been increased over the last 20 years as a result not just of the increase on productivity of plantations, but also as a result of the increase on diversity of cultivated varieties, allowing the expansion of cultivated area to other geographical regions. This geographical expansion demands the development of adapted varieties to different edaphoclimatic conditions, rendering the sustainability of sugarcane market supported by the continuously suppling to the market of new and local adapted germplasm and also by the capacity to control diseases and pests.

The conventional breeding methods are important for continuously supplying new varieties, but not sufficient to attend the need of the modern market since part of characteristics are not founded in the genetic background of the varieties, being fundamental the development of efficient methods to introduce exogenous genes. Due to the limitations of conventional breeding methods and the increasing need to rapidly and efficiently incorporate desirable traits, the use of genetic engineering (biotechnology) in sugarcane breeding programs has gained prominence, in particular due to the commercial success of incorporating desirable agronomic traits through genetic engineering into other plant species (soybean, corn, canola, beet and cotton, for example).

Plant genetic engineering involves the transfer of genes-of-interest into plant cells (genetic transformation) in such a way that a fertile and agronomically superior progeny maintain and stably express the gene responsible for the desired trait.

Despite the potential of sugarcane genetic alteration (incorporation of desirable characteristics) by genetic engineering [virus resistance (Guo et al., 2015; Zhu et al., 2011), insects (Kalunke, Kolge, Babu, & Prasad, 2009; Weng et al., 2011), herbicides (Enríquez-Obregon, Vazquez-Padron, Prieto-Samsonov, De la Riva, & Selman-Housein, 1998;

van der Vyver, Conradie, Kossmann, & Lloyd, 2013), drought tolerance (Molinari et al., 2007; Reis et al., 2014), salinity (Kumar, Uzma, Khan, Abbas, & Ali, 2014) and aluminum toxicity (Ribeiro, 2016), increased production and accumulation of sugar (Bewg, Poovaiah, Lan, Ralph, & Coleman, 2016; Mudge et al., 2013)], this approach is limited by intrinsic characteristics of sugarcane. Unlike maize, rice, wheat and other commercial cereals, sugarcane exhibits difficulties in tissue culture propagation, low rates of induction and regeneration of embryogenic calluses, and the impossibility of using the zygotic embryo as a target tissue in genetic transformation [(Anderson & Birch, 2012; Basnayake, Moyle, & Birch, 2011; Molinari et al., 2007)]. Low rates of transformation efficiency and high variability between sugarcane genotypes are frequently observed, and there are still numerous challenges to overcome in order to successfully incorporate desirable agronomic traits into sugarcane using genetic engineering.

Sugarcane is considered a recalcitrant species for genetic transformation, and although several genetic engineering approaches have been evaluated for this species, there are still no standard protocols that guarantee the production of transgenic events (Smith et al. 1992; Rathius & Birch 1992.; Chen et al. 1987; Arencibia 1998; Manickavasagam et al. 2004; Elliott et al. 1998).

In addition to the inherent limitations of the species that prevent the application of existing genetic engineering techniques, the complexity of the sugarcane genome (high ploidy level and aneuploidy), prevents trait introgression into specific cultivars through backcrossing (reconstitution of a specific genotype), as is commonly performed in other crops of commercial interest. Thus, for the "insertion" of the same trait in more than one germplasm, enabling gains in productivity in different geographical regions, it is necessary to carry out a new genetic transformation, incurring the same costs and risks associated with the first event obtained for that trait. Even today, there is very little predictability of success for the insertion of the same characteristic in different varieties of sugar cane, being an extremely genotype-dependent process, which makes the construction of a portfolio of genetically modified products for this species challenging.

The genotypic complexity of the species also significantly impacts the characterization of the events generated in order to ensure the necessary characteristics for their commercialization. The unambiguous identification of transgenic events is fundamental to ensure their traceability and monitoring, which is a regulatory requirement for their commercialization. The high polyploidy of the sugarcane genome and the high number of repeated regions, coupled with the lack of information on their organization and structure, make the characterization of the transgenic events generated even more difficult.

There are several technical challenges to be overcome in the field of sugarcane breeding to increase the predictability of results, even when applying widely known conventional and/or molecular/genetic techniques. Despite all the technical challenges for obtaining more productive varieties of sugarcane, there is no doubt about the urgency of obtaining improved varieties that have characteristics that significantly impact crop productivity and therefore its market.

Historically, agricultural pests are one of the main factors that cause losses in agriculture. In Brazil, the main sugarcane pest is the species *Diatraea saccharalis* (first described by Fabricius in 1794), popularly known as the common borer, cane borer or just borer. It is a member of the Crambidae family and Lepidoptera order. The borer is found practically everywhere the crop is cultivated, an area of approximately 10 million hectares for the 2019/20 crop season (CONAB, 2019).

After mating, the female sugarcane borer lays 200 to 400 eggs distributed on either side of the leaves as well as the leaf sheaths. After hatching, neonate larvae feed on the leaf parenchyma, migrating to the sheath region for shelter. They remain in this region for 7 to 10 days, feeding by scraping the leaf sheath or bark of the young internodes. After an ecdysis, the caterpillars pierce the stalk, penetrating inside. The insect creates tunnels inside the stalk, usually upwards as it feeds. Inside the culm, the caterpillar goes through approximately six ecdysis before becoming a winged adult (DINARDO-MIRANDA, 2014). This is the developmental stage of the insect that causes economic damage to the crop (Figure 1).

The attack of the sugarcane borer also causes serious secondary damage to the quality of the raw material used for sugar and alcohol production, because the drilling of the sugarcane stalk by the borer creates favorable conditions for fungal entry and opportunistic bacteria, especially *Fusarium moniliform* and *Colletotrichum falcatum,* causing red rot (Figure 2). Bacteria associated with the red rot raw material produce undesirable fermentations, resulting in products foreign to industrial alcoholic fermentation. Moreover, these bacteria also produce organic acids and gums (dextrans) from the sugars contained in the wort, which negatively affect the viability of yeast cells, requiring their replacement in fermentation reactors (Precectі and Terán 1983; Prececti et al., 1988; BOTELHO and MACEDO, 2002). Another problem arising from the presence of bacteria in fermentation reactors is the possibility of yeast flocculation occurring. In this case, the contaminating bacteria form mucilage that aggregates the yeast cells, causing them to flocculate. Finally, plants attacked by the borer/red rot complex also have high levels of phenolic compounds (METCALF and LUCKMANN, 1994; PRICE, 1997).

Assuming a 4% borer Infestation Infection Index (typical for Brazilian sugarcane fields), average agricultural losses, and pest control costs, it is estimated that the borer causes economic losses of more than R$5 billion annually to the sugar and ethanol production industries.

The sugarcane borer is difficult to control with chemical insecticides due to the feeding behavior of the larva in the stalk, which prevents the insecticide from effectively contacting the insect. As an alternative to chemical insecticides, insecticidal proteins, mainly identified from the bacterium *Bacillus thuringiensis* (Bt), have been used to control agricultural pests, including *Diatraea* sp. Among the insecticidal proteins derived from Bt strains, Cry crystalline proteins stand out for their specific toxicity to larvae of common lepidopteran, dipteran and coleopteran species. These proteins, produced as protoxins (65-149 KDa), are solubilized and activated in the intestines of susceptible insects by proteolysis and bind to the intestinal cell membrane, inducing osmotic lysis of the epithelium, which causes the insect to die.

Cry proteins are classified into several groups according to sequence homology, among them, the protein group classified as Cry1 presents high specificity against lepidopteran insects, making it an excellent candidate for introduction into sugarcane germplasm to produce sugarcane borer resistant varieties. The heterologous expression of Cry1 proteins in sugarcane varieties, although challenging, has great potential for sugarcane borer control, reducing economic losses to the sugarcane industry, as well as the release of chemical insecticides in the environment.

Therefore, there remains a need to develop strategies to mitigate the damage caused to sugarcane crops by pest infestation, especially by infestation by the sugarcane borer pest. By offering sugarcane growers varieties with both high yield potential and borer resistance traits, agricultural biotechnology makes an important contribution to the sugarcane industry and Brazilian sugarcane growers.

The genetically modified sugarcane event CTC79005-2 disclosed in the present invention was generated to provide to the sugarcane growers a new variety, resistant to sugarcane borer pest and with the genetic background of RB92579 (Plant Variety Protection/PVP - protocol number - 21806.000439/2000-45; PVP Certificate, № 480, granted at 23/07/2003). The cultivar RB92579 was launched in 2003 and had a planting area at 2017/18 of approximately 61,000ha, having been used in the renovation of sugarcane fields, especially in the Northeast region, where it produces 30-40% more cane than other varieties. RB92579 is adapted to the edaphoclimatic conditions of northeast region (drier region) as it is highly responsive to irrigation and water use.

Embodiments

In a first embodiment the invention provides polynucleotides that unambiguously identify the event CTC79005-2.

The invention also identifies primers pairs and probes able to identify polynucleotides that characterize the event CTC79005-2.

In a third embodiment the invention provides methods for detecting plant material derived from the CTC79005-2 event.

Other embodiment of the invention defines a kit for detecting the presence of event CTC79005-2 in a sample of plant material.

The fifth embodiment of the present invention is a genetic construct capable of imparting to a sugarcane (*Saccharum* spp.) plant, resistance to insect infestation, particularly by *Diatraea saccharalis* pest.

Also, the invention provides a genetically modified sugarcane, a plant part, plant cell, plant tissue, or seed comprising the genetic construct of interest located at a site defined in the genome of the transformed sugarcane plant, characterized by specific flanking sequences.

The seventh embodiment of the present invention is to provide a commodity product.

The eighth embodiment of the present invention is a method of producing an insect resistant plant.

Finally, the ninth and tenth embodiments of the invention are to provide a method of making and cultivating an insect resistant sugarcane plant and/or plant cell, plant part, or seed.

## Summary of the Invention

The first embodiment is achieved by providing polynucleotides comprising at least 14 to 26 contiguous nucleotides of sequences selected from the group consisting of SEQ ID NO: 5, SEQ ID NO: 12, SEQ ID NO: 13, SEQ ID NO: 18, SEQ ID NO: 19, and SEQ ID NO: 22.

The second embodiment of the invention is achieved by providing primers pairs wherein the forward primer consists of SEQ ID NO: 6 and the reverse primer consists of SEQ ID NO: 7 and/or the forward primer consists of SEQ ID NO: 8 and the reverse primer is SEQ ID NO: 9.

The third embodiment is achieved by a method of detecting plant material derived from event CTC79005-2 comprising the steps of:

a) obtaining a sample for analysis;
b) extracting DNA from the sample;
c) providing primer pairs comprising at least a forward and a reverse primer;
d) amplifying a region between the primer pair; and detecting the presence of a product from amplification.

Also to achieve the third embodiment the primer pairs in step c) are designed to bind to a polynucleotide comprising contiguous nucleotides of sequences selected from the group consisting of SEQ ID NO: 22 and SEQ ID NO: 29, wherein at least one pair of primers comprises contiguous nucleotides of sequences selected from the group consisting of SEQ ID NO: 23, SEQ ID NO: 24, SEQ ID NO: 30 and SEQ ID NO: 31.

Still to achieve the third embodiment, the present invention describes a method of detecting plant material derived from event CTC79005-2 comprising the steps of:

a) obtaining a plant material sample for analysis;
b) extracting DNA or RNA from the sample;
c) providing a probe designed to bind to the complement of a polynucleotide comprising at least 14 contiguous nucleotides of sequences selected from the group consisting of SEQ ID NO: 18, SEQ ID NO: 19, SEQ ID NO: 32 and SEQ ID NO: 33;
d) hybridizing said probe with the sample; and
e) detecting the actual hybridization of the probe.

The fourth embodiment of the invention is evidenced by a kit for detecting the presence of event CTC79005-2 in a plant sample, the kit comprising a means to detect the presence of a polynucleotide comprising, at least, 14 contiguous nucleotides of SEQ ID NO: 18 and/or of SEQ ID NO: 19 and/or a pesticidal crystal protein (Cry).

The fifth embodiment is achieved through providing a genetic construct comprising SEQ ID NO: 1 or 2.

The sixth embodiment is achieved through a genetically modified sugarcane (*Saccharum* spp.) plant, a plant part, plant cell, plant tissue, or seed comprising SEQ ID NO: 18 or SEQ ID NO: 19.

The seventh embodiment is achieved by providing a commodity product produced from the genetic modified sugarcane from the present invention.

The eighth embodiment is achieved by a method of producing an insect resistant plant comprising SEQ ID NO: 20 and SEQ ID NO: 21

The ninth embodiment of the present invention provides a method of making an insect resistant sugarcane plant comprising introducing a genetic modification to a sugarcane (*Saccharum* spp.) plant comprising SEQ ID NO: 5 or SEQ ID NO: 22 to produce a genetically modified sugarcane (*Saccharum* spp.) plant of event CTC79005-2.

Finally, the tenth embodiment of the invention describes a method of cultivating a genetically modified sugarcane (*Saccharum* spp.) plant of event CTC79005-2, comprising growing a genetically modified sugarcane (*Saccharum* spp.) plant of event CTC79005-2 comprising SEQ ID NO: 5 or SEQ ID NO: 22 under conditions comprising insect infestation.

**Brief Description of the Figures**

Figure 1 exemplifies the damage to sugarcane stalks caused by *D. saccharalis* (sugarcane borer).

Figure 2 exemplifies the red rot-borer complex due to *D. saccharalis* (sugarcane borer) attack.

Figure 3 represents the map of the T-DNA introduced in the event of the present invention.

Figure 4 represents the plasmid used as a base for constructing the plasmid used in the present invention.

Figure 5 represents the resulting plasmid used to obtain the event of interest.

Figure 6 is the graph of qPCR amplification via Taqman® (relative fluorescence x cycle) for the event of interest.

Figure 7 is the qPCR melting curve via SYBR GREEN ™ (relative fluorescence x temperature) for the event of interest. The arrows indicate the specific amplification peak of the event of interest, as well as the baseline indicate of no amplification for the other negative events and controls.

Figure 8 is the result of comparing means of Cry1Ac protein expression in leaves (Fresh tissue) of the event of the invention during the sugarcane cultivation cycle. Combined analysis for Barrinha, Piracicaba, Valparaiso (SP), Quirinopolis (GO), Mandaguaçu (PR) and Juazeiro (BA). Bars followed by the same letter do not differ by T test at the 5% probability level. Bars means SE.

Figure 9 is the result of comparing means of CrylAc protein expression in leaves (Dry tissue) of the event of the invention during the sugarcane cultivation cycle. Combined analysis for Barrinha (SP), Piracicaba (SP), Valparaiso (SP), Quirinopolis (GO), Mandaguaçu (PR) and Juazeiro (BA). Bars followed by the same letter do not differ by T test at the 5% probability level. Bars means SE.

Figure 10 is the expression means of the CrylAc protein in fresh (A) and dry base (B) of the stalk, leaf and root tissues of the CTC79005-2 event. Bars followed by the same letter do not differ by T test at the 5% probability level. The bars represent the mean ± standard error.

Figure 11 is the result of comparing means of NptII protein expression in leaves (Fresh tissue) of the event of the invention during the sugarcane cultivation cycle. Combined analysis for Barrinha (SP), Piracicaba (SP), Valparaiso (SP), Quirinopolis (GO), Mandaguaçu (PR) and Juazeiro (BA) locations. Bars followed by the same letter do not differ by T test at the 5% probability level.

Figure 12 is the result of comparing means of NptII protein expression in leaves (Dry tissue) of the event of the invention during the sugarcane cultivation cycle. Combined analysis for Barrinha (SP), Piracicaba (SP), Valparaiso (SP), Quirinopolis (GO), Mandaguaçu (PR) and Juazeiro (BA). Bars followed by the same letter do not differ by T test at the 5% probability level.

Figure 13 is the expression averages of the NptII protein in fresh (A) and dry base (B) of the stalk, leaf and root tissues of the CTC79005-2 event. Bars followed by the same letter do not differ by T test at the 5% probability level. The bars represent the mean ± standard error.

Figure 14 is the result of the Western blot methodology for identifying the CrylAc protein. M: molecular weight marker (kDa). R1 to R4: biological repetitions of event CTC79005-2. WT1 to WT4 (negative control): total protein extracted from parental cultivar, RB 92579, untransformed. CP (positive control): 1ng of purified CrylAc protein. WT + CP: 1ng of purified CrylAc protein diluted in total proteins extracted from WT. Ø: empty well.

Figure 15 is the result of the Western blot methodology for identification of NptII protein. M: molecular weight marker (kDa). R1 and R2: biological repeats of event CTC79005-2. CP: 5ng of NptII protein and WT + CP: 5ng of NptII protein diluted in total proteins extracted from the parental cultivar RB 92579. WT: total proteins extracted from the parental cultivar RB 92579 untransformed.

Figure 16 represents A) Infestation index (I.I.%), B) Relative length of damage from event CTC79005-2 and from controls (plants in the first harvest). The letters represent the result of the analysis of variance by the T test and the bars refer to the standard error.

Figure 17 represents Borer Control Effectiveness of event CTC79005-2 from the 4 locations (1.1% = infestation index). The bar refers to the standard error of the analysis.

Figure 18 shows in A: larvae mortality in the Leaf disc assay with plant material from the insect (sugarcane borer) resistant CTC79005-2 event in comparison to the parental non-genetically modified CTC79-TC (RB92579); B: Borer Control Effectiveness of event CTC79005-2; C: exemplary results of larval size and development after seven days of feeding in the Leaf disc assay. On left are exemplary larvae that were fed with plant material from the insect resistant CTC79005-2 event, and on right are exemplary larvae that were fed with plant material from the parental non-genetically modified CTC79-TC (RB92579).

Figure 19 demonstrates examples of cassettes for generation of event CTC79005-2 through gene editing approach. Cassette A comprises a sugarcane codon optimized Cas9 driven by pZmUbi promoter and T-35s terminator; Cassette B comprises crRNA for Cas9 driven by wheat U3 promoter and Cassette C comprises the HR template comprising CTC79005-2 -T-DNA region (SEQ ID NO 2) with homologous arms for site directed integration.

Figure 20 represents an example of a gene editing construction comprising Cas 9 and crRNA Cassettes.

Figure 21 shows an example of a gene editing construction comprising the HR template comprising CTC79005-2 -T-DNA region (SEQ ID NO 2) with homologous arms for site directed integration.

Figure 22 represents a gene editing construction comprising the all the cassettes for generation of event CTC79005-2: a codon optimized Cas9 driven by pZmUbi promoter and T-35s terminator; a crRNA for Cas9 driven by wheat U3 promoter and the HR template comprising CTC79005-2 -T-DNA region (SEQ ID NO 2) with homologous arms for site directed integration.

Figure 23 A) is a schematic representation of Southern blot strategy using *EcoRV, Hind*III and *BsrG*I restriction enzymes for identifying the T-DNA inserted in the event CTC79005-2. Gray arrows below the T-DNA scheme indicate the predicted sizes of the T-DNA fragments from CTC79005-2 event generated. B) is a schematic representation of Southern blot strategy for detecting vector (backbone) fragments using the restriction enzyme *Sph*I.

Figure 24 represents the Southern blot using the restriction enzymes *EcoRV* (left) *Hind*III (midle), and *BsrG*I (right). Results for the cry probe that recognizes the *cry1Ac* gene. M: molecular weight marker; WT: negative control; 1C: positive control 1 copy. T0: plant from the 1st harvest. T1: plant from the 2nd harvest; T2: plant from the 3rd harvest; T3: plant from the 4th harvest.

Figure 25 represents the Southern blot using the restriction enzymes *BsrGI* (left), *Hind*III (midle), and *EcoRV* (right). Results for the UBI-1 probe that recognizes UBI promoter. M: molecular weight marker; WT: negative control; 1C: positive control 1 copy. T0: plant from the 1st harvest. T1: plant from the 2nd harvest; T2: plant from the 3rd harvest; T3: plant from the 4th harvest.

Figure 26 represents the Southern blot using the restriction enzymes *EcoRV* (left) *Hind*III (midle), and *BsrGI* (right). Results for the nptII probe that recognizes the *nptII* gene. M: molecular weight marker; WT: negative control; 1C: positive control 1 copy; T0: plant from the 1st harvest. T1: plant from the 2nd harvest; T2: plant from the 3rd harvest; T3: plant from the 4th harvest.

Figure 27 A) represents the Southern blot using the restriction enzyme *Sph*I and the backbone probes BB1 and BB3. M: molecular weight marker; WT: negative control; 1C: positive control 1 copy with pCTC302. BB1: positive control for the probe backbone 1; BB2: positive control for the backbone 2 probe; BB3: positive control for the backbone probe 3. The red arrows indicate two repetitions of the CTC79005-2 event. Lane 1: other event. B) represents the Southern blot using the *Sph*I restriction enzyme and the BB2 backbone probe. M: molecular weight marker; WT: negative control; 1C: positive control 1 copy with pCTC302. BB1: positive control for the probe backbone 1; BB2: positive control for the backbone 2 probe; BB3: positive control for the backbone probe 3. The red arrows indicate two repetitions of the CTC79005-2 event. Lane 1 are other events.

Figure 28 represents the confirmation of the integrity of the flanking regions of the T-DNA inserted in the event CTC79005-2. (A) Confirmation of integrity of the left border (5’). (B) confirmation of integrity of the right border (3’). Event: DNA from event CTC79005-2; WT: DNA of the RB 92579 variety; C-: negative control.

## Detailed Description of the Invention

First, the term "event" refers to the transgenic plant produced through genetic transformation that stably expresses the desired trait conferred by the introduced transgene. More particularly, in the present case, the term "event" is considered to be the transgenic plant, preferably a sugarcane transgenic plant (*Saccharum* spp.) which, after genetic modification, expresses the characteristic of pest resistance, particularly resistance to the pest *Diatraea saccharalis*(sugarcane borer). In a preferred embodiment, the transgenic sugarcane produced through the genetic transformation is designated ’CTC79005-2’ and may alternately be referred to as "CTC79005-2 event".

Also, for reference purposes, unless expressly mentioned otherwise, "LB region" means the left border (edge) of T-DNA transfer (5’), and "RB region" means the right border (edge) of T-DNA transfer (3’).

Additionally, all biological sequences describe herein, except otherwise explicitly stated, encompass sequences having at least 80%, preferably 85%, 90%, 95%, 98%, 99% or 100% of identity with the described sequences.

Finally, "plant material" means any and all plant tissue or derivatives thereof, such as, but not limited to, seeds, stems, stalks, leaves, straws, bark, roots, cells, molecules of plant origin, among others. In addition, "plant material" may include any product of a plant or derivative thereof, for example, but not limited to, sap, sugar, ethanol, among others.

Recombinant DNA technology has enabled the isolation of genes and their stable insertion into a host genome. This technique, also called genetic transformation, can be defined as the controlled introduction of nucleic acids ("DNA" or DNA) into a recipient genome, excluding introduction by fertilization. It is a controlled process where a defined DNA fragment is introduced into the host (or recipient) genome and must be integrated into it. The stable insertion of these molecules into a host genome gives rise to an individual with the genome equal or substantially equal to the recipient (host) of the recombinant molecule, but with a new and particular feature. "Substantially equal" means a genome with more than 80%, preferably 85%, 90%, 95%, 98%, 99% or 100% of identity in relation to the recipient.

There are several plant genetic transformation techniques grouped into two main categories: indirect and direct gene transfer. Indirect transfer is when exogenous DNA is inserted into the genome by the action of a biological vector, while direct transfer is based on physical-biochemical processes.

Different tissues and/or cells could be used according to the genetic transformation technique and according to the species or genotypes to be transformed. Generally, these tissues or cells include, without limitation, embryogenic callus, callus, protoplasts, embryos, somatic embryos, meristematic tissues, and any other part, tissue or cell of plant with regenerative capacity.

Indirect transformation is based on the bacterium-mediated system of the genus *Agrobacterium* and has been the most widely used method for obtaining transgenic plants. Advantages to this method include the ability to transfer relatively long DNA segments without rearrangement while maintaining low copy number integration of the transgenes, thus ensuring greater genotypic stability for the generated events. Several *Agrobacterium* species and strains, plasmids and protocols have been developed and adapted for genetic transformation of several plant species. The advantages of these methods include higher probabilities to single copy events, stable integration, and genetic heritage of the introduced genetic traits, as well as, consistent genic expression through generations and lower rates of gene silencing.

*Agrobacterium tumefaciens* and *A. rhizogenes* are gram negative soil phytopathogenic bacteria belonging to the Rhizobiaceae family that cause diseases in dicotyledons, known as crown and hairy root galls, respectively. In this plant-pathogen interaction there is a process of natural gene transfer between the agrobacterium and the plant cell wherein fragments of bacterial DNA are transferred into the plant cell (T-DNA), integrating with the nuclear genome. In its natural form, the bacterium transfers T-DNA ("transferred DNA"), which is part of the bacterial plasmid called Ti ("tumor-inducing") and integrates into the genome of infected plant cells. The T-DNA fragment that is transferred to the plant cell is comprised of genes involved in the constitutive biosynthesis of phytohormones (auxins and cytokinins), which alter the normal developmental program of infected tissue and cause tumor formation. In addition, it also contains oncogenes for the synthesis of sugars and amino acids called opines, which serve as carbon and nitrogen sources for bacteria (Oger et al. 1997). Repeated ends of 25 base pairs (bp) at the right and left borders delimit the T-DNA and are essential for its transfer. Phenolic compounds released by injured plant tissues activate specific regions (vir regions), initiating the process of transfer of T-DNA to the plant cell. *Agrobacterium* also has chromosomal (chv) genes that promote binding between bacterial and host cells, allowing the formation of the pore passage of the T-DNA-containing complex (Sheng & Citovsky. 1996).

Since the segment to be transferred is defined by its borders, any sequence flanked by the borders can be transferred to a plant by means of agrobacteria, making it possible to manipulate these sequences in order to transfer coding sequences of interest. The replacement or deletion of the coding regions of wild-type T-DNA (oncogenes) allows for the generation of non-oncogenic (disarmed) *Agrobacterium* strains, which can carry the sequences of interest. The modified T-DNA is able to transfer the sequences of interest to plants because the virulence genes (vir region) remain intact.

Additionally, the *Agrobacterium* indirect transformation system allows for the transfer of artificial plasmid constructs to plants as long as the constructs contain such T-DNA borders, which enables the flexibility to use molecular tools and materials developed for other bacterial strains.

These artificial plasmid constructs have promoters from different origins, as for example, plant promoters, viral promoters, bacterial and or chimeric promoters, besides genes that confer antibiotic resistance, herbicide resistance or tolerance, or enzymatic activity (phosphomannose isomerase (PMI)/mannose (Man)), so these markers can be used for the selection of transformed cells or plants.

These constructions also can contain auxiliaries genes which interfere with relevant morphogenesis signaling pathways, enhancing the efficiency of the genetic transformation process and regeneration of vegetal tissues. Included, without limitations, LEAFY COTYLEDON1 (Lotan et al., 1998), Lecl (Lowe et al., 2002), LEAFY COTYLEDON2 (Stone et al., 2001), WUSCHEL (WUS; Zuo et al., 2002), e BABY BOOM (BBM; Boutilier et al., 2002), among others.

In a first aspect of the present invention, foreign or exogenous DNA to be introduced into the plant is cloned into a binary plasmid between the left and right border consensus sequences (T-DNA). The binary plasmid is transferred to an *Agrobacterium* cell, which is subsequently used to infect plant tissue. The T-DNA region of the vector comprising the exogenous DNA is inserted into the plant genome. The marker gene expression cassette and the characteristic gene expression cassette may be present in the same region of T-DNA, in different regions of T-DNA on the same plasmid, or in different regions of T-DNA on different plasmids. In one embodiment of the present invention, the cassettes are present in the same region as the T-DNA. One of skill in the art is familiar with the methods of indirect transformation by *Agrobacterium.*

Alternatively, direct DNA transfer can be used to directly introduce DNA into a plant cell. One method of direct DNA transfer is to bombard plant cells with a vector comprising DNA for insertion using a particle gun (particle-mediated biolistic transformation). Other methods for transformation of plant cells include protoplast transformation (optionally in the presence of polyethylene glycols); ultrasound treatment of plant tissues, cells, or protoplasts in a medium comprising the polynucleotide or the vector; microinjection of the polynucleotide or vector into plant material; microinjection, vacuum infiltration, sonication, use of silicon carbide, chemical transformation with PEG, electroporation of plant cells and the like. Between the disadvantages of direct transformation are challenges related to regeneration of plant tissue and the low transgene expression.

In addition, genetic transformation could be performed by site direct insertion through homologous recombination mediated by nucleases (genome editing). In recent years, genome editing technology based on use of engineered or chimeric nucleases has enabling the generation of genetically modified organisms in a more precise and specific way. The introduction of exogenous or foreign genes occur by homologous recombination through introduction of a Homologous recombination template (HR) having the exogeneous DNA linked to a DNA fragment homologous to the genome of the receptor organism. Between the tools available are the chimeric enzymatic system CRISPR(clustered, regularly interspaced, short palindromic repeats) - Cas, the Zinc finger (ZFN)nucleases and TAL effector nucleases (TALENs). Crispr-Cas systems are enzymatic systems comprising two main components: a endonuclease (Cas) and a guide-RNA (single-guide RNA - sgRNA; a guide to the specific cleavage site of Cas endonuclease). The guide RNA may also comprise of two components: a Crispr RNA (crRNA) - a sequence of 17-20 mer complementary to specific DNA genomic sequences and, optionally, of a tracr RNA. The specific cleavage performed by endonuclease and guide by the sgRNA would be repair by homologous recombination, specifically inserting the exogenous DNA flanked by the homologous sequences to the cleavage site. The introduction of this enzymatic system to the cell could occur by several manners, using plasmids, through direct or indirect transformation, or using carriers like proteins and other chemical agents. The expression of the system components would occur in a transient or stable manner, using the cellular machinery of the receptor organism or being realized in a exogeneous way, in vitro, delivering to the target cell or tissue all the components ready to use (endonucleases + sgRNA, in vitro transcribed and combined before cell delivery). The description presented herein is not exhaustive and should not limit the use of different variations, systems and methods of genome editing on scope of the present invention, known in the State of the Art and even the ones not yet discovered.

Following transformation, transgenic plants are regenerated from the transformed plant tissue and the progeny that have exogenous DNA can be selected using an appropriate marker such as kanamycin, geneticin or ammonium glufosinate resistance. One skilled in the art is familiar with the composition of suitable regeneration media.

Alternatively, other selection methods could be applied, without the insertion of any gene marker in the host genome (receptor organism) as described before.

In a preferred embodiment, genetic transformation is mediated through a bacterium of the genus *Agrobacterium.*

In an even more preferred embodiment, genetic transformation is mediated by *Agrobacterium tumefaciens.*

CTC79005-2 event exhibits a new genotype comprising two expression cassettes. The first expression cassette comprises a promoter suitable for plant expression operably linked to a gene encoding a Cry1Ac insecticide toxin useful in controlling lepidopteran insect pests and a suitable polyadenylation signal. The second expression cassette comprises a promoter suitable for plant expression operably linked to a gene encoding a protein used as a selective marker in obtaining the event of the present invention.

Promoters suitable for plant expression may be isolated from plants or from other organisms. Several promoters have been isolated or developed including constitutive promoters, "on and off" promoters, and promoters that are responsive to tissue-specific abiotic stresses, among others. Many of these promoters have intronic sequences described as relevant for proper gene expression. In a preferred aspect of the invention, promoters are constitutive promoters and may be selected from the non-limiting group consisting of CaMV 35s, CoYMV (*Commelina yellow mottle virus*), FMV 35s, Ubiquitin, Actin Rice Promoter (Act-1), Act -2, nopaline synthase promoter (NOS), octopine synthase promoter (OCS), corn alcohol dehydrogenase promoter (Adh-1), PvUbi1, among others.

In one embodiment of the invention, the promoter is the maize Ubiquitin (pUBI) gene promoter. In an even more preferred embodiment, the maize Ubiquitin promoter contains an intron in the 5' sequence of the leader RNA.

The promoter region of the present invention (UBI-1) has 1992 base pairs which are subdivided into: promoter fragment (899 bases), first exon of the polyubiquitin-1 gene (83 bases) and first intron (1,010 bases).

Additional elements such as introns, enhancer sequences and transporters (transporters) may also be incorporated into the expression cassette for the purpose of enhancing gene expression levels, for example, transcriptional or translation enhancers such as CaMV 35s enhancers, FMV 35s, Nos, supP, non-translated leader sequence from wheat major Chlorophyll a/b-Binding Polypeptide (L-Cab), kosak sequences 5' upstream from the translational start site, among others.

Terminator sequences are also contemplated on the expression cassette. Examples of suitable and functional plant polyadenylation signals include those from the *Agrobacterium tumefaciens* nopaline synthase gene (nos), proteinase inhibitor II gene rbcS (pea ribulose-1,5-bisphosphate carboxylase small subunit), Lhcb1 (tobacco chlorophyll a/b-binding proteins), CaMV 35S, octopine synthase, alpha-tubulin gene, among others.

In one embodiment of the present invention, the polyadenylation signal is that derived from the *Agrobacterium tumefaciens* nopaline synthase (nos) gene.

Preferably, the expression of *cry1Ac* and *nptII* genes is regulated by the maize ubiquitin gene promoter - UBI-1 (which has an endogenous intron). Both expression cassettes use the *Agrobacterium tumefaciens* nopaline synthase terminator - NOS.

**[0073]** The *cry1Ac* gene encodes a 615 amino acid toxin with an estimated molecular weight of 68 KDa, originating from *Bacillus thuringiensis* serovar kustaki (strain HD73), which confers resistance to *Diatraea saccharalis* (cane borer). The present invention contemplates gene modifications for expression of the active tryptic nucleus of native CrylAc protein only. Thus, in a preferred embodiment of the present invention, the polynucleotide encoding the CrylAc protein is truncated, encoding the 52 kDa tryptic insecticide nucleus. In a more preferred embodiment, the CrylAc protein is SEQ ID NO 34. The present invention also contemplates sequences having at least 80%, preferably 85%, 90%, 95%, 98%, 99% or 100% of identity with SEQ ID NO: 34. The tryptic nucleus is responsible for the insecticidal activity of the protein, binding to specific proteins of the insect's gut leading to disruption of the functional and anatomical integrity of this organ. Ingestion of the CrylAc protein by the target insect causes altered nutrient absorption, which leads to rapid toxicity and subsequent death of the insect.

According to the invention, the polynucleotide encoding the CrylAc protein may have optimized (or otherwise altered) codons to improve expression in plant material. Such codon optimization may be used to alter the predicted secondary structure of the RNA transcription product produced in any transformed cell or to destroy the cryptic RNA instability elements present in the unchanged transcription product, thereby enhancing the stability and/or availability of the transcription product in the transformed cell.

Preferably, the *cry1Ac* gene present at the event of the invention corresponds to a truncated synthetic DNA sequence optimized with preferred sugarcane codons. In an even more preferred aspect of the present invention, the *cry1Ac* gene has the sequence SEQ ID NO: 20. The invention also contemplates sequences having at least 80%, preferably 85%, 90%, 95%, 98%, 99% or 100% of identity with SEQ ID NO: 20.

Several marker genes for plant event selection have already been characterized, including some that confer tolerance to antibiotics and others that confer resistance to herbicides. Examples of marker genes that may be selected for use in the present invention include those that confer resistance or tolerance to hygromycin, kanamycin, gentamicin, geneticin, glyphosate, ammonium glufosinate or resistance to toxins such as eutypine. Other forms of selection are also available such as hormone-based selection systems, visual selection through expression of fluorescent proteins, mannose isomerase, xylose isomerase, among others. In one embodiment of the present invention, the event selection marker gene is one which confers tolerance to kanamycin and geneticin.

In a preferred embodiment of the invention, the marker gene used in the second expression cassette is the *nptII* gene, which encodes the 265 amino acid neomycin phosphotransferase II (NptII) enzyme with an estimated molecular weight of 29.2kDa. In an even more preferred aspect of the present invention, the *nptII* gene has the sequence SEQ ID NO 21. Neomycin phosphotransferase II confers resistance to antibiotics of the aminoglycoside class, such as kanamycin and geneticin. The *nptII* gene used as a selection marker in obtaining transformed events is derived from the Escherichia coli Tn5 transposon as described by BECK et al. (1982). The NptII protein is produced by several prokaryotes widely found in the environment, both in aquatic and terrestrial habitats, as well as in human and animal intestinal microflora. The NptII protein inactivates aminoglycoside antibiotics such as neomycin, gentamicin, paromycin and kanamycin A, B and C using adenosine triphosphate (ATP) to phosphorylate them, thus preventing them from causing injury to cells when exposed to the mentioned antibiotics. This mechanism allows its use as a marker for selecting transformed plants. In a preferred aspect of the present invention, the NptII protein has the sequence SEQ ID NO 35.

The use of selection marker genes, such as the *nptII* gene, is important for selecting cells transformed in the process of genetic modification (HORSCH et al., 1985). The purpose of inserting the *nptII* gene in the event of the present invention was, therefore, the selection of cells transformed with the *cry1Ac* gene.

In addition to the expression cassettes described, additional expression cassettes may also be used in event CTC79005-2.

The first and second expression cassettes comprised in event CTC79005-2 may be introduced into the plant on the same or on different plasmids. If the first and second expression cassettes are located on the same plasmid and are introduced into the plant by an *Agrobacterium-mediated* transformation method, they may be present within the same or different regions of T-DNA. In one embodiment of the present invention, the first and second expression cassettes are present in the same region as the T-DNA.

More particularly, the event of the present invention was obtained by *Agrobacterium tumefasciens*-mediated transformation with a genetic construct comprising a DNA fragment (T-DNA) containing the *cry1Ac* and *nptII* gene expression cassettes. Preferably, the genetic construct of the present invention comprises the nucleotide of sequence SEQ ID NO:1.

The event of the present invention was obtained by *Agrobacterium tumefasciens*-mediated transformation containing the T-DNA fragment as defined above (SEQ ID NO:1).

This T-DNA fragment was inserted into a binary plasmid that contains in its host spectrum the bacteria *Escherichia coli* and *Agrobacterium tumefaciens.* Specific genetic elements and the origins of the components of the original binary plasmid of the present invention are shown in Figure 4. The binary plasmid comprising the construct of the present invention is depicted in Figure 5.

In a preferred embodiment, the genetic construct of the present invention comprises the sequence of SEQ ID NO:14.

Said construct is transferred to an *Agrobacterium tumefaciens* (vector) strain by techniques known to one of ordinary skill in the art, such as electroporation or thermal shock, among others.

In an even more preferred embodiment, the vector is an *Agrobacterium tumefaciens* strain EHA105.

A method for producing the event of interest is further described. In a preferred embodiment, the said method comprising the steps of:

a) introducing a genetic construct into an *Agrobacterium* strain;
b) obtaining embryogenic callus from immature leaf rolls or top stalks of sugarcane (*Saccharum* spp.);
c) co-cultivating embryogenic callus with a culture of *Agrobacterium*;
d) selecting transformed cells containing the functional fragment in culture medium containing aminoglycoside antibiotics; and
e) regenerating transformed sugarcane plants.

In one embodiment, the step a) of the method of producing a genetically modified sugarcane (*Saccharum* spp.) plant of event CTC79005-2 comprises introducing a genetic construct comprising SEQ ID NO: 20 and SEQ ID NO: 21 into an *Agrobacterium* strain. Additionally, step e) of said method comprises regenerating transformed sugarcane plants, wherein the genetically modified sugarcane plants comprise SEQ ID NO: 20 and SEQ ID NO: 21. The invention also contemplates, a plant part, plant cell, plant tissue, or seed of the genetically modified sugarcane plants produced by the method described herein.

Those skilled in the art are familiar with the composition of suitable culture media for the generation of embryogenic callus (stage b), as well as the means of the co-cultivation stages (stage c: co-cultivation + rest), selection (stage d), and regeneration (stage e; regeneration + elongation). Preferably, the culture media used are based on compositions comprising ingredients such as MS salts (Murashige and Skoog, 1962), sucrose, and vitamins B5. Optionally, the following can also be added: amino acids selected from the group comprising proline and asparagine; casein hydrolysate; citric acid; mannitol; copper sulfate; glycine; gelling agent; auxins; antibiotics; acetosyringone; and selection agents. The use of auxins is especially important in embryogenic callus generation, co-cultivation and selection, as well as aminoglycoside antibiotics, preferable geneticin, in the selection medium.

The "co-cultivation" step refers to the incubation of plant tissue that has been infected or contacted with *Agrobacterium* to allow the transfer of *Agrobacterium* T-DNA to plant cells. This stage corresponds to the period from the moment immediately after inoculation (contact of *Agrobacterium* with plant tissue) until the moment the bacterium is removed or inactivated.

Inoculated tissue may be co-cultured for about 1 to 30 days, preferably from 1 to 20 days, or more preferably from 1 to 10 days.

During the co-cultivation step, the temperature may be any suitable temperature known in the art for the target plant. Illustratively, for sugarcane, the temperature may range from about 15 °C to about 30 °C and from about 16 °C to about 29 °C. In some embodiments, the co-cultivation step occurs in the absence of light.

Following co-cultivation with *Agrobacterium,* the medium is removed, and the cells are transferred to a culture medium lacking *Agrobacterium.* The cells are then incubated in the dark at a temperature between about 20 °C and about 26 °C for a period of 1 to 20 days.

The method provided herein further includes selecting cells comprising at least one copy of the genetic sequence of interest. "Select" as used herein means the situation in which a selective agent is used for transformants, wherein said selective agent will allow preferential growth of plant cells containing at least one copy of the marker gene positioned within the T-DNA. Whereas, those cells that were not transformed will not contain the marker gene that permits survival in the selective agent. As indicated above, any suitable selection marker may be used. Preferably, the selection marker gene used is the *nptII* gene, which encodes an enzyme that confers resistance to aminoglycoside antibiotics, such as geneticin.

In some embodiments, an agent that inhibits *Agrobacterium* growth is also added.

Selection may occur under light or dark conditions, depending on the plant species being transformed and, on the genotype, for example. In some cases, embryogenic callus or other tissues undergoing transformation may be subcultured at regular or irregular intervals in the same medium. In the case of callus transformation, individual calluses can be kept separate to ensure that only one plant is regenerated by each callus (thus ensuring that all regenerated plants are derived from independent transformation events). In a preferred embodiment, the selection step occurs in the dark using using geneticin as a selection agent as a selection agent for about 1 to 10 weeks. More preferably the selection step occurs for about 2 to 5 weeks.

After the selection period, plant tissue that has continued to grow in the presence of the selection agent, and has therefore been genetically modified, can be manipulated and regenerated by placing it in suitable culture media and growth conditions. The transgenic plants thus obtained can be tested for the presence of the DNA of interest. For the purpose of this invention, the term "regenerate" refers to the formation of a plant comprising both an aerial part and roots. Regenerated plants can be planted on suitable substrate (such as soil) and transferred to the greenhouse. As used herein, "genetically modified" or "transgenic" or "stably transformed" means a plant cell, plant part, plant tissue, or plant that comprises a DNA sequence of interest that is introduced into its genome by transformation.

In one embodiment, the bacterium is of the genus *Agrobacterium.*

In a more preferred embodiment, the bacterium is *Agrobacterium tumefaciens.*

In an even more preferred embodiment, the bacterium is an *Agrobacterium tumefaciens* strain EHA105.

The present invention also relates to the characterization of the selected event (CTC79005-2) and methods of detecting plant material derived therefrom. Analytical methods for detection and characterization of transgenic plants include indirect methods (protein-based detection methods) or direct methods (DNA-based detection methods).

The definition of the T-DNA stable integration site in the host cell genome and the characterization of its flanking sequences is necessary for the development and validation of methodologies for the unambiguous identification and characterization of the event.

To identify the flanking regions at the ends of the T-DNA insert in event CTC79005-2, several DNA amplification and sequencing experiments were performed. Inverse PCR (iPCR) assays were performed at both ends of the T-DNA to isolate and clone the flanking regions of the insert. Subsequently, the fragments obtained and isolated were sequenced using the Sanger method to validate the results obtained by iPCR. The genetic insertion map present in event CTC79005-2 resulting from the data generated by these experiments is shown in Figure 3 and SEQ ID NO: 2. The flanking sequences of event CTC79005-2 are shown in SEQ ID NO: 23 and SEQ ID NO: 24.

According to one aspect of the invention, a polynucleotide comprising at least 14 contiguous nucleotides of the 26 nucleotide sequence of SEQ ID NO: 18 is provided. In one embodiment, a polynucleotide comprising at least 15 contiguous nucleotides of the 26 nucleotide sequence of SEQ ID NO: 18 is provided. In one embodiment, a polynucleotide comprising at least 16 contiguous nucleotides of the 26 nucleotides of SEQ ID NO: 18 is provided. In one embodiment, said polynucleotide comprises at least 17 contiguous nucleotides of the 26 nucleotides of SEQ ID NO: 18. In one embodiment, said polynucleotide comprises at least 18 contiguous nucleotides of the 26 nucleotide sequence of SEQ ID NO: 18. In one embodiment, said polynucleotide comprises at least 19 contiguous nucleotides of the 26 nucleotide sequence of SEQ ID NO: 18. In one embodiment, said polynucleotide comprises at least 20 contiguous nucleotides of the 26 nucleotide sequence of SEQ ID NO: 18. In one embodiment, said polynucleotide comprises at least 21 contiguous nucleotides of the 26 nucleotide sequence of SEQ ID NO: 18. In one embodiment, said polynucleotide comprises at least 22 contiguous nucleotides of the 26 nucleotide sequence of SEQ ID NO: 18. In one embodiment, said polynucleotide comprises at least 23 contiguous nucleotides of the 26 nucleotide sequence of SEQ ID NO: 18. In one embodiment, said polynucleotide comprises at least 24 contiguous nucleotides of the 26 nucleotide sequence of SEQ ID NO: 18. In one embodiment, said polynucleotide comprises at least 25 contiguous nucleotides of the 26 nucleotide sequence of SEQ ID NO: 18. In one embodiment, said polynucleotide comprises SEQ ID NO: 18. In one further aspect of the invention, said polynucleotide comprises SEQ ID NO: 13.

According to one aspect of the invention, a polynucleotide comprising at least 14 contiguous nucleotides of the 26 nucleotide sequence of SEQ ID NO 19 is provided. In one embodiment, a polynucleotide comprising at least 15 contiguous nucleotides of the 26 nucleotide sequence of SEQ ID NO 19 is provided. According to one aspect of the invention, a polynucleotide comprising at least 16 contiguous nucleotides of the 26 nucleotide sequence of SEQ ID NO 19 is provided. In one embodiment, a polynucleotide comprising at least 17 contiguous nucleotides of the 26 nucleotide sequence of SEQ ID NO: 19 is provided. In one embodiment, a polynucleotide comprising at least 18 contiguous nucleotides of the 26 nucleotide sequence of SEQ ID NO: 19 is provided. In one embodiment, a polynucleotide comprising at least 19 contiguous nucleotides of the 26 nucleotide sequence of SEQ ID NO: 19 is provided. In one embodiment, a polynucleotide comprising at least 20 contiguous nucleotides of the 26 nucleotide sequence of SEQ ID NO: 19 is provided. In one embodiment, a polynucleotide comprising at least 21 contiguous nucleotides of the 26 nucleotide sequence of SEQ ID NO: 19 is provided. In one embodiment, a polynucleotide comprising at least 22 contiguous nucleotides of the 26nucleotide sequence of SEQ ID NO: 19 is provided. In one embodiment, a polynucleotide comprising at least 23 contiguous nucleotides of the 26 nucleotide sequence of SEQ ID NO: 19 is provided. In one embodiment, a polynucleotide comprising at least 24 contiguous nucleotides of the 26 nucleotide sequence of SEQ ID NO: 19 is provided. According to one aspect of the invention, a polynucleotide comprising at least 25 contiguous nucleotides of the 26 nucleotides of SEQ ID NO: 19 is provided. In one embodiment, a polynucleotide comprising SEQ ID NO: 19 is provided. In one aspect of the invention, a polynucleotide comprising SEQ ID NO: 12 is provided.

In a further aspect of the present invention a polynucleotide comprising the sequence of SEQ ID NO: 5 is provided. In still another aspect of the invention, a polynucleotide comprising the sequence SEQ ID NO: 22 is provided.

According to one aspect of the invention, there is provided a plant comprising at least 14 contiguous nucleotides of the 26 nucleotide sequence of SEQ ID NO: 18. In one embodiment, a plant comprising at least 15 contiguous nucleotides of the 26 nucleotide sequence of SEQ ID NO: 18 is provided. According to one aspect of the invention, there is provided a plant comprising at least 16 contiguous nucleotides of the 26 nucleotide sequence of SEQ ID NO: 18. In one embodiment, a plant comprising at least 17 contiguous nucleotides of the 26 nucleotide sequence of SEQ ID NO: 18 is provided. In one embodiment, a plant comprising at least 18 contiguous nucleotides of the 26 nucleotide sequence of SEQ ID NO: 18 is provided. In one embodiment, a plant comprising at least 19 contiguous nucleotides of the 26 nucleotide sequence of SEQ ID NO: 18 is provided. In one embodiment, a plant comprising at least 20 contiguous nucleotides of the 26 nucleotide sequence of SEQ ID NO: 18 is provided. In one embodiment, a plant comprising at least 21 contiguous nucleotides of the 26 nucleotide sequence of SEQ ID NO: 18 is provided. In one embodiment, a plant comprising at least 22 contiguous nucleotides of the 26 nucleotide sequence of SEQ ID NO: 18 is provided. In one embodiment, a plant comprising at least 23 contiguous nucleotides of the 26 nucleotide sequence of SEQ ID NO: 18 is provided. In one embodiment, a plant comprising at least 24 contiguous nucleotides of the 26 nucleotide sequence of SEQ ID NO: 18 is provided. In one embodiment, a plant comprising at least 25 contiguous nucleotides of the 26 nucleotide sequence of SEQ ID NO: 18 is provided. In one embodiment, a plant comprising SEQ ID NO: 18 is provided. In an additional embodiment, a plant comprising SEQ ID NO: 13 is provided.

According to one aspect of the invention, there is provided a plant comprising at least 14 contiguous nucleotides of the 26 nucleotide sequence of SEQ ID NO: 19. In one embodiment, a plant comprising at least 15 contiguous nucleotides of the 26 nucleotide sequence of SEQ ID NO: 19 is provided. In one embodiment, a plant comprising at least 16 contiguous nucleotides of the 26 nucleotide sequence of SEQ ID NO: 19 is provided. In one embodiment, a plant comprising at least 17 contiguous nucleotides of the 26 nucleotide sequence of SEQ ID NO: 19 is provided. In one embodiment, a plant comprising at least 18 contiguous nucleotides of the 26 nucleotide sequence of SEQ ID NO: 19. In one embodiment, a plant comprising at least 19 contiguous nucleotides of the 26 nucleotide sequence of SEQ ID NO: 19 is provided. In one embodiment, a plant comprising at least 20 contiguous nucleotides of the 26 nucleotide sequence of SEQ ID NO: 19 is provided. In one embodiment, a plant comprising at least 21 contiguous nucleotides of the 26 nucleotide sequence of SEQ ID NO: 19 is provided. In one embodiment, a plant comprising at least 22 contiguous nucleotides of the 26 nucleotide sequence of SEQ ID NO: 19 is provided. In one embodiment, a plant comprising at least 23 contiguous nucleotides of the 26 nucleotide sequence of SEQ ID NO: 19 is provided. In one embodiment, a plant comprising at least 24 contiguous nucleotides of the 26 nucleotides of sequence SEQ ID NO: 19 is provided. In one embodiment, a plant comprising at least 24 contiguous nucleotides of the 26 nucleotide sequence of SEQ ID NO: 19 is provided. In one embodiment, a plant comprising at least 25 contiguous nucleotides of the 26 nucleotide sequence of SEQ ID NO: 19 is provided. In one embodiment, a plant comprising SEQ ID NO: 19 is provided. In an additional embodiment, a plant comprising SEQ ID NO: 12 is provided.

In one embodiment of the present invention, said plant is a genetically modified sugarcane (*Saccharum* spp.) plant. Additionally, said plant is insect resistant and comprises the sequence SEQ ID NO: 5. Still in a further aspect, the insect resistant plant of the present invention comprises SEQ ID NO: 22. In a further embodiment, said plant is an insect-resistant sugarcane plant of event CTC79005-2 or a plant derived therefrom.

In one aspect of the invention, event CTC79005-2 is a sugarcane (*Saccharum* spp.) plant comprising SEQ ID NO: 5. In a further aspect, event CTC79005-2 comprises SEQ ID NO: 22.

In other embodiment a specific method for detection and identification of CTC79005-2 event is provided.

According to the present invention, there is provided a method of detecting plant material derived from genetically modified sugarcane of event CTC79005-2 comprising the steps of:

a) obtaining a plant material sample for analysis;

b) extracting DNA from the sample;

c) providing primer pairs comprising at least a forward and a reverse primer;

d) amplifying a region between the primer pairs; and

e) detecting the presence of a product from amplification.

In one embodiment, primer pairs (step c) according to the detection method described are designed to bind to a polynucleotide comprising contiguous nucleotides of SEQ ID NO 2.

In another embodiment, the primer pairs in step c) are designed to bind to a polynucleotide comprising contiguous nucleotides of sequences selected from the group consisting of SEQ ID NO: 22 and SEQ ID NO: 29, wherein at least one pair of primers comprises contiguous nucleotides sequences selected from the group consisting of SEQ ID NO: 23, SEQ ID NO: 24, SEQ ID NO: 30 and SEQ ID NO: 31.

In one embodiment, the primer pairs above (step c) are designed to bind to a polynucleotide comprising at least 14 contiguous nucleotides of sequences selected from the group consisting of SEQ ID NO: 22 and SEQ ID NO:

29, wherein at least one primer pair comprises at least 3 contiguous nucleotides of sequences selected from the group consisting of SEQ ID NO: 23, SEQ ID NO: 24, SEQ ID NO: 30 and SEQ ID NO: 31. In one embodiment, the primer pairs are designed to bind to a polynucleotide comprising at least 14 contiguous nucleotides of sequences selected from the group consisting of SEQ ID NO: 22 and SEQ ID NO: 29, wherein at least one primer pair comprises at least 7 contiguous nucleotides of sequences selected from the group consisting of SEQ ID NO: 23,SEQ ID NO: 24, SEQ ID NO: 30 and SEQ ID NO: 31. In addition, the primer pairs are designed to bind to a polynucleotide comprising at least 14 contiguous nucleotides of sequences selected from the group consisting of SEQ ID NO: 22 and SEQ ID NO: 29, wherein at least one primer pair comprises at least 14 contiguous nucleotides of sequences selected from the group consisting of SEQ ID NO: 23, SEQ ID NO: 24, SEQ ID NO: 30 and SEQ ID NO: 31.

Additionally, primer pairs according to the detection method described are designed to bind to a polynucleotide comprising contiguous nucleotides of sequences selected from the group consisting of SEQ ID NO: 22 and SEQ ID NO: 29, where at least one primer pair consists of a first primer comprising contiguous nucleotides of sequences selected from the group consisting of SEQ ID NO: 23, SEQ ID NO: 24, SEQ ID NO: 30 and SEQ ID NO: 31 and a second primer comprising contiguous nucleotides of sequences selected from the group consisting of SEQ ID NO: 2 and SEQ ID NO: 36.

In one embodiment, the primer pairs according to the detection method described are designed to bind to a polynucleotide comprising at least 14 contiguous nucleotides of sequences selected from the group consisting of SEQ ID NO: 22 and SEQ ID NO: 29, wherein at least one primer pair consists of a first primer comprising at least 3 contiguous nucleotides of sequences selected from the group consisting of SEQ ID NO: 23, SEQ ID NO: 24, SEQ ID NO: 30 and SEQ ID NO: 31 and a second primer comprising at least 3 contiguous nucleotides of sequences selected from the group consisting of SEQ ID NO: 2 and SEQ ID NO: 36. In an additional embodiment, primer pairs are designed to bind to a polynucleotide comprising at least 14 contiguous nucleotides of sequences selected from the group consisting of SEQ ID NO: 22 and SEQ ID NO: 29, wherein at least one primer pair consists of a first primer comprising at least 7 contiguous nucleotides of sequences selected from the group consisting of SEQ ID NO: 23, SEQ ID NO: 24, SEQ ID NO: 30 and SEQ ID NO: 31 and a second primer comprising at least 7 contiguous nucleotides of sequences selected from the group consisting of SEQ ID NO: 2 and SEQ ID NO: 36. In addition, primer pairs are designed to bind to a polynucleotide comprising at least 14 contiguous nucleotides of sequences selected from the group consisting of SEQ ID NO: 22 and SEQ ID NO: 29, wherein at least one primer pair consists of a first primer comprising at least 14 contiguous nucleotides of sequences selected from the group consisting of SEQ ID NO: 23,SEQ ID NO: 24, SEQ ID NO: 30 and SEQ ID NO: 31 and a second primer comprising at least 14 contiguous nucleotides of sequences selected from the group consisting of SEQ ID NO: 2and SEQ ID NO: 36.

In one embodiment, primer pairs according to the detection method described are designed to bind to a polynucleotide comprising contiguous nucleotides of sequences selected from the group consisting of SEQ ID NO: 5 and SEQ ID NO: 37, wherein at least one pair of primers comprises contiguous nucleotides of sequences selected from the group consisting of SEQ ID NO: 3, SEQ ID NO: 4, SEQ ID NO: 38 and SEQ ID NO: 39. In one embodiment, primer pairs according to the detection method described are designed to bind to a polynucleotide comprising at least 14 contiguous nucleotides of sequences selected from the group consisting of SEQ ID NO: 5 and SEQ ID NO: 37, wherein at least one primer pair comprises at least 3 contiguous nucleotides of sequences selected from the group consisting of SEQ ID NO: 3, SEQ ID NO: 4, SEQ ID NO: 38 and SEQ ID NO: 39. In one embodiment, primer pairs are designed to bind to a polynucleotide comprising at least 14 contiguous nucleotides of sequences selected from the group consisting of SEQ ID NO: 5 and SEQ ID NO: 37, wherein at least one primer pair comprises at least 7 contiguous nucleotides of sequences selected from the group consisting of SEQ ID NO: 3, SEQ ID NO: 4, SEQ ID NO: 38 and SEQ ID NO: 39. In addition, primer pairs are designed to bind to a polynucleotide comprising at least 14 contiguous nucleotides of sequences selected from the group consisting of SEQ ID NO: 5 and SEQ ID NO: 37, wherein at least one primer pair comprises at least 14 contiguous nucleotides of sequences selected from the group consisting of SEQ ID NO: 3, SEQ ID NO: 4, SEQ ID NO: 38 and SEQ ID NO: 39.

In one embodiment, primer pairs according to the detection method described are designed to bind to a polynucleotide comprising contiguous nucleotides of sequences selected from the group consisting of SEQ ID NO: 5 and SEQ ID NO: 37, wherein at least one primer pair consists of a first primer comprising contiguous nucleotides of sequences selected from the group consisting of SEQ ID NO: 3, SEQ ID NO: 4, SEQ ID NO: 38 and SEQ ID NO: 39 and a second primer comprising contiguous nucleotides of sequences selected from the group consisting of SEQ ID NO: 2 and SEQ ID NO: 36. In one embodiment, primer pairs, according to the detection method described, are designed to bind to a polynucleotide comprising at least 14 contiguous nucleotides of sequences selected from the group consisting of SEQ ID NO: 5 and SEQ ID NO: 37, wherein at least one primer pair consists of a first primer comprising at least 3 contiguous nucleotides of sequences selected from the group consisting of SEQ ID NO: 3, SEQ ID NO: 4, SEQ ID NO: 38 and SEQ ID NO: 39 and a second primer comprising at least 3 contiguous nucleotides of sequences selected from the group consisting of SEQ ID NO: 2 and SEQ ID NO: 36. In one embodiment, primer pairs are designed to bind to a polynucleotide comprising at least 14 contiguous nucleotides of sequences selected from the group consisting of SEQ ID NO: 2, SEQ ID NO: 5, SEQ ID NO: 36 and SEQ ID NO: 37, wherein at least one primer pair consists of a first primer comprising at least 7 contiguous nucleotides of sequences selected from the group consisting of SEQ ID NO: 3, SEQ ID NO: 4 SEQ ID NO: 38 and SEQ ID NO: 39 and a second primer comprising at least 7 contiguous nucleotides of sequences selected from the group consisting of SEQ ID NO: 2 and SEQ ID NO 36. Additionally, primer pairs are designed to bind to a polynucleotide comprising at least 14 contiguous nucleotides of sequences selected from the group consisting of SEQ ID NO: 5 and SEQ ID NO: 37, wherein at least one primer pair consists of a first primer comprising at least 14 contiguous nucleotides of sequences selected from the group consisting of SEQ ID NO: 3, SEQ ID NO: 4, SEQ ID NO: 38 and SEQ ID NO: 39 and a second primer comprising at least 14 contiguous nucleotides of sequences selected from the group consisting of SEQ ID NO: 2 and SEQ ID NO: 36.

It is well known, especially to those skilled in the art, that the DNA molecule (or DNA) is made up of two strands (of nucleotides) that are held together by hydrogen bridges between the nucleotide bases. Pairing occurs according to the complementarity of the bases, following the general rule of: Adenine with Thymine and Cytosine with Guanine. Thus, although representation of nucleotide sequences is performed for only one of the strands, their complementary strand or complementary sequence is included within the scope of this invention and is considered for the definition of primers and probes described herein.

Methods for obtaining samples for DNA extraction are widely known to one of ordinary skill in the art and include the collection of any plant material derived from the CTC79005-2 transgenic event such as stalks, roots, and leaves. Preferably, the samples are obtained from intact leaves. Plant DNA extraction methods include, without limitation, those based on the use of CTAB detergent (Alianabi et al., 1999), (optionally) followed by further sample purification with cesium chloride or ammonium acetate, as well as other commercially available methods.

Primer pairs suitable for use in this detection method may be designed using parameters well known to those skilled in the art of molecular biology now that SEQs ID Nos: 2, 3, 4, 5, 22, 23, 24, 29, 30, 31, 36, 37, 38 and 39 have become available. For example, one or both primers of the pair may be designed to be construct-specific, trait gene-specific, promoter-specific, sequence-specific to the junction between inserted DNA and genomic DNA, and/or flanking sequence-specific.

There are many amplification methods that can be used in accordance with this aspect of the invention. One of the most common amplification techniques known to those skilled in the art, is the polymerase chain reaction (PCR). The amplification product of a PCR reaction can be visualized by staining the nucleotide chain with a fluorescent tag such as ethidium bromide and then exciting it with UV light (typically after size separation using agarose gel electrophoresis).

One embodiment of the present invention employs variations of the PCR principle such as quantitative real-time PCR, nested PCR, inverse PCR (iPCR), digital PCR, Long PCR, Touchdown PCR, Hot Start PCR, Multiplex PCR, among others. The amplification product can also be detected by different methodologies which are contemplated in the present invention, such as the SYBR Green™ system which emits fluorescence when this reagent binds to double stranded DNA and the Taqman® system where detection is based on the interaction of fluorescent probes. The Taqman® methodology uses a probe that is complementary to the intended PCR product segment located between the reaction primers. During the hybridization stage of the PCR cycle the probe is bound to the target DNA, and during Taq polymerase extension, through its 5'-exonuclease activity, it removes the probe, releasing the fluorochrome and emitting fluorescence. Additional embodiments of this aspect of the present invention include, but are not limited to: loop-mediated isothermal amplification (LAMP), capillary gel electrophoresis (CGE), microarray technology Luminex, "DNA walking" and Next Generation Sequencing (NGS), Sanger method, Illumina, among others.

The present invention describes a specific detection methodology based on the quantitative real-time PCR (qPCR) technique known as "Plus-Minus" or "Presence - Absence," presenting two variations of the methodology: SYBR GREEN ™ and Taqman ® technology.

In one embodiment of the present invention, primer pairs are provided wherein the forward primer consists of SEQ ID NO: 6 and the reverse primer consists of SEQ ID NO: 7 and/or the forward primer is SEQ ID. NO: 8 and the reverse primer is SEQ ID NO: 9.

In an additional embodiment, the primer pairs used in step c) of the method of detecting plant material from genetically modified sugarcane of event CTC79005-2 comprise forward primer consists of SEQ ID NO: 6 and the reverse primer consists of SEQ ID NO: 7 and/or the forward primer is SEQ ID NO: 8 and the reverse primer is SEQ ID NO: 9. In addition, the amplicon (product from amplification) produced by the primers of SEQ ID NO: 6 and SEQ ID NO: 7 is viewed through a labeled probe of SEQ ID NO: 10. Alternatively, the amplicon produced by the primers of SEQ ID NO: 8 and SEQ ID NO: 9 is visualized through a labeled probe of SEQ ID NO 11. Thus, it is an aspect of the present invention that detection of the of the product from amplication obtained by the use of primers SEQ ID NO: 6 and SEQ ID NO: 7 and/or SEQ ID NO: 8 and SEQ ID NO: 9 is performed through hybridization of a probe comprising SEQ ID NO: 10 or SEQ ID NO: 11.

In one embodiment of the present invention, the region amplified by said method (the amplicon or product from amplification) is between 80 and 1000 base pairs in length. In an additional embodiment, the amplicon is between 100 and 300 base pairs in length. In one preferred embodiment, the amplicon obtained using the primers SEQ ID NO: 6 and SEQ ID NO: 7 is 126 base pairs in length, as defined by SEQ ID NO: 12. In another preferred embodiment, the amplicon obtained through the use of primers SEQ ID NO: 8 and SEQ ID NO: 9 is 104 base pairs in length, as defined by SEQ ID NO: 13.

Figures 6 (event-specific detection reaction of the invention via Taqman®) and 7 (SYBR GREEN™ assay) represent the validation of both Methods.

Primers and probes described in the present invention may be used in combination to detect the CTC79005-2 event. Thus, a further embodiment of the present invention involves the use of multiplex PCR to identify plant material from the CTC79005-2 event.

Alternative primers and probes for the detection and characterization of the CTC79005-2 event are included in the invention. These and other variations may be used with but are not limited to any of the direct detection methods described above.

Additionally, the CTC79005-2 event can be detected from plant material by hybridizing DNA samples to the probes. Specifically, the present invention describes a method of detecting material from the genetically modified sugarcane event CTC79005-2 which comprises the steps of:

a) obtaining a plant material sample for analysis;

b) DNA or RNA extraction from the sample;

c) providing a probe or a combination of probes designed to bind to a polynucleotide comprising contiguous nucleotides of sequences selected from the group consisting of SEQ ID NO 2, SEQ ID NO 3, SEQ ID NO 4, SEQ ID NO 5, SEQ ID NO 12, SEQ ID NO 13, SEQ ID NO: 18, SEQ ID NO: 19, SEQ ID NO 22, SEQ ID NO 23, SEQ ID NO 24, SEQ ID NO 29, SEQ ID NO 30, SEQ ID NO 31, SEQ ID NO: 32, SEQ ID NO: 33, SEQ ID NO 36, SEQ ID NO 37, SEQ ID NO 38 and SEQ ID NO 39, wherein the polynucleotide is single stranded;

d) hybridizing said probe or a combination of probes with the sample, and

e) detecting the actual hybridization of the probe or a combination of probes.

In preferred embodiment, the present invention describes a method of detecting material from the genetically modified sugarcane event CTC79005-2 which comprises the steps of:

a) obtaining a plant material sample for analysis;

b) DNA or RNA extraction from the sample;

c) providing a probe designed to bind to a polynucleotide comprising 14 or more contiguous nucleotides of sequences selected from the group consisting of SEQ ID NO: 18, SEQ ID NO: 19, SEQ ID NO: 32 and SEQ ID NO: 33, when the polynucleotide is single stranded;

d) hybridizing said probe with the sample, and

e) detecting the actual hybridization of the probe.

According to one aspect of the invention, a probe designed to bind to a polynucleotide comprising at least 15 contiguous nucleotides of sequences selected from the group consisting of SEQ ID NO: 18, SEQ ID NO: 19, SEQ ID NO: 32 and SEQ ID NO: 33 is provided. In one embodiment, a probe designed to bind to a polynucleotide comprising at least 16 contiguous nucleotides of sequences selected from the group consisting of SEQ ID NO: 18, SEQ ID NO: 19, SEQ ID NO: 32 and SEQ ID NO: 33 is provided. According to one aspect of the invention, a probe designed to bind to a polynucleotide comprising at least 17 contiguous nucleotides of sequences selected from the group consisting of SEQ ID NO: 18, SEQ ID NO: 19, SEQ ID NO: 32 and SEQ ID NO: 33 is provided. In one embodiment, said polynucleotide comprises at least 18 contiguous nucleotides of sequences selected from the group consisting of SEQ ID NO: 18, SEQ ID NO: 19, SEQ ID NO: 32 and SEQ ID NO: 33. In one embodiment, said polynucleotide comprises at least 19 contiguous nucleotides of sequences selected from the group consisting of SEQ ID NO: 18, SEQ ID NO: 19, SEQ ID NO: 32 and SEQ ID NO: 33. In one embodiment, said polynucleotide comprises at least 19 contiguous nucleotides of sequences selected from the group consisting of SEQ ID NO: 18, SEQ ID NO: 19, SEQ ID NO : 32 and SEQ ID NO: 33. In one embodiment, said polynucleotide comprises at least 20 contiguous nucleotides of sequences selected from the group consisting of SEQ ID NO: 18, SEQ ID NO: 19, SEQ ID NO: 32 and SEQ ID NO: 33. In one embodiment, said polynucleotide comprises at least 21 contiguous nucleotides of sequences selected from the group consisting of SEQ ID NO: 18, SEQ ID NO: 19, SEQ ID NO: 32 and SEQ ID NO: 33. In one embodiment, said polynucleotide comprises at least 22 contiguous nucleotides of sequences selected from the group consisting of SEQ ID NO: 18, SEQ ID NO: 19, SEQ ID NO: 32 and SEQ ID NO: 33. In one embodiment, said polynucleotide comprises at least 23 contiguous nucleotides of sequences selected from the group consisting of SEQ ID NO: 18, SEQ ID NO: 19, SEQ ID NO: 32 and SEQ ID NO: 33. In one embodiment, said polynucleotide comprises at least 24 contiguous nucleotides of sequences selected from the group consisting of SEQ ID NO: 18, SEQ ID NO: 19, SEQ ID NO: 32 and SEQ ID NO: 33. According to one aspect of the invention, a polynucleotide comprising at least 25 contiguous nucleotides of sequences selected from the group consisting of SEQ ID NO: 18, SEQ ID NO: 19, SEQ ID NO: 32 and SEQ ID NO: 33 is provided. According to one aspect of the invention, a polynucleotide comprising at least 26 contiguous nucleotides of sequences selected from the group consisting of SEQ ID NO: 18, SEQ ID NO: 19, SEQ ID NO: 32 and SEQ ID NO: 33 is provided.

The probe may be, for example, a PCR product or a restriction digest fragment. In a further embodiment, the probe as described herein may be labeled with a fluorescent, radioactive, enzymatic, or other label suitable to enable hybridization to be detected. The person skilled in the art will now know how to design suitable probes given the advantage of the present disclosure.

In an additional embodiment, a probe hybridization method is provided to the sample under stringent conditions (high specificity). Stringent hybridization conditions are well known to those skilled in the art. Examples of stringent conditions include: hybridization at a temperature of approximately 65 °C in a solution containing 6x SSC, 0.01% SDS and 0.25% skimmed milk powder followed by washing at the same temperature in a solution containing 0.2 x SSC and 0.1% SDS.

Suitable techniques for detecting plant material derived from event CTC79005-2 based on the hybridization principle include, but are not limited to, Southern blots and *in situ* hybridization. One of skill in the art is familiar with such techniques.

Typically, these techniques involve incubating a probe with a sample, washing to remove the unbound probe, and detecting whether the probe has hybridized. Said detection method is dependent upon the type of label attached to the probe. For example, a radio-labelled probe can be detected by exposure to and development of X-ray film. Alternatively, an enzymatically labeled probe may be detected by converting a substrate to affect a color change.

Additionally, another aspect of the invention contemplates a method for detecting plant material derived from event CTC79005-2, which comprises: obtaining a sample for analysis; providing an antibody designed to bind to a Cry or NptII protein contained within a plant comprising at least 14 contiguous nucleotides of SEQ ID NO: 18 and/or SEQ ID NO: 19; incubating said antibody with the sample; and detecting whether the antibody bound. In one embodiment of the present invention, said Cry protein is encoded by nucleotide sequence SEQ ID NO: 20 and said NptII protein is encoded by nucleotide sequence SEQ ID NO: 21. In an additional embodiment, said Cry protein comprises SEQ ID NO: 34 and said NptII protein comprises SEQ ID NO: 35.

Suitable methods for detecting plant material derived from the CTC79005 -2 event based on said antibody binding include (but are not limited to): western blots, ELISA (Enzyme-Linked ImmunoSorbent Assays), and mass spectrometry (e.g. surface-enhanced laser desorption/ionization (SELDI)). One of skill in the art is familiar with these immunological techniques. Typical steps include incubating a sample with an antibody that binds to the Cry or NptII protein, washing for removal of unbound antibody, and detecting whether the antibody has bound. Many such detection methods are based on enzymatic reactions: for example, the antibody may be linked with an enzyme such as peroxidase and upon application of a suitable substrate, a color change is detected. Such antibodies may be monoclonal or polyclonal.

Another aspect the invention contemplates a method for detecting plant material derived from event CTC79005-2, which comprises: obtaining a sample for analysis; providing a protein extract from the sample; providing test strips designed to detect the presence of a Cry or NptII protein in a plant comprising at least 14 contiguous nucleotides of SEQ ID NO: 18 and/or SEQ ID NO: 19; incubating the test strips with the sample; and detecting. In one embodiment of the present invention, said Cry protein is encoded by nucleotide sequence SEQ ID NO: 20 and the NptII protein is encoded by nucleotide sequence SEQ ID NO: 21. In an additional embodiment, said Cry protein comprises SEQ ID NO: 34 and said NptII protein comprises SEQ ID NO: 35.

In one embodiment of the invention there is provided a method for detecting plant material derived from the CTC79005-2 event, said method comprising: obtaining a sample derived from the CTC79005-2 event and a sample from a non-transgenic sugarcane variety for analysis (control); subjecting one or more insects of the species *Diatraea saccharallis* (susceptible to Cry1Ac) to the samples; detecting an insecticidal effect on the insects. In this aspect of the invention, "insecticide" refers to any inhibitory effect on the insect (including but not limited to): reduced feeding, retarded growth, reduced fecundity, paralysis, and death.

The method of detecting plant material from event CTC79005-2 includes, but is not limited to, biological leaf feeding assays where a leaf or other suitable part of the plant of event CTC79005-2, or any plant material derived from event CTC79005-2, is infested with one or more insect pests. Measurement of said detection can include: assessing leaf or plant damage after adjusted time periods, assessing mortality or assessing other insecticidal effects. Such biological assays may be performed in the field or greenhouses and may entail either natural or artificial insect infestation.

In another aspect of the invention, a kit for detecting in a plant sample the presence of event CTC79005-2 is provided, said kit comprising: a means for detecting the presence of a polynucleotide comprising at least 14 contiguous nucleotides of the sequence of SEQ ID NO: 18 and/or SEQ ID NO: 19 and/or a pesticidal crystal protein (Cry). In one embodiment of the present invention, said kit may comprise DNA amplification detection technology such as PCR, qPCR, or Taqman®. In a further embodiment of the present invention, said kit may comprise probe hybridization detection technology such as Southern Blots or *in situ* hybridization. In one aspect, the means to detect material from transgenic sugarcane comprising a Cry1Ac protein (event CTC79005-2) comprises primer pairs designed to bind to a polynucleotide comprising contiguous nucleotides of sequences selected from the group consisting of SEQ ID NO: 22 and SEQ ID NO: 29, wherein at least one pair of primers comprises contiguous nucleotides sequences selected from the group consisting of SEQ ID NO: 23, SEQ ID NO: 24, SEQ ID NO: 30 and SEQ ID NO: 31. Additionally, the means comprise primer pairs, wherein the forward primer comprises SEQ ID NO: 6 and the reverse primer comprises SEQ ID NO: 7, or the forward primer comprises SEQ ID NO: 8 and the reverse primer comprises SEQ ID NO: 9. In a further embodiment, the means to detect material from transgenic sugarcane comprising a CrylAc protein (event CTC79005-2) comprises a probe comprising SEQ ID NO: 10 or SEQ ID NO: 11. In another embodiment of the present invention, said kit may comprise antibody binding detection technology such as western blots, ELISAs, mass spectrometry (SELDI) or test strips. In a further embodiment of the present invention, said kit may comprise detection technology by biological insect testing such as leaf feeding biological assays or biological mortality assays. In a further embodiment of the present invention, said kit may comprise any combination of the detection technologies mentioned above.

The transgenic event as described in the present invention affect insects of one or more species of the group comprising insects of the order Lepidoptera. As a result, a reduced number of insecticidal sprays is required during cultivation of said plant compared with a non-transgenic sugarcane plant of the same variety.

The present invention is not itself bound to event CTC79005-2; rather, it is further extended to include any plant material derived therefrom, including seed, provided they contain at least one of the polynucleotides sequences of the present invention. In one embodiment, the present invention comprises a plant part, plant cell, plant tissue, or seed from the genetically modified sugarcane (*Saccharum* spp.) plant, wherein said plant part, plant cell, plant tissue, or seed comprises SEQ ID NO: 18 or SEQ ID NO: 19. In other embodiment, the invention contemplates plant part, plant cell, plant tissue, or seed comprising SEQ ID NO: 12 or SEQ ID NO: 13. Additionally, the invention includes a plant part, plant cell, plant tissue, or seed comprising SEQ ID NO: 5 or SEQ ID NO: 22. The present invention also includes, but is not limited to, plants that are derived from crossbred lineages with the CTC79005-2 event or a derivative thereof by conventional or other crossbreeding methods; thus, one embodiment of the present invention relates to the use of a plant, plant cell, plant part, or seed from the genetically modified sugarcane (*Saccharum* spp.) plant as described herein, which is used for regenerating a plant, planting, cultivating a field of plants, or producing a plant product. The present invention also contemplates a tissue culture of a genetically modified sugarcane (*Saccharum* spp.) plant comprising SEQ ID NO: 18 or SEQ ID NO: 19. In other embodiment, the invention includes a tissue culture of a genetically modified sugarcane (*Saccharum* spp.) plant comprising SEQ ID NO: 12 or SEQ ID NO: 13. Also contemplate in the present invention is a tissue culture of a genetically modified sugarcane (*Saccharum* spp.) plant comprising SEQ ID NO: 5 or SEQ ID NO: 22. Additionally, a genetic modified sugarcane (*Saccharum* spp.) plant regenerated from the tissue culture describe above is also included in the present invention, wherein the regenerate plant comprises SEQ ID NO: 18 or SEQ ID NO: 19. Examples of plant cells and plant parts include but are not limited to: suspension cells, callus, somatic embryos, meristematic tissue, top stalks, stalks, leaf, leaf discs, tiller, shoots. Another aspect contemplates a method for producing an insect-resistant sugarcane (*Saccharum* spp.) plant, comprising crossing a first sugarcane plant with a second sugar cane plant, wherein the second sugar cane plant is a plant comprising event CTC79005-2, and producing offspring sugarcane plants therefrom. The plant comprising event CTC79005-2 is a genetically modified sugarcane (*Saccharum* spp.) plant comprising SEQ ID NO: 18 or SEQ ID NO: 19. The present invention also contemplates a sugarcane (*Saccharum* spp.) plant and plant parts, plant cells, plant tissues, or seeds therefrom produced by the method for producing an insect-resistant sugarcane described above.

In a further embodiment, the present invention provides a commodity product, produced from a sugarcane plant comprising event CTC79005-2. Thus, the invention includes a commodity product, produced from a genetically modified sugarcane (*Saccharum* spp.) plant comprising SEQ ID NO: 18 or SEQ ID NO: 19. In one embodiment, the invention contemplates a commodity product, produced from a genetically modified sugarcane (*Saccharum* spp.) plant comprising SEQ ID NO: 12 or SEQ ID NO: 13. Additionally, the invention includes a commodity product, produced from a genetically modified sugarcane (*Saccharum* spp.) plant comprising SEQ ID NO: 5 or SEQ ID NO: 22. Examples of commodity products include but are not limited to: bagasse, sugarcane juice, syrup, first generation ethanol (produced from sugarcane juice), second generation ethanol (cellulosic ethanol; produced from biomass), biomass, sugar, raw sugar, refined sugar, molasses, vinasse, and fiber.

The present invention further provides a plant material from CTC79005-2 event comprising additional polynucleotide sequences, modified or smaller than CTC79005-2, or exhibit other phenotypic characteristics. For example, the plant material CTC79005-2 event could be transformed to produce a new event comprising additional characteristics, such as, a second insect resistance gene. This process is known as gene stacking. Such second insect resistance gene codes, for example, insecticidal lectins, insecticidal protease inhibitors and other insecticidal proteins derived from *Bacillus thuringiensis.*

The present invention further provides an insect control method comprising providing plant material derived from the CTC79005-2 event at a location where said insects feed. The invention further provides an insect control method comprising providing the CTC79005-2 derived plant material at the site where said insects feed and applying other agrochemical reagents to said plant material (e.g. herbicides, fungicides, and the like).

In other embodiment, the invention describes, a method of making a genetically modified sugarcane (*Saccharum* spp.) plant of event CTC79005-2, comprising introducing a genetic modification to a sugarcane (*Saccharum* spp.) plant comprising SEQ ID NO: 5 or SEQ ID NO: 22 to produce a genetically modified sugarcane (*Saccharum* spp.) plant of event CTC79005-2, wherein the genetically modified sugarcane (*Saccharum* spp.) plant has improved insect resistance as compared to a sugarcane (*Saccharum* spp.) plant without the genetic modification. In one additional embodiment, the invention provides a method of cultivating a genetically modified sugarcane (*Saccharum* spp.) plant of event CTC79005-2, comprising growing a genetically modified sugarcane (*Saccharum* spp.) plant of event CTC79005-2 comprising SEQ ID NO: 5 or SEQ ID NO: 22 under conditions comprising insect infestation, wherein the genetically modified sugarcane (*Saccharum* spp.) plant has an increase in insect resistance as compared to a sugarcane (*Saccharum* spp.) plant without the genetic modification grown under the same conditions. The invention also provides, a genetically modified sugarcane (*Saccharum* spp.) plant of event CTC79005-2 comprising SEQ ID NO: 5 or SEQ ID NO: 22.

Description of Transgenic Sugarcane 'CTC79005-2'

Transgenic hybrid sugarcane 'CTC79005-2' plants are genetically substantially equal and phenotypically similar to the recipient (host) of the recombinant molecule, the parental plant variety 'RB92579', a commercial Brazilian sugarcane variety (Plant Variety Protection/PVP - protocol number - 21806.000439/2000-45; PVP Certificate, № 480, granted at 23/07/2003), but with a new and particular feature (CrylAc expression), which guarantees the insect resistance to sugarcane borer *D. saccharalis* (Lepidoptera). As its parental variety 'RB92579', 'CTC79005-2' is a modern sugarcane hybrid that holds several desirable agronomic characteristics such as high productivity, genetic potential for high ratoon cane sprouting vigor, high cane yield, excellent ratooning, great recovery from dry periods, highly responsive to irrigation and good sucrose content. 'CTC79005-2'event, besides insect resistance, is also tolerant to leaf scald, brown and orange rust diseases, and moderate resistant to smut.

The average mature stalk height (measured at 330 Harvest Day after Planting - DAP, except for Juazeiro, measured at 210 DAP; measured from the crown until the insertion of leaf +1), stalk diameter (measured at 330 Harvest Day after Planting - DAP, expect for Juazeiro, measured at 210 DAP), number of tillers (at 30, 60, 90, 120, 150, 180, 210, 240, 270, 300 and 330 DAP; not measured in Juazeiro-BA), weight (measured at 330 DAP, expect for Juazeiro, measured at 210 DAP), sugar content (BRIX %; measured at 330 DAP, expect for Juazeiro, measured at 210 DAP; extracted juice), flowering (observations during all developmental cycle until 330 DAP, expect for Juazeiro, data collected until 210 DAP) and tons of pol% (% of sucrose in the sugarcane juice) per hectare (TPH; measured at 330 Harvest Day after Planting - DAP, expect for Juazeiro, measured at 210 DAP) were evaluate in six different locations in comparison with the parental variety 'RB92579'. TPH is calculated according to the formulae:

$$TPH = (Pol\,\%\,Cana\,x\,TCH)/100$$

For each data set, data across all sites were combined for statistical analysis. Combined site analysis was done using the following statistical model:

$$y_{ijk} = \mu + G_k + (S)_i + B(S)_{ij} + (SG)_{ik} + \varepsilon_{ijk},$$

,wherein $y_{ijk}$ is the measurement of replicate *j* on site *i* for treatment *k*; $\mu$ is the overall mean; $S_i$ is the effect of site *i* (*i* = 1 to 6); $B_j$ is the effect of replicate *j* (j = 1 to 4); $B(S)_{ij}$ is the effect of replicate *j* on site *i* (j = 1 to 4); $G_k$ is the effect of the treatment *k* (k = 1 to 7); $(SG)_{ik}$ is the interaction between site *i* and treatment *k;* $\varepsilon_{ijk}$ is the experimental residual error.

The main effects analysis and model interaction were performed as described by Kuznetsova et al. (2017). All data were analyzed using mixed linear model by package lme4 (Bates et al., 2015).

The results of the agronomic and phenotypic characteristics analysis are shown in the table below and corroborate the conclusion that 'CTC79005-2 is similar to its parental variety ('RB92579').

Table 01. Comparison of means for phenotypic and agronomic characteristics between CTC79005-2 and its parental variety RB92579. Combined analysis for Barrinha-SP, Piracicaba-SP, Valparaíso-SP, Quirinópolis-GO, Mandaguaçu-PR and Juazeiro-BA. Data from Juazeiro-BA collected at 210 DAP and other locations at 330 DAP.

| | Parameter | Mean | | SE | Range** | |
|---|---|---|---|---|---|---|
| | | CTC79005 | RB92579 | | Min | Max |
| Combined analysis | Height (m) | 2.72 | 2.6 | 0.2 | 1.71 | 3.66 |
| | Diameter (cm) | 23.26 * | 28 | 0.61 | 22.88 | 34.14 |
| | Tillers (330 DAP) † | 75.45 | 67.57 | 14.26 | 30.25 | 144 |
| | Weight (Kg) | 10.69 * | 15.04 | 1.19 | 7.87 | 23.7 |
| | BRIX (%) | 13.38 * | 14.79 | 0.54 | 13.56 | 20.27 |
| | TPH | 4.84 * | 7.14 | 1.35 | 7.63 | 10.13 |
| | Flowering (Total No. Panicles) | 0 | 0 | - | 0 | 0 |

* Significant difference between the transgenic event and the parental variety by the t test at the level of 5% ($p \leq 0.05$). EP: Standard Error; **Range: Average values minimum and maximum observed in 3 commercial reference varieties. † Not evaluated in Juazeiro.

## Examples

Example 1. CTC79005-2 event Generation - *Agrobacterium* transformation

Event CTC79005-2 was obtained by *Agrobacterium tumefasciens-mediated* genetic transformation of the RB92579 cultivar.

The RB 92579 cultivar is a commercial hybrid and is the donor genotype of the CTC79005-2 event (genetic background); that is, it represents the untransformed counterpart of the CTC79005-2 event. This cultivar has medium-late maturation and has been planting specially on Brazilian Northeast region. As with other commercial sugarcane hybrids, it is high-ploidy material with numerous chromosomes derived from its two parental varieties: S. *officinarum* and *S. spontaneum* (DANIELS and ROACH, 1987; SREENIVASAN et al., 1987).

CTC79005-2 event has the *cry1Ac* gene, which expresses a toxin to control *D. saccharalis,* and the *nptII* gene, used as a selection marker during the process of genetic modification. The expression of the *cry1Ac* and *nptII* genes is regulated by the maize ubiquitin gene promoter UBI-1, which has an endogenous intron. Both expression cassettes use the *Agrobacterium tumefaciens* nopaline synthase (nos) terminator.

1.1 Construct development using *cry1Ac* and *nptII* genes (Figure 5; SEQ ID NO 14).

Conventional gene cloning techniques using commercial bacterial plasmids, restriction enzyme digestion, and fragment ligation (with ligases) were used to develop the construct of the present invention (Figure 5).

The construct of the present invention was developed by joining the UBI-*cry1Ac*-NOS and UBI-*nptII*-NOS cassettes. T-DNA containing both cassettes was transferred from a cloning plasmid to the base plasmid (Figure 4: binary plasmid vector, which contains in its host spectrum the bacteria *Escherichia coli* and *Agrobacterium tumefaciens*) using restriction enzymes, generating the construct of the present invention (Figure 5; SEQ ID NO: 14).

After the final cloning step, the construct (SEQ ID NO: 14) was inserted into *Escherichia coli* strain DH5$\alpha$ using heat shock. An isolated colony containing the construct was inoculated into liquid LB medium supplemented with 150 $\mu$g/ml spectinomycin and incubated at 37 ° C while shaking at 250 rpm for a period of 16 hours. Stocks were then prepared containing bacterial suspension and 10% (v/v) glycerol, which were stored in an ultrafreezer at -80 °C.

The construct of the present invention was then transferred from *E. coli* to *Agrobacterium tumefaciens* strain EHA105 by isolation and purification of plasmid DNA and transformation of *Agrobacterium* by electroporation. As with the *E. coli* strain, stocks containing the bacterial suspension of *Agrobacterium* and 10% (v/v) glycerol were stored in an ultrafreezer at -80 °C.

1.2 *Agrobacterium*-mediated plant transformation

To obtain embryogenic callus, young RB92579 sugarcane leaf rolls, grown in the field or greenhouse for up to 12 months, were collected for isolation of the initial explants.

After surface disinfection, transverse sections about 0.05-5mm thick were cut from above the meristem under aseptic conditions. The sections were placed on the surface of the callus induction culture medium [MS - Murashige and Skoog, 1962; sucrose, vitamins B5, amino acids selected from the group comprising proline, casein hydrolyzate, citric acid, mannitol, copper sulfate, glycine, gelling agent, 2,4D]. The cultures were kept in the dark at 26 $\pm$ 2 °C and sub-cultured every 15 days for three to five cycles of 7-28 days each. One week before transformation, calli were again selected for embryogenic characteristics (nodular, compact, opaque and slightly yellowish).

*Agrobacterium* culture, comprising strain EHA105 transformed with the construct of the present invention, was started from a glycerol stock and kept in the dark at 28 °C for two to three days. The *Agrobacterium* suspension to infect plant material was prepared by resuspending the culture in MS liquid medium plus acetosyringone, adjusting to a final $OD_{600}$ of 0.1-1.0 (MS salts, sucrose, and vitamins B5).

The calli with embryogenic characteristics were visually selected and directly transferred to the *Agrobacterium* suspension, where they remained for 30 minutes in the dark with constant agitation at 50 rpm.

After this period, calli were separated from the *Agrobacterium* suspension and excess suspension was removed. Next, calli were cultured for 1-5 days in semi-solid (MS salts, sucrose, vitamins B5, citric acid, gelling agent, 2,4D and acetosyringone) at 22 °C in the dark.

After co-cultivation, callus was transferred to DT rest medium (MS salts; sucrose, B5 vitamins, amino acids selected from the group comprising proline and asparagine, casein hydrolyzate, citric acid, copper sulfate, glycine, gelling agent, 2,4D, timentin) and kept for 5-14 days at 26 °C in the dark.

Transformed cells were selected by successive sub-cultures in selection culture medium containing phytoregulators and the selective agent aminoglycoside antibiotics. (Selection medium with geneticin: MS salts, sucrose, vitamins B5, amino acids selected from the group comprising proline and asparagine, casein hydrolyzate, copper sulfate, glycine, gelling agent, 2,4D, timentin) The calli remained in this condition for 21 days at 26 °C in the dark, then the calli were transferred to the regeneration medium (equivalent to selection medium without 2,4D) and then to elongation medium (MS salts, sucrose, B5 vitamins, casein hydrolyzate, gelling agent, timentin). The calli were exposed to a 16-hour photoperiod at 4,000 lux in the presence of the selective agent, then they were multiplied, rooted, and acclimatized before transfer to the greenhouse. This process was used to generate the clone that eventually created the event CTC79005-2.

Example 2. Molecular Characterization of Event CTC79005-2

2.1 DNA extraction.

Approximately 10 mg of leaf tissue from event CTC79005-2 was used. Genomic DNA extraction was performed on the BioSprint 96 Nucleic Acid Extractor (Quiagen, GER) with the BioSprint 96 DNA Plant Kit Extraction Kit (Quiagen, GER) according to the manufacturer's instructions. The DNA was normalized to a concentration of 10 ng/$\mu$L in a Multiskan GO spectrometer (Thermo Scientific, USA).

2.2 Determination of the number of transgene copies inserted into the host plant germplasm.

The copy number of *cry1Ac* and *nptII* genes inserted into CTC79005-2 event was initially evaluated by quantitative Taqman® PCR (qPCR/Taqman®), and the results were confirmed via Southern blot and/or sequencing.

The Taqman® real time PCR reactions were realized with 7500 Real-Time PCR System (Applied Biosystems, USA) in the Fast mode. The primer pairs and probes used are shown at Table 02. As endogenous control of the *cry1Ac* and *nptII* reactions to confirm the presence and quality of the used DNA, as well as, effectiveness of the reaction, it was used the sugarcane polyubiquitin gene (forward primer: 5' ACCATTACCCTGGAGGTTGAGA 3' (SEQ ID No: 15); reverse primer: 5' GTCCTGGATCTTCGCCTTCA 3' (SEQ ID No: 16); probe: VIC -5' CTCTGACACCATCGAC 3'-MGB (SEQ ID No: 17)) in multiplex mode.

Table 02. Primers and probes from the Taqman® assay used to determine the number of copies of the *cry1Ac* and *nptII* genes and vector structures (backbone) present in the CTC79005-2 event.

| Assay | *Primers* / probe | Sequence 5' - 3' | *Amplicon* (bp) |
|---|---|---|---|
| cry1Ac - 0 | pCTC00 1_Cry1Ac.F | GAGGTGCCAATCCACTTCCC - SEQ ID NO 40 | 82 |
| | pCTC001_ Cry1Ac.R | GAGTTACCCCAGTTCACGTTGAG - SEQ ID NO 41 | |
| | Cry1Ac_probe | FAM-ACCTCCACCAGGTACAG-MGB - SEQ ID NO 42 | |
| nptII - 0 | nptII.F | TGCCGAATATCATGGTGGAA - SEQ ID NO 43 | 55 |
| | nptII.R | CGGCCACAGTCGATGAATC - SEQ ID NO 44 | |
| | nptII.probe | FAM-TGGCCGCTTTTCT-MGB - SEQ ID NO 45 | |
| c-StaA (*backbone*) | c-StaA.F | GCCGCTTGTAGCCTTCCA - SEQ ID NO 46 | 64 |
| | c-StaA.R | CCGCCGACCTGGTGGA - SEQ ID NO 47 | |
| | c-StaA _BB_probe | FAM-CGTGACCTCAATGCG-MGB - SEQ ID NO 48 | |
| aad-A (*backbone*) | aad-A BB F | CACAATCGTCACCTCAACCG - SEQ ID NO 49 | 75 |
| | aad-A BB R | ATCAACGACCTTCTGGAAACG - SEQ ID NO 50 | |
| | aad-A _BB_probe | NED-CGCAGGATTTCGCTCTCG-MGB - SEQ ID NO 51 | |

The qPCR reactions used IX TaqMan® Fast PCR Master Mix II (Applied Biosystems, USA), 300 nM from each primer and 200 nM from the corresponding probes. The cycling used was: a 50 °C cycle for 2 minutes for uracil N-glycosylase activation, a 95 °C cycle for 20 seconds for DNA polymerase activation, 40 cycles of 95 °C for 3 seconds (denaturation), and 60 °C for 30 seconds (annealing and extension).

Data analysis was performed by manually entering the threshold at the exponential phase of the amplification curve. For *cry1Ac* and *nptII* genes, the copy number was inferred from DeltaCt (dCt) analysis, in which the Ct (cycle at which the fluorescence signal emitted by the amplification product reaches the threshold) of the endogenous gene is subtracted from the Ct of the target gene. In this type of analysis, the number of copies is assumed to double every Ct and the reference number of control copies of the same variety whose value is known is taken as a reference.

As a result, both assays pointed to the presence of 1 copy for the *cry1Ac* gene in the CTC79005-2 event genome. The same copy number (i.e., 1 copy) was detected for the *nptII* gene, the assay of which is based on detection of the gene promoter.

For the Southern blot, 10 μg of genomic DNA from the CTC79005-2 event and the parental variety RB 92579 were digested separately with distinct restriction enzymes (*EcoRV, Hind*III, *BsrG*I *and Shp*I), following the cold probe tagging procedure (Digoxigenin - DIG). Briefly, genomic DNAs of the event CTC79005-2 and the parental variety RB92579 were extracted using the *NucleoSpin® Plant II* kit in tubes, following the recommendations of the manufacturer (*Macherey-Nagel GmbH* & *Co. KG,* Germany). The quality of the extracted DNA was checked on a 1% agarose gel in TAE IX buffer (Tris-Acetate EDTA) and 10 μg of DNA were digested using 100 units of restriction enzyme (10 U/μg of gDNA) per sample, in a final volume of 400 μl of reaction.

The enzymatic reaction was carried out following the manufacturer's recommendations (Thermo Fisher, USA). The digestion product was precipitated with 2 volumes of ethanol and 10% 7.5 M ammonium acetate, and incubated for 48 h at -20 °C. The precipitated DNA was centrifuged at 14,000 x g for 30 min and the pellet formed was resuspended in 35 μl of milli-Q water until complete dissolution. The quality of digestion was visualized on a 1% agarose gel with IX TAE buffer (Tris-Acetate - EDTA). The *Eco*RV, *Hind*III and *BsrG*I enzymes were used to detect the cassettes containing the *cry1Ac* and *nptII* genes (Figure 23 A). *Sph*I enzyme was used to detect vector backbone (Figure 23 B).

Probes were design to detect *cry1Ac* gene (probe CrylAc - 1,079 bp), *nptII* gene (*npt*II probe 679 bp), UBI promoter region (1029 bp), presented in the CTC79005-2 event. The probes for detecting fragments of the vector (backbone) were designed throughout the vector, covering ~ 98% of the backbone (BB1 probe - 1,875 bp; BB2 probe -2,305 bp; BB3 probe -2,282 bp; Figure 23 B). The probes were prepared and used to detect targets using the *PCR DIG Probe Synthesis Kit* reagents (Roche, cat # 11636090910, Switzerland). About 100 pg of the linearized vector were used as a template for the amplification of the regions of interest and incorporation of the digoxigenin molecule (DIG) by PCR. The primers used for the production of the probes, the hybridization temperatures (Thyb) and the expected size of each amplicon are listed in Table 03.

DNA from RB92579 variety was used as a negative control. As a positive control, plasmid DNA containing the construction used to obtain event CTC79005-2 was added to RB 92579 genomic DNA samples. Plasmid DNA was previously linearized with the restriction enzyme used in each assay.

Table 03. List of primers used in the synthesis of probes marked with DIG.

| Probe | Enzyme | Sequence 5'→ 3' | Target | Tm | Amplicon | Thyb |
|---|---|---|---|---|---|---|
| Cry | *EcoRV/ HindIII/ BsrGI* | CAACAACCCAAACATCAACG - SEQ ID NO 65 | T-DNA | 58 °C | 1,079 bp | 50 °C |
| | | GGGTCCTGTAGACACCCTGA - SEQ ID NO 86 | | | | |
| nptII | *EcoRV/ HindIII/ BsrGI* | GCTCACCCTGTTGTTTGGTGTT - SEQ ID NO 87 | T-DNA | 58 °C | 679 bp | 50 °C |
| | | CGGCCACAGTCGATGAATC - SEQ ID NO 44 | | | | |
| UBI | *EcoRV/ HindIII/ BsrGI* | CTGTCGGCATCCAGAAATTG - SEQ ID NO 88 | T-DNA | 58 °C | 1,029 bp | 50 °C |
| | | TCAGTAAAACCCACATCAAC - SEQ ID NO 71 | | | | |
| *Backbone 1* | *Sph*I | TCCCCTCGGGATCAAAGTA - SEQ ID NO 89 | Vector | 57 °C | 1,875 bp | 50 °C |
| | | TAGCGTGTTTGTGCTTTTGC - SEQ ID NO 90 | | | | |
| *Backbone 2* | *Sph*I | GGCCGCTGAAATTAAATCAA - SEQ ID NO 91 | Vector | 58 °C | 2,305 bp | 50 °C |
| | | GAGTCAGTGAGCGAGGAAGC - SEQ ID NO 92 | | | | |
| *Backbone 3* | *Sph*I | CGGTGAAAACCTCTGACACA - SEQ ID NO 93 | Vector | 57 °C | 2,282 bp | 50 °C |
| | | ATACAGGCAGCCCATCAGTC - SEQ ID NO 94 | | | | |

The transfer of the digested DNA to the *nylon* membrane was carried out according to well established protocols. Briefly, electrophoresis of the digested DNA in 1% agarose gel with TAE IX buffer was performed at 40 V for approximately 20 hours, using *SYBR Safe DNA gel stain* (Invitrogen # S33102, USA) as an intercalant agent. Then, the agarose gel was treated with four different solutions in the following order:

- Depurination solution (1.1% HCL); 10-15 min;
- Denaturation solution (0.5 N NaOH; 1.5 M NaCl); 30-40 min;
- Neutralization solution (1.5 M NaCl; 0.5 M Tris); 30-40 min;
- SSC 20x (Saline Sodium Citrate); 20 min

The DNA transfer to the *nylon* membrane was performed using *TurboBlotter Transfer System* 20 x 25 (Sigma # WHA10416324, USA) according manufacturer instructions. The membrane with the transferred DNA samples was placed in the *UV Crosslinker* equipment (UVP- Analytik-Jena, Germany) for DNA fixation (2 cycles of 700 x 100 μJ/cm$^2$ Prehybridization and hybridization were then performed.

The Prehybridization treatment consists of incubation of the membrane with *DIG Easy Hyb* solution (Roche # 11603558001, Switzerland) at 40 °C for 3 hours, with denatured salmon sperm DNA in the final concentration of 100 μg.mL$^{-1}$ and constant rotation (0.5 x g). After, Hybridization was performed with the same Pre-hybridization solution (*DIG Easy Hyb* with salmon sperm DNA) added to the probe marked with DIG and denatured. The concentration of the probes used was 65 μg.mL$^{-1}$. The hybridization temperature was 50 °C.

Hybridization was carried out in a hybridization oven for approximately 16 hours with constant rotation (0.5 x g). The hybridized membranes were washed and blocked. After blocking solution was discarded and the membrane was covered with a new blocking solution with the antibody *Anti-Digoxigenin AP fragments* (Roche # 11093274910, Switzerland) diluted in a 1:20,000 ratio. The membrane was then incubated for 30 min at room temperature and slightly shaken. The antibody solution was discarded, and the membrane was washed and incubated with the *Detection buffer* (Roche # 11585762001, Switzerland) plus *CDP-Star ready to use* (Roche # 12041677001, Switzerland) for a final concentration of IX according to manufacturer instructions. A photographic film was added to detect chemiluminescence (Roche # 11666657001, Switzerland) in contact with the membrane for at least 16 hours and then immersed 1x Developing Solution (Kodak # 1900943, USA) for a visualization of the bands. The 1x Fixing Solution (Kodak # 1901875, USA) was used to fix the band images detected.

In hybridization with the Cry1Ac, for the materials digested with *Eco*RV, *Hind*III and *BsrGI,* a single band greater than 8.5 kb was observed for all samples from CTC79005-2 event (Figure 24). In hybridization with the pUBI probe, for the materials digested with *Hind*III and *EcoRV,* a single band greater than 8.5 kb was observed for all samples from CTC79005-2 event (Figure 25). The samples digested with *BsrG*I had two bands, one relative to the fragment containing the cry1Ac element greater than 8.5 kb and the other with the fragment containing *nptII,* with an approximate size of ~ 4.9 kb (Figure 25). For hybridization with the nptII probe, for the materials digested with *Hind*III and *Eco*RV, unique fragments with the size greater than 8.5 kb was observed. For the digestion with *BsrGI,* hybridization with the nptII probe presented a single fragment of size ~ 4.9 kb (Figure 26).

The results of the Southern blot, using the enzymes *Eco*RV, *Hind*III and *BsrG*I in combination with the probes that recognize the *cry1Ac, nptII* and UBI-1 promoter, confirm the results found using qPCR, which indicate that the CTC79005-2 event has only one integration of T-DNA with one copy of the *cry1Ac* gene and one copy of the *nptII* gene in the genome. In addition, the presence of the same fragments in all generations of the CTC79005-2 event from samples collected in consecutive vegetative generations (T0 - T3; Figures 24, 25 and 26), confirm the genetic stability of the insertion in the event over 4 generations of vegetative multiplication. Sequences of the vector backbone was not detected in the event CTC79005-2 (Figure 27).

2.3 Definition of flanking sequences.

To isolate the flanking regions at the ends of the T-DNA insert present in event CTC79005-2, several DNA sequencing experiments were performed. The map of the genetic insertion of event CTC79005-2 generated from the data of these experiments is shown in Figure 3.

Inverse PCR (iPCR) assays were performed for Right border (3') end of the T-DNA to isolate and clone the flanking region of the insert. Left border was identified by capture sequencing methodology, as described below. The iPCR methodology is based on genomic DNA digestion using enzymes that cleave the T-DNA sequence and a random event genome sequence. The cleavage products are circularized and subjected to multiple nested PCR cycles using primers for known T-DNA regions (Table 04). The isolated fragments were then isolated, cloned and sequenced by Sanger methodology. Finally, a consensus sequence of the flanking regions was assembled (SEQ ID NO: 23 and SEQ ID NO: 24).

Table 04. Restriction enzyme and primer nucleotide sequences used to perform the iPCR (inverse PCR) to identify the flanking regions of the CTC79005-2 event, and conditions of the amplification reactions.

| T-DNA Border | Restriction Enzyme | | Oligonucleotide Sequence |
|---|---|---|---|
| Right border | *Bgl*II | Nested PCR1 | 5'-TTTGACAACAGGACTCTAC - 3' - SEQ ID NO 52<br>5' -TGCAATGCTCATTATCTCTAG - 3' - SEQ ID NO 53 |
| | | Nested PCR2 | 5'-TAGCTTCACCTATATAATACTTC - 3' - SEQ ID NO 54<br>5'-TGCACTGCAGGCATCGATC - 3' - SEQ ID NO 55 |
| | | Nested PCR3 | 5'-TCGTCGACTCTAGACTCGAG - 3' - SEQ ID NO 56<br>5' - GATAATGCCAGCCTGTTAAAC- 3' - SEQ ID NO 57 |
| Right border | *Bsp*OI | Nested PCR1 | 5' - TTTGACAACAGGACTCTAC - 3' - SEQ ID NO 52<br>5'-TGCAATGCTCATTATCTCTAG - 3'- SEQ ID NO 53 |
| | | Nested PCR2 | 5'-TAGCTTCACCTATATAATACTTC - 3' - SEQ ID NO 54<br>5'-TGCACTGCAGGCATCGATC - 3' - SEQ ID NO 55 |

| Cycle Number | Denaturation | Annealing | Extension |
|---|---|---|---|
| **1st** | 94 °C, 5 min | - | - |
| **2-36th** | 94 °C, 30 sec | 50 °C 45 sec | 72 °C, 3 min |
| **37th** | - | - | 72 °C, 7 min |

In parallel, as there is currently no fully sequenced genome that could be used as a reference for RB92579 germplasm, a capture sequencing methodology was adopted as an additional effort to isolate the T-DNA inserted into the event CTC79005-2 and its flanking regions. In this strategy, small overlapping polynucleotide fragments (probes) were developed to cover the entire T-DNA sequence. These probes were hybridized to the fractionated genomic DNA of both RB92579 and CTC79005-2 cultivars, and hybrid sequences were isolated. Isolated fragments were then sequenced using Illumina® technology according to standard protocol. The data obtained were aligned with the T-DNA sequence present in the transformation vector and, together with the iPCR data mentioned above, the complete T-DNA consensus sequence (SEQ ID NO: 2) of the CTC79005-2 event and its flanking sequences (SEQ ID NO: 22, SEQ ID NO: 5, SEQ ID NO: 23, SEQ ID NO: 24) were obtained.

2.4 Method for the detection and characterization of event CTC79005-2 (event-specific assay)

For the validation of the methodology, both non-GMO control (WT) plants and other genetically-modified events having the same construct were used as experimental controls. DNA extraction occurred as described above.

Real-time PCR assays for identification of the CTC79005-2 event were designed and validated based upon the molecular characterization of the T-DNA insertion genomic flanking sequences. For the development of specific detection methodology, the real-time PCR (qPCR) technique known as "Plus-Minus" or "Presence - Absence" was chosen, validating the two variations of the methodology: via SYBR GREEN™ and via Taqman® technology. Specific primer pairs have been designed to generate information about the insertion of T-DNA in both methodologies, such that one primer binds in the construct and the second primer binds in the host genome. For the use of Taqman® technology, specific probes were designed between the primers.

In a preferred embodiment, the primers designed are the primers as defined on the SEQ ID Nos: 6 to 9, wherein the primer of SEQ ID NO: 6 is a forward primer of the right border (RB), the primer of SEQ ID NO: 7 is a reverse primer of the right border, the primer of SEQ ID NO: 8 is a forward primer of the left border (LB) and the primer of SEQ ID NO: 9 is a reverse primer of the left border (Table 05).

In a preferred embodiment, the probe employed in Taqman® PCR technology consists of SEQ ID NO: 10 in the RB region (right border) and/or SEQ ID NO: 11 in the LB region (left border).

Table 05. List of primers and probes used in Real-time PCR assays.

| | Primer/Probe | Oligonucleotide sequence | Amplicon |
|---|---|---|---|
| Detection of left board (LB) | Forward<br>Probe LB<br>Reverse | CAGGTTGACGTGACTGGGTTG - SEQ ID NO 08<br>FAM-TGGTGTAAACAAATTGACGC-MGB - SEQ ID NO 11<br>CGTCCGCAATGTGTTATTAAGTTG - SEQ ID NO 09 | 104 bp |
| Detection of right board (RB) | Forward<br>Probe RB<br>Reverse | CAGGAGCTCGTCGACTCTAGACT - SEQ ID NO 06<br>FAM-CATTGGGAGGAGGAAGA-MGB - SEQ ID NO 10<br>GTGTGCCCTTCCCTCGTG - SEQ ID NO 07 | 126 bp |
| Endogenous control (pUBI) | Forward<br>Probe RB<br>Reverse | ACCATTACCCTGGAGGTTGAGA- SEQ ID NO 15<br>VIC - CTCTGACACCATCGAC - MGB-SEQ ID NO 17<br>GTCCTGGATCTTCGCCTTCA - SEQ ID NO 16 | |

Taqman® real-time PCR reactions were performed using *QuantStudio 6 Flex* from *Applied Biosystems.*

The sugarcane poly-ubiquitin gene (endogenous gene) was used as an internal reaction control to confirm the presence and quality of the DNA used. The following reagents were multiplexed with the assay developed for the event: forward primer (SEQ ID NO: 15); reverse primer (SEQ ID NO: 16); probe (SEQ ID NO: 17).

qPCR reactions used IX TaqMan® Fast PCR Master Mix II (Applied Biosystems, USA), 150nM from each event-specific primer and 100nM from the corresponding probe, 300nM from the primers for the endogenous poly-ubiquitin gene and 300nM of its probe, 100-200ng of DNA and enough water to complete the 20 $\mu$L volume. The following PCR program was used: a 50°C cycle for 2 minutes for uracil N-glycosylase activation, a 95°C cycle for 20 seconds for DNA polymerase activation, 35 cycles of 95° C for 3 seconds (denaturation) and 60 °C for 30 seconds (annealing and extension). Singleplex reaction could also be performed. For Singleplex reactions, 150nM from each event-specific primer or endogenous primer and 100nM from the corresponding probe were used. The reaction conditions are the same for multiplex reactions.

As a result, it was possible to validate the event-specific detection reaction of CTC79005-2 through high-accuracy Taqman®, as illustrated in Figure 6. Taqman® technology probes specifically bind to DNA and are released during DNA amplification, thus generating the fluorescence signal captured by the equipment during this process.

qPCR reactions using SYBR GREEN™ were performed using the 7500 Real-Time PCR System (Applied Biosystems, USA) and *QuantStudio 6 Flex* from *Applied Biosystems* in its standard mode for this type of assay. Reactions were performed using 1 X QuantiFast SYBR Green™ PCR Kit (QIAGEN ™), 100nM forward and reverse primers, 20ng DNA and sufficient water for a final volume of 25$\mu$L. The reactions were performed using the event of the invention, the other events transformed with the same construct as the event of the invention (negative controls), wild type sugarcane (WT; untransformed) samples, and experimental controls (extraction and reaction blank).

The following PCR program was used: a DNA denaturation cycle at 95°C for 5 minutes, 35 cycles for annealing and amplification at 95°C for 15 seconds and 60°C for 1 minute and a dissociation cycle for generation melting peak of 95°C for 15 sec, 60°C for 1 min, 95°C for 15 sec. The reaction with SYBR safe does not allow the use of multiplex; therefore it is necessary to prepare a separated endogenous gene amplification reaction, using the same DNA, to eliminate false negatives.

Samples corresponding to the event of the invention showed specific amplification: having well-defined amplification curve formation and characteristic sigmoidal shape; whereas samples from other events, WT, and extraction and reaction blanks did not show event-specific amplification. As expected, the endogenous control presented amplification for all samples except extraction and reaction blanks, demonstrating both the quality of the DNA used in the reaction, as well as the quality of the reaction and cycling.

The SYBR GREEN™ assay showed specific amplification of the event samples at the expected melting temperature. Samples of the other events, as well as WT and blanks did not have peaks at this temperature (Figure 7).

Example 3. Event CTC79005-2 Generation - Genome editing (GE)

The event of the invention, CTC79005-2, is generated using a genome editing (GE) approach, thus recreating the event generated using the preferred *Agrobacterium-mediated* transformation methods described herein.

In this way, the event CTC79005-2 is recreated with the insertion of the *cry1Ac* gene into the same location of the genome as the CTC79005-2 event. The *cry1Ac* gene expression is regulated by a promoter or a promoter region and a terminator capable to drive the CrylAc protein expression at levels sufficient to control infestation of the target pest. Additionally, a marker gene or selection system is also inserted (transiently or stably) to enable event selection. Preferably, the T-DNA of the claimed invention (SEQ ID NO: 2) is inserted into the same location of the genome as the CTC79005-2 event. Thus, the event CTC79005-2 is recreated with the insertion of the *cry1Ac* gene, which expresses a toxin to control *D. saccharalis,* and the *nptII* gene. The expression of the cry1Ac and *nptII* genes is regulated by the maize ubiquitin gene promoter UBI-1, which has an endogenous intron. Both expression cassettes use the *Agrobacterium tumefaciens* nopaline synthase (nos) terminator.

In the case that the aforementioned genome editing approaches to generating event CTC79005-2 result in low-efficiency integration of the T-DNA at the target site, developmental genes or other regulatory elements could be delivered in conjunction with the GE reagents in order to improve the integration efficiency.

3.1 Constructs development.

Conventional gene cloning techniques using commercial plasmids, restriction enzyme digestion, fragment ligation (with ligases) and other known methodologies are used to develop the constructs (plasmids) of the present invention.

The GE reagents can be delivered on multiple plasmids, each one comprised of an element of the enzymatic complex (endonuclease, crRNA or guide RNA, and the homologous recombination (HR) template; Figure 19).

In one embodiment, the HR template constructs comprises the T-DNA (SEQ ID NO: 2) of the claimed invention flanked by approximately 1kb of DNA homologous to the flanking sequences described for CTC79005-2 (SEQ ID Nos: 23 and 24) located on either side of the T-DNA. In a preferred embodiment, the HR template constructs comprises the SEQ ID NO: 26 (Figure 21). The invention also comprises a second construct comprising an endonuclease expression cassette. In a preferred embodiment, the endonuclease expression cassette comprises a Cas9 endonuclease sequence. In more preferred embodiment the Cas9 sequence is codon optimized for sugarcane expression. In one embodiment, the Cas 9 construct comprises additionally the guide/ crRNA sequence. Preferably, the Cas 9 construct comprises the crRNA sequence SEQ ID NO: 28. In a more specific embodiment, the Cas 9 construct comprises the SEQ ID NO: 27 (Figure 20). A third construct comprising the guide RNA expression cassette alone is also contemplated in the present invention and comprises SEQ ID NO: 28.

In one additional embodiment the genome editing construct comprises HR template, the nuclease and the guide RNA expression cassettes, delivering all the GE reagents in a single construct. In one embodiment, the single construct comprises the T-DNA (SEQ ID NO: 2) of the claimed invention flanked by approximately 1kb of DNA homologous to the flanking sequences described for CTC79005-2 (SEQ ID Nos: 23 and 24) located on either side of the T-DNA. In a preferred embodiment, the construct comprises the SEQ ID NO: 25 (Figure 22).

Optionally, the constructs also comprise fluorescent/selection markers and/or other genetic engineering systems to remove marker genes and/or nucleases cassettes, such as a Cre/loxP recombination system from the bacteriophage P1. In this case, the marker/nuclease gene cassette, which should be deleted, is flanked by the loxP regions, while Cre recombinase removes this fragment during a transient expression.

3.2 Direct Delivery

In one embodiment, the event of the invention is generated using methodologies for direct delivery of proteins, RNA or plasmids. The methodologies for direct delivery are selected from the group consisting of particle bombardment, electroporation, lipofection and protoplast transfection; however, other delivery methodologies known to those of skill in the art could also be utilized.

3.2.1 Direct delivery - RNP approach

In one embodiment, the event of the invention is generated using ribonucleoprotein (RNP) delivery. The preferred methodologies for RNP delivery are selected of the group consisting of particle bombardment of sugarcane calli and protoplast transfection. Moreover, other sugarcane cell types can also be used for transformation including (but not limited to): leaf disc, meristem, and calli-derived suspension cells.

Using the RNP approach to genome editing, the endonuclease and crRNA or guide RNA are delivered in RNP form, separate from the HR template, which is delivered via plasmid.

The guide RNA may be preliminarily produced by in vitro transcription or be chemically synthesized as ribooligonucleotide, while the corresponding nuclease may be produced *in vivo* with further purification (bacterial expression) or purchased from any manufacturer of such products. In a preferred embodiment, the guide RNA comprises SEQ ID NO: 28. In another embodiment the nuclease is Cas9 nuclease.

A ready ribonucleoprotein (RNP) complex consisting of the corresponding nuclease and guide RNA, and the HR template plasmid are absorbed on golden particles and direct delivery to the cells or tissue.

3.2.2 Direct delivery - Plasmid

In another embodiment of the invention, the event of the invention is generated using plasmid delivery, wherein the GE reagents will be expressed in a transient manner, thus achieving the site-directed integration of CTC79005-2 without the integration of additional transgenes associated with the GE approach.

Methodologies for plasmid delivery is selected from the group consisting of particle bombardment (biolistic) of sugarcane calli or polyethylene glycol transformation of protoplasts; however, other methodologies known to those of skill in the art could also be utilized. Moreover, other sugarcane cell types can also be used for transformation including (but not limited to): protoplast, leaf disc, meristem, and calli-derived suspension cells.

In yet another embodiment of the invention, the event of the invention is generated using plasmid delivery, where the GE reagents are stably expressed, thus requiring the excision of the integrated GE reagents and selectable marker using, for example, a Cre/Lox system. Using this approach, LoxP sites will remain in the genome of the event CTC79005-2 plant. Other DNA excision approaches known to those skilled in the art may also be used to remove the GE reagent DNA from the genome of the event CTC79005-2 plant.

3.3 Indirect Delivery

In one embodiment, the event of the invention is generated using methodologies for indirect delivery of plasmids, as_*Agrobacterium* transformation. *Agrobacterium tumefaciens* and *Agrobacterium rhizogenes* can be used. Plant viruses also can be used for indirect delivery of plasmids to plant cells and tissues. For example, genetically modified plant geminiviruses make it possible to achieve higher transformation efficiency, specially without stable insertion into a genome. Moreover, different tissue and cell types can also be used for transformation including (but not limited to): calli, protoplast, leaf disc, meristem, and calli-derived suspension cells.

In a preferred embodiment the *Agrobacterium* transformation is performed as describe at Example 1.

In another preferred embodiment, the event of the invention is generated using plasmid delivery by *Agrobacterium* transformation, wherein the GE reagents will be expressed in a transient manner, thus achieving the site-directed integration of CTC79005-2 without the integration of additional transgenes associated with the GE approach.

The GE reagents can be delivered on multiple plasmids, but preferably on a single plasmid. In a preferred embodiment, the constructs is SEQ ID NO: 25 and comprises a selectable marker, a nuclease, crRNA or guide RNA, and homologous recombination (HR) template (Figure 22). The HR template comprises T-DNA (SEQ ID NO: 2) of the claimed invention flanked by of the DNA homologous to the flanking sequences described for CTC79005-2 (SEQ ID Nos: 23 and 24) located on either side of the expression cassettes.

In a preferred embodiment, the event of the invention is generated using plasmid delivery, where the GE reagents are stably expressed, thus requiring the excision of the integrated GE reagents and selectable marker using, for example, a Cre/Lox system. Using this approach, LoxP sites will remain in the genome of the event CTC79005-2 plant. Other DNA excision approaches known to those skilled in the art may also be used to remove the GE reagent DNA from the genome of the event CTC79005-2 plant.

With all the transformation processes described above, the resulting transformed cells will be regenerated to form a plant containing the event of the invention.

3.4 Molecular characterization.

The event CTC79005-2, generated using genome editing, is evaluated for accurate insertion of the T-DNA into the target site of the genome using the primers and probes of the invention (SEQ ID NO: 6-9) as is described in the specification herein.

Additionally, pair of primers designed to amplify the outermost sequences in the flanking sequences of event CTC79005-2 are validated and could be used to evaluate the integrity of the recombination site (Table 06, Figure 28)

Table 06. Pair of primers designed for Flanking sequences of CTC79005-2.

| Position | *Primer Forward* / *Primer Reverse* | 5' → 3' | Amplicon (pb) |
|---|---|---|---|
| 5' Flanking region | 79-005 FSLB 1 FW 1 | GGTAATCCTTCAAAGGATTTTAAAAAATAC -SEQ ID NO 58 | 517 |
| | LBRV2 | GGCCGTTACTAGTGGATCG-SEQ ID NO 63 | |
| | 79-005 FSLB 2 FW 1 | AGATAGAGAGCGGGAAATTGAGATGTAAC-SEQ ID NO 59 | 442 |
| | LBRV2 | GGCCGTTACTAGTGGATCG-SEQ ID NO 63 | |
| | 79-005 FSLB 3 FW 1 | TCGTCCGATGCAGGATCAC-SEQ ID NO 60 | 523 |
| | LBRV2 | GGCCGTTACTAGTGGATCG - SEQ ID NO 63 | |
| | 79-005 FSLB 4 FW 2 | GGTTGACGTGACTGGGTTG-SEQ ID NO 61 | 173 |
| | LBRV1 | GCCGTTACTAGTGGATCGAG-SEQ ID NO 64 | |
| 5' Flanking region + T-DNA | 79-005 FSLB 1 FW 1 | GGTAATCCTTCAAAGGATTTTAAAAAATAC-SEQ ID NO 58 | 5760 |
| | Fw-1 _SB_ Cry1 | CAACAACCCAAACATCAACG-SEQ ID NO 65 | |
| | 79-005 FSLB 2 FW 1 | AGATAGAGAGCGGGAAATTGAGATGTAAC-SEQ ID NO 59 | 5685 |
| | Fw-1 _SB_ Cry1 | CAACAACCCAAACATCAACG-SEQ ID NO 65 | |
| | 79-005 FSLB 3 FW 1 | TCGTCCGATGCAGGATCAC-SEQ ID NO 60 | 5496 |
| | Fw-1 _SB_ Cry1 | CAACAACCCAAACATCAACG-SEQ ID NO 65 | |
| | 79-005 FSLB 4 FW 2 | GGTTGACGTGACTGGGTTG-SEQ ID NO 61 | 5417 |
| | Fw-1 _SB_ Cry1 | CAACAACCCAAACATCAACG- SEQ ID NO 65 | |

(continued)

| Position | *Primer Forward* / *Primer Reverse* | 5' → 3' | Amplicon (pb) |
|---|---|---|---|
| 3' Flanking region | 79-005 FSRB 1 RW 1 | GGTAATCCTTCAAAGGATTTTAAAAAATAC-SEQ ID NO 58 | 953 |
| | RB007 FW 1 | ATGGCTGCAGGAGCTCGTCGACTCTA-SEQ ID NO 66 | |
| | 79-005 FSRB 2 RW 1 | AGATAGAGAGCGGGAAATTGAGATGTAAC-SEQ ID NO 59 | 818 |
| | RB007 FW 1 | ATGGCTGCAGGAGCTCGTCGACTCTA-SEQ ID NO 66 | |
| | 79-005 FSRB 3 RW 1 | TCGTCCGATGCAGGATCAC-SEQ ID NO 60 | 613 |
| | RB007 FW 1 | ATGGCTGCAGGAGCTCGTCGACTCTA-SEQ ID NO 66 | |
| | 79-005 FSRB 3 RW 1- | GTTGACGTGACTGGGTTGC - SEQ ID NO 62 | 238 |
| | RB007 FW 1 | ATGGCTGCAGGAGCTCGTCGACTCTA--SEQ ID NO 66 | |
| | 79-005 FSRB 4 RW 1 | GGTAATCCTTCAAAGGATTTTAAAAAATAC-SEQ ID NO 58 | 328 |
| | RB007 FW 1 | ATGGCTGCAGGAGCTCGTCGACTCTA-SEQ ID NO 66 | |

(continued)

| Position | Primer Forward / Primer Reverse | 5' → 3' | Amplicon (pb) |
|---|---|---|---|
| 3' Flanking region + T-DNA | 79-005 FSRB 1 RW 1 | GGTAATCCTTCAAAGGATTTTAAAAAATAC-SEQ ID NO 58 | 3906 |
| | Rv-4_SB_ Cry1Ab | CGTCGGTGTAGATGGTGATG-SEQ ID NO 67 | |
| | 79-005 FSRB 2 RW 1 | AGATAGAGAGCGGGAAATTGAGATGTAAC-SEQ ID NO 59 | 3771 |
| | Rv-4_SB_ Cry1Ab | CGTCGGTGTAGATGGTGATG-SEQ ID NO 67 | |
| | 79-005 FSRB 3 RW 1 | TCGTCCGATGCAGGATCAC-SEQ ID NO 60 | 3568 |
| | Rv-4 _SB_ Cry1Ab | CGTCGGTGTAGATGGTGATG-SEQ ID NO 67 | |
| | 79-005 FSRB 3 RW 1- | GTTGACGTGACTGGGTTGC - SEQ ID NO 62 | 3191 |
| | Rv-4_SB_ Cry1Ab | CGTCGGTGTAGATGGTGATG-SEQ ID NO 67 | |
| | 79-005 FSRB 4 RW 1 | GGTAATCCTTCAAAGGATTTTAAAAAATAC-SEQ ID NO 58 | 3281 |
| | Rv-4_SB_ Cry1Ab | CGTCGGTGTAGATGGTGATG-SEQ ID NO 67 | |

PCR reactions use 0.2 μM of primers (Table 06), 20ng of DNA, IX *Dream Taq- Thermo Fisher buffer* (Thermo Fisher Scientific Inc - USA), Taq polymerase (final volume 20 μL). Reaction conditions are: one cycle at 94°C for 1 min; 35 cycles at 94°C for 30 seconds, 65°C for 45 seconds and 72°C for 3 min; and one cycle of extension at 72°C for 7 minutes. Amplicons with more than 2kb, the reactions are performed with 1U de Takara LA Taq enzyme - Takara (Takara Bio Inc-USA), 1 X LA PCR Buffer (Takara Bio Inc - USA), 2.5 mM MgCl2, 2.5mM dNTP, 0.5 μM of primers and 200 ng of DNA (final volume 50 μL).

Amplicons generated by PCR reactions with a combination of "T-DNA" and "Flanking regions" primers from Table 06, could be further sequencing by Sanger, using the pair of primers described at Table 07 (pair of primers designed to anneal to the T-DNA sequence of CTC79005-2 event) to confirm integrity of the insert.

Table 07. Primers located internally to T-DNA, used to amplify the insertion of the event CTC79005-2.

| *Primer* | Sequence (5' → 3') | Element |
|---|---|---|
| CLNG P04 FW | TGATGCATATACAGAGATGCTTTTT - SEQ ID NO 68 | *UBI* |
| CLNG P01 FW | TTCATCCATTTTATTAGTACATCCA - SEQ ID NO 69 | *UBI* |
| CLNG P03 FW | ACGGATGCGACCTGTACG - SEQ ID NO 70 | *UBI* |
| CLNG P07 RV | TCAGTAAAACCCACATCAAC - SEQ ID NO 71 | *UBI* |
| CLNG P08 RV | GACCACATCATCACAACCAAG - SEQ ID NO 72 | *UBI* |

(continued)

| *Primer* | Sequence (5' → 3') | Element |
|---|---|---|
| CLNG P09 RV | GCTCCGAACAACACGAGGTTG - SEQ ID NO 73 | *UBI* |
| CLNG P10 RV | ATGAAGTATTATATAGGTGAAG - SEQ ID NO 74 | *UBI* |
| CLNG P19 FW | AGAGGCTATTCGGCTATGAC - SEQ ID NO 75 | *nptII* |
| CLNG P20 FW | CAGCCGAACTGTTCGCCAGG - SEQ ID NO 76 | *nptII* |
| CLNG P21 RV | AGCACGAGGAAGCGGTCAGC - SEQ ID NO 77 | *nptII* |
| CLNG P22 RV | TGAGATGACAGGAGATCCTG - SEQ ID NO 78 | *nptII* |
| CLNG P65 FW | CAACAGCTCCGTGAGCATCATC - SEQ ID NO 79 | *Cry1Ac* |
| CLNG P66 FW | CCAGCGACTTCGGTTACTTC - SEQ ID NO 80 | *Cry1Ac* |
| CLNG P70 RV | CTCTCGGCGTAGATTTGGTAG - SEQ ID NO 81 | *Cry1Ac* |
| CLNG P55 FW | CAGAACAACAACGTGCCACC - SEQ ID NO 82 | *Cry1Ab/Ac* |
| CLNG P64 FW | AGCATCACCATCTACACCGAC - SEQ ID NO 83 | *Cry1Ac/ab* |
| CLNG P68 RV | GATCCAGGAGAACATCGGAGC - SEQ ID NO 84 | *Cry1Ac* |
| CLNG P67 RV | GTCACTGGGATGAACTCGAAC- SEQ ID NO 85 | *Cry1Ac* |
| CLNG P68 RV | GATCCAGGAGAACATCGGAGC- SEQ ID NO 84 | *Cry1Ac* |
| Rv-4_ SB_Cry1Ab | CGTCGGTGTAGATGGTGATG- SEQ ID NO 67 | *Cry1Ac/ab* |
| FW-1_ SB_CRY1 | CAACAACCCAAACATCAACG- SEQ ID NO 65 | *CrylAc/ab* |

Example 4. Evaluation of the gene expression product inserted in the event of the invention.

The gene expression product in the event of the present invention has been characterized detail using ELISA and Western blot to determine the concentration of CrylAc and NptII proteins and to confirm the identities of these heterologous proteins.

**(ELISA) Enzyme-Linked Immunosorbent Assay**

To evaluate *cry1Ac* and *nptII* gene expression via ELISA, different sugarcane tissues were studied at different stages of crop development. To produce tissue samples of the event of the invention and the parental control, the experimental tests conducted was AGRO/PHENO.

AGRO/PHENO trials were conducted at six representative sites of the parental control cultivation area, three in the state of São Paulo (Barrinha, Piracicaba and Valparaiso), one in the state of Goiás (Quirinopolis), one in the state of Bahia (Juazeiro), and one in the state of Paraná (Mandaguaçu). The experiments were conducted in a randomized complete block design with 4 replications. The plots were composed of four 8 meters rows and the spacing between rows was 1.5 meter.

Table 08. *Assay* information used for sample collection for analysis of *cry1Ac* and *nptII* gene expression produced by the event of the invention. (Number of Days After Planting (DAP) represents time of sample collection for analysis)

| Assay Type | Location | Tissue | DAP |
|---|---|---|---|
| AGRO/PHENO | Barrinha-SP | leaf<br>stalk<br>root | 100, 200, 300,330<br>330<br>330 |
| | Piracicaba-SP | leaf<br>stalk<br>root | 100, 200, 300,330<br>330<br>330 |
| | Valparaiso-SP | leaf<br>stalk | 100, 200, 300,330<br>330 |

(continued)

| Assay Type | Location | Tissue | DAP |
|---|---|---|---|
| | | root | 330 |
| | Quirinópolis-GO | leaf<br>stalk<br>root | 100, 200, 300,330<br>330<br>330 |
| | Juazeiro-BA | leaf<br>stalk<br>root | 100, 200<br>200<br>200 |
| | Mandaguaçu-PR | leaf<br>stalk<br>root | 100, 200, 300,330<br>330<br>330 |

The expression analysis of CrylAc and NptII proteins produced by the event of the invention was investigated at different periods of sugarcane plant development. The conditions evaluated were:

- Expression of heterologous proteins in leaves over a cultivation cycle of the event of the invention (100, 200, 300 DAP);
- Expression of heterologous proteins in leaves, stalks and roots at 330 DAP of the sugarcane cycle. In Juazeiro at 200 DAP.

Leaf samples were collected on experimental treatment plots (CTC79005-2. and parental control - RB 92579) in the AGRO/PHENO assays at 100, 200, 300 and 330 DAP. Stalk and root samples were collected only at 330 DAP in the AGRO/PHENO assays. With exception of Juazeiro where the leaves, stalks and roots were collected at 200DAP. After collection, the samples were sent for ELISA analysis to determine CrylAc and NptII protein expression levels.

Leaf Samples: 30 cm of tissue were collected from the tip of 5 to 10 "diagnostic" leaves on zigzag lines 2 and 3 avoiding diseased leaves. After removal of the central rib, the leaves were chopped into pieces, homogenized and packed in previously identified ziplock bags.

Stalk Samples: 10 whole sugarcanes were collected. After removing the dried leaves and pointers, the canes were cut into small tails, homogenized, and packed into labelled packages.

Root Samples: A representative clump from rows 2 and 3 of the experimental plot was collected. The soil was crushed, and the roots were washed with clean water to remove excess soil. The clean roots were then minced into pieces, homogenized, and packaged into labelled plastic bags.

All samples (from leaf, stalk, and root tissues) were transferred to dry ice in a Styrofoam box within 15 min of sampling. The genetic identity of all clumps sampled was confirmed by event-specific assay as described above.

For the analysis of the Cry1Ac protein 30 ± 1 mg of leaf, 200± 1 mg of stalks, and 20 ± 1 mg of root tissue were macerated using TissueLyser equipment. To the macerated leaf tissue was added 750 μL saline phosphate extraction buffer (PBS) supplemented with Tween 20 (0.138 M NaCl; 0.027 mM KCl; 0.05% Tween 20, pH 7.4) diluted according to manufacturer's instructions (Envirologix ™, USA). For stalk 375 μL of the same buffer was used and for root samples, 1,500 μL were added.

For the analysis of the NptII protein in leaves, 40 ± 1 mg were weighted and macerated and 200 ± 1 mg were weighted for stalk and root samples and macerated. To the macerated leaf tissue samples, 750 μL of the extraction buffer were added; while in the stalk and root samples, 1,500 μL of the same buffer were added. The extraction buffer used in this case was PEB1 1x (pH 7.0), and the dilution was made according to manufacturer's instruction (PathoScreen® Kit for NPT II,Agdia, USA).

After buffer addition, vortex homogenization was performed and then centrifuge for 20 minutes at maximum speed. The resulting supernatant was collected, and total protein was quantified using the Bradford assay (CrylAc) and BCA (NptII).

The standards used for obtaining the calibration curve were the already-diluted commercial BSA (Bovine Serum Albumin) standards supplied with the kit described above. The 2000, 1000, 500, 250, 125, and 0 μg/mL calibrators (prepared in PBST buffer) were used. 10 μL of each standard calibrator was added in triplicate to plate wells. In total, 6 curves were generated from independent dilutions. For the samples, 10μL of the 3 individual protein extractions were used in each well. Then 200μL of Coomassie Plus Reagent Solution was added to each well containing the calibrators and samples. The plates were covered and incubated for 5 minutes at room temperature. Absorbance was read at 595 nanometers (nm) using SoftmaxPro 7.0 software (Molecular Device, USA).

Total proteins were obtained in triplicate for each sample studied. After the total protein quantification of each replicate, the sample with the smallest variation of the median quantification value was chosen for ELISA analysis. After quantification of total proteins, leaf and stalks samples were diluted 700x; while root samples for CrylAc analysis were diluted 70x. For NptII analysis, leaves and stalk samples were diluted 5x, while root samples were used directly, without dilution.

Results were obtained by 96-well plate spectrometry reading at two different wavelengths: 450 nm and 630 nm for CrylAc and 450 nm for NptII on a SpectraMax Plate reader (Molecular Devices, USA). For CrylAc the Envirologix AP003 CRBS kit is used for protein detection and quantification. For NptII the *PathoScreen® Kit for NPT II* (Agdia, USA) kit was used. In all cases, the manufacturers recommendations were followed.

The analysis was based on the association of the absorbance values of the test samples with the predicted values in an equation estimated by measuring the absorbance of a standard curve. Synthetic proteins were diluted to desired concentrations in PBST buffer. Analyzes were performed in experimental duplicate for each sample. Cry1Ac and NptII protein concentrations were presented based on Total Protein (μg/mg), Fresh Tissue (μg/g) and Dry Tissue (μg/g).

The results of the statistical analyzes for the expression of the proteins CrylAc and NptII are shown in Tables 09 and 10, respectively. The protein expression data were obtained from the average of four biological replicates (experimental plots) in duplicate. Table 09 presents the results of individual and combined analyzes by location for CrylAc protein expression data, in sugarcane leaves collected over a year of cultivation (100, 200, 300 and 330 DAP). Table 10 shows the results of the analyzes for the NptII protein, under the same conditions.

Table 09. Comparison of the means of expression of Cry1Ac in leaves of the event CTC79005-2 over a year of cultivation (100, 200 and 300 DAP; DAP = Days After Planting) and at 330 DAP. Individual and joint Combined statistical analysis for the 6 tested sites. Data not available in Juazeiro-BA at 300 and 330 DAP in individual and Combined analyzes. Means followed by the same letter do not differ by t test at the 5% level ($p \leq 0.05$). (SE: standard error).

| Location | DAP | μg Cry 1Ac / g Fresh Tissue | SE | μg Cry 1Ac / g Dry Tissue | SE |
|---|---|---|---|---|---|
| Barrinha-SP | 100 | 48.36 a | 2.86 | 137.20 a | 8.24 |
| | 200 | 51.20 ab | 2.86 | 128.98 a | 8.24 |
| | 300 | 58.71 b | 2.86 | 185.38 b | 8.24 |
| | 330 | 79.40 b | 0.98 | 211.38 b | 2.8 |
| Piracicaba-SP | 100 | 64.44 a | 6.96 | 184.11 a | 20.11 |
| | 200 | 37.32 b | 6.96 | 95.22 b | 20.11 |
| | 300 | 56.97 ab | 6.96 | 166.72 a | 20.11 |
| | 330 | 64.08 b | 2.26 | 196.15 b | 6.98 |
| Valparaiso-SP | 100 | 68.45 a | 7.15 | 248.90 a | 25.85 |
| | 200 | 56.98 a | 6.19 | 191.67 a | 22.38 |
| | 300 | 50.06 a | 6.19 | 188.04 a | 22.38 |
| | 330 | 56.53 b | 6 | 173.45 b | 18.43 |
| Quirinópolis-GO | 100 | 70.31 a | 8.24 | 213.06 a | 24.57 |
| | 200 | 81.41 a | 8.24 | 230.78 a | 24.57 |
| | 300 | 65.10 a | 8.24 | 200.14 a | 24.57 |
| | 330 | 51.58 b | 4.58 | 144.29 b | 12.93 |
| Mandaguaçu-PR | 100 | 70.58 a | 5.09 | 249.82 a | 17.81 |
| | 200 | 49.52 b | 5.09 | 154.13 b | 17.81 |
| | 300 | 70.91 a | 5.09 | 245.11 a | 17.81 |
| | 330 | 50.56 b | 1.35 | 137.67 b | 3.93 |

(continued)

| Location | DAP | μg Cry 1Ac / g Fresh Tissue | SE | μg Cry 1Ac / g Dry Tissue | SE |
|---|---|---|---|---|---|
| Juazeiro-BA | 100 | 66.48 a | 4.98 | 221.62 a | 15.07 |
| | 200 | 58.54 a | 4.98 | 175.40 a | 15.07 |
| Combined Analysis | 100 | 64.68 a | 4.49 | 208.62 a | 16.84 |
| | 200 | 55.83 a | 4.46 | 162.70 b | 16.75 |
| | 300 | 60.51 a | 4.85 | 198.61 ab | 17.96 |
| | 330 | 60.12 b | 2.54 | 173.06 b | 7 |

Table 10. Comparison of the averages of NptII expression in leaves of the event CTC79005-2 over a year of cultivation (100, 200 and 300 DAP) and at 330 DAP. Individual and Combined statistical analysis for the 6 tested sites. Data not available in Juazeiro-BA at 300 and 330 DAP in individual and Combined analyzes. Means followed by the same letter do not differ by t test at the 5% level ($p \leq 0.05$). (SE: standard error).

| Locations | DAP | μg NptII/g Fresh Tissue | SE | μg NptII /g Dry Tissue | SE |
|---|---|---|---|---|---|
| Barrinha-SP | 100 | 0.28 a | 0.02 | 0.79 a | 0.05 |
| | 200 | 0.17 b | 0.02 | 0.44 b | 0.05 |
| | 300 | 0.25 a | 0.02 | 0.80 a | 0.05 |
| | 330 | 0.28 b | 0.02 | 0.75 b | 0.05 |
| Piracicaba-SP | 100 | 0.29 a | 0.03 | 0.83 a | 0.08 |
| | 200 | 0.15 b | 0.03 | 0.38 b | 0.08 |
| | 300 | 0.32 a | 0.03 | 0.93 a | 0.08 |
| | 330 | 0.29 b | 0 | 0.88 b | 0.02 |
| Valparaiso-SP | 100 | 0.21 a | 0.01 | 0.78 a | 0.04 |
| | 200 | 0.16 b | 0.01 | 0.53 b | 0.03 |
| | 300 | 0.16 b | 0.01 | 0.62 b | 0.03 |
| | 330 | 0.15 b | 0.01 | 0.48 b | 0.03 |
| Quirinópolis-GO | 100 | 0.19 a | 0.01 | 0.58 a | 0.04 |
| | 200 | 0.09 b | 0.01 | 0.24 b | 0.04 |
| | 300 | 0.26 c | 0.01 | 0.81 c | 0.04 |
| | 330 | 0.28 b | 0.01 | 0.77 b | 0.04 |
| Mandaguaçu-PR | 100 | 0.19 a | 0.02 | 0.66 a | 0.07 |
| | 200 | 0.22 ab | 0.02 | 0.70 a | 0.07 |
| | 300 | 0.28 b | 0.02 | 0.98 b | 0.07 |
| | 330 | 0.29 b | 0.01 | 0.80 b | 0.03 |
| Juazeiro - SP | 100 | 0.18 a | 0.02 | 0.60 a | 0.07 |
| | 200 | 0.23 a | 0.02 | 0.69 a | 0.07 |

(continued)

| Locations | DAP | μg NptII/g Fresh Tissue | SE | μg NptII /g Dry Tissue | SE |
|---|---|---|---|---|---|
| Combined Analysis | 100 | 0.22 ab | 0.02 | 0.71 a | 0.06 |
| | 200 | 0.17 a | 0.02 | 0.50 b | 0.06 |
| | 300 | 0.26 b | 0.02 | 0.83 a | 0.07 |
| | 330 | 0.25 b | 0.01 | 0.73 b | 0.03 |

Cry1Ac expression data (leaves; μg/g fresh and dry tissue) from the combined analysis of the 6 locations of the event of the invention over a year of cane cultivation (Table 09) are shown in Figure 8 (in μg protein/g fresh tissue) and Figure 9 (in μg protein/g dry tissue). NptII expression data (leaves; μg/g fresh and dry tissue) from the combined analysis of the 6 locations of the event of the invention over a year of cane cultivation (Table 10) are shown in Figurell (in μg protein/g fresh tissue) and Figure 12 (in μg protein/g dry tissue).

The average expression of CrylAc protein in leaves from CTC79005-2 event over a sugarcane cycle in all locations (with exception of Juazeiro at 300 and 330 DAP - no data) was 60.34 μg/g of fresh tissue and 185.37 μg/g of dry tissue. For NptII, the average expression in leaves from CTC79005-2 event over a sugarcane cycle in all locations (with exception of Juazeiro at 300 and 330 DAP - no data) was 0.22 μg/g of fresh tissue e de 0.68 μg/g of dry tissue.

Cry1Ac protein expression data in leaves from the event CTC79005-2 harvested at 330 DAP (200 DAP for Juazeiro) are shown in Table 09 and in Figure 10 (in μg protein/g of fresh and dry tissue). NptII protein expression data in leaves from the event CTC79005-2 harvested at 330 DAP (200 DAP for Juazeiro) are shown in Table 10 and Figure 13 (in μg protein/g of fresh and dry tissue).

CrylAc protein expression data in mature stalks from the event of the invention harvested at 330 DAP are shown in Table 11 and Figure 10 (μg/g fresh and dry tissue).

Table 11. Comparison of the average expression of the CrylAc protein in mature stalks of the event CTC79005-2 (330 DAP). Individual and Combined statistical analysis for the six tested sites. Sample collection in Juazeiro-BA was carried out at 200 DAP. Means followed by the same letter do not differ by t test at the 5% level ($p \leq 0.05$). (SE: standard error).

| Location | μg Cry1Ac / g Fresh Tissue | SE | μg CrylAc / g Dry Tissue | SE |
|---|---|---|---|---|
| Barrinha-SP | 8.11 a | 0.98 | 42.15 a | 2.8 |
| Piracicaba-SP | 4.56 a | 2.26 | 24.33 a | 6.98 |
| Valparaiso-SP | 5.61 a | 6 | 27.62 a | 18.43 |
| Quirinópolis-GO | 6.65 a | 4.58 | 26.45 a | 12.93 |
| Mandaguaçu-PR | 7.50 a | 1.35 | 36.44 a | 3.93 |
| Juazeiro - BA | 5.64 a | 3.91 | 22.42 a | 11.71 |
| Combined Analysis | 6.35 a | 2.54 | 29.90 a | 7 |

NptII protein expression data from mature stalks of the event of the invention collected at 330 DAP are shown in Table 12 and Figure 13 (μg/g fresh and dry tissue).

Table 12. Comparison of averages of NPTII protein expression in mature stalk from event CTC79005-2 to 330DAP. Sample collection in Juazeiro-BA was carried out at 200 DAP. Individual and Combined statistical analysis for the 6 tested sites. Means followed by the same letter do not differ by t test at the 5% level ($p \leq 0.05$). (SE: standard error).

| Locations | μg NptII / g Fresh Tissue | SE | μg NptII / g Dry Tissue | SE |
|---|---|---|---|---|
| Barrinha-SP | 0.01 a | 0.02 | 0.04 a | 0.05 |
| Piracicaba-SP | 0.01 a | 0 | 0.07 a | 0.02 |
| Valparaiso-SP | 0.01 a | 0.01 | 0.06 a | 0.03 |

(continued)

| Locations | μg NptII / g Fresh Tissue | SE | μg NptII / g Dry Tissue | SE |
|---|---|---|---|---|
| Quirinópolis-GO | 0.01 a | 0.01 | 0.04 a | 0.04 |
| Mandaguaçu-PR | 0.01 a | 0.01 | 0.05 a | 0.03 |
| Juazeiro - BA | 0.01 a | 0.02 | 0.06 a | 0.06 |
| Combined Analysis | 0.01 a | 0.01 | 0.05 a | 0.03 |

CrylAc protein expression data in roots from the event CTC79005-2 harvested at 330 DAP are shown in Table 13 and and Figure 10 (μg/g fresh and dry tissue).

Table 13. Comparison of the average expression of the CrylAc protein at the root of the CTC79005-2 event. Individual and Combined statistical analysis for the six tested sites. Sample collection in Juazeiro-BA was carried out at 200 DAP. Means followed by the same letter do not differ by t test at the 5% level ($p \leq 0.05$). (SE: standard error).

| Locations | μg Cry1Ac / g Fresh Tissue | SE | μg CrylAc / g Dry Tissue | SE |
|---|---|---|---|---|
| Barrinha-SP | 2.79 c | 0.98 | 9.25 c | 2.8 |
| Piracicaba-SP | 1.08 a | 2.26 | 5.40 a | 6.98 |
| Valparaiso-SP | 1.97 a | 6 | 9.55 a | 18.43 |
| Quirinópolis-GO | 4.36 a | 4.58 | 11.73 a | 12.93 |
| Mandaguaçu-PR | 2.23 c | 1.35 | 10.66 c | 3.93 |
| Juazeiro - BA | 1.83 a | 3.91 | 8.76 a | 11.71 |
| Combined Analysis | 2.38 a | 2.54 | 9.22 a | 7 |

NptII protein expression data in roots of the event of the invention collected at 330 DAP are shown in Table 14 and Figure 13 (μg/g fresh and dry tissue).

Table 14. Comparison of the averages of expression of the NPTII protein in root of the event CTC79005-2 to the 330DAP. Sample collection in Juazeiro-BA was carried out at 200 DAP. Individual and combined statistical analysis for the 6 tested sites. Means followed by the same letter do not differ by t test at the 5% level ($p \leq 0.05$). (SE: standard error).

| Location | μg NptII / g Fresh Tissue | SE | μg NptII / g Dry Tissue | SE |
|---|---|---|---|---|
| Barrinha-SP | 0.01 a | 0.02 | 0.02 a | 0.05 |
| Piracicaba-SP | 0.01 a | 0 | 0.05 a | 0.02 |
| Valparaiso-SP | 0.01 a | 0,01 | 0.04 a | 0.03 |
| Quirinópolis-GO | 0.01 a | 0.01 | 0.02 a | 0.04 |
| Mandaguaçu-PR | 0.01 a | 0.01 | 0.04 a | 0.03 |
| Juazeiro - BA | 0.01 a | 0.02 | 0.04 a | 0.06 |
| Combined Analysis | 0.01 a | 0.01 | 0.03 a | 0.03 |

The average expression of CrylAc protein in stalks from CTC79005-2 event in all locations was 6.35 μg/g of fresh tissue and 29.90 μg/g of dry tissue. For NptII, the average expression in stalks from CTC79005-2 event in all locations was 0.01 μg/g of fresh tissue and 0.06 μg/g of dry tissue.

The average expression of CrylAc protein in roots from CTC79005-2 event in all locations was 2.38 μg/g of fresh tissue and 9.23 μg/g of dry tissue. For NptII, the average expression in roots from CTC79005-2 event in all locations was 0.01 μg/g of fresh tissue and de 0.04 μg/g of dry tissue.

The results obtained for leaf, stem, and roots indicate that the event of the invention has Cry1Ac protein expression levels much higher than NptII expression.

The average concentration of Cry1Ac from leaves of the event of the invention at 200 DAP is lower than the average concentration at 100 DAP (dry tissue). Also, for NptII the average expression levels in leaves at 200 DAP is lower than the average concentration at other DAPs analyzed (100 and 300 DAP).

Cry1Ac and NptII protein expression levels from CTC79005-2 event at 330 DAP were much higher in leaves than in stalk and roots. Expression data of CrylAc and NptII proteins did not differ significantly between stalk and roots.

It is therefore concluded that the expression levels of CrylAc and NptII proteins from the event of the invention was characterized at different times, tissues and planting sites representative of its cultivation in Brazil. CrylAc protein expression levels, especially in the leaves of the event of the invention, remain high throughout the cultivation cycle, ensuring the intended effect of resistance to *Diatraea saccharalis*.

**Western blot**

For identification of the heterologous proteins expressed by the event of the invention, 50mg ($\pm$0,5 mg) of leaf frozen in liquid nitrogen was used for CryAc and 300 ($\pm$0,5 mg) for NptII. Maceration was performed in the TissueLyser equipment for 10 minutes at 25Hz, with the addition of three steel beads (3mm - Qiagen, DEU). To the macerated tissue, 500$\mu$l of Tween 20-supplemented saline phosphate extraction buffer (PBS) was added (0.138 M NaCl; 0.027 mM KCl; 0.05% Tween 20, pH 7.4) diluted according to manufacturer's instructions (Envirologix™, USA). After buffer addition, vortex homogenization was performed, and the mixture was centrifuged for 20 minutes at 4,000 RPM, 4 °C. The resulting supernatant was collected, and total protein was quantified. For NptII, the protein extract was concentrated before proceeding (Amicon® Ultra-0.5 Centrifugal Filter Devices (3000 NMWL).

Quantitation adopted for analysis of Cry1Ac and NptII proteins was performed according to the recommendations of ThermoScientific™ Coomassie Plus (Bradford) Protein Assay Kit (23236) - Microplate Procedure and BCA. Thus, the standards used for obtaining the calibration curve were the already-diluted commercial BSA (Bovine Serum Albumin) standards supplied with the kit described above. The 2000, 1000, 500, 250, 125, and 0 $\mu$g/ml calibrators prepared in PBST buffer were used. 10 $\mu$L of each standard calibrator was added to the plate wells in triplicate. The plates were covered and incubated for 5 minutes at room temperature. Absorbance was read at 595 nanometers (nm) using SoftmaxPro 7.0 software (Molecular Device, USA). Once the extraction of total proteins was carried out, 3 $\mu$g of protein extract were mixed with the 2X Laemmli Sample Buffer (Bio-Rad, USA) and denatured by heating at 100 °C for 5 minutes to identify the Cry1Ac protein present in the sample. In the case of nptII detection and identification, 40 $\mu$g of the total protein extract was mixed with the sample buffer (2X Laemmli Sample Buffer) and denatured.

As a negative control of the presence of heterologous proteins, 3 $\mu$g of protein extract from the conventional parental variety (WT - RB 92579) was used for CryAc experiments and 40$\mu$g for NptII assays. In addition, positive controls were prepared to detect Cry1Ac and *NptII* proteins. The first positive control was prepared using either 1 ng of CrylAc (~69 kDa, in house production) or 5 ng of NptII (~29 kDa, Bon Opus Biosciences, USA), diluted in total protein solution extracted from conventional parental variety (WT - RB92579) leaves. The second positive control was made by diluting 1 ng of purified CrylAc protein or 5 ng of NptII protein in PBST extraction buffer.

Samples were denatured and applied on 4-20% polyacrylamide gel (Mini-PROTEAN® TGXTM Precast Gel) submerged in Tris/glycine/SDS running buffer (Bio-Rad, USA) and separated by electrophoresis at 50 V for 5 minutes and then at 120V for approximately 90 minutes. Next, the polyacrylamide gels were equilibrated in Tris/Glycine Transfer Buffer (Bio-Rad, USA), which was added with 20% methanol for 10-15 minutes. The PVDF membrane was treated with absolute methanol. The transfer system was mounted in a container filled with the cold Transfer Buffer for immersion transfer ("wet transfer") at a constant voltage of 50 V for 3 hours. Upon completion of the transfer, the membrane was blocked for 16 hours at 4 °C, under constant agitation, in blocking solution [5% skimmed milk powder (Bio-Rad, USA) and TBS/T (20mM Tris, 150mM NaCl, 1% Tween20)] to prevent possible nonspecific membrane binding.

In the next step, the membrane was incubated with the primary antibody for 90 minutes to detect and confirm the presence and integrity of the Cry1Ac and NptII proteins. The polyclonal antibodies used in this assay were rabbit Anti-Cry1Ab (Fitzgerald, USA), which bind both Cry1Ac and Cry1Ab proteins; and rabbit Anti- NptII (#N6537- Sigma Aldrich USA), which reacts with NptII protein, diluted 1: 1000 in TBS/T (v/v).

The membrane was washed 3 times per 5 minute (3x5) in TBS/T and incubated with goat-produced HRP-conjugated secondary Anti-Rabbit antibody (Sigma Aldrich, USA) at a concentration of 1:20,000 or at a concentration of 1:5,000 - v/v (Fitzgerald, USA) for 60 minutes. After incubations, the membrane was again washed with TBS/T (3x5 minutes), and the enzyme-linked immunoassay was verified on Amersham Hyperfilm ECL X-ray films (GE Healthcare, USA) by Clarit Western ECL Substrate Kit substrate reaction (Biorad, USA) according to the manufacturer's instructions. X-ray film exposure to membrane ranged from 15 seconds to 3 minutes.

Western blot assays detected the expression of Cry1Ac and NptII proteins in the CTC79005-2 event (Figure 14). The expression profile of CrylAc protein appears as two immunoreactive bands from approximately ~66 kDa and ~69 kDa, which is commonly known as doublet and accepted as a product of intracellular proteolytic cleavage of Cry proteins in plant leaves, generally produced by removing of terminal amino acid residues by proteases released during plant tissue processing. Two other smaller bands with an approximate weight of ~ 45kDa and 32kDa were observed, which are also considered the product of proteolytic cleavage in plant leaves. All samples (R1-R4) are biological replicates of the event of the invention, obtained from four experimental plots at the Piracicaba site. As expected, the negative control (WT) showed no immunoreactivity. The positive control (WT+CP) where the CrylAc protein was added to the total protein extracted from the parental cultivar, presented the same two immunoreactive bands, from approximately 69 and 66kDa.

The protein for the *nptII* selection gene was also detected by the Western blot assay for the presence of an immunoreactive band in the expected size of ~ 29 kDa, in the two biological repetitions of the CTC79005-2 event used in the assay (Figure 15). The diagnostic band corresponding to NptII protein is also present in positive control diluted in the protein extract from the parental variety (RB92579).

Immunoreactive bands of molecular weight ~ 150, 75 and 50 kDa are observed in all samples comprising proteins derived from RB92579 (event specific, positive control and negative control), and is considered a non-specific immunoreaction of the antibody solution used in the assay with some protein present in the RB92579 variety protein profile (Figure 14).

The results of Western blot assays confirmed the presence and integrity of proteins Cry1Ac and NptII expressed by the event of the invention, CTC79005-2. The proteins expressed by the event of the invention are of the expected size, and no evidence of unexpected alternative forms of these proteins being expressed by said event was found.

Example 5. Biological tests: Susceptibility to the sugarcane borer *(D. saccharalis)*

Biological Assays (bioassays) with target pest D. saccharalis (cane borer) can also be used for detection and characterization of event CTC79005-2, demonstrating the efficacy on the pest control provided by the expressed insecticidal protein CrylAc. Different bioassays may be contemplated within the scope of the present invention: for example, Leaf Disk Assay, Screenhouse bioassays, Tissue Dilution Assays, among others.

For leaf disc assay, leaves of event CTC79005-2 plants were collected, cut into discs of 16 $mm^2$ and distributed in bioassay plates containing gelled agar. Each well from culture plates was infested with *D. saccharalis* (0-24h old) neonate and incubated at 27 ± 1°C, relative humidity 60 ± 10%, and photoperiod 12:12h (light: dark) for a period of 7 days. At the end of incubation, larval mortality and inhibition of larval development of surviving individuals were evaluated, and the relative efficacy was calculated:

$$\text{LDA Relative efficacy} = 100 - \left(\frac{\text{\%Live larvae of event}}{\text{\%Live larvae of control}}\right) \text{x } 100$$

The surviving larvae were submitted to image analysis by Digimizer software (v 4.6.1) for assessment of larval stage based on width of cephalic capsule. The larvae that did not reach the first instar were considered dead. Non-transgenic sugarcane varieties that are genetically very similar to the evaluated transgenic event can be used as assay controls.

To characterize event efficacy in controlling target pest *D. saccharalis* in laboratory, leaf disc assays were performed with plant tissue from CTC79005-2 event (30 DAP). Non-transgenic sugarcane RB92579 was used as control (CTC79-TC). The experimental design was completely randomized with four replicates per treatment. An average of 92% of mortality rate was observed when comparing the conventional variety (CT79-TC) and CTC79005-2 event after 7 days feeding with leaf discs. These data, when normalized (ABBOTT, 1925) by the results obtained by the conventional variety, present 92% relative effectiveness (Figure 18). Also, based on measurement of cephalic capsule width, it was observed that 100% of the surviving individuals did not develop beyond the first instar, evidencing high suppression in the development of *D. saccharalis* after feeding with the transgenic event (Figure 18).

For tissue dilution assays, the leaves of the CTC79005-2 event were sampled in the Agro/pheno fields of the Piracicaba, Valparaiso, Barrinha (SP) and Quirinópolis(GO), with sampling points at 100, 200 and 300 DAP. These samples were chopped, dehydrated in a freeze dryer and then macerated to obtain a uniform green powder. To prepare the bioassay plates, each quadrant of 16 cells received the sample diluted 25x with MS diet (Multiple Species), filling approximately 1 ml per cell. For infestation, 2 neonate larvae (0-24h of age) were transferred in each cell (32 caterpillars per quadrant). The plates were identified and incubated at a temperature of 27 ± 1 ° C, relative humidity 60 ± 10% and a 12:12 photoperiod (light: dark), for a period of 10 days. At the end of the incubation, each quadrant was evaluated for effective mortality and average mass of larvae. Effective mortality was calculated from the equation:

$$\text{Effective mortality} = \frac{(\text{n dead larvae} + \text{n larvae on 1st instar})}{\text{total number of live larvae}} \text{x } 100$$

Relative effectiveness for dilution tests were calculated using the equation:

$$\text{Relative effectiveness in dilution} = 100 - \left(\frac{\%\text{Live larvae of the event}}{\%\text{Live larvae of the control}}\right) \text{x } 100$$

Through the analysis of tissue dilution from the fields of Agro/pheno, it was possible to verify that the effectiveness, both for effective mortality and for mass reduction of larvae, of the event CTC79005-2 is 23% and 93%, respectively.

For screenhouse bioassays, seedlings of the transgenic event are planted in a screened nursery, where the plants are planted in the soil similar to natural environmental conditions, but in controlled environment to prevent the occurrence of natural infestations. At least 5 infestations are performed, containing 20-35 *D. saccharalis* eggs per tiller. The evaluation occurs when all infected stalks are harvested and cutting longitudinally to quantify the damage. Infestation Intensity is calculated dividing the number of internodes with damage by the total number of internodes, and the result was multiplied by 100 (Infestation Intensity). Percentage of Effective Damage was calculated, considering the total of internodes with damage caused by the insect divided by the total number of stalks evaluated in the plot.

Screenhouse trials were performed to characterize the efficacy of CTC79005-2 event in controlling borer attack in comparison to its parental variety RB 92579 (WT; non-transgenic) in a randomized block design, with 4 replications. Each experimental plot was composed by eight clumps of cane-plant that received 10 artificial infestations with approximately 30 eggs of *D. saccharalis* every 15 days. After eight months, the Infestation Intensity (II) and the effective Damage for both varieties were calculated. Relative efficacy was calculated according to the formula below:

$$relative\ efficacy\ (\%) = 1 - \left(\frac{\text{(average I.I.\% of the event)}}{\text{average I.I.\% of the controls}}\right)$$

Under the artificial infestation the event CTC79005-2 presented relative efficacy in controlling infestation by *D. saccharalis* of 99.2% and in controlling stalk damage (length) was almost 100% in relation to its non-transgenic parental variety RB92579 (Control). The damage caused by *D. saccharalis* in CTC79005-2 stalks was visibly lower than in the conventional sugarcane RB92579. There were statistically differences (t-test, $P < 0.05$) between CTC79005-2 and the non-transgenic variety RB 92579 in both parameters evaluated, showing that under massive infestation with *D. Saccharalis* the event suppressed the damages caused by the pest (Figure 16).

On sugarcane conventional production, *D. saccharalis* is considered controlled when the infestation intensity is lower than 3% (Gallo et al., 2002). For CTC79005-2, the intensity of infestation was lower than 0.2% reinforcing the event effectiveness to control its main target pest.

In addition to employing bioassays that use artificial infestations to observe the degree of infestation and damage caused by *D. saccharalis,* observations of infestation and damage percentage can also be made based on information collected directly from the fields where the event CTC79005-2 is grown. The I.I. (infestation intensity), for example, can be calculated for natural infestation evaluation by defining an experimental area for stalk sampling cutting and quantification the number of internodes with and without damages to obtain the infestation intensity (I.I.).

In the tests performed for the event of the invention, the infestation intensity (I.I.) was calculated in four experimental areas: Piracicaba, Barrinha, and Valparaiso (SP) and Quirinopolis (GO). The conventional variety RB92579 (parental; non transgenic) was used as control. This assay illustrates the resistance of the event CTC79005-2 to *D. saccharalis* infestation compared to the parental variety (RB92579): it was observed a lower intensity of infestation for CTC79005-2 plants in comparison to the parental variety (RB92579) in all the four experimental areas (Combined analysis; Figure 17). Event CTC79005-2 presented relative efficacy in controlling infestation by *D. saccharalis* of almost 100% in all locations, except for Barrinha, which showed 99.4% effectiveness. Thus, it appears that the event CTC79005-2 was, on average in the 4 locations, 99.8% more effective than conventional materials, for controlling *D. saccharalis* (Figure 17).

Having described examples of preferred embodiments, it should be understood that the scope of the present invention encompasses other possible variations and is limited only by the content of the appended claims, including the possible equivalents thereof.

SEQUENCE LISTING

SEQ ID NO: 1

tggcaggatatattgtggtgtaaacaaattgacgcttagacaacttaataacacattgcggacgtttttaatgtactgaattagtactg
atatcggtacccggggcggccgcgagctcgatccactagtaacggccgccagtgtgctggaattcgcccttggcgcgccgatct
agtaacatagatgacaccgcgcgcgataatttatcctagtttgcgcgctatattttgttttctatcgcgtattaaatgtataattgcggga
ctctaatcataaaaacccatctcataaataacgtcatgcattacatgttaattattacatgcttaacgtaattcaacagaaattatatga
taatcatcgcaagaccggcaacaggattcaatcttaagaaactttattgccaaatgtttgaacgatctcagaagaactcgtcaaga
aggcgatagaaggcgatgcgctgcgaatcgggagcggcgataccgtaaagcacgaggaagcggtcagcccattcgccgcc
aagctcttcagcaatatcacgggtagccaacgctatgtcctgatagcggtccgccacacccagccggccacagtcgatgaatcc
agaaaagcggccattttccaccatgatattcggcaagcaggcatcgccatgggtcacgacgagatcctcgccgtcgggcatgcg
cgccttgagcctggcgaacagttcggctggcgcgagcccctgatgctcttcgtccagatcatcctgatcgacaagaccggcttcca
tccgagtacgtgctcgctcgatgcgatgtttcgcttggtggtcgaatgggcaggtagccggatcaagcgtatgcagccgccgcatt
gcatcagccatgatggatactttctcggcaggagcaaggtgagatgacaggagatcctgccccggcacttcgcccaatagcagc
cagtcccttcccgcttcagtgacaacgtcgagcacagctgcgcaaggaacgcccgtcgtggccagccacgatagccgcgctgc
ctcgtcctgcagttcattcagggcaccggacaggtcggtcttgacaaaaagaaccgggcgcccctgcgctgacagccggaaca
cggcggcatcagagcagccgattgtctgttgtgcccagtcatagccgaatagcctctccacccaagcggccggagaacctgcgt
gcaatccatcttgttcaatccacattctagagtcgacctgcagaagtaacaccaaacaacagggtgagcatcgacaaaagaaa
cagtaccaagcaaataaatagcgtatgaaggcagggctaaaaaaatccacatatagctgctgcatatgccatcatccaagtata
tcaagatcaaaataattataaaacatacttgtttattataatagataggtactcaaggttagagcatatgaatagatgctgcatatgcc
atcatgtatatgcatcagtaaaacccacatcaacatgtatacctatcctagatcgatatttccatccatcttaaactcgtaactatgaa
gatgtatgacacacacatacagttccaaaattaataaatacaccaggtagtttgaaacagtattctactccgatctagaacgaatg
aacgaccgcccaaccacaccacatcatcacaaccaagcgaacaaaaagcatctctgtatatgcatcagtaaaacccgcatca
acatgtatacctatcctagatcgatatttccatccatcatcttcaattcgtaactatgaatatgtatggcacacacatacagatccaaa
attaataaatccaccaggtagtttgaaacagaattctactccgatctagaacgaccgcccaaccagaccacatcatcacaacca
agacaaaaaaaagcatgaaaagatgacccgacaaacaagtgcacggcatatattgaaataaaggaaaagggcaaaccaa
accctatgcaacgaaacaaaaaaaatcatgaaatcgatcccgtctgcggaacggctagagccatcccaggattccccaaaga
gaaacactggcaagttagcaatcagaacgtgtctgacgtacaggtcgcatccgtgtacgaacgctagcagcacggatctaaca
caaacacggatctaacacaaacatgaacagaagtagaactaccgggccctaaccatggaccggaacgccgatctagagaa
ggtagagaggggggggggggggaggacgagcggcgtaccttgaagcggaggtgccgacgggtggatttgggggagatctggt
tgtgtgtgtgtgcgctccgaacaacacgaggttggggaaagagggtgtggagggggtgtctatttattacggcgggcgaggaag
ggaaagcgaaggagcggtgggaaaggaatcccccgtagctgccgtgccgtgagaggaggaggaggccgcctgccgtgccg
gctcacgtctgccgctccgccacgcaatttctggatgccgacagcggagcaagtccaacggtggagcggaactctcgagaggg
gtccagaggcagcgacagagatgccgtgccgtctgcttcgcttggcccgacgcgacgctgctggttcgctggttggtgtccgttag
actcgtcgacggcgtttaacaggctggcattatctactcgaaacaagaaaaatgtttccttagtttttttaatttcttaaagggtatttgttt
aatttttagtcactttattttattctattttatatctaaattattaaataaaaaaactaaaatagagttttagttttcttaatttagaggctaaaat
agaataaaatagatgtactaaaaaaattagtctataaaaaccattaaccctaaaccctaaatggatgtactaataaaatggatga
agtattatataggtgaagctatttgcaaaaaaaaaggagaacacatgcacactaaaaagataaaactgtagagtcctgttgtcaa
aatactcaattgtcctttagaccatgtctaactgttcatttatatgattctctaaaacactgatattattgtagtactatagattatattattcgt
agagtaaagtttaaatatatgtataaagatagataaactgcacttcaaacaagtgtgacaaaaaaaatatgtggtaatttttttataac
ttagacatgcaatgctcattatctctagagaggggcacgaccgggtcacgctgcactgcaggcatacgcgtgatccactagtaac
ggccgccagtgtgctggaattcgcccttggcgcgccgatctagtaacatagatgacaccgcgcgcgataatttatcctagtttgcgc
gctatattttgttttctatcgcgtattaaatgtataattgcgggactctaatcataaaaacccatctcataaataacgtcatgcattacatg
ttaattattacatgcttaacgtaattcaacagaaattatatgataatcatcgcaagaccggcaacaggattcaatcttaagaaacttta
ttgccaaatgtttgaacgatctcactcagcctcgagggtggcggtcactgggatgaactcgaacctgtcgatgatcacgccggcg
gtgccgctgaagttcctcacgcccacgatgttaccgagggaggaggtgaaagcgttggcgctctcgaagtaaccgaagtcgctg

gattggaggttgtccagggaggtagcggtagctggcacggtgttggagaagatggaggagttaccccagttcacgttgaggtgg
atcggggtcacggaagcgtacctcacgcgcaccctgtacctggtggaggtggatgggaagtggattggcacctcgatgtagccc
ctgttctggatgttgttgccgctgctgttgagcctcacgaggtcgccaccggtgaagcctgggccggaaatgacggaaccgttgaa
gaggaagttgcccttcacggccgggatttgggtgatgctgtcggaggcgatgatgttgttgaactcagcgctgcggtggatccagg
agaacatcggagccctgatgatgctcacggagctgttgctgaagccggagcggaacatggacacgtggctcagcctgtgggag
aagccttgcctgggtggcacgttgttgttctgtggtgggatctcgtccagggagtccacggtgccgctcttcctgtagacagcggatg
gcaggttggaggaggtgccgtaggcgaactcggtgccgtcgagcacggacagttgctggttgttgataccgatgttgaagggcct
cctgtacagggtggaggacagggtcctgtagacaccctgacccagttgagccacgatgcgttgctgtggagcggcgttgcccat
ggtgccgtagagcgggaaggtgaactcggggccgctgaagcccactggggaggccatgatctggtggccggaccagtagtac
tcgcccctgtgagcgtcggtgtagatggtgatgctgttcaggatgtccatcaggtgtgggctcctgatggagccctcgataccctgg
gcggaaccgcggaagctaccgtcgaagttctccagcactgggttggtgtagatctccctggtgagttgggacacggtgcggatcg
ggtaggtcctggagtcgtagttcgggaagagggacacaatgtccagcacggtgagggtcaactccctcctgaactggttgtacct
gatccagtccctggagtccggaccccagacgcgctccaggccggtgttgtaccagcgcacagcgtggtcggtgtagttgccaat
cagcctggtcaggtcgttgtagcggctgttgatggtggcagcatcgaagccccacctttggccgaacacgctcacgtcgcgcagc
acgctgaggtgcaggttagcggcttgcacgtacacggacaggagcggcacttggtagttctggacggcgaacagtgggatagc
ggtggtcagggcgctgttcatgtcgttgaattgaatgcgcatttcctcgcggagagctgggttggtcgggtcggcctcccactccctg
aagctctcggcgtagatttggtagaggttgctcaggccctccagcctggagatggcctggttcctggcgaactcttcgatcctctggtt
gatcagctgctcgatttgcaccaggaaggcgtcccattgggatggaccgaagataccccagatgatgtccaccaggccgagca
cgaagccagcacctggcacgaactcgctgagcaggaactgggtcaaggacagggagatgtcgatgggggtgtaaccggtctc
gatgcgctcgccacccagcacctccacctctgggttgctcaggcagttgtatgggatgcactcgttgatgtttgggttgttgtccattct
agagtcgacctgcagaagtaacaccaaacaacagggtgagcatcgacaaaagaaacagtaccaagcaaataaatagcgta
tgaaggcagggctaaaaaaatccacatatagctgctgcatatgccatcatccaagtatatcaagatcaaaataattataaaacat
acttgtttattataatagataggtactcaaggttagagcatatgaatagatgctgcatatgccatcatgtatatgcatcagtaaaaccc
acatcaacatgtatacctatcctagatcgatatttccatccatcttaaactcgtaactatgaagatgtatgacacacacatacagttcc
aaaattaataaatacaccaggtagtttgaaacagtattctactccgatctagaacgaatgaacgaccgcccaaccacaccacat
catcacaaccaagcgaacaaaaagcatctctgtatatgcatcagtaaaacccgcatcaacatgtatacctatcctagatcgatatt
tccatccatcatcttcaattcgtaactatgaatatgtatggcacacacatacagatccaaaattaataaatccaccaggtagtttgaa
acagaattctactccgatctagaacgaccgcccaaccagaccacatcatcacaaccaagacaaaaaaaagcatgaaaagat
gacccgacaaacaagtgcacggcatatattgaaataaaggaaaagggcaaaccaaaccctatgcaacgaaacaaaaaaa
atcatgaaatcgatcccgtctgcggaacggctagagccatcccaggattccccaaagagaaacactggcaagttagcaatcag
aacgtgtctgacgtacaggtcgcatccgtgtacgaacgctagcagcacggatctaacacaaacacggatctaacacaaacatg
aacagaagtagaactaccgggccctaaccatggaccggaacgccgatctagagaaggtagagagggggggggggggagg
acgagcggcgtaccttgaagcggaggtgccgacgggtggatttgggggagatctggttgtgtgtgtgtgcgctccgaacaacac
gaggttggggaaagagggtgtggagggggtgtctatttattacggcgggcgaggaagggaaagcgaaggagcggtgggaaa
ggaatcccccgtagctgccgtgccgtgagaggaggaggaggccgcctgccgtgccggctcacgtctgccgctccgccacgca
atttctggatgccgacagcggagcaagtccaacggtggagcggaactctcgagaggggtccagaggcagcgacagagatgc
cgtgccgtctgcttcgcttggcccgacgcgacgctgctggttcgctggttggtgtccgttagactcgtcgacggcgtttaacaggctg
gcattatctactcgaaacaagaaaaatgtttccttagtttttttaatttcttaaagggtatttgtttaatttttagtcactttattttattctattttat
atctaaattattaaataaaaaaactaaaatagagttttagttttcttaatttagaggctaaaatagaataaaatagatgtactaaaaaa
attagtctataaaaaccattaaccctaaaccctaaatggatgtactaataaaatggatgaagtattatataggtgaagctatttgcaa
aaaaaaaggagaacacatgcacactaaaaagataaaactgtagagtcctgttgtcaaaatactcaattgtcctttagaccatgtct
aactgttcatttatatgattctctaaaacactgatattattgtagtactatagattatattattcgtagagtaaagtttaaatatatgtataaa
gatagataaactgcacttcaaacaagtgtgacaaaaaaaaatatgtggtaattttttataacttagacatgcaatgctcattatctctag
agaggggcacgaccgggtcacgctgcactgcaggcatcgatcgaagcttacgcgtccatggctgcaggagctcgtcgactcta
gactcgagggcgcgccgacaggatatattggcgggtaaacc

SEQ ID NO: 2

ttgtggtgtaaacaaattgacgcttagacaacttaataacacattgcggacgtttttaatgtactgaattagtactgatatcggtacccg
gggcggccgcgagctcgatccactagtaacggccgccagtgtgctggaattcgcccttggcgcgccgatctagtaacatagatg
acaccgcgcgcgataatttatcctagtttgcgcgctatattttgttttctatcgcgtattaaatgtataattgcgggactctaatcataaaa
acccatctcataaataacgtcatgcattacatgttaattattacatgcttaacgtaattcaacagaaattatatgataatcatcgcaag
accggcaacaggattcaatcttaagaaactttattgccaaatgtttgaacgatctcagaagaactcgtcaagaaggcgatagaag
gcgatgcgctgcgaatcgggagcggcgataccgtaaagcacgaggaagcggtcagcccattcgccgccaagctcttcagcaa
tatcacgggtagccaacgctatgtcctgatagcggtccgccacacccagccggccacagtcgatgaatccagaaaagcggcc
attttccaccatgatattcggcaagcaggcatcgccatgggtcacgacgagatcctcgccgtcgggcatgcgcgccttgagcctg
gcgaacagttcggctggcgcgagcccctgatgctcttcgtccagatcatcctgatcgacaagaccggcttccatccgagtacgtgc
tcgctcgatgcgatgtttcgcttggtggtcgaatgggcaggtagccggatcaagcgtatgcagccgccgcattgcatcagccatga
tggatactttctcggcaggagcaaggtgagatgacaggagatcctgccccggcacttcgcccaatagcagccagtcccttcccg
cttcagtgacaacgtcgagcacagctgcgcaaggaacgcccgtcgtggccagccacgatagccgcgctgcctcgtcctgcagtt
cattcagggcaccggacaggtcggtcttgacaaaaagaaccgggcgcccctgcgctgacagccggaacacggcggcatcag
agcagccgattgtctgttgtgcccagtcatagccgaatagcctctccacccaagcggccggagaacctgcgtgcaatccatcttgt
tcaatccacattctagagtcgacctgcagaagtaacaccaaacaacagggtgagcatcgacaaaagaaacagtaccaagca
aataaatagcgtatgaaggcagggctaaaaaaatccacatatagctgctgcatatgccatcatccaagtatatcaagatcaaaat
aattataaaacatacttgtttattataatagataggtactcaaggttagagcatatgaatagatgctgcatatgccatcatgtatatgca
tcagtaaaacccacatcaacatgtatacctatcctagatcgatatttccatccatcttaaactcgtaactatgaagatgtatgacaca
cacatacagttccaaaattaataaatacaccaggtagtttgaaacagtattctactccgatctagaacgaatgaacgaccgccca
accacaccacatcatcacaaccaagcgaacaaaaagcatctctgtatatgcatcagtaaaacccgcatcaacatgtatacctat
cctagatcgatatttccatccatcatcttcaattcgtaactatgaatatgtatggcacacacatacagatccaaaattaataaatccac
caggtagtttgaaacagaattctactccgatctagaacgaccgcccaaccagaccacatcatcacaaccaagacaaaaaaa
gcatgaaaagatgacccgacaaacaagtgcacggcatatattgaaataaaggaaagggcaaaccaaaccctatgcaacg
aaacaaaaaaaatcatgaaatcgatcccgtctgcggaacggctagagccatcccaggattccccaaagagaaacactggca
agttagcaatcagaacgtgtctgacgtacaggtcgcatccgtgtacgaacgctagcagcacggatctaacacaaacacggatct
aacacaaacatgaacagaagtagaactaccgggccctaaccatggaccggaacgccgatctagagaaggtagagagggg
gggggggggaggacgagcggcgtaccttgaagcggaggtgccgacgggtggatttgggggagatctggttgtgtgtgtgtgcg
ctccgaacaacacgaggttggggaaagagggtgtggagggggtgtctatttattacggcgggcgaggaagggaaagcgaag
gagcggtgggaaaggaatcccccgtagctgccgtgccgtgagaggaggaggaggccgcctgccgtgccggctcacgtctgcc
gctccgccacgcaatttctggatgccgacagcggagcaagtccaacggtggagcggaactctcgagaggggtccagaggca
gcgacagagatgccgtgccgtctgcttcgcttggcccgacgcgacgctgctggttcgctggttggtgtccgttagactcgtcgacgg
cgtttaacaggctggcattatctactcgaaacaagaaaaatgtttccttagtttttttaatttcttaaagggtatttgtttaatttttagtcactt
tattttattctattttatatctaaattattaaataaaaaaactaaaatagagttttagttttcttaatttagaggctaaaatagaataaaatag
atgtactaaaaaaattagtctataaaaaccattaaccctaaaccctaaatggatgtactaataaaatggatgaagtattatataggt
gaagctatttgcaaaaaaaaaggagaacacatgcacactaaaaagataaaactgtagagtcctgttgtcaaaatactcaattgtc
ctttagaccatgtctaactgttcatttatatgattctctaaaacactgatattattgtagtactatagattatattattcgtagagtaaagttta
aatatatgtataaagatagataaactgcacttcaaacaagtgtgacaaaaaaaaatatgtggtaatttttataacttagacatgcaat
gctcattatctctagagaggggcacgaccgggtcacgctgcactgcaggcatacgcgtgatccactagtaacggccgccagtgt
gctggaattcgcccttggcgcgccgatctagtaacatagatgacaccgcgcgcgataatttatcctagtttgcgcgctatattttgtttt
ctatcgcgtattaaatgtataattgcgggactctaatcataaaaacccatctcataaataacgtcatgcattacatgttaattattacat
gcttaacgtaattcaacagaaattatatgataatcatcgcaagaccggcaacaggattcaatcttaagaaactttattgccaaatgtt
tgaacgatctcactcagcctcgagggtggcggtcactgggatgaactcgaacctgtcgatgatcacgccggcggtgccgctgaa
gttcctcacgcccacgatgttaccgagggaggaggtgaaagcgttggcgctctcgaagtaaccgaagtcgctggattggaggttg
tccagggaggtagcggtagctggcacggtgttggagaagatggaggagttaccccagttcacgttgaggtggatcggggtcacg
gaagcgtacctcacgcgcaccctgtacctggtggaggtggatgggaagtggattggcacctcgatgtagcccctgttctggatgtt
gttgccgctgctgttgagcctcacgaggtcgccaccggtgaagcctgggccggaaatgacggaaccgttgaagaggaagttgc

ccttcacggccgggatttgggtgatgctgtcggaggcgatgatgttgttgaactcagcgctgcggtggatccaggagaacatcgg agccctgatgatgctcacggagctgttgctgaagccggagcggaacatggacacgtggctcagcctgtgggagaagccttgcct gggtggcacgttgttgttctgtggtgggatctcgtccagggagtccacggtgccgctcttcctgtagacagcggatggcaggttgga ggaggtgccgtaggcgaactcggtgccgtcgagcacggacagttgctggttgttgataccgatgttgaagggcctcctgtacagg gtggaggacagggtcctgtagacaccctgacccagttgagccacgatgcgttgctgtggagcggcgttgcccatggtgccgtag agcgggaaggtgaactcggggccgctgaagcccactggggaggccatgatctggtggccggaccagtagtactcgcccctgt gagcgtcggtgtagatggtgatgctgttcaggatgtccatcaggtgtgggctcctgatggagccctcgataccctgggcggaaccg cggaagctaccgtcgaagttctccagcactgggttggtgtagatctccctggtgagttgggacacggtgcggatcgggtaggtcct ggagtcgtagttcgggaagagggacacaatgtccagcacggtgagggtcaactccctcctgaactggttgtacctgatccagtcc ctggagtccggaccccagacgcgctccaggccggtgttgtaccagcgcacagcgtggtcggtgtagttgccaatcagcctggtc aggtcgttgtagcggctgttgatggtggcagcatcgaagccccacctttggccgaacacgctcacgtcgcgcagcacgctgaggt gcaggttagcggcttgcacgtacacggacaggagcggcacttggtagttctggacggcgaacagtgggatagcggtggtcagg gcgctgttcatgtcgttgaattgaatgcgcatttcctcgcggagagctgggttggtcgggtcggcctcccactccctgaagctctcgg cgtagatttggtagaggttgctcaggccctccagcctggagatggcctggttcctggcgaactcttcgatcctctggttgatcagctgc tcgatttgcaccaggaaggcgtcccattgggatggaccgaagataccccagatgatgtccaccaggccgagcacgaagccag cacctggcacgaactcgctgagcaggaactgggtcaaggacagggagatgtcgatgggggtgtaaccggtctcgatgcgctcg ccacccagcacctccacctctgggttgctcaggcagttgtatgggatgcactcgttgatgtttgggttgttgtccattctagagtcgacc tgcagaagtaacaccaaacaacagggtgagcatcgacaaaagtgctgcatatgccatcatccaagtatatcaagatcaaaata attataaaacatacttgtttattataatagataggtactcaaggttagagcatatgaatagatgctgcatatgccatcatgtatatgcat cagtaaaacccacatcaacatgtatacctatcctagatcgatatttccatccatcttaaactcgtaactatgaagatgtatgacacac acatacagttccaaaattaataaatacaccaggtagtttgaaacagtattctactccgatctagaacgaatgaacgaccgcccaa ccacaccacatcatcacaaccaagcgaacaaaaagcatctctgtatatgcatcagtaaaacccgcatcaacatgtatacctatc ctagatcgatatttccatccatcatcttcaattcgtaactatgaatatgtatggcacacacatacagatccaaaattaataaatccacc aggtagtttgaaacagaattctactccgatctagaacgaccgcccaaccagaccacatcatcacaaccaagacaaaaaaaag catgaaaagatgacccgacaaacaagtgcacggcatatattgaaataaaggaaaagggcaaaccaaaccctatgcaacga aacaaaaaaaaatcatgaaatcgatcccgtctgcggaacggctagagccatcccaggattccccaaagagaaacactggcaa gttagcaatcagaacgtgtctgacgtacaggtcgcatccgtgtacgaacgctagcagcacggatctaacacaaacacggatcta acacaaacatgaacagaagtagaactaccgggccctaaccatggaccggaacgccgatctagagaaggtagagaggggg gggggggaggacgagcggcgtaccttgaagcggaggtgccgacgggtggatttgggggagatctggttgtgtgtgtgtgcgct ccgaacaacacgaggttggggaaagagggtgtggaggggggtgtctatttattacggcgggcgaggaagggaaagcgaagga gcggtgggaaaggaatcccccgtagctgccgtgccgtgagaggaggaggaggccgcctgccgtgccggctcacgtctgccgc tccgccacgcaatttctggatgccgacagcggagcaagtccaacggtggagcggaactctcgagaggggtccagaggcagc gacagagatgccgtgccgtctgcttcgcttggcccgacgcgacgctgctggttcgctggttggtgtccgttagactcgtcgacggcg tttaacaggctggcattatctactcgaaacaagaaaaatgtttccttagtttttttaatttcttaaagggtatttgtttaatttttagtcactttat tttattctattttatatctaaattattaaataaaaaaaactaaaatagagttttagttttcttaatttagaggctaaaatagaataaaatagat gtactaaaaaaattagtctataaaaaccattaaccctaaaccctaaatggatgtactaataaaatggatgaagtattatataggtga agctatttgcaaaaaaaaaggagaacacatgcacactaaaaagataaaactgtagagtcctgttgtcaaaatactcaattgtcctt tagaccatgtctaactgttcatttatatgattctctaaaacactgatattattgtagtactatagattatattattcgtagagtaaagtttaaa tatatgtataaagatagataaactgcacttcaaacaagtgtgacaaaaaaaaatatgtggtaatttttataacttagacatgcaatgct cattatctctagagaggggcacgaccgggtcacgctgcactgcaggcatcgatcgaagcttacgcgtccatggctgcaggagct cgtcgactctagactcgagggcgagcc

SEQ ID NO: 3

ggagaggcgaagccctaggccggcgctgtcgccacgccaggatcggtcgcccaggttgacgtgactgggttgcctgcgctgaa cctaataacacattgctgggttgtggtgtaaacaaattgacgcttagacaacttaataacacattgcggacgtttttaatgtactgaatt agtactgatatcggtacccggggcggccgcgag

SEQ ID NO: 4

ctagagaggggcacgaccgggtcacgctgcactgcaggcatcgatcgaagcttacgcgtccatggctgcaggagctcgtcgac
tctagactcgagggcgagccccgagcgggggcagctgaggcgccattgggaggaggaagagggcagcgctccggggcgg
aagtcgtggggcggcacgagggaagggcacacgacggacggacg

SEQ ID NO: 5:

ggagaggcgaagccctaggccggcgctgtcgccacgccaggatcggtcgcccaggttgacgtgactgggttgcctgcgctgaa
cctaataacacattgctgggttgtggtgtaaacaaattgacgcttagacaacttaataacacattgcggacgtttttaatgtactgaatt
agtactgatatcggtacccggggcggccgcgagctcgatccactagtaacggccgccagtgtgctggaattcgcccttggcgcg
ccgatctagtaacatagatgacaccgcgcgcgataatttatcctagtttgcgcgctatattttgttttctatcgcgtattaaatgtataatt
gcgggactctaatcataaaaacccatctcataaataacgtcatgcattacatgttaattattacatgcttaacgtaattcaacagaaa
ttatatgataatcatcgcaagaccggcaacaggattcaatcttaagaaactttattgccaaatgtttgaacgatctcagaagaactc
gtcaagaaggcgatagaaggcgatgcgctgcgaatcgggagcggcgataccgtaaagcacgaggaagcggtcagcccattc
gccgccaagctcttcagcaatatcacgggtagccaacgctatgtcctgatagcggtccgccacacccagccggccacagtcgat
gaatccagaaaagcggccattttccaccatgatattcggcaagcaggcatcgccatgggtcacgacgagatcctcgccgtcggg
catgcgcgccttgagcctggcgaacagttcggctggcgcgagcccctgatgctcttcgtccagatcatcctgatcgacaagaccg
gcttccatccgagtacgtgctcgctcgatgcgatgtttcgcttggtggtcgaatgggcaggtagccggatcaagcgtatgcagccg
ccgcattgcatcagccatgatggatactttctcggcaggagcaaggtgagatgacaggagatcctgccccggcacttcgcccaat
agcagccagtcccttcccgcttcagtgacaacgtcgagcacagctgcgcaaggaacgcccgtcgtggccagccacgatagcc
gcgctgcctcgtcctgcagttcattcagggcaccggacaggtcggtcttgacaaaaagaaccgggcgcccctgcgctgacagcc
ggaacacggcggcatcagagcagccgattgtctgttgtgcccagtcatagccgaatagcctctccacccaagcggccggagaa
cctgcgtgcaatccatcttgttcaatccacattctagagtcgacctgcagaagtaacaccaaacaacagggtgagcatcgacaaa
agaaacagtaccaagcaaataaatagcgtatgaaggcagggctaaaaaaatccacatatagctgctgcatatgccatcatcca
agtatatcaagatcaaaataattataaaacatacttgtttattataatagataggtactcaaggttagagcatatgaatagatgctgca
tatgccatcatgtatatgcatcagtaaaacccacatcaacatgtatacctatcctagatcgatatttccatccatcttaaactcgtaact
atgaagatgtatgacacacacatacagttccaaaattaataaatacaccaggtagtttgaaacagtattctactccgatctagaac
gaatgaacgaccgcccaaccacaccacatcatcacaaccaagcgaacaaaaagcatctctgtatatgcatcagtaaaacccg
catcaacatgtatacctatcctagatcgatatttccatccatcatcttcaattcgtaactatgaatatgtatggcacacacatacagatc
caaaattaataaatccaccaggtagtttgaaacagaattctactccgatctagaacgaccgcccaaccagaccacatcatcaca
accaagacaaaaaaaagcatgaaaagatgacccgacaaacaagtgcacggcatatattgaaataaaggaaaagggcaaa
ccaaaccctatgcaacgaaacaaaaaaaatcatgaaatcgatcccgtctgcggaacggctagagccatcccaggattcccca
aagagaaacactggcaagttagcaatcagaacgtgtctgacgtacaggtcgcatccgtgtacgaacgctagcagcacggatct
aacacaaacacggatctaacacaaacatgaacagaagtagaactaccgggccctaaccatggaccggaacgccgatctag
agaaggtagagagggggggggggggaggacgagcggcgtaccttgaagcggaggtgccgacgggtggatttgggggagat
ctggttgtgtgtgtgtgcgctccgaacaacacgaggttggggaaagagggtgtggagggggtgtctatttattacggcgggcgag
gaagggaaagcgaaggagcggtgggaaaggaatcccccgtagctgccgtgccgtgagaggaggaggaggccgcctgccg
tgccggctcacgtctgccgctccgccacgcaatttctggatgccgacagcggagcaagtccaacggtggagcggaactctcga
gaggggtccagaggcagcgacagagatgccgtgccgtctgcttcgcttggcccgacgcgacgctgctggttcgctggttggtgtc
cgttagactcgtcgacggcgtttaacaggctggcattatctactcgaaacaagaaaaatgtttccttagtttttttaatttcttaaagggt
atttgtttaatttttagtcactttattttattctattttatatctaaattattaaataaaaaaactaaaatagagttttagttttcttaatttagaggc
taaaatagaataaaatagatgtactaaaaaaattagtctataaaaaccattaaccctaaaccctaaatggatgtactaataaaatg
gatgaagtattatataggtgaagctatttgcaaaaaaaaaaggagaacacatgcacactaaaaagataaaactgtagagtcctgtt
gtcaaaatactcaattgtcctttagaccatgtctaactgttcatttatatgattctctaaaacactgatattattgtagtactatagattatatt
attcgtagagtaaagtttaaatatatgtataaagatagataaactgcacttcaaacaagtgtgacaaaaaaaatatgtggtaattttttt
ataacttagacatgcaatgctcattatctctagagaggggcacgaccgggtcacgctgcactgcaggcatacgcgtgatccacta

gtaacggccgccagtgtgctggaattcgcccttggcgcgccgatctagtaacatagatgacaccgcgcgcgataatttatcctagtt tgcgcgctatattttgttttctatcgcgtattaaatgtataattgcgggactctaatcataaaaacccatctcataaataacgtcatgcatt acatgttaattattacatgcttaacgtaattcaacagaaattatatgataatcatcgcaagaccggcaacaggattcaatcttaagaa actttattgccaaatgtttgaacgatctcactcagcctcgagggtggcggtcactgggatgaactcgaacctgtcgatgatcacgcc ggcggtgccgctgaagttcctcacgcccacgatgttaccgagggaggaggtgaaagcgttggcgctctcgaagtaaccgaagt cgctggattggaggttgtccagggaggtagcggtagctggcacggtgttggagaagatggaggagttaccccagttcacgttgag gtggatcggggtcacggaagcgtacctcacgcgcaccctgtacctggtggaggtggatgggaagtggattggcacctcgatgta gcccctgttctggatgttgttgccgctgctgttgagcctcacgaggtcgccaccggtgaagcctgggccggaaatgacggaaccgt tgaagaggaagttgcccttcacggccgggatttgggtgatgctgtcggaggcgatgatgttgttgaactcagcgctgcggtggatc caggagaacatcggagccctgatgatgctcacggagctgttgctgaagccggagcggaacatggacacgtggctcagcctgtg ggagaagccttgcctgggtggcacgttgttgttctgtggtgggatctcgtccagggagtccacggtgccgctcttcctgtagacagc ggatggcaggttggaggaggtgccgtaggcgaactcggtgccgtcgagcacggacagttgctggttgttgataccgatgttgaag ggcctcctgtacagggtggaggacagggtcctgtagacaccctgacccagttgagccacgatgcgttgctgtggagcggcgttg cccatggtgccgtagagcgggaaggtgaactcggggccgctgaagcccactggggaggccatgatctggtggccggaccagt agtactcgcccctgtgagcgtcggtgtagatggtgatgctgttcaggatgtccatcaggtgtgggctcctgatggagccctcgatac cctgggcggaaccgcggaagctaccgtcgaagttctccagcactgggttggtgtagatctccctggtgagttgggacacggtgcg gatcgggtaggtcctggagtcgtagttcgggaagagggacacaatgtccagcacggtgagggtcaactccctcctgaactggttg tacctgatccagtccctggagtccggaccccagacgcgctccaggccggtgttgtaccagcgcacagcgtggtcggtgtagttgc caatcagcctggtcaggtcgttgtagcggctgttgatggtggcagcatcgaagccccacctttggccgaacacgctcacgtcgcg cagcacgctgaggtgcaggttagcggcttgcacgtacacggacaggagcggcacttggtagttctggacggcgaacagtggga tagcggtggtcagggcgctgttcatgtcgttgaattgaatgcgcatttcctcgcggagagctgggttggtcgggtcggcctcccactc cctgaagctctcggcgtagatttggtagaggttgctcaggccctccagcctggagatggcctggttcctggcgaactcttcgatcctc tggttgatcagctgctcgatttgcaccaggaaggcgtcccattgggatggaccgaagataccccagatgatgtccaccaggccg agcacgaagccagcacctggcacgaactcgctgagcaggaactgggtcaaggacagggagatgtcgatgggggtgtaaccg gtctcgatgcgctcgccacccagcacctccacctctgggttgctcaggcagttgtatgggatgcactcgttgatgtttgggttgttgtcc attctagagtcgacctgcagaagtaacaccaaacaacagggtgagcatcgacaaaagtgctgcatatgccatcatccaagtata tcaagatcaaaataattataaaacatacttgtttattataatagataggtactcaaggttagagcatatgaatagatgctgcatatgcc atcatgtatatgcatcagtaaaacccacatcaacatgtatacctatcctagatcgatatttccatccatcttaaactcgtaactatgaa gatgtatgacacacacatacagttccaaaattaataaatacaccaggtagtttgaaacagtattctactccgatctagaacgaatg aacgaccgcccaaccacaccacatcatcacaaccaagcgaacaaaaagcatctctgtatatgcatcagtaaaacccgcatca acatgtatacctatcctagatcgatatttccatccatcatcttcaattcgtaactatgaatatgtatggcacacacatacagatccaaa attaataaatccaccaggtagtttgaaacagaattctactccgatctagaacgaccgcccaaccagaccacatcatcacaacca agacaaaaaaaagcatgaaaagatgacccgacaaacaagtgcacggcatatattgaaataaaggaaaagggcaaaccaa accctatgcaacgaaacaaaaaaaatcatgaaatcgatcccgtctgcggaacggctagagccatcccaggattccccaaaga gaaacactggcaagttagcaatcagaacgtgtctgacgtacaggtcgcatccgtgtacgaacgctagcagcacggatctaaca caaacacggatctaacacaaacatgaacagaagtagaactaccgggccctaaccatggaccggaacgccgatctagagaa ggtagagaggggggggggggggaggacgagcggcgtaccttgaagcggaggtgccgacgggtggatttgggggagatctggt tgtgtgtgtgtgcgctccgaacaacacgaggttggggaaagagggtgtggagggggtgtctatttattacggcgggcgaggaag ggaaagcgaaggagcggtgggaaaggaatcccccgtagctgccgtgccgtgagaggaggaggaggccgcctgccgtgccg gctcacgtctgccgctccgccacgcaatttctggatgccgacagcggagcaagtccaacggtggagcggaactctcgagaggg gtccagaggcagcgacagagatgccgtgccgtctgcttcgcttggcccgacgcgacgctgctggttcgctggttggtgtccgttag actcgtcgacggcgtttaacaggctggcattatctactcgaaacaagaaaaatgtttccttagtttttttaatttcttaaagggtatttgttt aatttttagtcactttattttattctattttatatctaaattattaaataaaaaaactaaaatagagttttagttttcttaatttagaggctaaaat agaataaaatagatgtactaaaaaaattagtctataaaaaccattaaccctaaaccctaaatggatgtactaataaaatggatga agtattatataggtgaagctatttgcaaaaaaaaaggagaacacatgcacactaaaaagataaaactgtagagtcctgttgtcaa aatactcaattgtcctttagaccatgtctaactgttcatttatatgattctctaaaacactgatattattgtagtactatagattatattattcgt

agagtaaagtttaaatatatgtataaagatagataaactgcacttcaaacaagtgtgacaaaaaaaatatgtggtaattttttataac
ttagacatgcaatgctcattatctctagagaggggcacgaccgggtcacgctgcactgcaggcatcgatcgaagcttacgcgtcc
atggctgcaggagctcgtcgactctagactcgagggcgagccccgagcgggggcagctgaggcgccattgggaggaggaag
agggcagcgctccggggcggaagtcgtggggcggcacgagggaagggcacacgacggacggacg

SEQ ID NO: 6
caggagctcgtcgactctagact
SEQ ID NO: 7
gtgtgcccttccctcgtg
SEQ ID NO: 8
caggttgacgtgactgggttg
SEQ ID NO: 9
cgtccgcaatgtgttattaagttg
SEQ ID NO: 10
cattgggaggaggaaga
SEQ ID NO: 11
tggtgtaaacaaattgacgc

SEQ ID NO: 12

caggagctcgtcgactctagactcgagggcgagccccgagcgggggcagctgaggcgccattgggaggaggaagagggca
gcgctccggggcggaagtcgtggggcggcacgagggaagggcacac

SEQ ID NO: 13

caggttgacgtgactgggttgcctgcgctgaacctaataacacattgctgggttgtggtgtaaacaaattgacgcttagacaactta
ataacacattgcggacg

SEQ ID NO: 14

tggcaggatatattgtggtgtaaacaaattgacgcttagacaacttaataacacattgcggacgtttttaatgtactgaattagtactg
atatcggtacccggggcggccgcgagctcgatccactagtaacggccgccagtgtgctggaattcgcccttggcgcgccgatct
agtaacatagatgacaccgcgcgcgataatttatcctagtttgcgcgctatattttgttttctatcgcgtattaaatgtataattgcggga
ctctaatcataaaaacccatctcataaataacgtcatgcattacatgttaattattacatgcttaacgtaattcaacagaaattatatga
taatcatcgcaagaccggcaacaggattcaatcttaagaaactttattgccaaatgtttgaacgatctcagaagaactcgtcaaga
aggcgatagaaggcgatgcgctgcgaatcgggagcggcgataccgtaaacacattgctgggaagcacgaggaagcggtca
gcccattcgccgccaagctcttcagcaatatcacgggtagccaacgctatgtcctgatagcggtccgccacacccagccggcca
cagtcgatgaatccagaaaagcggccattttccaccatgatattcggcaagcaggcatcgccatgggtcacgacgagatcctcg
ccgtcgggcatgcgcgccttgagcctggcgaacagttcggctggcgcgagcccctgatgctcttcgtccagatcatcctgatcgac
aagaccggcttccatccgagtacgtgctcgctcgatgcgatgtttcgcttggtggtcgaatgggcaggtagccggatcaagcgtat
gcagccgccgcattgcatcagccatgatggatactttctcggcaggagcaaggtgagatgacaggagatcctgccccggcactt

cgcccaatagcagccagtcccttcccgcttcagtgacaacgtcgagcacagctgcgcaaggaacgcccgtcgtggccagcca
cgatagccgcgctgcctcgtcctgcagttcattcagggcaccggacaggtcggtcttgacaaaaagaaccgggcgcccctgcgc
tgacagccggaacacggcggcatcagagcagccgattgtctgttgtgcccagtcatagccgaatagcctctccacccaagcgg
ccggagaacctgcgtgcaatccatcttgttcaatccacattctagagtcgacctgcagaagtaacaccaaacaacagggtgagc
atcgacaaaagaaacagtaccaagcaaataaatagcgtatgaaggcagggctaaaaaaatccacatatagctgctgcatatg
ccatcatccaagtatatcaagatcaaaataattataaaacatacttgtttattataatagataggtactcaaggttagagcatatgaat
agatgctgcatatgccatcatgtatatgcatcagtaaaacccacatcaacatgtatacctatcctagatcgatatttccatccatctta
aactcgtaactatgaagatgtatgacacacacatacagttccaaaattaataaatacaccaggtagtttgaaacagtattctactcc
gatctagaacgaatgaacgaccgcccaaccacaccacatcatcacaaccaagcgaacaaaaagcatctctgtatatgcatca
gtaaaacccgcatcaacatgtatacctatcctagatcgatatttccatccatcatcttcaattcgtaactatgaatatgtatggcacac
acatacagatccaaaattaataaatccaccaggtagtttgaaacagaattctactccgatctagaacgaccgcccaaccagacc
acatcatcacaaccaagacaaaaaaaagcatgaaaagatgacccgacaaacaagtgcacggcatatattgaaataaagga
aaagggcaaaccaaaccctatgcaacgaaacaaaaaaaatcatgaaatcgatcccgtctgcggaacggctagagccatccc
aggattccccaaagagaaacactggcaagttagcaatcagaacgtgtctgacgtacaggtcgcatccgtgtacgaacgctagc
agcacggatctaacacaaacacggatctaacacaaacatgaacagaagtagaactaccgggccctaaccatggaccggaa
cgccgatctagagaaggtagagagggggggggggggggaggacgagcggcgtaccttgaagcggaggtgccgacgggtgga
tttgggggagatctggttgtgtgtgtgtgcgctccgaacaacacgaggttggggaaagagggtgtggagggggtgtctatttattac
ggcgggcgaggaagggaaagcgaaggagcggtgggaaaggaatcccccgtagctgccgtgccgtgagaggaggaggag
gccgcctgccgtgccggctcacgtctgccgctccgccacgcaatttctggatgccgacagcggagcaagtccaacggtggagc
ggaactctcgagaggggtccagaggcagcgacagagatgccgtgccgtctgcttcgcttggcccgacgcgacgctgctggttcg
ctggttggtgtccgttagactcgtcgacggcgtttaacaggctggcattatctactcgaaacaagaaaaatgtttccttagtttttttaatt
tcttaaagggtatttgtttaatttttagtcactttattttattctattttatatctaaattattaaataaaaaaactaaaatagagttttagttttctt
aatttagaggctaaaatagaataaaatagatgtactaaaaaaattagtctataaaaaccattaaccctaaaccctaaatggatgta
ctaataaaatggatgaagtattatataggtgaagctatttgcaaaaaaaaaggagaacacatgcacactaaaaagataaaactg
tagagtcctgttgtcaaaatactcaattgtcctttagaccatgtctaactgttcatttatatgattctctaaaacactgatattattgtagtac
tatagattatattattcgtagagtaaagtttaaatatatgtataaagatagataaactgcacttcaaacaagtgtgacaaaaaaaata
tgtggtaatttttataacttagacatgcaatgctcattatctctagagaggggcacgaccgggtcacgctgcactgcaggcatacgc
gtgatccactagtaacggccgccagtgtgctggaattcgcccttggcgcgccgatctagtaacatagatgacaccgcgcgcgata
atttatcctagtttgcgcgctatattttgttttctatcgcgtattaaatgtataattgcgggactctaatcataaaaacccatctcataaata
acgtcatgcattacatgttaattattacatgcttaacgtaattcaacagaaattatatgataatcatcgcaagaccggcaacaggatt
caatcttaagaaactttattgccaaatgtttgaacgatctcactcagcctcgagggtggcggtcactgggatgaactcgaacctgtc
gatgatcacgccggcggtgccgctgaagttcctcacgcccacgatgttaccgagggaggaggtgaaagcgttggcgctctcga
agtaaccgaagtcgctggattggaggttgtccagggaggtagcggtagctggcacggtgttggagaagatggaggagttacccc
agttcacgttgaggtggatcggggtcacggaagcgtacctcacgcgcaccctgtacctggtggaggtggatgggaagtggattg
gcacctcgatgtagcccctgttctggatgttgttgccgctgctgttgagcctcacgaggtcgccaccggtgaagcctgggccggaa
atgacggaaccgttgaagaggaagttgcccttcacggccgggatttgggtgatgctgtcggaggcgatgatgttgttgaactcagc
gctgcggtggatccaggagaacatcggagccctgatgatgctcacggagctgttgctgaagccggagcggaacatggacacgt
ggctcagcctgtgggagaagccttgcctgggtggcacgttgttgttctgtggtgggatctcgtccagggagtccacggtgccgctctt
cctgtagacagcggatggcaggttggaggaggtgccgtaggcgaactcggtgccgtcgagcacggacagttgctggttgttgat
accgatgttgaagggcctcctgtacagggtggaggacagggtcctgtagacaccctgacccagttgagccacgatgcgttgctgt
ggagcggcgttgcccatggtgccgtagagcgggaaggtgaactcggggccgctgaagcccactggggaggccatgatctggt
ggccggaccagtagtactcgcccctgtgagcgtcggtgtagatggtgatgctgttcaggatgtccatcaggtgtgggctcctgatgg
agccctcgataccctgggcggaaccgcggaagctaccgtcgaagttctccagcactgggttggtgtagatctccctggtgagttgg
gacacggtgcggatcgggtaggtcctggagtcgtagttcgggaagagggacacaatgtccagcacggtgagggtcaactccct
cctgaactggttgtacctgatccagtccctggagtccggaccccagacgcgctccaggccggtgttgtaccagcgcacagcgtgg
tcggtgtagttgccaatcagcctggtcaggtcgttgtagcggctgttgatggtggcagcatcgaagccccacctttggccgaacac

gctcacgtcgcgcagcacgctgaggtgcaggttagcggcttgcacgtacacggacaggagcggcacttggtagttctggacgg
cgaacagtgggatagcggtggtcagggcgctgttcatgtcgttgaattgaatgcgcatttcctcgcggagagctgggttggtcgggt
cggcctcccactccctgaagctctcggcgtagatttggtagaggttgctcaggccctccagcctggagatggcctggttcctggcg
aactcttcgatcctctggttgatcagctgctcgatttgcaccaggaaggcgtcccattgggatggaccgaagataccccagatgat
gtccaccaggccgagcacgaagccagcacctggcacgaactcgctgagcaggaactgggtcaaggacagggagatgtcga
tgggggtgtaaccggtctcgatgcgctcgccacccagcacctccacctctgggttgctcaggcagttgtatgggatgcactcgttga
tgtttgggttgttgtccattctagagtcgacctgcagaagtaacaccaaacaacagggtgagcatcgacaaaagaaacagtacc
aagcaaataaatagcgtatgaaggcagggctaaaaaaatccacatatagctgctgcatatgccatcatccaagtatatcaagat
caaaataattataaaacatacttgtttattataatagataggtactcaaggttagagcatatgaatagatgctgcatatgccatcatgt
atatgcatcagtaaaacccacatcaacatgtatacctatcctagatcgatatttccatccatcttaaactcgtaactatgaagatgtat
gacacacacatacagttccaaaattaataaatacaccaggtagtttgaaacagtattctactccgatctagaacgaatgaacgac
cgcccaaccacaccacatcatcacaaccaagcgaacaaaaagcatctctgtatatgcatcagtaaaacccgcatcaacatgta
tacctatcctagatcgatatttccatccatcatcttcaattcgtaactatgaatatgtatggcacacacatacagatccaaaattaataa
atccaccaggtagtttgaaacagaattctactccgatctagaacgaccgcccaaccagaccacatcatcacaaccaagacaaa
aaaaagcatgaaaagatgacccgacaaacaagtgcacggcatatattgaaataaaggaaaagggcaaaccaaaccctatg
caacgaaacaaaaaaaatcatgaaatcgatcccgtctgcggaacggctagagccatcccaggattccccaaagagaaacac
tggcaagttagcaatcagaacgtgtctgacgtacaggtcgcatccgtgtacgaacgctagcagcacggatctaacacaaacac
ggatctaacacaaacatgaacagaagtagaactaccgggccctaaccatggaccggaacgccgatctagagaaggtagaga
gggggggggggggaggacgagcggcgtaccttgaagcggaggtgccgacgggtggatttgggggagatctggttgtgtgtgtg
tgcgctccgaacaacacgaggttggggaaagagggtgtggagggggtgtctatttattacggcgggcgaggaagggaaagcg
aaggagcggtgggaaaggaatcccccgtagctgccgtgccgtgagaggaggaggaggccgcctgccgtgccggctcacgtc
tgccgctccgccacgcaatttctggatgccgacagcggagcaagtccaacggtggagcggaactctcgagaggggtccagag
gcagcgacagagatgccgtgccgtctgcttcgcttggcccgacgcgacgctgctggttcgctggttggtgtccgttagactcgtcga
cggcgtttaacaggctggcattatctactcgaaacaagaaaaatgtttccttagtttttttaatttcttaaagggtatttgtttaatttttagtc
actttattttattctattttatatctaaattattaaataaaaaaactaaaatagagttttagttttcttaatttagaggctaaaatagaataaa
atagatgtactaaaaaaattagtctataaaaaccattaaccctaaaccctaaatggatgtactaataaaatggatgaagtattatat
aggtgaagctatttgcaaaaaaaaaggagaacacatgcacactaaaaagataaaactgtagagtcctgttgtcaaaatactca
attgtcctttagaccatgtctaactgttcatttatatgattctctaaaacactgatattattgtagtactatagattatattattcgtagagtaa
agtttaaatatatgtataaagatagataaactgcacttcaaacaagtgtgacaaaaaaaatatgtggtaatttttataacttagacat
gcaatgctcattatctctagagaggggcacgaccgggtcacgctgcactgcaggcatcgatcgaagcttacgcgtccatggctgc
aggagctcgtcgactctagactcgagggcgcgccgacaggatatattggcgggtaaaccttagaataacggatatttaaaaggg
cgtgaaaaggtttatccgttcgtccatttgtatgtgcatgccaaccacagggttcccctcgggatcaaagtactttgatccaacccctc
cgctgctatagtgcagtcggcttctgacgttcagtgcagccgtcttctgaaaacgacatgtcgcacaagtcctaagttacgcgacag
gctgccgccctgcccttttcctggcgttttcttgtcgcgtgttttagtcgcataaagtagaatacttgcgactagaaccggagacattac
gccatgaacaagagcgccgccgctggcctgctgggctatgcccgcgtcagcaccgacgaccaggacttgaccaaccaacgg
gccgaactgcacgcggccggctgcaccaagctgttttccgagaagatcaccggcaccaggcgcgaccgcccggagctggcc
aggatgcttgaccacctacgccctggcgacgttgtgacagtgaccaggctagaccgcctggcccgcagcacccgcgacctact
ggacattgccgagcgcatccaggaggccggcgcgggcctgcgtagcctggcagagccgtgggccgacaccaccacgccgg
ccggccgcatggtgttgaccgtgttcgccggcattgccgagttcgagcgttccctaatcatcgaccgcacccggagcgggcgcga
ggccgccaaggcccgaggcgtgaagtttggcccccgccctaccctcaccccggcacagatcgcgcacgcccgcgagctgatc
gaccaggaaggccgcaccgtgaaagaggcggctgcactgcttggcgtgcatcgctcgaccctgtaccgcgcacttgagcgca
gcgaggaagtgacgcccaccgaggccaggcggcgcggtgccttccgtgaggacgcattgaccgaggccgacgccctggcg
gccgccgagaatgaacgccaagaggaacaagcatgaaaccgcaccaggacggccaggacgaaccgtttttcattaccgaa
gagatcgaggcggagatgatcgcggccgggtacgtgttcgagccgcccgcgcacgtctcaaccgtgcggctgcatgaaatcct
ggccggtttgtctgatgccaagctggcggcctggccggccagcttggccgctgaagaaaccgagcgccgccgtctaaaaggt
gatgtgtatttgagtaaaacagcttgcgtcatgcggtcgctgcgtatatgatgcgatgagtaaataaacaaatacgcaaggggaa

cgcatgaaggttatcgctgtacttaaccagaaaggcgggtcaggcaagacgaccatcgcaacccatctagcccgcgccctgca
actcgccggggccgatgttctgttagtcgattccgatccccagggcagtgcccgcgattgggcggccgtgcgggaagatcaacc
gctaaccgttgtcggcatcgaccgcccgacgattgaccgcgacgtgaaggccatcggccggcgcgacttcgtagtgatcgacg
gagcgccccaggcggcggacttggctgtgtccgcgatcaaggcagccgacttcgtgctgattccggtgcagccaagcccttacg
acatatgggccaccgccgacctggtggagctggttaagcagcgcattgaggtcacggatggaaggctacaagcggcctttgtcg
tgtcgcgggcgatcaaaggcacgcgcatcggcggtgaggttgccgaggcgctggccgggtacgagctgcccattcttgagtccc
gtatcacgcagcgcgtgagctacccaggcactgccgccgccggcacaaccgttcttgaatcagaacccgagggcgacgctgc
ccgcgaggtccaggcgctggccgctgaaattaaatcaaaactcatttgagttaatgaggtaaagagaaaatgagcaaaagcac
aaacacgctaagtgccggccgtccgagcgcacgcagcagcaaggctgcaacgttggccagcctggcagacacgccagccat
gaagcgggtcaactttcagttgccggcggaggatcacaccaagctgaagatgtacgcggtacgccaaggcaagaccattacc
gagctgctatctgaatacatcgcgcagctaccagagtaaatgagcaaatgaataaatgagtagatgaattttagcggctaaagg
aggcggcatggaaaatcaagaacaaccaggcaccgacgccgtggaatgccccatgtgtggaggaacgggcggttggccag
gcgtaagcggctgggttgtctgccggccctgcaatggcactggaacccccaagcccgaggaatcggcgtgacggtcgcaaac
catccggcccggtacaaatcggcgcggcgctgggtgatgacctggtggagaagttgaaggccgcgcaggccgcccagcggc
aacgcatcgaggcagaagcacgccccggtgaatcgtggcaagcggccgctgatcgaatccgcaaagaatcccggcaaccg
ccggcagccggtgcgccgtcgattaggaagccgcccaagggcgacgagcaaccagatttttttcgttccgatgctctatgacgtgg
gcacccgcgatagtcgcagcatcatggacgtggccgttttccgtctgtcgaagcgtgaccgacgagctggcgaggtgatccgcta
cgagcttccagacgggcacgtagaggtttccgcagggccggccggcatggccagtgtgtgggattacgacctggtactgatggc
ggtttcccatctaaccgaatccatgaaccgataccgggaagggaagggagacaagcccggccgcgtgttccgtccacacgttg
cggacgtactcaagttctgccggcgagccgatggcggaaagcagaaagacgacctggtagaaacctgcattcggttaaacac
cacgcacgttgccatgcagcgtacgaagaaggccaagaacggccgcctggtgacggtatccgagggtgaagccttgattagc
cgctacaagatcgtaaagagcgaaaccgggcggccggagtacatcgagatcgagctagctgattggatgtaccgcgagatca
cagaaggcaagaacccggacgtgctgacggttcaccccgattactttttgatcgatcccggcatcggccgttttctctaccgcctgg
cacgccgcgccgcaggcaaggcagaagccagatggttgttcaagacgatctacgaacgcagtggcagcgccggagagttca
agaagttctgtttcaccgtgcgcaagctgatcgggtcaaatgacctgccggagtacgatttgaaggaggaggcggggcaggctg
gcccgatcctagtcatgcgctaccgcaacctgatcgagggcgaagcatccgccggttcctaatgtacggagcagatgctagggc
aaattgccctagcaggggaaaaaggtcgaaaaggtctctttcctgtggatagcacgtacattgggaacccaaagccgtacattg
ggaaccggaacccgtacattgggaacccaaagccgtacattgggaaccggtcacacatgtaagtgactgatataaaagagaa
aaaaggcgatttttccgcctaaaactctttaaaacttattaaaactcttaaaacccgcctggcctgtgcataactgtctggccagcgc
acagccgaagagctgcaaaaagcgcctacccttcggtcgctgcgctccctacgccccgccgcttcgcgtcggcctatcgcggcc
gctggccgctcaaaaatggctggcctacggccaggcaatctaccagggcgcggacaagccgcgccgtcgccactcgaccgc
cggcgcccacatcaaggcaccctgcctcgcgcgtttcggtgatgacggtgaaaacctctgacacatgcagctcccggagacggt
cacagcttgtctgtaagcggatgccgggagcagacaagcccgtcagggcgcgtcagcgggtgttggcgggtgtcggggcgca
gccatgacccagtcacgtagcgatagcggagtgtatactggcttaactatgcggcatcagagcagattgtactgagagtgcacca
tatgcggtgtgaaataccgcacagatgcgtaaggagaaaataccgcatcaggcgctcttccgcttcctcgctcactgactcgctgc
gctcggtcgttcggctgcggcgagcggtatcagctcactcaaaggcggtaatacggttatccacagaatcaggggataacgcag
gaaagaacatgtgagcaaaaggccagcaaaaggccaggaaccgtaaaaaggccgcgttgctggcgtttttccataggctccg
cccccctgacgagcatcacaaaaatcgacgctcaagtcagaggtggcgaaacccgacaggactataaagataccaggcgttt
ccccctggaagctccctcgtgcgctctcctgttccgaccctgccgcttaccggatacctgtccgcctttctcccttcgggaagcgtgg
cgctttctcatagctcacgctgtaggtatctcagttcggtgtaggtcgttcgctccaagctgggctgtgtgcacgaaccccccgttcag
cccgaccgctgcgccttatccggtaactatcgtcttgagtccaacccggtaagacacgacttatcgccactggcagcagccactg
gtaacaggattagcagagcgaggtatgtaggcggtgctacagagttcttgaagtggtggcctaactacggctacactagaagga
cagtatttggtatctgcgctctgctgaagccagttaccttcggaaaaagagttggtagctcttgatccggcaaacaaaccaccgctg
gtagcggtggtttttttgtttgcaagcagcagattacgcgcagaaaaaaaggatctcaagaagatcctttgatcttttctacggggtct
gacgctcagtggaacgaaaactcacgttaagggattttggtcatgcattctaggtattatttgccaacgaccttcgtgatctcgccctt
gacatagtggacaaattcttcgagctggtcggcccgggacgcgagacggtcttcttcttggcccagataggcttggcgcgcttcga

ggatcacgggctggtattgcgccggaaggcgctccatcgcccagtcggcggcgacatccttcggcgcgatcttgccggtaaccg ccgagtaccaaatccggctcagcgtaaggaccacattgcgctcatcgcccgcccaatccggcggggagttccacagggtcagc gtctcgttcagtgcttcgaacagatcctgttccggcaccgggtcgaaaagttcctcggccgcggggccgacgagggccacgctat gctcccgggccttggtgagcaggatcgccagatcaatgtcgatggtggccggttcaaagatacccgccagaatatcattacgctg ccattcgccgaactggagttcgcgtttggccggatagcgccaggggatgatgtcatcgtgcaccacaatcgtcacctcaaccgcg cgcaggatttcgctctcgccgggggaggcggacgtttccagaaggtcgttgataagcgcgcggcgcgtggtctcgtcgagacgg acggtaacggtgacaagcaggtcgatgtccgaatggggcttaaggccgccgtcaacggcgctaccatacagatgcacggcga ggagggtcggttcgaggtggcgctcgatgacacccacgacttccgacagctgggtggacacctcggcgatgaccgcttcaccc atttattatttccttcctcttttctacagtatttaaagataccccaagaagctaattataacaagacgaactccaattcactgttccttgcat tctaaaaccttaaataccagaaaacagctttttcaaagttgttttcaaagttggcgtataacatagtatcgacggagccgattttgaaa ccgcggtgatcacaggcagcaacgctctgtcatcgttacaatcaacatgctaccctccgcgagatcatccgtgtttcaaacccggc agcttagttgccgttcttccgaatagcatcggtaacatgagcaaagtctgccgccttacaacggctctcccgctgacgccgtcccgg actgatgggctgcctgtatcgagtggtgattttgtgccgagctgccggtcggggagctgttggctggctgg

SEQ ID NO: 15
accattaccctggaggttgaga
SEQ ID NO: 16
gtcctggatcttcgccttca
SEQ ID NO: 17
ctctgacaccatcgac
SEQ ID NO: 18
acacattgctgggttgtggtgtaaa
SEQ ID NO: 19
tcgagggcgagccccgagcgggggca

SEQ ID NO: 20

atggacaacaacccaaacatcaacgagtgcatcccatacaactgcctgagcaacccagaggtggaggtgctgggtggcgag cgcatcgagaccggttacacccccatcgacatctccctgtccttgacccagttcctgctcagcgagttcgtgccaggtgctggcttc gtgctcggcctggtggacatcatctggggtatcttcggtccatcccaatgggacgccttcctggtgcaaatcgagcagctgatcaac cagaggatcgaagagttcgccaggaaccaggccatctccaggctggagggcctgagcaacctctaccaaatctacgccgaga gcttcagggagtgggaggccgacccgaccaacccagctctccgcgaggaaatgcgcattcaattcaacgacatgaacagcgc cctgaccaccgctatcccactgttcgccgtccagaactaccaagtgccgctcctgtccgtgtacgtgcaagccgctaacctgcacc tcagcgtgctgcgcgacgtgagcgtgttcggccaaaggtggggcttcgatgctgccaccatcaacagccgctacaacgacctga ccaggctgattggcaactacaccgaccacgctgtgcgctggtacaacaccggcctggagcgcgtctggggtccggactccagg gactggatcaggtacaaccagttcaggagggagttgaccctcaccgtgctggacattgtgtccctcttcccgaactacgactccag gacctacccgatccgcaccgtgtcccaactcaccagggagatctacaccaacccagtgctggagaacttcgacggtagcttccg cggttccgcccagggtatcgagggctccatcaggagcccacacctgatggacatcctgaacagcatcaccatctacaccgacg ctcacaggggcgagtactactggtccggccaccagatcatggcctccccagtgggcttcagcggccccgagttcaccttcccgct ctacggcaccatgggcaacgccgctccacagcaacgcatcgtggctcaactgggtcagggtgtctacaggaccctgtcctccac cctgtacaggaggcccttcaacatcggtatcaacaaccagcaactgtccgtgctcgacggcaccgagttcgcctacggcacctc

ctccaacctgccatccgctgtctacaggaagagcggcaccgtggactccctggacgagatcccaccacagaacaacaacgtg ccacccaggcaaggcttctcccacaggctgagccacgtgtccatgttccgctccggcttcagcaacagctccgtgagcatcatca gggctccgatgttctcctggatccaccgcagcgctgagttcaacaacatcatcgcctccgacagcatcacccaaatcccggccgt gaagggcaacttcctcttcaacggttccgtcatttccggcccaggcttcaccggtggcgacctcgtgaggctcaacagcagcggc aacaacatccagaacaggggctacatcgaggtgccaatccacttcccatccacctccaccaggtacagggtgcgcgtgaggta cgcttccgtgaccccgatccacctcaacgtgaactggggtaactcctccatcttctccaacaccgtgccagctaccgctacctccct ggacaacctccaatccagcgacttcggttacttcgagagcgccaacgctttcacctcctccctcggtaacatcgtgggcgtgagga acttcagcggcaccgccggcgtgatcatcgacaggttcgagttcatcccagtgaccgccaccctcgaggctgagtga

SEQ ID NO: 21

tcagaagaactcgtcaagaaggcgatagaaggcgatgcgctgcgaatcgggagcggcgataccgtaaagcacgaggaagcggtcagcccattcgccgccaagctcttcagcaatatcacgggtagccaacgctatgtcctgatagcggtccgccacacccagccggccacagtcgatgaatccagaaaagcggccattttccaccatgatattcggcaagcaggcatcgccatgggtcacgacgagatcctcgccgtcgggcatgcgcgccttgagcctggcgaacagttcggctggcgcgagcccctgatgctcttcgtccagatcatcctgatcgacaagaccggcttccatccgagtacgtgctcgctcgatgcgatgtttcgcttggtggtcgaatgggcaggtagccggatcaagcgtatgcagccgccgcattgcatcagccatgatggatactttctcggcaggagcaaggtgagatgacaggagatcctgccccggcacttcgcccaatagcagccagtcccttcccgcttcagtgacaacgtcgagcacagctgcgcaaggaacgcccgtcgtggccagccacgatagccgcgctgcctcgtcctgcagttcattcagggcaccggacaggtcggtcttgacaaaaagaaccgggcgcccctgcgctgacagccggaacacggcggcatcagagcagccgattgtctgttgtgcccagtcatagccgaatagcctctccacccaagcggccggagaacctgcgtgcaatccatcttgttcaatccacat

SEQ ID NO: 22

ggtaatccttcaaaggattttaaaaaatactaaatctgagctaggatctgcagatgaggaagaaagacgaagacgagatagagagcgggaaattgagatgtaaccctagctgatggaacgggcacaggcacaaatacgggcatgggccgagatcgaggggcggcgcatcagcagcagtgcagtaggcgagcagggccgccgcattggtgggccgcccatgccggtgcaccgccgccgctctcgcgggggccgccgtgccatggctcgtccgatgcaggatcaccggcgacggagaggcgaagccctaggccggcgctgtcgccacgccaggatcggtcgcccaggttgacgtgactgggttgcctgcgctgaacctaataacacattgctgggttgtggtgtaaacaaattgacgcttagacaacttaataacacattgcggacgtttttaatgtactgaattagtactgatatcggtacccggggcggccgcgagctcgatccactagtaacggccgccagtgtgctggaattcgcccttggcgcgccgatctagtaacatagatgacaccgcgcgcgataatttatcctagtttgcgcgctatattttgttttctatcgcgtattaaatgtataattgcgggactctaatcataaaaacccatctcataaataacgtcatgcattacatgttaattattacatgcttaacgtaattcaacagaaattatatgataatcatcgcaagaccggcaacaggattcaatcttaagaaactttattgccaaatgtttgaacgatctcagaagaactcgtcaagaaggcgatagaaggcgatgcgctgcgaatcgggagcggcgataccgtaaagcacgaggaagcggtcagcccattcgccgccaagctcttcagcaatatcacgggtagccaacgctatgtcctgatagcggtccgccacacccagccggccacagtcgatgaatccagaaaagcggccattttccaccatgatattcggcaagcaggcatcgccatgggtcacgacgagatcctcgccgtcgggcatgcgcgccttgagcctggcgaacagttcggctggcgcgagcccctgatgctcttcgtccagatcatcctgatcgacaagaccggcttccatccgagtacgtgctcgctcgatgcgatgtttcgcttggtggtcgaatgggcaggtagccggatcaagcgtatgcagccgccgcattgcatcagccatgatggatactttctcggcaggagcaaggtgagatgacaggagatcctgccccggcacttcgcccaatagcagccagtcccttcccgcttcagtgacaacgtcgagcacagctgcgcaaggaacgcccgtcgtggccagccacgatagccgcgctgcctcgtcctgcagttcattcagggcaccggacaggtcggtcttgacaaaaagaaccgggcgcccctgcgctgacagccggaacacggcggcatcagagcagccgattgtctgttgtgcccagtcatagccgaatagcctctccacccaagcggccggagaacctgcgtgcaatccatcttgttcaatccacattctagagtcgacctgcagaagtaacaccaaacaacagggtgagcatcgacaaaagaaacagtaccaagcaaataaatagcgtatgaaggcagggctaaaaaaatccacatatagctgctgcatatgccatcatccaagtatatcaagatcaaaataattataaaacatacttgtttattataatagataggtactcaaggttagagcatatgaatagatgctgcatatgccatcatgtatatgcatcagtaaaacccacatcaacatgtatacctatcctagatcgatatttccatccatcttaaactcgtaactatgaagatgtatgacacacacatacagttccaaaattaataaatacaccaggtagtttgaaacagtattctactccgatctagaacgaatgaacgaccgcccaaccacaccacatcatcacaac

caagcgaacaaaaagcatctctgtatatgcatcagtaaaacccgcatcaacatgtatacctatcctagatcgatatttccatccatc
atcttcaattcgtaactatgaatatgtatggcacacacatacagatccaaaattaataaatccaccaggtagtttgaaacagaattct
actccgatctagaacgaccgcccaaccagaccacatcatcacaaccaagacaaaaaaaagcatgaaaagatgacccgaca
aacaagtgcacggcatatattgaaataaaggaaaagggcaaaccaaaccctatgcaacgaaacaaaaaaaatcatgaaatc
gatcccgtctgcggaacggctagagccatcccaggattccccaaagagaaacactggcaagttagcaatcagaacgtgtctga
cgtacaggtcgcatccgtgtacgaacgctagcagcacggatctaacacaaacacggatctaacacaaacatgaacagaagta
gaactaccgggccctaaccatggaccggaacgccgatctagagaaggtagagaggggggggggggggaggacgagcggc
gtaccttgaagcggaggtgccgacgggtggatttgggggagatctggttgtgtgtgtgtgcgctccgaacaacacgaggttgggg
aaagagggtgtggagggggtgtctatttattacggcgggcgaggaagggaaagcgaaggagcggtgggaaaggaatccccc
gtagctgccgtgccgtgagaggaggaggaggccgcctgccgtgccggctcacgtctgccgctccgccacgcaatttctggatgc
cgacagcggagcaagtccaacggtggagcggaactctcgagaggggtccagaggcagcgacagagatgccgtgccgtctg
cttcgcttggcccgacgcgacgctgctggttcgctggttggtgtccgttagactcgtcgacggcgtttaacaggctggcattatctact
cgaaacaagaaaaatgtttccttagtttttttaatttcttaaagggtatttgtttaatttttagtcactttattttattctattttatatctaaattatta
aataaaaaaactaaaatagagttttagttttcttaatttagaggctaaaatagaataaaatagatgtactaaaaaaattagtctataa
aaaccattaaccctaaaccctaaatggatgtactaataaaatggatgaagtattatataggtgaagctatttgcaaaaaaaaagg
agaacacatgcacactaaaaagataaaactgtagagtcctgttgtcaaaatactcaattgtcctttagaccatgtctaactgttcattt
atatgattctctaaaacactgatattattgtagtactatagattatattattcgtagagtaaagtttaaatatatgtataaagatagataaa
ctgcacttcaaacaagtgtgacaaaaaaaaatatgtggtaatttttataacttagacatgcaatgctcattatctctagagaggggca
cgaccgggtcacgctgcactgcaggcatacgcgtgatccactagtaacggccgccagtgtgctggaattcgcccttggcgcgcc
gatctagtaacatagatgacaccgcgcgcgataatttatcctagtttgcgcgctatattttgttttctatcgcgtattaaatgtataattgc
gggactctaatcataaaaacccatctcataaataacgtcatgcattacatgttaattattacatgcttaacgtaattcaacagaaatta
tatgataatcatcgcaagaccggcaacaggattcaatcttaagaaactttattgccaaatgtttgaacgatctcactcagcctcgag
ggtggcggtcactgggatgaactcgaacctgtcgatgatcacgccggcggtgccgctgaagttcctcacgcccacgatgttaccg
agggaggaggtgaaagcgttggcgctctcgaagtaaccgaagtcgctggattggaggttgtccagggaggtagcggtagctgg
cacggtgttggagaagatggaggagttaccccagttcacgttgaggtggatcggggtcacggaagcgtacctcacgcgcaccct
gtacctggtggaggtggatgggaagtggattggcacctcgatgtagcccctgttctggatgttgttgccgctgctgttgagcctcacg
aggtcgccaccggtgaagcctgggccggaaatgacggaaccgttgaagaggaagttgcccttcacggccgggatttgggtgat
gctgtcggaggcgatgatgttgttgaactcagcgctgcggtggatccaggagaacatcggagccctgatgatgctcacggagctg
ttgctgaagccggagcggaacatggacacgtggctcagcctgtgggagaagccttgcctgggtggcacgttgttgttctgtggtgg
gatctcgtccagggagtccacggtgccgctcttcctgtagacagcggatggcaggttggaggaggtgccgtaggcgaactcggt
gccgtcgagcacggacagttgctggttgttgataccgatgttgaagggcctcctgtacagggtggaggacagggtcctgtagaca
ccctgacccagttgagccacgatgcgttgctgtggagcggcgttgcccatggtgccgtagagcgggaaggtgaactcggggcc
gctgaagcccactggggaggccatgatctggtggccggaccagtagtactcgcccctgtgagcgtcggtgtagatggtgatgctg
ttcaggatgtccatcaggtgtgggctcctgatggagccctcgataccctgggcggaaccgcggaagctaccgtcgaagttctcca
gcactgggttggtgtagatctccctggtgagttgggacacggtgcggatcgggtaggtcctggagtcgtagttcgggaagaggga
cacaatgtccagcacggtgagggtcaactccctcctgaactggttgtacctgatccagtccctggagtccggaccccagacgcgc
tccaggccggtgttgtaccagcgcacagcgtggtcggtgtagttgccaatcagcctggtcaggtcgttgtagcggctgttgatggtg
gcagcatcgaagccccacctttggccgaacacgctcacgtcgcgcagcacgctgaggtgcaggttagcggcttgcacgtacac
ggacaggagcggcacttggtagttctggacggcgaacagtgggatagcggtggtcagggcgctgttcatgtcgttgaattgaatg
cgcatttcctcgcggagagctgggttggtcgggtcggcctcccactccctgaagctctcggcgtagatttggtagaggttgctcagg
ccctccagcctggagatggcctggttcctggcgaactcttcgatcctctggttgatcagctgctcgatttgcaccaggaaggcgtccc
attgggatggaccgaagataccccagatgatgtccaccaggccgagcacgaagccagcacctggcacgaactcgctgagca
ggaactgggtcaaggacagggagatgtcgatgggggtgtaaccggtctcgatgcgctcgccacccagcacctccacctctgggt
tgctcaggcagttgtatgggatgcactcgttgatgtttgggttgttgtccattctagagtcgacctgcagaagtaacaccaaacaaca
gggtgagcatcgacaaaagtgctgcatatgccatcatccaagtatatcaagatcaaaataattataaaacatacttgtttattataat
agataggtactcaaggttagagcatatgaatagatgctgcatatgccatcatgtatatgcatcagtaaaacccacatcaacatgtat

acctatcctagatcgatatttccatccatcttaaactcgtaactatgaagatgtatgacacacacatacagttccaaaattaataaata
caccaggtagtttgaaacagtattctactccgatctagaacgaatgaacgaccgcccaaccacaccacatcatcacaaccaag
cgaacaaaaagcatctctgtatatgcatcagtaaaacccgcatcaacatgtatacctatcctagatcgatatttccatccatcatcttc
aattcgtaactatgaatatgtatggcacacacatacagatccaaaattaataaatccaccaggtagtttgaaacagaattctactcc
gatctagaacgaccgcccaaccagaccacatcatcacaaccaagacaaaaaaaagcatgaaaagatgacccgacaaaca
agtgcacggcatatattgaaataaaggaaaagggcaaaccaaaccctatgcaacgaaacaaaaaaaatcatgaaatcgatc
ccgtctgcggaacggctagagccatcccaggattccccaaagagaaacactggcaagttagcaatcagaacgtgtctgacgta
caggtcgcatccgtgtacgaacgctagcagcacggatctaacacaaacacggatctaacacaaacatgaacagaagtagaa
ctaccgggccctaaccatggaccggaacgccgatctagagaaggtagagagggggggggggggaggacgagcggcgtac
cttgaagcggaggtgccgacgggtggatttgggggagatctggttgtgtgtgtgtgcgctccgaacaacacgaggttggggaaa
gagggtgtggagggggtgtctatttattacggcgggcgaggaagggaaagcgaaggagcggtgggaaaggaatcccccgta
gctgccgtgccgtgagaggaggaggaggccgcctgccgtgccggctcacgtctgccgctccgccacgcaatttctggatgccga
cagcggagcaagtccaacggtggagcggaactctcgagaggggtccagaggcagcgacagagatgccgtgccgtctgcttc
gcttggcccgacgcgacgctgctggttcgctggttggtgtccgttagactcgtcgacggcgtttaacaggctggcattatctactcga
aacaagaaaaatgtttccttagtttttttaatttcttaaagggtatttgtttaatttttagtcactttattttattctattttatatctaaattattaaat
aaaaaaactaaaatagagttttagttttcttaatttagaggctaaaatagaataaaatagatgtactaaaaaaattagtctataaaaa
ccattaaccctaaaccctaaatggatgtactaataaaatggatgaagtattatataggtgaagctatttgcaaaaaaaaaggaga
acacatgcacactaaaaagataaaactgtagagtcctgttgtcaaaatactcaattgtcctttagaccatgtctaactgttcatttatat
gattctctaaaacactgatattattgtagtactatagattatattattcgtagagtaaagtttaaatatatgtataaagatagataaactg
cacttcaaacaagtgtgacaaaaaaaatatgtggtaatttttttataacttagacatgcaatgctcattatctctagagaggggcacga
ccgggtcacgctgcactgcaggcatcgatcgaagcttacgcgtccatggctgcaggagctcgtcgactctagactcgagggcga
gccccgagcgggggcagctgaggcgccattgggaggaggaagagggcagcgctccggggcggaagtcgtggggcggcac
gagggaagggcacacgacggacggacggaggaagaagaagagcgcggaggtagctggccgagcgtgacctgcatgtca
agtcaatggtcgcgggtgcgagtagaaaagagggaggacgaccggacacaccgccgcgtcaggtcaccgatgacctaacgt
gtctggtcactattcacgcgctaaaaacacttactagactcgatctaacgcgggcagccctctgagtccgatcatttattctgtgcgtc
cggtcatcgacgtgtgcgggaaagcgttggctgttaacggctagttttgaatcgtgtgacgtggcacatcgtggttgctcgactgtta
cgaccggacacggggctgagtccggtcgatgagtccgatcacccccgagctgcgtaacggttggtttttcagaggggggtctttaa
atagaccttggtcggctccaagccttttctcttggtattttgatcatcttgacatcctcttaagctaagcatacttcctcccactcatctctctt
gcttggttgtgaatccaaagtgagattgagtgattccaagtgtatttgcttgtgagtttgcatatagtggcacttggggatcgtttgagct
gcggaattcttgttactcttggtggctaccgccacctagacagcttggagcaacagatgagttgtggcaagagttggtgattatttttg
gtcacctcccggtgattgtgaggggtttttgtgcctttcccggcagagcgttaaaggcaactctagtggattgcgcatgtcattgagct
acctcacttatgggtcggttcttgcggtttccaacgtgtggacgaggttc

SEQ ID NO: 23

ggtaatccttcaaaggattttaaaaaatactaaatctgagctaggatctgcagatgaggaagaaagacgaagacgagatagag
agcgggaaattgagatgtaaccctagctgatggaacgggcacaggcacaaatacgggcatgggccgagatcgaggggcgg
cgcatcagcagcagtgcagtaggcgagcagggccgccgcattggtgggccgcccatgccggtgcaccgccgccgctctcgcg
ggggccgccgtgccatggctcgtccgatgcaggatcaccggcgacggagaggcgaagccctaggccggcgctgtcgccacg
ccaggatcggtcgcccaggttgacgtgactgggttgcctgcgctgaacctaataacacattgctggg

SEQ ID NO: 24

ccgagcgggggcagctgaggcgccattgggaggaggaagagggcagcgctccggggcggaagtcgtggggcggcacga
gggaagggcacacgacggacggacggaggaagaagaagagcgcggaggtagctggccgagcgtgacctgcatgtcaagt
caatggtcgcgggtgcgagtagaaaagagggaggacgaccggacacaccgccgcgtcaggtcaccgatgacctaacgtgtc
tggtcactattcacgcgctaaaaacacttactagactcgatctaacgcgggcagccctctgagtccgatcatttattctgtgcgtccg
gtcatcgacgtgtgcgggaaagcgttggctgttaacggctagttttgaatcgtgtgacgtggcacatcgtggttgctcgactgttacg

accggacacggggctgagtccggtcgatgagtccgatcacccccgagctgcgtaacggttggtttttcagagggggtctttaaatagaccttggtcggctccaagccttttctcttggtattttgatcatcttgacatcctcttaagctaagcatacttcctcccactcatctctcttgcttggttgtgaatccaaagtgagattgagtgattccaagtgtatttgcttgtgagtttgcatatagtggcacttggggatcgtttgagctgcggaattcttgttactcttggtggctaccgccacctagacagcttggagcaacagatgagttgtggcaagagttggtgattatttttggtcacctcccggtgattgtgaggggtttttgtgcctttcccggcagagcgttaaaggcaactctagtggattgcgcatgtcattgagctacctcacttatgggtcggttcttgcggtttccaacgtgtggacgaggttc

SEQ ID NO: 25

cttagaataacggatatttaaaagggcgtgaaaaggtttatccgttcgtccatttgtatgtgcatgccaaccacagggttcccctcgggatcaaagtactttgatccaacccctccgctgctatagtgcagtcggcttctgacgttcagtgcagccgtcttctgaaaacgacatgtcgcacaagtcctaagttacgcgacaggctgccgccctgcccttttcctggcgttttcttgtcgcgtgttttagtcgcataaagtagaatacttgcgactagaaccggagacattacgccatgaacaagagcgccgccgctggcctgctgggctatgcccgcgtcagcaccgacgaccaggacttgaccaaccaacgggccgaactgcacgcggccggctgcaccaagctgttttccgagaagatcaccggcaccaggcgcgaccgcccggagctggccaggatgcttgaccacctacgccctggcgacgttgtgacagtgaccaggctagaccgcctggcccgcagcacccgcgacctactggacattgccgagcgcatccaggaggccggcgcgggcctgcgtagcctggcagagccgtgggccgacaccaccacgccggccggccgcatggtgttgaccgtgttcgccggcattgccgagttcgagcgttccctaatcatcgaccgcacccggagcgggcgcgaggccgccaaggcccgaggcgtgaagtttggcccccgccctaccctcaccccggcacagatcgcgcacgcccgcgagctgatcgaccaggaaggccgcaccgtgaaagaggcggctgcactgcttggcgtgcatcgctcgaccctgtaccgcgcacttgagcgcagcgaggaagtgacgcccaccgaggccaggcggcgcggtgccttccgtgaggacgcattgaccgaggccgacgccctggcggccgccgagaatgaacgccaagaggaacaagcatgaaaccgcaccaggacggccaggacgaaccgtttttcattaccgaagagatcgaggcggagatgatcgcggccgggtacgtgttcgagccgcccgcgcacgtctcaaccgtgcggctgcatgaaatcctggccggtttgtctgatgccaagctggcggcctggccggccagcttggccgctgaagaaaccgagcgccgccgtctaaaaaggtgatgtgtatttgagtaaaacagcttgcgtcatgcggtcgctgcgtatatgatgcgatgagtaaataaacaaatacgcaaggggaacgcatgaaggttatcgctgtacttaaccagaaaggcgggtcaggcaagacgaccatcgcaacccatctagcccgcgccctgcaactcgccggggccgatgttctgttagtcgattccgatccccagggcagtgcccgcgattgggcggccgtgcgggaagatcaaccgctaaccgttgtcggcatcgaccgcccgacgattgaccgcgacgtgaaggccatcggccggcgcgacttcgtagtgatcgacggagcgccccaggcggcggacttggctgtgtccgcgatcaaggcagccgacttcgtgctgattccggtgcagccaagcccttacgacatatgggccaccgccgacctggtggagctggttaagcagcgcattgaggtcacggatggaaggctacaagcggcctttgtcgtgtcgcgggcgatcaaaggcacgcgcatcggcggtgaggttgccgaggcgctggccgggtacgagctgcccattcttgagtcccgtatcacgcagcgcgtgagctacccaggcactgccgccgccggcacaaccgttcttgaatcagaacccgagggcgacgctgcccgcgaggtccaggcgctggccgctgaaattaaatcaaaactcatttgagttaatgaggtaaagagaaaatgagcaaaagcacaaacacgctaagtgccggccgtccgagcgcacgcagcagcaaggctgcaacgttggccagcctggcagacacgccagccatgaagcgggtcaactttcagttgccggcggaggatcacaccaagctgaagatgtacgcggtacgccaaggcaagaccattaccgagctgctatctgaatacatcgcgcagctaccagagtaaatgagcaaatgaataaatgagtagatgaattttagcggctaaaggaggcggcatggaaaatcaagaacaaccaggcaccgacgccgtggaatgccccatgtgtggaggaacgggcggttggccaggcgtaagcggctgggttgtctgccggccctgcaatggcactggaacccccaagcccgaggaatcggcgtgacggtcgcaaaccatccggcccggtacaaatcggcgcggcgctgggtgatgacctggtggagaagttgaaggccgcgcaggccgcccagcggcaacgcatcgaggcagaagcacgccccggtgaatcgtggcaagcggccgctgatcgaatccgcaaagaatcccggcaaccgccggcagccggtgcgccgtcgattaggaagccgcccaagggcgacgagcaaccagattttttcgttccgatgctctatgacgtgggcacccgcgatagtcgcagcatcatggacgtggccgttttccgtctgtcgaagcgtgaccgacgagctggcgaggtgatccgctacgagcttccagacgggcacgtagaggtttccgcagggccggccggcatggccagtgtgtgggattacgacctggtactgatggcggtttcccatctaaccgaatccatgaaccgataccgggaagggaagggagacaagcccggccgcgtgttccgtccacacgttgcggacgtactcaagttctgccggcgagccgatggcggaaagcagaaagacgacctggtagaaacctgcattcggttaaacaccacgcacgttgccatgcagcgtacgaagaaggccaagaacggccgcctggtgacggtatccgagggtgaagccttgattagccgctacaagatcgtaaagagcgaaaccgggcggccggagtacatcgagatcgagctagctgattggatgtaccgcgagatcacagaaggcaagaacccggacgtgctgacggttcaccccgattactttttgatcgatcccggcatc

ggccgttttctctaccgcctggcacgccgcgccgcaggcaaggcagaagccagatggttgttcaagacgatctacgaacgcagt
ggcagcgccggagagttcaagaagttctgtttcaccgtgcgcaagctgatcgggtcaaatgacctgccggagtacgatttgaagg
aggaggcggggcaggctggcccgatcctagtcatgcgctaccgcaacctgatcgagggcgaagcatccgccggttcctaatgt
acggagcagatgctagggcaaattgccctagcaggggaaaaaggtcgaaaaggtctctttcctgtggatagcacgtacattggg
aacccaaagccgtacattgggaaccggaacccgtacattgggaacccaaagccgtacattgggaaccggtcacacatgtaag
tgactgatataaaagagaaaaaaggcgatttttccgcctaaaactctttaaaacttattaaaactcttaaaacccgcctggcctgtgc
ataactgtctggccagcgcacagccgaagagctgcaaaaagcgcctacccttcggtcgctgcgctccctacgccccgccgcttc
gcgtcggcctatcgcggccgctggccgctcaaaaatggctggcctacggccaggcaatctaccagggcgcggacaagccgcg
ccgtcgccactcgaccgccggcgcccacatcaaggcaccctgcctcgcgcgtttcggtgatgacggtgaaaacctctgacacat
gcagctcccggagacggtcacagcttgtctgtaagcggatgccgggagcagacaagcccgtcagggcgcgtcagcgggtgtt
ggcgggtgtcggggcgcagccatgacccagtcacgtagcgatagcggagtgtatactggcttaactatgcggcatcagagcag
attgtactgagagtgcaccatatgcggtgtgaaataccgcacagatgcgtaaggagaaaataccgcatcaggcgctcttccgctt
cctcgctcactgactcgctgcgctcggtcgttcggctgcggcgagcggtatcagctcactcaaaggcggtaatacggttatccaca
gaatcaggggataacgcaggaaagaacatgtgagcaaaaggccagcaaaaggccaggaaccgtaaaaaggccgcgttgc
tggcgtttttccataggctccgcccccctgacgagcatcacaaaaatcgacgctcaagtcagaggtggcgaaacccgacagga
ctataaagataccaggcgtttccccctggaagctccctcgtgcgctctcctgttccgaccctgccgcttaccggatacctgtccgcctt
tctcccttcgggaagcgtggcgctttctcatagctcacgctgtaggtatctcagttcggtgtaggtcgttcgctccaagctgggctgtgt
gcacgaaccccccgttcagcccgaccgctgcgccttatccggtaactatcgtcttgagtccaacccggtaagacacgacttatcg
ccactggcagcagccactggtaacaggattagcagagcgaggtatgtaggcggtgctacagagttcttgaagtggtggcctaac
tacggctacactagaaggacagtatttggtatctgcgctctgctgaagccagttaccttcggaaaaagagttggtagctcttgatccg
gcaaacaaaccaccgctggtagcggtggtttttttgtttgcaagcagcagattacgcgcagaaaaaaaggatctcaagaagatc
ctttgatcttttctacggggtctgacgctcagtggaacgaaaactcacgttaagggattttggtcatgcattctaggtattatttgccaac
gaccttcgtgatctcgcccttgacatagtggacaaattcttcgagctggtcggcccgggacgcgagacggtcttcttcttggcccag
ataggcttggcgcgcttcgaggatcacgggctggtattgcgccggaaggcgctccatcgcccagtcggcggcgacatccttcgg
cgcgatcttgccggtaaccgccgagtaccaaatccggctcagcgtaaggaccacattgcgctcatcgcccgcccaatccggcg
gggagttccacagggtcagcgtctcgttcagtgcttcgaacagatcctgttccggcaccgggtcgaaaagttcctcggccgcggg
gccgacgagggccacgctatgctcccgggccttggtgagcaggatcgccagatcaatgtcgatggtggccggttcaaagatacc
cgccagaatatcattacgctgccattcgccgaactggagttcgcgtttggccggatagcgccaggggatgatgtcatcgtgcacca
caatcgtcacctcaaccgcgcgcaggatttcgctctcgccgggggaggcggacgtttccagaaggtcgttgataagcgcgcggc
gcgtggtctcgtcgagacggacggtaacggtgacaagcaggtcgatgtccgaatggggcttaaggccgccgtcaacggcgcta
ccatacagatgcacggcgaggagggtcggttcgaggtggcgctcgatgacacccacgacttccgacagctgggtggacacctc
ggcgatgaccgcttcacccatttattatttccttcctcttttctacagtatttaaagataccccaagaagctaattataacaagacgaac
tccaattcactgttccttgcattctaaaaccttaaataccagaaaacagctttttcaaagttgttttcaaagttggcgtataacatagtatc
gacggagccgattttgaaaccgcggtgatcacaggcagcaacgctctgtcatcgttacaatcaacatgctaccctccgcgagatc
atccgtgtttcaaacccggcagcttagttgccgttcttccgaatagcatcggtaacatgagcaaagtctgccgccttacaacggctct
cccgctgacgccgtcccggactgatgggctgcctgtatcgagtggtgattttgtgccgagctgccggtcggggagctgttggctggc
tggtggcaggatatattgtggtgtaaacaaattgacgcttagacaacttaataacacattgcggacgtttttaatgtactgaattagta
ctgataaatggcgcgccaagctttggcaaacagctattatgggtattatgggtggtaccacgcgtcgatccactagtaacggccgc
cagtgtgctggaattcgcccttggcgcgccgatctagtaacatagatgacaccgcgcgcgataatttatcctagtttgcgcgctatat
tttgttttctatcgcgtattaaatgtataattgcgggactctaatcataaaaacccatctcataaataacgtcatgcattacatgttaattat
tacatgcttaacgtaattcaacagaaattatatgataatcatcgcaagaccggcaacaggattcaatcttaagaaactttattgcca
aatgtttgaacgatctcagaagaactcgtcaagaaggcgatagaaggcgatgcgctgcgaatcgggagcggcgataccgtaa
agcacgaggaagcggtcagcccattcgccgccaagctcttcagcaatatcacgggtagccaacgctatgtcctgatagcggtcc
gccacacccagccggccacagtcgatgaatccagaaaagcggccattttccaccatgatattcggcaagcaggcatcgccatg
ggtcacgacgagatcctcgccgtcgggcatgcgcgccttgagcctggcgaacagttcggctggcgcgagcccctgatgctcttcg
tccagatcatcctgatcgacaagaccggcttccatccgagtacgtgctcgctcgatgcgatgtttcgcttggtggtcgaatgggcag

gtagccggatcaagcgtatgcagccgccgcattgcatcagccatgatggatactttctcggcaggagcaaggtgagatgacagg
agatcctgccccggcacttcgcccaatagcagccagtcccttcccgcttcagtgacaacgtcgagcacagctgcgcaaggaac
gcccgtcgtggccagccacgatagccgcgctgcctcgtcctgcagttcattcagggcaccggacaggtcggtcttgacaaaaag
aaccgggcgcccctgcgctgacagccggaacacggcggcatcagagcagccgattgtctgttgtgcccagtcatagccgaata
gcctctccacccaagcggccggagaacctgcgtgcaatccatcttgttcaatccacatggtggtgtgacctgcagaagtaacacc
aaacaacagggtgagcatcgacaaaagaaacagtaccaagcaaataaatagcgtatgaaggcagggctaaaaaaatccac
atatagctgctgcatatgccatcatccaagtatatcaagatcaaaataattataaaacatacttgtttattataatagataggtactcaa
ggttagagcatatgaatagatgctgcatatgccatcatgtatatgcatcagtaaaacccacatcaacatgtatacctatcctagatcg
atatttccatccatcttaaactcgtaactatgaagatgtatgacacacacatacagttccaaaattaataaatacaccaggtagtttg
aaacagtattctactccgatctagaacgaatgaacgaccgcccaaccacaccacatcatcacaaccaagcgaacaaaaagc
atctctgtatatgcatcagtaaaacccgcatcaacatgtatacctatcctagatcgatatttccatccatcatcttcaattcgtaactatg
aatatgtatggcacacacatacagatccaaaattaataaatccaccaggtagtttgaaacagaattctactccgatctagaacgac
cgcccaaccagaccacatcatcacaaccaagacaaaaaaaagcatgaaaagatgacccgacaaacaagtgcacggcatat
attgaaataaaggaaaagggcaaaccaaaccctatgcaacgaaacaaaaaaaatcatgaaatcgatcccgtctgcggaacg
gctagagccatcccaggattccccaaagagaaacactggcaagttagcaatcagaacgtgtctgacgtacaggtcgcatccgt
gtacgaacgctagcagcacggatctaacacaaacacggatctaacacaaacatgaacagaagtagaactaccgggccctaa
ccatggaccggaacgccgatctagagaaggtagagagggggggggggggaggacgagcggcgtaccttgaagcggaggt
gccgacgggtggatttgggggagatctggttgtgtgtgtgtgcgctccgaacaacacgaggttggggaaagagggtgtggaggg
ggtgtctatttattacggcgggcgaggaagggaaagcgaaggagcggtgggaaaggaatcccccgtagctgccgtgccgtga
gaggaggaggaggccgcctgccgtgccggctcacgtctgccgctccgccacgcaatttctggatgccgacagcggagcaagt
ccaacggtggagcggaactctcgagaggggtccagaggcagcgacagagatgc

cgtgccgtctgcttcgcttggcccgacgcgacgctgctggttcgctggttggtgtccgttagactcgtcgacggcgtttaacaggctg
gcattatctactcgaaacaagaaaaatgtttccttagttttttaatttcttaaagggtatttgtttaatttttagtcactttattttattctattttat
atctaaattattaaataaaaaaactaaaatagagttttagttttcttaatttagaggctaaaatagaataaaatagatgtactaaaaaa
attagtctataaaaaccattaaccctaaaccctaaatggatgtactaataaaatggatgaagtattatataggtgaagctatttgcaa
aaaaaaaggagaacacatgcacactaaaaagataaaactgtagagtcctgttgtcaaaatactcaattgtcctttagaccatgtct
aactgttcatttatatgattctctaaaacactgatattattgtagtactatagattatattattcgtagagtaaagtttaaatatatgtataaa
gatagataaactgcacttcaaacaagtgtgacaaaaaaaatatgtggtaatttttataacttagacatgcaatgctcattatctctag
agaggggcacgaccgggtcacgctgcactgcagggatccgatctagtaacatagatgacaccgcgcgcgataatttatcctagt
ttgcgcgctatattttgttttctatcgcgtattaaatgtataattgcgggactctaatcataaaaacccatctcataaataacgtcatgcat
tacatgttaattattacatgcttaacgtaattcaacagaaattatatgataatcatcgcaagaccggcaacaggattcaatcttaaga
aactttattgccaaatgtttgaacgatccctaggacgatctcacttgtacagctcgtccatgccgtgggtgatgccagctgcggtgac
gaactccagcaggaccatgtggtcgcgcttctcgttggggtccttgctcagagcggactgggtgctcaggtagtggttgtcgggca
gcagcacgggaccgtcgccgatgggcgtgttctgctggtagtggtcggcgagctggacgctgccgtcctcgatgttgtggcggat
cttgaagttgaccttgatgccgttcttctgcttgtcagccatgatgtagacgttgtggctgttgtagttgtactccagcttgtgccccagga
tgttgccgtcctccttgaagtcgatgcccttcagctcgatgcggttcaccagggtgtcgccctcgaacttcacctcggctcgggtcttgt
agttgccgtcgtccttgaagaagatggtgcgctcctggacgtagccttcgggcatggcggacttgaagaagtcgtgctgcttcatgt
ggtcggggtagcggctgaagcactgcacgccgtaggtgaaggtggtcacgagggtgggccagggcacgggcagcttgccggt
ggtgcagatgaacttcagggtcagcttgccgtaggtggcgtcgccctcgccctcgccgctgacgctgaacttgtggccgttcacgt
cgccgtccagctcgaccaggatgggcaccaccccagtgaacagctcctcgcccttgctcactacaaaaaagctccgcacgag
gctgcatttgtcacaaatcatgaaaagaaaaactaccgatgaacaatgctgagggattcaaattctacccacaaaaagaagaa
agaaagatctagcacatctaagcctgacgaagcagcagaaatatataaaaatataaaccatagtgcccttttcccctcttcctgat
cttgtttagcacggcggaaattttaaaccccccatcatctcccccaacaacggcggatcgcagatctacatccgagagccccattc
cccgcgagatccgggccggatccacgccggcgagagccccagccgcgagatcccgcccctcccgcgcaccgatctgggcg
cgcacgaagccgcctctcgcccacccaaactaccaaggccaaagatcgagaccgagacggaaaaaaaaacggagaaag
aaagaggagaggggcggggtggttaccggcggcggcggaggcctcccttggatcttatggtgtgttgtccctgtgtgttctccaat

agtgtggcttgagtgtgtggaagatggttctagaggatctgctagagtcagcttgtcagcgtgtcctctccaaatgaaatgaacttcct
tatatagaggaagggtcttgcgaaggatagtgggattgtgcgtcatcccttacgtcagtggagatatcacatcaatccacttgctttg
aagacgtggttggaacgtcttctttttccacgatgctcctcgtgggtgggggtccatctttgggaccactgtcggcagaggcatcttca
acgatggcctttcctttatcgcaatgatggcatttgtaggagccaccttccttttccactatcttcacaataaagtgacagatagctggg
caatggaatccgaggaggtttccggatattaccctttgttgaaaagtctcaatcggaccatcacatcaatccacttgctttgaagacg
tggttggaacgtcttctttttccacgatgctcctcgtgggtgggggtccatctttgggaccactgtcggcagaggcatcttcaacgatg
gcctttcctttatcgcaatgatggcatttgtaggagccaccttccttttccactatcttcacaataaagtgacagatagctgggcaatgg
aatccgaggaggtttccggatattaccctttgttgaaaagtctcaatcggacccccctcagcctgcagtgcagcgtgacccggtcgtg
cccctctctagagataatgagcattgcatgtctaagttataaaaaattaccacatattttttttgtcacacttgtttgaagtgcagtttatct
atctttatacatatatttaaactttactctacgaataatataatctatagtactacaataatatcagtgttttagagaatcatataaatgaa
cagttagacatggtctaaaggacaattgagtattttgacaacaggactctacagttttatctttttagtgtgcatgtgttctcctttttttttgc
aaatagcttcacctatataatacttcatccattttattagtacatccatttagggtttagggttaatggtttttatagactaatttttttagtaca
tctattttattctattttagcctctaaattaagaaaactaaaactctattttagtttttttatttaataatttagatataaaatagaataaaataa
agtgactaaaaattaaacaaataccctttaagaaattaaaaaaactaaggaaacatttttcttgtttcgagtagataatgccagcct
gttaaacgccgtcgacgagtctaacggacaccaaccagcgaaccagcagcgtcgcgtcgggccaagcgaagcagacggca
cggcatctctgtcgctgcctctggacccctctcgagagttccgctccaccgttggacttgctccgctgtcggcatccagaaattgcgt
ggcggagcggcagacgtgagccggcacggcaggcggcctcctcctcctctcacggcacggcagctacgggggattcctttccc
accgctccttcgctttcccttcctcgcccgccgtaataaatagacacccccctccacaccctctttccccaacctcgtgttgttcggagc
gcacacacacacaaccagatctcccccaaatccacccgtcggcacctccgcttcaaggtacgccgctcgtcctcccccccccc
cctctctaccttctctagatcggcgttccggtccatggttagggcccggtagttctacttctgttcatgtttgtgttagatccgtgtttgtgtta
gatccgtgctgctagcgttcgtacacggatgcgacctgtacgtcagacacgttctgattgctaacttgccagtgtttctctttggggaat
cctgggatggctctagccgttccgcagacgggatcgatttcatgattttttttgtttcgttgcatagggtttggtttgcccttttcctttatttca
atatatgccgtgcacttgtttgtcgggtcatcttttcatgcttttttttgtcttggttgtgatgatgtggtctggttgggcggtcgttctagatcgg
agtagaattctgtttcaaactacctggtggatttattaattttggatctgtatgtgtgtgccatacatattcatagttacgaattgaagatga
tggatggaaatatcgatctaggataggtatacatgttgatgcgggttttactgatgcatatacagagatgctttttgttcgcttggttgtga
tgatgtggtgtggttgggcggtcgttcattcgttctagatcggagtagaatactgtttcaaactacctggtgtatttattaattttggaactg
tatgtgtgtgtcatacatcttcatagttacgagtttaagatggatggaaatatcgatctaggataggtatacatgttgatgtgggttttact
gatgcatatacatgatggcatatgcagcatctattcatatgctctaaccttgagtacctatctattataataaacaagtatgttttataatt
attttgatcttgatatacttggatgatggcatatgcagcagctatatgtggatttttttagccctgccttcatacgctatttatttgcttggtact
gtttcttttgtcgatgctcaccctgttgtttggtgttacttctgcaggcgatcgccacaccaccatgccgaagaagaagcgcaaggtca
tggacaagaagtactccatcggcctggacatcggcaccaacagcgtgggctgggccgtcatcaccgacgagtacaaggtgcc
ctccaagaagttcaaggtcctcggcaacaccgacaggcacagcatcaagaagaacctgatcggcgccctgctgttcgactccg
gcgagactgcggaggctaccaggctgaagcgcactgctcgcaggcgctacaccaggcgcaagaaccgcatctgctacctcca
ggagattttctccaacgagatggccaaggtggacgactccttcttccaccgcctggaggagagcttcctggtcgaggaagacaa
gaagcacgagcgccaccctatcttcggcaacatcgtggacgaggtcgcctaccacgagaagtacccaaccatctaccacctcc
gcaagaagctggtggactccaccgacaaggccgacctgaggctcatctacctggccctcgcccacatgatcaagttccgcggc
cacttcctcatcgagggcgacctgaacccggacaacagcgacgtggacaagctcttcatccagctggtccagacctacaacca
gctgttcgaggagaaccccatcaacgcctccggcgtggacgctaaggctatcctcagcgctaggctgtccaagagcaggcgcc
tggagaacctcatcgcccagctcccgggcgagaagaagaacggcctcttcggcaacctgatcgctctgtccctcggcctgaccc
ccaacttcaagagcaacttcgacctggccgaggacgccaagctccagctgtccaaggacacctacgacgacgacctcgacaa
cctgctcgcccagatcggcgaccagtacgccgacctcttcctggccgccaagaacctctccgacgccatcctgctcagcgacat
cctgagggtgaacaccgagatcaccaaggccccgctgtccgccagcatgatcaagcgctacgacgagcaccaccaggacct
cactctcctgaaggccctcgtccgccagcagctgcccgagaagtacaaggagattttcttcgaccagagcaagaacggctacg
cgggctacatcgatggcggcgcctcccaggaagagttctacaagttcatcaagcctatcctggagaagatggacggcaccgag
gagctgctcgtgaagctgaaccgcgaggacctgctccgcaagcagaggaccttcgacaacggcagcatccctcaccagatcc
acctgggcgagctgcacgctatcctccgccgccaggaagacttctacccattcctgaaggacaaccgcgagaagatcgagaa

gatcctcaccttccgcatcccgtactacgtgggccccctggcccgcggcaactccaggttcgcctggatgaccaggaagagcga
ggagaccatcaccccgtggaacttcgaggaagtggtggacaagggcgcctccgctcagagcttcatcgagcgcatgaccaact
tcgacaagaacctccctaacgagaaggtgctgccaaagcactccctgctctacgagtacttcaccgtctacaacgagctgacca
aggtgaagtatgtgaccgagggcatgaggaagcccgccttcctcagcggcgagcagaagaaggccatcgtggacctgctcttc
aagaccaaccgcaaggtgaccgtcaagcagctgaaggaagactacttcaagaagatcgagtgcttcgactccgtggagatca
gcggcgtggaggaccgcttcaacgcctccctcggcacctaccacgacctgctcaagatcatcaaggacaaggacttcctcgac
aacgaggagaacgaggacatcctggaggacatcgtgctcaccctgaccctcttcgaggaccgcgagatgatcgaggagagg
ctcaagacctacgcccacctgttcgacgacaaggtcatgaagcagctgaagaggcgcaggtacactggctggggccgcctca
gcaggaagctgatcaacggcatcagggacaagcagtccggcaagaccatcctggacttcctcaagagcgacggcttcgccaa
ccgcaacttcatgcagctcatccacgacgactccctgaccttcaaggaagacatccagaaggctcaggtgtccggccagggcg
acagcctccacgagcacatcgctaacctggcgggcagccctgccatcaagaagggcatcctccagaccgtgaaggtggtgga
cgagctggtgaaggtcatgggccgccacaagccagagaacatcgtcatcgagatggccagggagaaccagaccacccaga
agggtcagaagaactcccgcgagaggatgaagaggatcgaggaaggcatcaaggagctgggcagccagatcctgaagga
gcacccggtggagaacacccagctccagaacgagaagctgtacctctactacctgcagaacggccgcgacatgtatgtggac
caggagctggacatcaacaggctgtccgactacgacgtggaccacatcgtccctcagtccttcctcaaggacgacagcatcgac
aacaaggtgctgacccgcagcgacaagaacaggggcaagtccgacaacgtcccaagcgaggaagtggtcaagaagatga
agaactactggcgccagctgctcaacgccaagctcatcacccagcgcaagttcgacaacctgactaaggcggagaggggcg
gcctgtccgagctggacaaggctggcttcatcaagcgccagctcgtggagaccaggcagatcaccaagcacgtcgcccagat
cctggacagcaggatgaacaccaagtacgacgagaacgacaagctcatccgcgaggtgaaggtcatcaccctcaagtccaa
gctggtgagcgacttccgcaaggacttccagttctacaaggtcagggagatcaacaactaccaccacgcccacgatgcttacct
caacgcggtggtgggcaccgccctcatcaagaagtaccctaagctggagagcgagttcgtgtacggcgactacaaggtgtacg
acgtccgcaagatgatcgccaagtccgagcaggagatcggcaaggccaccgccaagtacttcttctacagcaacatcatgaac
ttcttcaagaccgagatcaccctcgccaacggcgagatccgcaagaggccactgatcgagaccaacggcgagactggcgag
atcgtgtgggacaagggcagggacttcgccaccgtgaggaaggtcctgtccatgcctcaggtgaacatcgtcaagaagaccga
ggtccagaccggcggcttctccaaggagagcatcctcccaaagcgcaacagcgacaagctgatcgccaggaagaaggactg
ggacccgaagaagtacggtggcttcgactcccctactgtggcttacagcgtcctggtggtcgccaaggtggagaagggcaagtc
caagaagctgaagagcgtcaaggagctgctcggcatcaccatcatggagaggtccagcttcgagaagaacccgatcgacttc
ctggaggccaagggctacaaggaagtgaagaaggacctgatcatcaagctgcccaagtacagcctgttcgagctggagaacg
gccgcaagaggatgctcgcctccgctggcgagctgcagaagggcaacgagctggccctcccgtccaagtatgtgaacttcctgt
acctcgcctcccactacgagaagctgaagggcagccccgaggacaacgagcagaagcagctcttcgtcgagcagcacaag
cactacctggacgagatcatcgagcagatcagcgagttcagcaagcgcgtgatcctcgccgacgccaacctcgacaaggtcct
gtccgcctacaacaagcaccgcgacaagcctatcagggagcaggccgagaacatcatccacctgttcaccctcaccaacctg
ggcgccccagctgccttcaagtacttcgacaccaccatcgaccgcaagaggtacaccagcaccaaggaagtgctggacgcca
ccctgatccaccagtccatcaccggcctgtacgagactcgcatcgacctcagccagctgggcggcgacccgaagaagaagcg
caaagtctgagggaccctcgatcgacaagctcgagtttctccataataatgtgtgagtagttcccagataagggaattagggttcct
atagggtttcgctcatgtgttgagcatataagaaacccttagtatgtatttgtatttgtaaaatacttctatcaataaaatttctaattccta
aaaccaaaatccagtactaaaatccagatcccccgaattaacctgcaggggcggcagggagagttttaacattgactagcgtgc
tgataatttgtgagaaataataattgacaagtagatactgacatttgagaagagcttctgaactgttattagtaacaaaaatggaaa
gctgatgcacggaaaaaggaaagaaaaagccatacttttttttaggtaggaaaagaaaaagccatacgagactgatgtctctca
gatgggccgggatctgtctatctagcaggcagcagccctaccaacctcacgggccagcaattacgagtccttctaaaacgtccc
gccgagggcgcgtggccgtgctgtgcagcagcacgtctaacattagtcccacctcgccagtttacagggagcagaaccagctta
taagcggaggcgcggcaccaagaagcagcgctccggggcggaagtcggttttagagctagaaatagcaagttaaaataagg
ctagtccgttatcaacttgaaaaagtggcaccgagtcggtgcttttttttcctcgagggcgctccggggcggaagtcgtgggtaatcc
ttcaaaggattttaaaaaatactaaatctgagctaggatctgcagatgaggaagaaagacgaagacgagatagagagcggga
aattgagatgtaaccctagctgatggaacgggcacaggcacaaatacgggcatgggccgagatcgaggggcggcgcatcag
cagcagtgcagtaggcgagcagggccgccgcattggtgggccgcccatgccggtgcaccgccgccgctctcgcgggggccg

ccgtgccatggctcgtccgatgcaggatcaccggcgacggagaggcgaagccctaggccggcgctgtcgccacgccaggatc
ggtcgcccaggttgacgtgactgggttgcctgcgctgaacctaataacacattgctgggttgtggtgtaaacaaattgacgcttaga
caacttaataacacattgcggacgtttttaatgtactgaattagtactgatatcggtacccggggcggccgcgagctcgatccacta
gtaacggccgccagtgtgctggaattcgcccttggcgcgccgatctagtaacatagatgacaccgcgcgcgataatttatcctagtt
tgcgcgctatattttgttttctatcgcgtattaaatgtataattgcgggactctaatcataaaaacccatctcataaataacgtcatgcatt
acatgttaattattacatgcttaacgtaattcaacagaaattatatgataatcatcgcaagaccggcaacaggattcaatcttaagaa
actttattgccaaatgtttgaacgatctcagaagaactcgtcaagaaggcgatagaaggcgatgcgctgcgaatcgggagcggc
gataccgtaaagcacgaggaagcggtcagcccattcgccgccaagctcttcagcaatatcacgggtagccaacgctatgtcctg
atagcggtccgccacacccagccggccacagtcgatgaatccagaaaagcggccattttccaccatgatattcggcaagcagg
catcgccatgggtcacgacgagatcctcgccgtcgggcatgcgcgccttgagcctggcgaacagttcggctggcgcgagcccct
gatgctcttcgtccagatcatcctgatcgacaagaccggcttccatccgagtacgtgctcgctcgatgcgatgtttcgcttggtggtcg
aatgggcaggtagccggatcaagcgtatgcagccgccgcattgcatcagccatgatggatactttctcggcaggagcaaggtga
gatgacaggagatcctgccccggcacttcgcccaatagcagccagtcccttcccgcttcagtgacaacgtcgagcacagctgcg
caaggaacgcccgtcgtggccagccacgatagccgcgctgcctcgtcctgcagttcattcagggcaccggacaggtcggtcttg
acaaaaagaaccgggcgcccctgcgctgacagccggaacacggcggcatcagagcagccgattgtctgttgtgcccagtcat
agccgaatagcctctccacccaagcggccggagaacctgcgtgcaatccatcttgttcaatccacattctagagtcgacctgcag
aagtaacaccaaacaacagggtgagcatcgacaaaagaaacagtaccaagcaaataaatagcgtatgaaggcagggctaa
aaaaatccacatatagctgctgcatatgccatcatccaagtatatcaagatcaaaataattataaaacatacttgtttattataataga
taggtactcaaggttagagcatatgaatagatgctgcatatgccatcatgtatatgcatcagtaaaacccacatcaacatgtatacc
tatcctagatcgatatttccatccatcttaaactcgtaactatgaagatgtatgacacacacatacagttccaaaattaataaatacac
caggtagtttgaaacagtattctactccgatctagaacgaatgaacgaccgcccaaccacaccacatcatcacaaccaagcga
acaaaaagcatctctgtatatgcatcagtaaaacccgcatcaacatgtatacctatcctagatcgatatttccatccatcatcttcaatt
cgtaactatgaatatgtatggcacacacatacagatccaaaattaataaatccaccaggtagtttgaaacagaattctactccgatc
tagaacgaccgcccaaccagaccacatcatcacaaccaagacaaaaaaaagcatgaaaagatgacccgacaaacaagtg
cacggcatatattgaaataaaggaaaagggcaaaccaaaccctatgcaacgaaacaaaaaaaatcatgaaatcgatcccgt
ctgcggaacggctagagccatcccaggattccccaaagagaaacactggcaagttagcaatcagaacgtgtctgacgtacag
gtcgcatccgtgtacgaacgctagcagcacggatctaacacaaacacggatctaacacaaacatgaacagaagtagaactac
cgggccctaaccatggaccggaacgccgatctagagaaggtagagagggggggggggggggaggacgagcggcgtaccttg
aagcggaggtgccgacgggtggatttgggggagatctggttgtgtgtgtgtgcgctccgaacaacacgaggttggggaaagag
ggtgtggagggggtgtctatttattacggcgggcgaggaagggaaagcgaaggagcggtgggaaaggaatcccccgtagctg
ccgtgccgtgagaggaggaggaggccgcctgccgtgccggctcacgtctgccgctccgccacgcaatttctggatgccgacag
cggagcaagtccaacggtggagcggaactctcgagaggggtccagaggcagcgacagagatgccgtgccgtctgcttcgctt
ggcccgacgcgacgctgctggttcgctggttggtgtccgttagactcgtcgacggcgtttaacaggctggcattatctactcgaaac
aagaaaaatgtttccttagtttttttaatttcttaaagggtatttgtttaatttttagtcactttattttattctattttatatctaaattattaaataaa
aaaactaaaatagagttttagttttcttaatttagaggctaaaatagaataaaatagatgtactaaaaaaattagtctataaaaacca
ttaaccctaaaccctaaatggatgtactaataaaatggatgaagtattatataggtgaagctatttgcaaaaaaaaaggagaaca
catgcacactaaaaagataaaactgtagagtcctgttgtcaaaatactcaattgtcctttagaccatgtctaactgttcatttatatgatt
ctctaaaacactgatattattgtagtactatagattatattattcgtagagtaaagtttaaatatatgtataaagatagataaactgcactt
caaacaagtgtgacaaaaaaaatatgtggtaatttttataacttagacatgcaatgctcattatctctagagaggggcacgaccgg
gtcacgctgcactgcaggcatacgcgtgatccactagtaacggccgccagtgtgctggaattcgcccttggcgcgccgatctagt
aacatagatgacaccgcgcgcgataatttatcctagtttgcgcgctatattttgttttctatcgcgtattaaatgtataattgcgggactct
aatcataaaaacccatctcataaataacgtcatgcattacatgttaattattacatgcttaacgtaattcaacagaaattatatgataat
catcgcaagaccggcaacaggattcaatcttaagaaactttattgccaaatgtttgaacgatctcactcagcctcgagggtggcgg
tcactgggatgaactcgaacctgtcgatgatcacgccggcggtgccgctgaagttcctcacgcccacgatgttaccgagggagg
aggtgaaagcgttggcgctctcgaagtaaccgaagtcgctggattggaggttgtccagggaggtagcggtagctggcacggtgtt
ggagaagatggaggagttaccccagttcacgttgaggtggatcggggtcacggaagcgtacctcacgcgcaccctgtacctggt

ggaggtggatgggaagtggattggcacctcgatgtagcccctgttctggatgttgttgccgctgctgttgagcctcacgaggtcgcc accggtgaagcctgggccggaaatgacggaaccgttgaagaggaagttgcccttcacggccgggatttgggtgatgctgtcgg aggcgatgatgttgttgaactcagcgctgcggtggatccaggagaacatcggagccctgatgatgctcacggagctgttgctgaa gccggagcggaacatggacacgtggctcagcctgtgggagaagccttgcctgggtggcacgttgttgttctgtggtgggatctcgt ccagggagtccacggtgccgctcttcctgtagacagcggatggcaggttggaggaggtgccgtaggcgaactcggtgccgtcg agcacggacagttgctggttgttgataccgatgttgaagggcctcctgtacagggtggaggacagggtcctgtagacaccctgac ccagttgagccacgatgcgttgctgtggagcggcgttgcccatggtgccgtagagcgggaaggtgaactcggggccgctgaag cccactggggaggccatgatctggtggccggaccagtagtactcgcccctgtgagcgtcggtgtagatggtgatgctgttcaggat gtccatcaggtgtgggctcctgatggagccctcgataccctgggcggaaccgcggaagctaccgtcgaagttctccagcactgg gttggtgtagatctccctggtgagttgggacacggtgcggatcgggtaggtcctggagtcgtagttcgggaagagggacacaatgt ccagcacggtgagggtcaactccctcctgaactggttgtacctgatccagtccctggagtccggaccccagacgcgctccaggc cggtgttgtaccagcgcacagcgtggtcggtgtagttgccaatcagcctggtcaggtcgttgtagcggctgttgatggtggcagcat cgaagccccacctttggccgaacacgctcacgtcgcgcagcacgctgaggtgcaggttagcggcttgcacgtacacggacag gagcggcacttggtagttctggacggcgaacagtgggatagcggtggtcagggcgctgttcatgtcgttgaattgaatgcgcatttc ctcgcggagagctgggttggtcgggtcggcctcccactccctgaagctctcggcgtagatttggtagaggttgctcaggccctcca gcctggagatggcctggttcctggcgaactcttcgatcctctggttgatcagctgctcgatttgcaccaggaaggcgtcccattggga tggaccgaagataccccagatgatgtccaccaggccgagcacgaagccagcacctggcacgaactcgctgagcaggaactg ggtcaaggacagggagatgtcgatgggggtgtaaccggtctcgatgcgctcgccacccagcacctccacctctgggttgctcag gcagttgtatgggatgcactcgttgatgtttgggttgttgtccattctagagtcgacctgcagaagtaacaccaaacaacagggtga gcatcgacaaaagtgctgcatatgccatcatccaagtatatcaagatcaaaataattataaaacatacttgtttattataatagatag gtactcaaggttagagcatatgaatagatgctgcatatgccatcatgtatatgcatcagtaaaacccacatcaacatgtatacctatc ctagatcgatatttccatccatcttaaactcgtaactatgaagatgtatgacacacacatacagttccaaaattaataaatacaccag gtagtttgaaacagtattctactccgatctagaacgaatgaacgaccgcccaaccacaccacatcatcacaaccaagcgaaca aaaagcatctctgtatatgcatcagtaaaacccgcatcaacatgtatacctatcctagatcgatatttccatccatcatcttcaattcgt aactatgaatatgtatggcacacacatacagatccaaaattaataaatccaccaggtagtttgaaacagaattctactccgatcta gaacgaccgcccaaccagaccacatcatcacaaccaagacaaaaaaaagcatgaaaagatgacccgacaaacaagtgca cggcatatattgaaataaaggaaaagggcaaaccaaaccctatgcaacgaaacaaaaaaaatcatgaaatcgatcccgtctg cggaacggctagagccatcccaggattccccaaagagaaacactggcaagttagcaatcagaacgtgtctgacgtacaggtc gcatccgtgtacgaacgctagcagcacggatctaacacaaacacggatctaacacaaacatgaacagaagtagaactaccg ggccctaaccatggaccggaacgccgatctagagaaggtagagagggggggggggggaggacgagcggcgtaccttgaa gcggaggtgccgacgggtggatttgggggagatctggttgtgtgtgtgcgctccgaacaacacgaggttggggaaagagggt gtggagggggtgtctatttattacggcgggcgaggaagggaaagcgaaggagcggtgggaaaggaatcccccgtagctgccg tgccgtgagaggaggaggaggccgcctgccgtgccggctcacgtctgccgctccgccacgcaatttctggatgccgacagcgg agcaagtccaacggtggagcggaactctcgagaggggtccagaggcagcgacagagatgccgtgccgtctgcttcgcttggc ccgacgcgacgctgctggttcgctggttggtgtccgttagactcgtcgacggcgtttaacaggctggcattatctactcgaaacaag aaaaatgtttccttagtttttttaatttcttaaagggtatttgtttaatttttagtcactttattttattctattttatatctaaattattaaataaaaaa actaaaatagagttttagttttcttaatttagaggctaaaatagaataaaatagatgtactaaaaaaattagtctataaaaaccattaa ccctaaaccctaaatggatgtactaataaaatggatgaagtattatataggtgaagctatttgcaaaaaaaaaggagaacacatg cacactaaaaagataaaactgtagagtcctgttgtcaaaatactcaattgtcctttagaccatgtctaactgttcatttatatgattctct aaaacactgatattattgtagtactatagattatattattcgtagagtaaagtttaaatatatgtataaagatagataaactgcacttca aacaagtgtgacaaaaaaaaatatgtggtaattttttataacttagacatgcaatgctcattatctctagagaggggcacgaccgggt cacgctgcactgcaggcatcgatcgaagcttacgcgtccatggctgcaggagctcgtcgactctagactcgagggcgagcccc gagcgggggcagctgaggcgccattgggaggaggaagagggcagcgctccggggcggaagtcgtgcggcggcacgagg gaagggcacacgacggacggacggaggaagaagaagagcgcggaggtagctggccgagcgtgacctgcatgtcaagtca atggtcgcgggtgcgagtagaaaagagggaggacgaccggacacaccgccgcgtcaggtcaccgatgacctaacgtgtctg gtcactattcacgcgctaaaaacacttactagactcgatctaacgcgggcagccctctgagtccgatcatttattctgtgcgtccggt

catcgacgtgtgcgggaaagcgttggctgttaacggctagttttgaatcgtgtgacgtggcacaccacgacttccgccccggagc gcggtaccactagtattaattaagtttaaacggcgcgccaagggcgaattccagcacactggcggccgttactagtggatcgagc tcgtcgactctagactcgagggcgcgcctgacaggatatattggcgggtaaac

SEQ ID NO: 26

cttagaataacggatatttaaaagggcgtgaaaaggtttatccgttcgtccatttgtatgtgcatgccaaccacagggttcccctcgg gatcaaagtactttgatccaacccctccgctgctatagtgcagtcggcttctgacgttcagtgcagccgtcttctgaaaacgacatgt cgcacaagtcctaagttacgcgacaggctgccgccctgcccttttcctggcgttttcttgtcgcgtgttttagtcgcataaagtagaata cttgcgactagaaccggagacattacgccatgaacaagagcgccgccgctggcctgctgggctatgcccgcgtcagcaccgac gaccaggacttgaccaaccaacgggccgaactgcacgcggccggctgcaccaagctgttttccgagaagatcaccggcacca ggcgcgaccgcccggagctggccaggatgcttgaccacctacgccctggcgacgttgtgacagtgaccaggctagaccgcctg gcccgcagcacccgcgacctactggacattgccgagcgcatccaggaggccggcgcgggcctgcgtagcctggcagagccg tgggccgacaccaccacgccggccggccgcatggtgttgaccgtgttcgccggcattgccgagttcgagcgttccctaatcatcg accgcacccggagcgggcgcgaggccgccaaggcccgaggcgtgaagtttggcccccgccctaccctcaccccggcacag atcgcgcacgcccgcgagctgatcgaccaggaaggccgcaccgtgaaagaggcggctgcactgcttggcgtgcatcgctcga ccctgtaccgcgcacttgagcgcagcgaggaagtgacgcccaccgaggccaggcggcgcggtgccttccgtgaggacgcatt gaccgaggccgacgccctggcggccgccgagaatgaacgccaagaggaacaagcatgaaaccgcaccaggacggccag gacgaaccgtttttcattaccgaagagatcgaggcggagatgatcgcggccgggtacgtgttcgagccgcccgcgcacgtctca accgtgcggctgcatgaaatcctggccggtttgtctgatgccaagctggcggcctggccggccagcttggccgctgaagaaacc gagcgccgccgtctaaaaaggtgatgtgtatttgagtaaaacagcttgcgtcatgcggtcgctgcgtatatgatgcgatgagtaaat aaacaaatacgcaaggggaacgcatgaaggttatcgctgtacttaaccagaaaggcgggtcaggcaagacgaccatcgcaa cccatctagcccgcgccctgcaactcgccggggccgatgttctgttagtcgattccgatccccagggcagtgcccgcgattgggc ggccgtgcgggaagatcaaccgctaaccgttgtcggcatcgaccgcccgacgattgaccgcgacgtgaaggccatcggccgg cgcgacttcgtagtgatcgacggagcgccccaggcggcggacttggctgtgtccgcgatcaaggcagccgacttcgtgctgattc cggtgcagccaagcccttacgacatatgggccaccgccgacctggtggagctggttaagcagcgcattgaggtcacggatgga aggctacaagcggcctttgtcgtgtcgcgggcgatcaaaggcacgcgcatcggcggtgaggttgccgaggcgctggccgggta cgagctgcccattcttgagtcccgtatcacgcagcgcgtgagctacccaggcactgccgccgccggcacaaccgttcttgaatca gaacccgagggcgacgctgcccgcgaggtccaggcgctggccgctgaaattaaatcaaaactcatttgagttaatgaggtaaa gagaaaatgagcaaaagcacaaacacgctaagtgccggccgtccgagcgcacgcagcagcaaggctgcaacgttggcca gcctggcagacacgccagccatgaagcgggtcaactttcagttgccggcggaggatcacaccaagctgaagatgtacgcggt acgccaaggcaagaccattaccgagctgctatctgaatacatcgcgcagctaccagagtaaatgagcaaatgaataaatgagt agatgaattttagcggctaaaggaggcggcatggaaaatcaagaacaaccaggcaccgacgccgtggaatgccccatgtgtg gaggaacgggcggttggccaggcgtaagcggctgggttgtctgccggccctgcaatggcactggaacccccaagcccgagga atcggcgtgacggtcgcaaaccatccggcccggtacaaatcggcgcggcgctgggtgatgacctggtggagaagttgaaggc cgcgcaggccgcccagcggcaacgcatcgaggcagaagcacgccccggtgaatcgtggcaagcggccgctgatcgaatcc gcaaagaatcccggcaaccgccggcagccggtgcgccgtcgattaggaagccgcccaagggcgacgagcaaccagatttttt cgttccgatgctctatgacgtgggcacccgcgatagtcgcagcatcatggacgtggccgttttccgtctgtcgaagcgtgaccgac gagctggcgaggtgatccgctacgagcttccagacgggcacgtagaggtttccgcagggccggccggcatggccagtgtgtgg gattacgacctggtactgatggcggtttcccatctaaccgaatccatgaaccgataccgggaagggaagggagacaagcccgg ccgcgtgttccgtccacacgttgcggacgtactcaagttctgccggcgagccgatggcggaaagcagaaagacgacctggtag aaacctgcattcggttaaacaccacgcacgttgccatgcagcgtacgaagaaggccaagaacggccgcctggtgacggtatcc gagggtgaagccttgattagccgctacaagatcgtaaagagcgaaaccgggcggccggagtacatcgagatcgagctagctg attggatgtaccgcgagatcacagaaggcaagaacccggacgtgctgacggttcaccccgattactttttgatcgatcccggcatc ggccgttttctctaccgcctggcacgccgcgccgcaggcaaggcagaagccagatggttgttcaagacgatctacgaacgcagt ggcagcgccggagagttcaagaagttctgtttcaccgtgcgcaagctgatcgggtcaaatgacctgccggagtacgatttgaagg aggaggcggggcaggctggcccgatcctagtcatgcgctaccgcaacctgatcgagggcgaagcatccgccggttcctaatgt

acggagcagatgctagggcaaattgccctagcaggggaaaaaggtcgaaaaggtctctttcctgtggatagcacgtacattggg
aacccaaagccgtacattgggaaccggaacccgtacattgggaacccaaagccgtacattgggaaccggtcacacatgtaag
tgactgatataaaagagaaaaaaggcgatttttccgcctaaaactctttaaaacttattaaaactcttaaaacccgcctggcctgtgc
ataactgtctggccagcgcacagccgaagagctgcaaaaagcgcctacccttcggtcgctgcgctccctacgccccgccgcttc
gcgtcggcctatcgcggccgctggccgctcaaaaatggctggcctacggccaggcaatctaccagggcgcggacaagccgcg
ccgtcgccactcgaccgccggcgcccacatcaaggcaccctgcctcgcgcgtttcggtgatgacggtgaaaacctctgacacat
gcagctcccggagacggtcacagcttgtctgtaagcggatgccgggagcagacaagcccgtcagggcgcgtcagcgggtgtt
ggcgggtgtcggggcgcagccatgacccagtcacgtagcgatagcggagtgtatactggcttaactatgcggcatcagagcag
attgtactgagagtgcaccatatgcggtgtgaaataccgcacagatgcgtaaggagaaaataccgcatcaggcgctcttccgctt
cctcgctcactgactcgctgcgctcggtcgttcggctgcggcgagcggtatcagctcactcaaaggcggtaatacggttatccaca
gaatcaggggataacgcaggaaagaacatgtgagcaaaaggccagcaaaaggccaggaaccgtaaaaaggccgcgttgc
tggcgtttttccataggctccgcccccctgacgagcatcacaaaaatcgacgctcaagtcagaggtggcgaaacccgacagga
ctataaagataccaggcgtttccccctggaagctccctcgtgcgctctcctgttccgaccctgccgcttaccggatacctgtccgcctt
tctcccttcgggaagcgtggcgctttctcatagctcacgctgtaggtatctcagttcggtgtaggtcgttcgctccaagctgggctgtgt
gcacgaaccccccgttcagcccgaccgctgcgccttatccggtaactatcgtcttgagtccaacccggtaagacacgacttatcg
ccactggcagcagccactggtaacaggattagcagagcgaggtatgtaggcggtgctacagagttcttgaagtggtggcctaac
tacggctacactagaaggacagtatttggtatctgcgctctgctgaagccagttaccttcggaaaaagagttggtagctcttgatccg
gcaaacaaaccaccgctggtagcggtggtttttttgtttgcaagcagcagattacgcgcagaaaaaaaggatctcaagaagatc
ctttgatcttttctacggggtctgacgctcagtggaacgaaaactcacgttaagggattttggtcatgcattctaggtattatttgccaac
gaccttcgtgatctcgcccttgacatagtggacaaattcttcgagctggtcggcccgggacgcgagacggtcttcttcttggcccag
ataggcttggcgcgcttcgaggatcacgggctggtattgcgccggaaggcgctccatcgcccagtcggcggcgacatccttcgg
cgcgatcttgccggtaaccgccgagtaccaaatccggctcagcgtaaggaccacattgcgctcatcgcccgcccaatccggcg
gggagttccacagggtcagcgtctcgttcagtgcttcgaacagatcctgttccggcaccgggtcgaaaagttcctcggccgcggg
gccgacgagggccacgctatgctcccgggccttggtgagcaggatcgccagatcaatgtcgatggtggccggttcaaagatacc
cgccagaatatcattacgctgccattcgccgaactggagttcgcgtttggccggatagcgccaggggatgatgtcatcgtgcacca
caatcgtcacctcaaccgcgcgcaggatttcgctctcgccgggggaggcggacgtttccagaaggtcgttgataagcgcgcggc
gcgtggtctcgtcgagacggacggtaacggtgacaagcaggtcgatgtccgaatggggcttaaggccgccgtcaacggcgcta
ccatacagatgcacggcgaggagggtcggttcgaggtggcgctcgatgacacccacgacttccgacagctgggtggacacctc
ggcgatgaccgcttcacccatttattatttccttcctcttttctacagtatttaaagataccccaagaagctaattataacaagacgaac
tccaattcactgttccttgcattctaaaaccttaaataccagaaaacagctttttcaaagttgttttcaaagttggcgtataacatagtatc
gacggagccgattttgaaaccgcggtgatcacaggcagcaacgctctgtcatcgttacaatcaacatgctaccctccgcgagatc
atccgtgtttcaaacccggcagcttagttgccgttcttccgaatagcatcggtaacatgagcaaagtctgccgccttacaacggctct
cccgctgacgccgtcccggactgatgggctgcctgtatcgagtggtgattttgtgccgagctgccggtcggggagctgttggctggc
tggtggcaggatatattgtggtgtaaacaaattgacgcttagacaacttaataacacattgcggacgtttttaatgtactgaattagta
ctgataaatggcgcgccaagctttggcaaacagctattatgggtattatgggtggtaccacgcgtcggatccgatctagtaacata
gatgacaccgcgcgcgataatttatcctagtttgcgcgctatattttgttttctatcgcgtattaaatgtataattgcgggactctaatcat
aaaaacccatctcataaataacgtcatgcattacatgttaattattacatgcttaacgtaattcaacagaaattatatgataatcatcg
caagaccggcaacaggattcaatcttaagaaactttattgccaaatgtttgaacgatccctaggacgatctcacttgtagagctcgt
ccatgccgtagaggaacaggtgatggcggccctcggacctctcgtactgctcaacaatagtgtaatcctcgttatggctagtaatat
ccagcttagtatccacgtagtagtagccagggagctgaaccggcttcttggccatgtagatagtcttgaactccaccaggtaatgg
ccaccatccttcagcttgagagcctgatgaatctcgcccttcagaacgccatccctagggtagagcctctcagtggaggcctccca
ccccatggtcttcttctgcatgacagggccgtctggagggaagttggtgccgcgcatcttcaccttgtagatgagggtgccatcttgg
agggagctatcctgagtcacagtaaccaggccaccatcttcaaagttcatgacgcgttcccacttgaagccttctgggaaggaga
gcttcttgtaatcaggaatatcagcagggtgcttcacgtaggctttggagccgtacatgaactgaggggagaggatatcccaggc
gaaagggagagggccgcccttagtcactttgagcttagcagtctgggtgccttcataagggcggccctcgccctcaccttcaatct
cgaactcatggccgttcatggagccctccatcctgaccttgaagcgcatgaactccttgatcacggccatgttgttgtcctcgctgga

ggcggtgccgctggagccgctgccagtggagccagtgccgtggccgaggaacaggtggtgcctgccctcgctgcgctcgtact
gctccacgatggtgtagtcctcgttgtgggaggtgatgtcgagcttggtgtcgacgtagtagtagcctggcagctgcacaggcttctt
ggccatgtagatggtcttgaactcgaccaggtagtggccaccgtccttgagcttcagggcctggtggatctcgcccttgagcacgc
cgtccctggggtacagcctctcagtgctagcctcccagcccatggtcttcttctgcatgaccgggccatcgggcgggaagttagtgc
ccctcatcttcaccttgtagatgagggtgccatcctggaggctggagtcctgagtgacggtcacgaggccaccgtcctcgaagttc
atgacgcgctcccacttgaagccctcggggaaggacagcttcttgtagtcggggatgtcggccgggtgcttcacgtaggccttgct
gccgtacatgaactgcggggagaggatgtcccaagcgaatggcagcgggccgcccttagtgaccttgagcttggcggtctgggt
gccctcgtaaggcctgccctcgccctcgccctcgatctcgaactcgtggccgttcatgctgccctccatcctcaccttgaagcgcat
gaactccttgatcacttcctcgcccttggacaccactacaaaaaagctccgcacgaggctgcatttgtcacaaatcatgaaaaga
aaaactaccgatgaacaatgctgagggattcaaattctacccacaaaaagaagaaagaaagatctagcacatctaagcctga
cgaagcagcagaaatatataaaaatataaaccatagtgcccttttcccctcttcctgatcttgtttagcacggcggaaattttaaacc
ccccatcatctcccccaacaacggcggatcgcagatctacatccgagagccccattccccgcgagatccgggccggatccacg
ccggcgagagccccagccgcgagatcccgcccctcccgcgcaccgatctgggcgcgcacgaagccgcctctcgcccaccca
aactaccaaggccaaagatcgagaccgagacggaaaaaaaaacggagaaagaaagaggagaggggcggggtggttac
cggcggcggcggaggcctcccttggatcttatggtgtgttgtccctgtgtgttctccaatagtgtggcttgagtgtgtggaagatggttc
tagaggatctgctagagtcagcttgtcagcgtgtcctctccaaatgaaatgaacttccttatatagaggaagggtcttgcgaaggat
agtgggattgtgcgtcatcccttacgtcagtggagatatcacatcaatccacttgctttgaagacgtggttggaacgtcttctttttccac
gatgctcctcgtgggtgggggtccatctttgggaccactgtcggcagaggcatcttcaacgatggcctttcctttatcgcaatgatgg
catttgtaggagccaccttccttttccactatcttcacaataaagtgacagatagctgggcaatggaatccgaggaggtttccggata
ttaccctttgttgaaaagtctcaatcggaccatcacatcaatccacttgctttgaagacgtggttggaacgtcttctttttccacgatgctc
ctcgtgggtgggggtccatctttgggaccactgtcggcagaggcatcttcaacgatggcctttcctttatcgcaatgatggcatttgta
ggagccaccttccttttccactatcttcacaataaagtgacagatagctgggcaatggaatccgaggaggtttccggatattaccctt
tgttgaaaagtctcaatcggacccccctcagcctgcacctcgagggcgctccggggcggaagtcgtgggtaatccttcaaaggattt
taaaaaatactaaatctgagctaggatctgcagatgaggaagaaagacgaagacgagatagagagcgggaaattgagatgt
aaccctagctgatggaacgggcacaggcacaaatacgggcatgggccgagatcgaggggcggcgcatcagcagcagtgca
gtaggcgagcagggccgccgcattggtgggccgcccatgccggtgcaccgccgccgctctcgcgggggccgccgtgccatgg
ctcgtccgatgcaggatcaccggcgacggagaggcgaagccctaggccggcgctgtcgccacgccaggatcggtcgcccag
gttgacgtgactgggttgcctgcgctgaacctaataacacattgctgggttgtggtgtaaacaaattgacgcttagacaacttaataa
cacattgcggacgtttttaatgtactgaattagtactgatatcggtacccggggcggccgcgagctcgatccactagtaacggccg
ccagtgtgctggaattcgcccttggcgcgccgatctagtaacatagatgacaccgcgcgcgataatttatcctagtttgcgcgctata
ttttgttttctatcgcgtattaaatgtataattgcgggactctaatcataaaaacccatctcataaataacgtcatgcattacatgttaatta
ttacatgcttaacgtaattcaacagaaattatatgataatcatcgcaagaccggcaacaggattcaatcttaagaaactttattgcca
aatgtttgaacgatctcagaagaactcgtcaagaaggcgatagaaggcgatgcgctgcgaatcgggagcggcgataccgtaa
agcacgaggaagcggtcagcccattcgccgccaagctcttcagcaatatcacgggtagccaacgctatgtcctgatagcggtcc
gccacacccagccggccacagtcgatgaatccagaaaagcggccattttccaccatgatattcggcaagcaggcatcgccatg
ggtcacgacgagatcctcgccgtcgggcatgcgcgccttgagcctggcgaacagttcggctggcgcgagcccctgatgctcttcg
tccagatcatcctgatcgacaagaccggcttccatccgagtacgtgctcgctcgatgcgatgtttcgcttggtggtcgaatgggcag
gtagccggatcaagcgtatgcagccgccgcattgcatcagccatgatggatactttctcggcaggagcaaggtgagatgacagg
agatcctgccccggcacttcgcccaatagcagccagtcccttcccgcttcagtgacaacgtcgagcacagctgcgcaaggaac
gcccgtcgtggccagccacgatagccgcgctgcctcgtcctgcagttcattcagggcaccggacaggtcggtcttgacaaaaag
aaccgggcgcccctgcgctgacagccggaacacggcggcatcagagcagccgattgtctgttgtgcccagtcatagccgaata
gcctctccacccaagcggccggagaacctgcgtgcaatccatcttgttcaatccacattctagagtcgacctgcagaagtaacac
caaacaacagggtgagcatcgacaaaagaaacagtaccaagcaaataaatagcgtatgaaggcagggctaaaaaaatcca
catatagctgctgcatatgccatcatccaagtatatcaagatcaaaataattataaaacatacttgtttattataatagataggtactca
aggttagagcatatgaatagatgctgcatatgccatcatgtatatgcatcagtaaaacccacatcaacatgtatacctatcctagatc
gatatttccatccatcttaaactcgtaactatgaagatgtatgacacacacatacagttccaaaattaataaatacaccaggtagttt

gaaacagtattctactccgatctagaacgaatgaacgaccgcccaaccacaccacatcatcacaaccaagcgaacaaaaag
catctctgtatatgcatcagtaaaacccgcatcaacatgtatacctatcctagatcgatatttccatccatcatcttcaattcgtaactat
gaatatgtatggcacacacatacagatccaaaattaataaatccaccaggtagtttgaaacagaattctactccgatctagaacg
accgcccaaccagaccacatcatcacaaccaagacaaaaaaaagcatgaaaagatgacccgacaaacaagtgcacggca
tatattgaaataaaggaaaagggcaaaccaaaccctatgcaacgaaacaaaaaaaatcatgaaatcgatcccgtctgcggaa
cggctagagccatcccaggattccccaaagagaaacactggcaagttagcaatcagaacgtgtctgacgtacaggtcgcatcc
gtgtacgaacgctagcagcacggatctaacacaaacacggatctaacacaaacatgaacagaagtagaactaccgggccct
aaccatggaccggaacgccgatctagagaaggtagagaggggggggggggggaggacgagcggcgtaccttgaagcggag
gtgccgacgggtggatttgggggagatctggttgtgtgtgtgcgctccgaacaacacgaggttggggaaagagggtgtggag
ggggtgtctatttattacggcgggcgaggaagggaaagcgaaggagcggtgggaaaggaatcccccgtagctgccgtgccgt
gagaggaggaggaggccgcctgccgtgccggctcacgtctgccgctccgccacgcaatttctggatgccgacagcggagcaa
gtccaacggtggagcggaactctcgagaggggtccagaggcagcgacagagatgccgtgccgtctgcttcgcttggcccgacg
cgacgctgctggttcgctggttggtgtccgttagactcgtcgacggcgtttaacaggctggcattatctactcgaaacaagaaaaat
gtttccttagtttttttaatttcttaaagggtatttgtttaatttttagtcactttattttattctattttatatctaaattattaaataaaaaaactaaa
atagagttttagttttcttaatttagaggctaaaatagaataaaatagatgtactaaaaaaattagtctataaaaaccattaaccctaa
accctaaatggatgtactaataaaatggatgaagtattatataggtgaagctatttgcaaaaaaaaaggagaacacatgcacact
aaaaagataaaactgtagagtcctgttgtcaaaatactcaattgtcctttagaccatgtctaactgttcatttatatgattctctaaaaca
ctgatattattgtagtactatagattatattattcgtagagtaaagtttaaatatatgtataaagatagataaactgcacttcaaacaagt
gtgacaaaaaaaatatgtggtaatttttataacttagacatgcaatgctcattatctctagagaggggcacgaccgggtcacgctg
cactgcaggcatacgcgtgatccactagtaacggccgccagtgtgctggaattcgcccttggcgcgccgatctagtaacatagat
gacaccgcgcgcgataatttatcctagtttgcgcgctatattttgttttctatcgcgtattaaatgtataattgcgggactctaatcataaa
aacccatctcataaataacgtcatgcattacatgttaattattacatgcttaacgtaattcaacagaaattatatgataatcatcgcaa
gaccggcaacaggattcaatcttaagaaactttattgccaaatgtttgaacgatctcactcagcctcgagggtggcggtcactggg
atgaactcgaacctgtcgatgatcacgccggcggtgccgctgaagttcctcacgcccacgatgttaccgagggaggaggtgaa
agcgttggcgctctcgaagtaaccgaagtcgctggattggaggttgtccagggaggtagcggtagctggcacggtgttggagaa
gatggaggagttaccccagttcacgttgaggtggatcggggtcacggaagcgtacctcacgcgcaccctgtacctggtggaggt
ggatgggaagtggattggcacctcgatgtagcccctgttctggatgttgttgccgctgctgttgagcctcacgaggtcgccaccggt
gaagcctgggccggaaatgacggaaccgttgaagaggaagttgcccttcacggccgggatttgggtgatgctgtcggaggcga
tgatgttgttgaactcagcgctgcggtggatccaggagaacatcggagccctgatgatgctcacggagctgttgctgaagccgga
gcggaacatggacacgtggctcagcctgtgggagaagccttgcctgggtggcacgttgttgttctgtggtgggatctcgtccaggg
agtccacggtgccgctcttcctgtagacagcggatggcaggttggaggaggtgccgtaggcgaactcggtgccgtcgagcacg
gacagttgctggttgttgataccgatgttgaagggcctcctgtacagggtggaggacagggtcctgtagacaccctgacccagttg
agccacgatgcgttgctgtggagcggcgttgcccatggtgccgtagagcgggaaggtgaactcggggccgctgaagcccactg
gggaggccatgatctggtggccggaccagtagtactcgcccctgtgagcgtcggtgtagatggtgatgctgttcaggatgtccatc
aggtgtgggctcctgatggagccctcgataccctgggcggaaccgcggaagctaccgtcgaagttctccagcactgggttggtgt
agatctccctggtgagttgggacacggtgcggatcgggtaggtcctggagtcgtagttcgggaagagggacacaatgtccagca
cggtgagggtcaactccctcctgaactggttgtacctgatccagtccctggagtccggaccccagacgcgctccaggccggtgttg
taccagcgcacagcgtggtcggtgtagttgccaatcagcctggtcaggtcgttgtagcggctgttgatggtggcagcatcgaagcc
ccacctttggccgaacacgctcacgtcgcgcagcacgctgaggtgcaggttagcggcttgcacgtacacggacaggagcggc
acttggtagttctggacggcgaacagtgggatagcggtggtcagggcgctgttcatgtcgttgaattgaatgcgcatttcctcgcgg
agagctgggttggtcgggtcggcctcccactccctgaagctctcggcgtagatttggtagaggttgctcaggccctccagcctgga
gatggcctggttcctggcgaactcttcgatcctctggttgatcagctgctcgatttgcaccaggaaggcgtcccattgggatggaccg
aagataccccagatgatgtccaccaggccgagcacgaagccagcacctggcacgaactcgctgagcaggaactgggtcaag
gacagggagatgtcgatgggggtgtaaccggtctcgatgcgctcgccacccagcacctccacctctgggttgctcaggcagttgt
atgggatgcactcgttgatgtttgggttgttgtccattctagagtcgacctgcagaagtaacaccaaacaacagggtgagcatcga
caaaagtgctgcatatgccatcatccaagtatatcaagatcaaaataattataaaacatacttgtttattataatagataggtactcaa

ggttagagcatatgaatagatgctgcatatgccatcatgtatatgcatcagtaaaacccacatcaacatgtatacctatcctagatcg atatttccatccatcttaaactcgtaactatgaagatgtatgacacacacatacagttccaaaattaataaatacaccaggtagtttg aaacagtattctactccgatctagaacgaatgaacgaccgcccaaccacaccacatcatcacaaccaagcgaacaaaaagc atctctgtatatgcatcagtaaaacccgcatcaacatgtatacctatcctagatcgatatttccatccatcatcttcaattcgtaactatg aatatgtatggcacacacatacagatccaaaattaataaatccaccaggtagtttgaaacagaattctactccgatctagaacgac cgcccaaccagaccacatcatcacaaccaagacaaaaaaaagcatgaaaagatgacccgacaaacaagtgcacggcatat attgaaataaaggaaaagggcaaaccaaaccctatgcaacgaaacaaaaaaaatcatgaaatcgatcccgtctgcggaacg gctagagccatcccaggattccccaaagagaaacactggcaagttagcaatcagaacgtgtctgacgtacaggtcgcatccgt gtacgaacgctagcagcacggatctaacacaaacacggatctaacacaaacatgaacagaagtagaactaccgggccctaa ccatggaccggaacgccgatctagagaaggtagagaggggggggggggggaggacgagcggcgtaccttgaagcggaggt gccgacgggtggatttgggggagatctggttgtgtgtgtgtgcgctccgaacaacacgaggttggggaaagagggtgtggaggg ggtgtctatttattacggcgggcgaggaagggaaagcgaaggagcggtgggaaaggaatcccccgtagctgccgtgccgtga gaggaggaggaggccgcctgccgtgccggctcacgtctgccgctccgccacgcaatttctggatgccgacagcggagcaagt ccaacggtggagcggaactctcgagaggggtccagaggcagcgacagagatgccgtgccgtctgcttcgcttggcccgacgc gacgctgctggttcgctggttggtgtccgttagactcgtcgacggcgtttaacaggctggcattatctactcgaaacaagaaaaatgt ttccttagtttttttaatttcttaaagggtatttgtttaatttttagtcactttattttattctattttatatctaaattattaaataaaaaaactaaaat agagttttagttttcttaatttagaggctaaaatagaataaaatagatgtactaaaaaaattagtctataaaaaccattaaccctaaac cctaaatggatgtactaataaaatggatgaagtattatataggtgaagctatttgcaaaaaaaaaggagaacacatgcacactaa aaagataaaactgtagagtcctgttgtcaaaatactcaattgtcctttagaccatgtctaactgttcatttatatgattctctaaaacact gatattattgtagtactatagattatattattcgtagagtaaagtttaaatatatgtataaagatagataaactgcacttcaaacaagtgt gacaaaaaaaatatgtggtaatttttttataacttagacatgcaatgctcattatctctagagaggggcacgaccgggtcacgctgca ctgcaggcatcgatcgaagcttacgcgtccatggctgcaggagctcgtcgactctagactcgagggcgagccccgagcggggg cagctgaggcgccattgggaggaggaagagggcagcgctccggggcggaagtcgtgcggcggcacgagggaagggcac acgacggacggacggaggaagaagaagagcgcggaggtagctggccgagcgtgacctgcatgtcaagtcaatggtcgcgg gtgcgagtagaaaagagggaggacgaccggacacaccgccgcgtcaggtcaccgatgacctaacgtgtctggtcactattca cgcgctaaaaacacttactagactcgatctaacgcgggcagccctctgagtccgatcatttattctgtgcgtccggtcatcgacgtgt gcgggaaagcgttggctgttaacggctagttttgaatcgtgtgacgtggcacaccacgacttccgccccggagcgcggtaccact agtattaattaagtttaaacggcgcgccaagggcgaattccagcacactggcggccgttactagtggatcgagctcgtcgactcta gactcgagggcgcgcctgacaggatatattggcgggtaaac

SEQ ID NO: 27

cttagaataacggatatttaaaagggcgtgaaaaggtttatccgttcgtccatttgtatgtgcatgccaaccacagggttcccctcgg gatcaaagtactttgatccaacccctccgctgctatagtgcagtcggcttctgacgttcagtgcagccgtcttctgaaaacgacatgt cgcacaagtcctaagttacgcgacaggctgccgccctgccttttcctggcgttttcttgtcgcgtgttttagtcgcataaagtagaata cttgcgactagaaccggagacattacgccatgaacaagagcgccgccgctggcctgctgggctatgcccgcgtcagcaccgac gaccaggacttgaccaaccaacgggccgaactgcacgcggccggctgcaccaagctgttttccgagaagatcaccggcacca ggcgcgaccgcccggagctggccaggatgcttgaccacctacgccctggcgacgttgtgacagtgaccaggctagaccgcctg gcccgcagcacccgcgacctactggacattgccgagcgcatccaggaggccggcgcgggcctgcgtagcctggcagagccg tgggccgacaccaccacgccggccggccgcatggtgttgaccgtgttcgccggcattgccgagttcgagcgttccctaatcatcg accgcacccggagcgggcgcgaggccgccaaggcccgaggcgtgaagtttggcccccgccctaccctcaccccggcacag atcgcgcacgcccgcgagctgatcgaccaggaaggccgcaccgtgaaagaggcggctgcactgcttggcgtgcatcgctcga ccctgtaccgcgcacttgagcgcagcgaggaagtgacgcccaccgaggccaggcggcgcggtgccttccgtgaggacgcatt gaccgaggccgacgccctggcggccgccgagaatgaacgccaagaggaacaagcatgaaaccgcaccaggacggccag gacgaaccgtttttcattaccgaagagatcgaggcggagatgatcgcggccgggtacgtgttcgagccgcccgcgcacgtctca accgtgcggctgcatgaaatcctggccggtttgtctgatgccaagctggcggcctggccggccagcttggccgctgaagaaacc gagcgccgccgtctaaaaaggtgatgtgtatttgagtaaaacagcttgcgtcatgcggtcgctgcgtatatgatgcgatgagtaaat

aaacaaatacgcaaggggaacgcatgaaggttatcgctgtacttaaccagaaaggcgggtcaggcaagacgaccatcgcaa cccatctagcccgcgccctgcaactcgccggggccgatgttctgttagtcgattccgatccccagggcagtgcccgcgattgggc ggccgtgcgggaagatcaaccgctaaccgttgtcggcatcgaccgcccgacgattgaccgcgacgtgaaggccatcggccgg cgcgacttcgtagtgatcgacggagcgccccaggcggcggacttggctgtgtccgcgatcaaggcagccgacttcgtgctgattc cggtgcagccaagcccttacgacatatgggccaccgccgacctggtggagctggttaagcagcgcattgaggtcacggatgga aggctacaagcggcctttgtcgtgtcgcgggcgatcaaaggcacgcgcatcggcggtgaggttgccgaggcgctggccgggta cgagctgcccattcttgagtcccgtatcacgcagcgcgtgagctacccaggcactgccgccgccggcacaaccgttcttgaatca gaacccgagggcgacgctgcccgcgaggtccaggcgctggccgctgaaattaaatcaaaactcatttgagttaatgaggtaaa gagaaaatgagcaaaagcacaaacacgctaagtgccggccgtccgagcgcacgcagcagcaaggctgcaacgttggcca gcctggcagacacgccagccatgaagcgggtcaactttcagttgccggcggaggatcacaccaagctgaagatgtacgcggt acgccaaggcaagaccattaccgagctgctatctgaatacatcgcgcagctaccagagtaaatgagcaaatgaataaatgagt agatgaattttagcggctaaaggaggcggcatggaaaatcaagaacaaccaggcaccgacgccgtggaatgccccatgtgtg gaggaacgggcggttggccaggcgtaagcggctgggttgtctgccggccctgcaatggcactggaacccccaagcccgagga atcggcgtgacggtcgcaaaccatccggcccggtacaaatcggcgcggcgctgggtgatgacctggtggagaagttgaaggc cgcgcaggccgcccagcggcaacgcatcgaggcagaagcacgccccggtgaatcgtggcaagcggccgctgatcgaatcc gcaaagaatcccggcaaccgccggcagccggtgcgccgtcgattaggaagccgcccaagggcgacgagcaaccagattttt cgttccgatgctctatgacgtgggcacccgcgatagtcgcagcatcatggacgtggccgttttccgtctgtcgaagcgtgaccgac gagctggcgaggtgatccgctacgagcttccagacgggcacgtagaggtttccgcagggccggccggcatggccagtgtgtgg gattacgacctggtactgatggcggtttcccatctaaccgaatccatgaaccgataccgggaagggaagggagacaagcccgg ccgcgtgttccgtccacacgttgcggacgtactcaagttctgccggcgagccgatggcggaaagcagaaagacgacctggtag aaacctgcattcggttaaacaccacgcacgttgccatgcagcgtacgaagaaggccaagaacggccgcctggtgacggtatcc gagggtgaagccttgattagccgctacaagatcgtaaagagcgaaaccgggcggccggagtacatcgagatcgagctagctg attggatgtaccgcgagatcacagaaggcaagaacccggacgtgctgacggttcaccccgattactttttgatcgatcccggcatc ggccgttttctctaccgcctggcacgccgcgccgcaggcaaggcagaagccagatggttgttcaagacgatctacgaacgcagt ggcagcgccggagagttcaagaagttctgtttcaccgtgcgcaagctgatcgggtcaaatgacctgccggagtacgatttgaagg aggaggcggggcaggctggcccgatcctagtcatgcgctaccgcaacctgatcgagggcgaagcatccgccggttcctaatgt acggagcagatgctagggcaaattgccctagcaggggaaaaaggtcgaaaaggtctctttcctgtggatagcacgtacattggg aacccaaagccgtacattgggaaccggaacccgtacattgggaacccaaagccgtacattgggaaccggtcacacatgtaag tgactgatataaaagagaaaaaaggcgatttttccgcctaaaactctttaaaacttattaaaactcttaaaacccgcctggcctgtgc ataactgtctggccagcgcacagccgaagagctgcaaaaagcgcctacccttcggtcgctgcgctccctacgccccgccgcttc gcgtcggcctatcgcggccgctggccgctcaaaaatggctggcctacggccaggcaatctaccagggcgcggacaagccgcg ccgtcgccactcgaccgccggcgcccacatcaaggcaccctgcctcgcgcgtttcggtgatgacggtgaaaacctctgacacat gcagctcccggagacggtcacagcttgtctgtaagcggatgccgggagcagacaagcccgtcagggcgcgtcagcgggtgtt ggcgggtgtcggggcgcagccatgacccagtcacgtagcgatagcggagtgtatactggcttaactatgcggcatcagagcag attgtactgagagtgcaccatatgcggtgtgaaataccgcacagatgcgtaaggagaaaataccgcatcaggcgctcttccgctt cctcgctcactgactcgctgcgctcggtcgttcggctgcggcgagcggtatcagctcactcaaaggcggtaatacggttatccaca gaatcaggggataacgcaggaaagaacatgtgagcaaaaggccagcaaaaggccaggaaccgtaaaaaggccgcgttgc tggcgtttttccataggctccgcccccctgacgagcatcacaaaaatcgacgctcaagtcagaggtggcgaaacccgacagga ctataaagataccaggcgtttccccctggaagctccctcgtgcgctctcctgttccgaccctgccgcttaccggatacctgtccgcctt tctcccttcgggaagcgtggcgctttctcatagctcacgctgtaggtatctcagttcggtgtaggtcgttcgctccaagctgggctgtgt gcacgaaccccccgttcagcccgaccgctgcgccttatccggtaactatcgtcttgagtccaacccggtaagacacgacttatcg ccactggcagcagccactggtaacaggattagcagagcgaggtatgtaggcggtgctacagagttcttgaagtggtggcctaac tacggctacactagaaggacagtatttggtatctgcgctctgctgaagccagttaccttcggaaaaagagttggtagctcttgatccg gcaaacaaaccaccgctggtagcggtggtttttttgtttgcaagcagcagattacgcgcagaaaaaaaggatctcaagaagatc ctttgatcttttctacggggtctgacgctcagtggaacgaaaactcacgttaagggattttggtcatgcattctaggtattatttgccaac gaccttcgtgatctcgcccttgacatagtggacaaattcttcgagctggtcggcccgggacgcgagacggtcttcttcttggcccag

ataggcttggcgcgcttcgaggatcacgggctggtattgcgccggaaggcgctccatcgcccagtcggcggcgacatccttcgg
cgcgatcttgccggtaaccgccgagtaccaaatccggctcagcgtaaggaccacattgcgctcatcgcccgcccaatccggcg
gggagttccacagggtcagcgtctcgttcagtgcttcgaacagatcctgttccggcaccgggtcgaaaagttcctcggccgcggg
gccgacgagggccacgctatgctcccgggccttggtgagcaggatcgccagatcaatgtcgatggtggccggttcaaagatacc
cgccagaatatcattacgctgccattcgccgaactggagttcgcgtttggccggatagcgccaggggatgatgtcatcgtgcacca
caatcgtcacctcaaccgcgcgcaggatttcgctctcgccgggggaggcggacgtttccagaaggtcgttgataagcgcgcggc
gcgtggtctcgtcgagacggacggtaacggtgacaagcaggtcgatgtccgaatggggcttaaggccgccgtcaacggcgcta
ccatacagatgcacggcgaggagggtcggttcgaggtggcgctcgatgacacccacgacttccgacagctgggtggacacctc
ggcgatgaccgcttcacccatttattatttccttcctcttttctacagtatttaaagataccccaagaagctaattataacaagacgaac
tccaattcactgttccttgcattctaaaaccttaaataccagaaaacagctttttcaaagttgttttcaaagttggcgtataacatagtatc
gacggagccgattttgaaaccgcggtgatcacaggcagcaacgctctgtcatcgttacaatcaacatgctaccctccgcgagatc
atccgtgtttcaaacccggcagcttagttgccgttcttccgaatagcatcggtaacatgagcaaagtctgccgccttacaacggctct
cccgctgacgccgtcccggactgatgggctgcctgtatcgagtggtgattttgtgccgagctgccggtcggggagctgttggctggc
tggtggcaggatatattgtggtgtaaacaaattgacgcttagacaacttaataacacattgcggacgtttttaatgtactgaattagta
ctgataaatggcgcgccaagctttggcaaacagctattatgggtattatgggtggtaccacgcgtcgatccactagtaacggccgc
cagtgtgctggaattcgcccttggcgcgccgatctagtaacatagatgacaccgcgcgcgataatttatcctagtttgcgcgctatat
tttgttttctatcgcgtattaaatgtataattgcgggactctaatcataaaaacccatctcataaataacgtcatgcattacatgttaattat
tacatgcttaacgtaattcaacagaaattatatgataatcatcgcaagaccggcaacaggattcaatcttaagaaactttattgcca
aatgtttgaacgatctcagaagaactcgtcaagaaggcgatagaaggcgatgcgctgcgaatcgggagcggcgataccgtaa
agcacgaggaagcggtcagcccattcgccgccaagctcttcagcaatatcacgggtagccaacgctatgtcctgatagcggtcc
gccacacccagccggccacagtcgatgaatccagaaaagcggccattttccaccatgatattcggcaagcaggcatcgccatg
ggtcacgacgagatcctcgccgtcgggcatgcgcgccttgagcctggcgaacagttcggctggcgcgagcccctgatgctcttcg
tccagatcatcctgatcgacaagaccggcttccatccgagtacgtgctcgctcgatgcgatgtttcgcttggtggtcgaatgggcag
gtagccggatcaagcgtatgcagccgccgcattgcatcagccatgatggatactttctcggcaggagcaaggtgagatgacagg
agatcctgccccggcacttcgcccaatagcagccagtcccttcccgcttcagtgacaacgtcgagcacagctgcgcaaggaac
gcccgtcgtggccagccacgatagccgcgctgcctcgtcctgcagttcattcagggcaccggacaggtcggtcttgacaaaaag
aaccgggcgcccctgcgctgacagccggaacacggcggcatcagagcagccgattgtctgttgtgcccagtcatagccgaata
gcctctccacccaagcggccggagaacctgcgtgcaatccatcttgttcaatccacatggtggtgtgacctgcagaagtaacacc
aaacaacagggtgagcatcgacaaaagaaacagtaccaagcaaataaatagcgtatgaaggcagggctaaaaaaatccac
atatagctgctgcatatgccatcatccaagtatatcaagatcaaaataattataaaacatacttgtttattataatagataggtactcaa
ggttagagcatatgaatagatgctgcatatgccatcatgtatatgcatcagtaaaacccacatcaacatgtatacctatcctagatcg
atatttccatccatcttaaactcgtaactatgaagatgtatgacacacacatacagttccaaaattaataaatacaccaggtagtttg
aaacagtattctactccgatctagaacgaatgaacgaccgcccaaccacaccacatcatcacaaccaagcgaacaaaaagc
atctctgtatatgcatcagtaaaacccgcatcaacatgtatacctatcctagatcgatatttccatccatcatcttcaattcgtaactatg
aatatgtatggcacacacatacagatccaaaattaataaatccaccaggtagtttgaaacagaattctactccgatctagaacgac
cgcccaaccagaccacatcatcacaaccaagacaaaaaaaagcatgaaaagatgacccgacaaacaagtgcacggcatat
attgaaataaaggaaaagggcaaaccaaaccctatgcaacgaaacaaaaaaaatcatgaaatcgatcccgtctgcggaacg
gctagagccatcccaggattccccaaagagaaacactggcaagttagcaatcagaacgtgtctgacgtacaggtcgcatccgt
gtacgaacgctagcagcacggatctaacacaaacacggatctaacacaaacatgaacagaagtagaactaccgggccctaa
ccatggaccggaacgccgatctagagaaggtagagagggggggggggggaggacgagcggcgtaccttgaagcggaggt
gccgacgggtggatttgggggagatctggttgtgtgtgtgtgcgctccgaacaacacgaggttggggaaagagggtgtggaggg
ggtgtctatttattacggcgggcgaggaagggaaagcgaaggagcggtgggaaaggaatcccccgtagctgccgtgccgtga
gaggaggaggaggccgcctgccgtgccggctcacgtctgccgctccgccacgcaatttctggatgccgacagcggagcaagt
ccaacggtggagcggaactctcgagaggggtccagaggcagcgacagagatgccgtgccgtctgcttcgcttggcccgacgc
gacgctgctggttcgctggttggtgtccgttagactcgtcgacggcgtttaacaggctggcattatctactcgaaacaagaaaaatgt
ttccttagtttttttaatttcttaaagggtatttgtttaatttttagtcactttattttattctattttatatctaaattattaaataaaaaaactaaaat

agagttttagttttcttaatttagaggctaaaatagaataaaatagatgtactaaaaaaattagtctataaaaaccattaaccctaaac
cctaaatggatgtactaataaaatggatgaagtattatataggtgaagctatttgcaaaaaaaaaggagaacacatgcacactaa
aaagataaaactgtagagtcctgttgtcaaaatactcaattgtcctttagaccatgtctaactgttcatttatatgattctctaaaacact
gatattattgtagtactatagattatattattcgtagagtaaagtttaaatatatgtataaagatagataaactgcacttcaaacaagtgt
gacaaaaaaaaatatgtggtaatttttataacttagacatgcaatgctcattatctctagagaggggcacgaccgggtcacgctgca
ctgcagggatccgatctagtaacatagatgacaccgcgcgcgataatttatcctagtttgcgcgctatattttgttttctatcgcgtatta
aatgtataattgcgggactctaatcataaaaacccatctcataaataacgtcatgcattacatgttaattattacatgcttaacgtaatt
caacagaaattatatgataatcatcgcaagaccggcaacaggattcaatcttaagaaactttattgccaaatgtttgaacgatccct
aggacgatctcacttgtacagctcgtccatgccgtgggtgatgccagctgcggtgacgaactccagcaggaccatgtggtcgcgc
ttctcgttggggtccttgctcagagcggactgggtgctcaggtagtggttgtcgggcagcagcacgggaccgtcgccgatgggcgt
gttctgctggtagtggtcggcgagctggacgctgccgtcctcgatgttgtggcggatcttgaagttgaccttgatgccgttcttctgcttg
tcagccatgatgtagacgttgtggctgttgtagttgtactccagcttgtgccccaggatgttgccgtcctccttgaagtcgatgcccttca
gctcgatgcggttcaccagggtgtcgccctcgaacttcacctcggctcgggtcttgtagttgccgtcgtccttgaagaagatggtgcg
ctcctggacgtagccttcgggcatggcggacttgaagaagtcgtgctgcttcatgtggtcggggtagcggctgaagcactgcacg
ccgtaggtgaaggtggtcacgagggtgggccagggcacgggcagcttgccggtggtgcagatgaacttcagggtcagcttgcc
gtaggtggcgtcgccctcgccctcgccgctgacgctgaacttgtggccgttcacgtcgccgtccagctcgaccaggatgggcacc
accccagtgaacagctcctcgcccttgctcactacaaaaaagctccgcacgaggctgcatttgtcacaaatcatgaaaagaaaa
actaccgatgaacaatgctgagggattcaaattctacccacaaaaagaagaaagaaagatctagcacatctaagcctgacga
agcagcagaaatatataaaaatataaaccatagtgcccttttcccctcttcctgatcttgtttagcacggcggaaattttaaaccccc
atcatctcccccaacaacggcggatcgcagatctacatccgagagccccattccccgcgagatccgggccggatccacgccgg
cgagagccccagccgcgagatcccgcccctcccgcgcaccgatctgggcgcgcacgaagccgcctctcgcccacccaaact
accaaggccaaagatcgagaccgagacggaaaaaaaaacggagaaagaaagaggagaggggcggggtggttaccggc
ggcggcggaggcctcccttggatcttatggtgtgttgtccctgtgtgttctccaatagtgtggcttgagtgtgtggaagatggttctaga
ggatctgctagagtcagcttgtcagcgtgtcctctccaaatgaaatgaacttccttatatagaggaagggtcttgcgaaggatagtg
ggattgtgcgtcatcccttacgtcagtggagatatcacatcaatccacttgctttgaagacgtggttggaacgtcttctttttccacgatg
ctcctcgtgggtgggggtccatctttgggaccactgtcggcagaggcatcttcaacgatggcctttcctttatcgcaatgatggcattt
gtaggagccaccttccttttccactatcttcacaataaagtgacagatagctgggcaatggaatccgaggaggtttccggatattac
cctttgttgaaaagtctcaatcggaccatcacatcaatccacttgctttgaagacgtggttggaacgtcttctttttccacgatgctcctc
gtgggtgggggtccatctttgggaccactgtcggcagaggcatcttcaacgatggcctttcctttatcgcaatgatggcatttgtagga
gccaccttccttttccactatcttcacaataaagtgacagatagctgggcaatggaatccgaggaggtttccggatattaccctttgtt
gaaaagtctcaatcggacccccctcagcctgcagtgcagcgtgacccggtcgtgcccctctctagagataatgagcattgcatgtct
aagttataaaaaattaccacatattttttttgtcacacttgtttgaagtgcagtttatctatctttatacatatatttaaactttactctacgaat
aatataatctatagtactacaataatatcagtgttttagagaatcatataaatgaacagttagacatggtctaaaggacaattgagtat
tttgacaacaggactctacagttttatctttttagtgtgcatgtgttctcctttttttttgcaaatagcttcacctatataatacttcatccattttat
tagtacatccatttagggtttagggttaatggtttttatagactaattttttagtacatctattttattctattttagcctctaaattaagaaaac
taaaactctattttagttttttatttaataatttagatataaaatagaataaaataaagtgactaaaaattaaacaaataccctttaaga
aattaaaaaaactaaggaaacatttttcttgtttcgagtagataatgccagcctgttaaacgccgtcgacgagtctaacggacacc
aaccagcgaaccagcagcgtcgcgtcgggccaagcgaagcagacggcacggcatctctgtcgctgcctctggacccctctcg
agagttccgctccaccgttggacttgctccgctgtcggcatccagaaattgcgtggcggagcggcagacgtgagccggcacggc
aggcggcctcctcctcctctcacggcacggcagctacgggggattcctttcccaccgctccttcgctttcccttcctcgcccgccgta
ataaatagacaccccctccacaccctctttccccaacctcgtgttgttcggagcgcacacacacacaaccagatctcccccaaat
ccacccgtcggcacctccgcttcaaggtacgccgctcgtcctcccccccccccctctctaccttctctagatcggcgttccggtcca
tggttagggcccggtagttctacttctgttcatgtttgtgttagatccgtgtttgtgttagatccgtgctgctagcgttcgtacacggatgcg
acctgtacgtcagacacgttctgattgctaacttgccagtgtttctctttggggaatcctgggatggctctagccgttccgcagacggg
atcgatttcatgattttttttgtttcgttgcatagggtttggtttgcccttttcctttatttcaatatatgccgtgcacttgtttgtcgggtcatcttttc
atgctttttttttgtcttggttgtgatgatgtggtctggttgggcggtcgttctagatcggagtagaattctgtttcaaactacctggtggattta

ttaattttggatctgtatgtgtgtgccatacatattcatagttacgaattgaagatgatggatggaaatatcgatctaggataggtataca tgttgatgcgggttttactgatgcatatacagagatgctttttgttcgcttggttgtgatgatgtggtgtggttgggcggtcgttcattcgttct agatcggagtagaatactgtttcaaactacctggtgtatttattaattttggaactgtatgtgtgtgtcatacatcttcatagttacgagttt aagatggatggaaatatcgatctaggataggtatacatgttgatgtgggttttactgatgcatatacatgatggcatatgcagcatcta ttcatatgctctaaccttgagtacctatctattataataaacaagtatgttttataattattttgatcttgatatacttggatgatggcatatgc agcagctatatgtggatttttttagccctgccttcatacgctatttatttgcttggtactgtttcttttgtcgatgctcaccctgttgtttggtgtta cttctgcaggcgatcgccacaccaccatgccgaagaagaagcgcaaggtcatggacaagaagtactccatcggcctggacat cggcaccaacagcgtgggctgggccgtcatcaccgacgagtacaaggtgccctccaagaagttcaaggtcctcggcaacacc gacaggcacagcatcaagaagaacctgatcggcgccctgctgttcgactccggcgagactgcggaggctaccaggctgaagc gcactgctcgcaggcgctacaccaggcgcaagaaccgcatctgctacctccaggagattttctccaacgagatggccaaggtg gacgactccttcttccaccgcctggaggagagcttcctggtcgaggaagacaagaagcacgagcgccaccctatcttcggcaa catcgtggacgaggtcgcctaccacgagaagtacccaaccatctaccacctccgcaagaagctggtggactccaccgacaag gccgacctgaggctcatctacctggccctcgcccacatgatcaagttccgcggccacttcctcatcgagggcgacctgaacccgg acaacagcgacgtggacaagctcttcatccagctggtccagacctacaaccagctgttcgaggagaaccccatcaacgcctcc ggcgtggacgctaaggctatcctcagcgctaggctgtccaagagcaggcgcctggagaacctcatcgcccagctcccgggcg agaagaagaacggcctcttcggcaacctgatcgctctgtccctcggcctgacccccaacttcaagagcaacttcgacctggccg aggacgccaagctccagctgtccaaggacacctacgacgacgacctcgacaacctgctcgcccagatcggcgaccagtacg ccgacctcttcctggccgccaagaacctctccgacgccatcctgctcagcgacatcctgagggtgaacaccgagatcaccaag gccccgctgtccgccagcatgatcaagcgctacgacgagcaccaccaggacctcactctcctgaaggccctcgtccgccagca gctgcccgagaagtacaaggagattttcttcgaccagagcaagaacggctacgcgggctacatcgatggcggcgcctcccagg aagagttctacaagttcatcaagcctatcctggagaagatggacggcaccgaggagctgctcgtgaagctgaaccgcgaggac ctgctccgcaagcagaggaccttcgacaacggcagcatccctcaccagatccacctgggcgagctgcacgctatcctccgccg ccaggaagacttctacccattcctgaaggacaaccgcgagaagatcgagaagatcctcaccttccgcatcccgtactacgtggg ccccctggcccgcggcaactccaggttcgcctggatgaccaggaagagcgaggagaccatcaccccgtggaacttcgagga agtggtggacaagggcgcctccgctcagagcttcatcgagcgcatgaccaacttcgacaagaacctccctaacgagaaggtgc tgccaaagcactccctgctctacgagtacttcaccgtctacaacgagctgaccaaggtgaagtatgtgaccgagggcatgagga agcccgccttcctcagcggcgagcagaagaaggccatcgtggacctgctcttcaagaccaaccgcaaggtgaccgtcaagca gctgaaggaagactacttcaagaagatcgagtgcttcgactccgtggagatcagcggcgtggaggaccgcttcaacgcctccct cggcacctaccacgacctgctcaagatcatcaaggacaaggacttcctcgacaacgaggagaacgaggacatcctggagga catcgtgctcaccctgaccctcttcgaggaccgcgagatgatcgaggagaggctcaagacctacgcccacctgttcgacgacaa ggtcatgaagcagctgaagaggcgcaggtacactggctggggccgcctcagcaggaagctgatcaacggcatcagggacaa gcagtccggcaagaccatcctggacttcctcaagagcgacggcttcgccaaccgcaacttcatgcagctcatccacgacgactc cctgaccttcaaggaagacatccagaaggctcaggtgtccggccagggcgacagcctccacgagcacatcgctaacctggcg ggcagccctgccatcaagaagggcatcctccagaccgtgaaggtggtggacgagctggtgaaggtcatgggccgccacaag ccagagaacatcgtcatcgagatggccagggagaaccagaccacccagaagggtcagaagaactcccgcgagaggatga agaggatcgaggaaggcatcaaggagctgggcagccagatcctgaaggagcacccggtggagaacacccagctccagaa cgagaagctgtacctctactacctgcagaacggccgcgacatgtatgtggaccaggagctggacatcaacaggctgtccgacta cgacgtggaccacatcgtccctcagtccttcctcaaggacgacagcatcgacaacaaggtgctgacccgcagcgacaagaac aggggcaagtccgacaacgtcccaagcgaggaagtggtcaagaagatgaagaactactggcgccagctgctcaacgccaa gctcatcacccagcgcaagttcgacaacctgactaaggcggagaggggcggcctgtccgagctggacaaggctggcttcatca agcgccagctcgtggagaccaggcagatcaccaagcacgtcgcccagatcctggacagcaggatgaacaccaagtacgac gagaacgacaagctcatccgcgaggtgaaggtcatcaccctcaagtccaagctggtgagcgacttccgcaaggacttccagttc tacaaggtcagggagatcaacaactaccaccacgcccacgatgcttacctcaacgcggtggtgggcaccgccctcatcaaga agtaccctaagctggagagcgagttcgtgtacggcgactacaaggtgtacgacgtccgcaagatgatcgccaagtccgagcag gagatcggcaaggccaccgccaagtacttcttctacagcaacatcatgaacttcttcaagaccgagatcaccctcgccaacggc gagatccgcaagaggccactgatcgagaccaacggcgagactggcgagatcgtgtgggacaagggcagggacttcgccac

cgtgaggaaggtcctgtccatgcctcaggtgaacatcgtcaagaagaccgaggtccagaccggcggcttctccaaggagagc
atcctcccaaagcgcaacagcgacaagctgatcgccaggaagaaggactgggacccgaagaagtacggtggcttcgactcc
cctactgtggcttacagcgtcctggtggtcgccaaggtggagaagggcaagtccaagaagctgaagagcgtcaaggagctgct
cggcatcaccatcatggagaggtccagcttcgagaagaacccgatcgacttcctggaggccaagggctacaaggaagtgaag
aaggacctgatcatcaagctgcccaagtacagcctgttcgagctggagaacggccgcaagaggatgctcgcctccgctggcga
gctgcagaagggcaacgagctggccctcccgtccaagtatgtgaacttcctgtacctcgcctcccactacgagaagctgaaggg
cagccccgaggacaacgagcagaagcagctcttcgtcgagcagcacaagcactacctggacgagatcatcgagcagatcag
cgagttcagcaagcgcgtgatcctcgccgacgccaacctcgacaaggtcctgtccgcctacaacaagcaccgcgacaagcct
atcagggagcaggccgagaacatcatccacctgttcaccctcaccaacctgggcgccccagctgccttcaagtacttcgacacc
accatcgaccgcaagaggtacaccagcaccaaggaagtgctggacgccaccctgatccaccagtccatcaccggcctgtacg
agactcgcatcgacctcagccagctgggcggcgacccgaagaagaagcgcaaagtctgagggaccctcgatcgacaagctc
gagtttctccataataatgtgtgagtagttcccagataagggaattagggttcctatagggtttcgctcatgtgttgagcatataagaa
acccttagtatgtatttgtatttgtaaaatacttctatcaataaaatttctaattcctaaaaccaaaatccagtactaaaatccagatccc
ccgaattaacctgcaggggcggcagggagagttttaacattgactagcgtgctgataatttgtgagaaataataattgacaagtag
atactgacatttgagaagagcttctgaactgttattagtaacaaaaatggaaagctgatgcacggaaaaaggaaagaaaaagc
catacttttttttaggtaggaaaagaaaaagccatacgagactgatgtctctcagatgggccgggatctgtctatctagcaggcagc
agccctaccaacctcacgggccagcaattacgagtccttctaaaacgtcccgccgagggcgcgtggccgtgctgtgcagcagc
acgtctaacattagtcccacctcgccagtttacagggagcagaaccagcttataagcggaggcgcggcaccaagaagcagcg
ctccggggcggaagtcggttttagagctagaaatagcaagttaaaataaggctagtccgttatcaacttgaaaaagtggcaccga
gtcggtgcttttttttcctcgaggggcgataggaacacgtacaacggccgttgtacgtgttcctatcgccggtaccactagtattaatta
agtttaaacggcgcgccaagggcgaattccagcacactggcggccgttactagtggatcgagctcgtcgactctagactcgagg
gcgcgcctgacaggatatattggcgggtaaac

SEQ ID NO: 28
gcgctccggggcggaagtcg

SEQ ID NO: 29

gaacctcgtccacacgttggaaaccgcaagaaccgacccataagtgaggtagctcaatgacatgcgcaatccactagagttgc
ctttaacgctctgccgggaaaggcacaaaaacccctcacaatcaccgggaggtgaccaaaaataatcaccaactcttgccaca
actcatctgttgctccaagctgtctaggtggcggtagccaccaagagtaacaagaattccgcagctcaaacgatccccaagtgcc
actatatgcaaactcacaagcaaatacacttggaatcactcaatctcactttggattcacaaccaagcaagagagatgagtggga
ggaagtatgcttagcttaagaggatgtcaagatgatcaaaataccaagagaaaaggcttggagccgaccaaggtctatttaaag
accccctctgaaaaaccaaccgttacgcagctcgggggtgatcggactcatcgaccggactcagccccgtgtccggtcgtaaca
gtcgagcaaccacgatgtgccacgtcacacgattcaaaactagccgttaacagccaacgctttcccgcacacgtcgatgaccgg
acgcacagaataaatgatcggactcagagggctgcccgcgttagatcgagtctagtaagtgtttttagcgcgtgaatagtgacca
gacacgttaggtcatcggtgacctgacgcggcggtgtgtccggtcgtcctccctcttttctactcgcacccgcgaccattgacttgac
atgcaggtcacgctcggccagctacctccgcgctcttcttcttcctccgtccgtccgtcgtgtgcccttccctcgtgccgccccacgac
ttccgccccggagcgctgccctcttcctcctcccaatggcgcctcagctgcccccgctcggggctcgccctcgagtctagagtcga
cgagctcctgcagccatggacgcgtaagcttcgatcgatgcctgcagtgcagcgtgacccggtcgtgcccctctctagagataat
gagcattgcatgtctaagttataaaaaattaccacatattttttttgtcacacttgtttgaagtgcagtttatctatctttatacatatatttaa
actttactctacgaataatataatctatagtactacaataatatcagtgttttagagaatcatataaatgaacagttagacatggtctaa
aggacaattgagtattttgacaacaggactctacagttttatctttttagtgtgcatgtgttctcctttttttttgcaaatagcttcacctatata
atacttcatccattttattagtacatccatttagggtttagggttaatggtttttatagactaatttttttagtacatctattttattctattttagcct
ctaaattaagaaaactaaaactctattttagttttttttatttaataatttagatataaaatagaataaaataaagtgactaaaaattaaac
aaataccctttaagaaattaaaaaaactaaggaaacatttttcttgtttcgagtagataatgccagcctgttaaacgccgtcgacga
gtctaacggacaccaaccagcgaaccagcagcgtcgcgtcgggccaagcgaagcagacggcacggcatctctgtcgctgcct

ctggacccctctcgagagttccgctccaccgttggacttgctccgctgtcggcatccagaaattgcgtggcggagcggcagacgt
gagccggcacggcaggcggcctcctcctcctctcacggcacggcagctacgggggattcctttcccaccgctccttcgctttccctt
cctcgcccgccgtaataaatagacacccctccacaccctctttccccaacctcgtgttgttcggagcgcacacacacacaacca
gatctcccccaaatccacccgtcggcacctccgcttcaaggtacgccgctcgtcctcccccccccccctctctaccttctctagatc
ggcgttccggtccatggttagggcccggtagttctacttctgttcatgtttgtgttagatccgtgtttgtgttagatccgtgctgctagcgttc
gtacacggatgcgacctgtacgtcagacacgttctgattgctaacttgccagtgtttctctttggggaatcctgggatggctctagccg
ttccgcagacgggatcgatttcatgattttttttgtttcgttgcatagggtttggtttgcccttttcctttatttcaatatatgccgtgcacttgttt
gtcgggtcatcttttcatgcttttttttgtcttggttgtgatgatgtggtctggttgggcggtcgttctagatcggagtagaattctgtttcaaac
tacctggtggatttattaattttggatctgtatgtgtgtgccatacatattcatagttacgaattgaagatgatggatggaaatatcgatct
aggataggtatacatgttgatgcgggttttactgatgcatatacagagatgctttttgttcgcttggttgtgatgatgtggtgtggttgggc
ggtcgttcattcgttctagatcggagtagaatactgtttcaaactacctggtgtatttattaattttggaactgtatgtgtgtgtcatacatctt
catagttacgagtttaagatggatggaaatatcgatctaggataggtatacatgttgatgtgggttttactgatgcatatacatgatggc
atatgcagcatctattcatatgctctaaccttgagtacctatctattataataaacaagtatgttttataattattttgatcttgatatacttgg
atgatggcatatgcagcacttttgtcgatgctcaccctgttgtttggtgttacttctgcaggtcgactctagaatggacaacaacccaa
acatcaacgagtgcatcccatacaactgcctgagcaacccagaggtggaggtgctgggtggcgagcgcatcgagaccggtta
cacccccatcgacatctccctgtccttgacccagttcctgctcagcgagttcgtgccaggtgctggcttcgtgctcggcctggtggac
atcatctggggtatcttcggtccatcccaatgggacgccttcctggtgcaaatcgagcagctgatcaaccagaggatcgaagagtt
cgccaggaaccaggccatctccaggctggagggcctgagcaacctctaccaaatctacgccgagagcttcagggagtgggag
gccgacccgaccaacccagctctccgcgaggaaatgcgcattcaattcaacgacatgaacagcgccctgaccaccgctatccc
actgttcgccgtccagaactaccaagtgccgctcctgtccgtgtacgtgcaagccgctaacctgcacctcagcgtgctgcgcgac
gtgagcgtgttcggccaaaggtggggcttcgatgctgccaccatcaacagccgctacaacgacctgaccaggctgattggcaac
tacaccgaccacgctgtgcgctggtacaacaccggcctggagcgcgtctggggtccggactccagggactggatcaggtacaa
ccagttcaggagggagttgaccctcaccgtgctggacattgtgtccctcttcccgaactacgactccaggacctacccgatccgca
ccgtgtcccaactcaccagggagatctacaccaacccagtgctggagaacttcgacggtagcttccgcggttccgcccagggtat
cgagggctccatcaggagcccacacctgatggacatcctgaacagcatcaccatctacaccgacgctcacaggggcgagtact
actggtccggccaccagatcatggcctccccagtgggcttcagcggccccgagttcaccttcccgctctacggcaccatgggcaa
cgccgctccacagcaacgcatcgtggctcaactgggtcagggtgtctacaggaccctgtcctccaccctgtacaggaggcccttc
aacatcggtatcaacaaccagcaactgtccgtgctcgacggcaccgagttcgcctacggcacctcctccaacctgccatccgctg
tctacaggaagagcggcaccgtggactccctggacgagatcccaccacagaacaacaacgtgccacccaggcaaggcttctc
ccacaggctgagccacgtgtccatgttccgctccggcttcagcaacagctccgtgagcatcatcagggctccgatgttctcctggat
ccaccgcagcgctgagttcaacaacatcatcgcctccgacagcatcacccaaatcccggccgtgaagggcaacttcctcttcaa
cggttccgtcatttccggcccaggcttcaccggtggcgacctcgtgaggctcaacagcagcggcaacaacatccagaacaggg
gctacatcgaggtgccaatccacttcccatccacctccaccaggtacagggtgcgcgtgaggtacgcttccgtgaccccgatcca
cctcaacgtgaactggggtaactcctccatcttctccaacaccgtgccagctaccgctacctccctggacaacctccaatccagcg
acttcggttacttcgagagcgccaacgctttcacctcctccctcggtaacatcgtgggcgtgaggaacttcagcggcaccgccggc
gtgatcatcgacaggttcgagttcatcccagtgaccgccaccctcgaggctgagtgagatcgttcaaacatttggcaataaagtttc
ttaagattgaatcctgttgccggtcttgcgatgattatcatataatttctgttgaattacgttaagcatgtaataattaacatgtaatgcatg
acgttatttatgagatgggtttttatgattagagtcccgcaattatacatttaatacgcgatagaaaacaaaatatagcgcgcaaact
aggataaattatcgcgcgcggtgtcatctatgttactagatcggcgcgccaagggcgaattccagcacactggcggccgttacta
gtggatcacgcgtatgcctgcagtgcagcgtgacccggtcgtgcccctctctagagataatgagcattgcatgtctaagttataaaa
aattaccacatatttttttgtcacacttgtttgaagtgcagtttatctatctttatacatatatttaaactttactctacgaataatataatctat
agtactacaataatatcagtgttttagagaatcatataaatgaacagttagacatggtctaaaggacaattgagtattttgacaacag
gactctacagttttatctttttagtgtgcatgtgttctcctttttttttgcaaatagcttcacctatataatacttcatccattttattagtacatcca
tttagggtttagggttaatggtttttatagactaatttttttagtacatctattttattctattttagcctctaaattaagaaaactaaaactctatt
ttagtttttttatttaataatttagatataaaatagaataaaataaagtgactaaaaattaaacaaataccctttaagaaattaaaaaa
ctaaggaaacatttttcttgtttcgagtagataatgccagcctgttaaacgccgtcgacgagtctaacggacaccaaccagcgaac

cagcagcgtcgcgtcgggccaagcgaagcagacggcacggcatctctgtcgctgcctctggacccctctcgagagttccgctcc
accgttggacttgctccgctgtcggcatccagaaattgcgtggcggagcggcagacgtgagccggcacggcaggcggcctcct
cctcctctcacggcacggcagctacgggggattcctttcccaccgctccttcgctttcccttcctcgcccgccgtaataaatagacac
cccctccacaccctctttccccaacctcgtgttgttcggagcgcacacacacacaaccagatctcccccaaatccacccgtcggc
acctccgcttcaaggtacgccgctcgtcctcccccccccccctctctaccttctctagatcggcgttccggtccatggttagggccc
ggtagttctacttctgttcatgtttgtgttagatccgtgtttgtgttagatccgtgctgctagcgttcgtacacggatgcgacctgtacgtca
gacacgttctgattgctaacttgccagtgtttctctttggggaatcctgggatggctctagccgttccgcagacgggatcgatttcatga
ttttttttgtttcgttgcatagggtttggtttgcccttttcctttatttcaatatatgccgtgcacttgtttgtcgggtcatcttttcatgcttttttttgtct
tggttgtgatgatgtggtctggttgggcggtcgttctagatcggagtagaattctgtttcaaactacctggtggatttattaattttggatct
gtatgtgtgtgccatacatattcatagttacgaattgaagatgatggatggaaatatcgatctaggataggtatacatgttgatgcggg
ttttactgatgcatatacagagatgctttttgttcgcttggttgtgatgatgtggtgtggttgggcggtcgttcattcgttctagatcggagta
gaatactgtttcaaactacctggtgtatttattaattttggaactgtatgtgtgtgtcatacatcttcatagttacgagtttaagatggatgg
aaatatcgatctaggataggtatacatgttgatgtgggttttactgatgcatatacatgatggcatatgcagcatctattcatatgctcta
accttgagtacctatctattataataaacaagtatgttttataattattttgatcttgatatacttggatgatggcatatgcagcagctatat
gtggatttttttagccctgccttcatacgctatttatttgcttggtactgtttcttttgtcgatgctcaccctgttgtttggtgttacttctgcaggtc
gactctagaatgtggattgaacaagatggattgcacgcaggttctccggccgcttgggtggagaggctattcggctatgactgggc
acaacagacaatcggctgctctgatgccgccgtgttccggctgtcagcgcaggggcgcccggttctttttgtcaagaccgacctgt
ccggtgccctgaatgaactgcaggacgaggcagcgcggctatcgtggctggccacgacgggcgttccttgcgcagctgtgctcg
acgttgtcactgaagcgggaagggactggctgctattgggcgaagtgccggggcaggatctcctgtcatctcaccttgctcctgcc
gagaaagtatccatcatggctgatgcaatgcggcggctgcatacgcttgatccggctacctgcccattcgaccaccaagcgaaa
catcgcatcgagcgagcacgtactcggatggaagccggtcttgtcgatcaggatgatctggacgaagagcatcaggggctcgc
gccagccgaactgttcgccaggctcaaggcgcgcatgcccgacggcgaggatctcgtcgtgacccatggcgatgcctgcttgcc
gaatatcatggtggaaaatggccgcttttctggattcatcgactgtggccggctgggtgtggcggaccgctatcaggacatagcgtt
ggctacccgtgatattgctgaagagcttggcggcgaatgggctgaccgcttcctcgtgctttacggtatcgccgctcccgattcgca
gcgcatcgccttctatcgccttcttgacgagttcttctgagatcgttcaaacatttggcaataaagtttcttaagattgaatcctgttgccg
gtcttgcgatgattatcatataatttctgttgaattacgttaagcatgtaataattaacatgtaatgcatgacgttatttatgagatgggtttttt
atgattagagtcccgcaattatacatttaatacgcgatagaaaacaaaatatagcgcgcaaactaggataaattatcgcgcgcgg
tgtcatctatgttactagatcggcgcgccaagggcgaattccagcacactggcggccgttactagtggatcgagctcgcggccgc
cccgggtaccgatatcagtactaattcagtacattaaaaacgtccgcaatgtgttattaagttgtctaagcgtcaatttgtttacaccac
aacccagcaatgtgttattaggttcagcgcaggcaacccagtcacgtcaacctgggcgaccgatcctggcgtggcgacagcgc
cggcctagggcttcgcctctccgtcgccggtgatcctgcatcggacgagccatggcacggcggcccccgcgagagcggcggc
ggtgcaccggcatgggcggcccaccaatgcggcggccctgctcgcctactgcactgctgctgatgcgccgcccctcgatctcgg
cccatgcccgtatttgtgcctgtgcccgttccatcagctagggttacatctcaatttcccgctctctatctcgtcttcgtctttcttcctcatct
gcagatcctagctcagatttagtatttttttaaaatcctttgaaggattacc

SEQ ID NO: 30

cccagcaatgtgttattaggttcagcgcaggcaacccagtcacgtcaacctgggcgaccgatcctggcgtggcgacagcgccg
gcctagggcttcgcctctccgtcgccggtgatcctgcatcggacgagccatggcacggcggcccccgcgagagcggcggcggt
gcaccggcatgggcggcccaccaatgcggcggccctgctcgcctactgcactgctgctgatgcgccgcccctcgatctcggccc
atgcccgtatttgtgcctgtgcccgttccatcagctagggttacatctcaatttcccgctctctatctcgtcttcgtctttcttcctcatctgca
gatcctagctcagatttagtatttttttaaaatcctttgaaggattacc

SEQ ID NO: 31

gaacctcgtccacacgttggaaaccgcaagaaccgacccataagtgaggtagctcaatgacatgcgcaatccactagagttgc
ctttaacgctctgccgggaaaggcacaaaaacccctcacaatcaccgggaggtgaccaaaaataatcaccaactcttgccaca
actcatctgttgctccaagctgtctaggtggcggtagccaccaagagtaacaagaattccgcagctcaaacgatccccaagtgcc

actatatgcaaactcacaagcaaatacacttggaatcactcaatctcactttggattcacaaccaagcaagagagatgagtggga ggaagtatgcttagcttaagaggatgtcaagatgatcaaaataccaagagaaaaggcttggagccgaccaaggtctatttaaag accccctctgaaaaaccaaccgttacgcagctcgggggtgatcggactcatcgaccggactcagccccgtgtccggtcgtaaca gtcgagcaaccacgatgtgccacgtcacacgattcaaaactagccgttaacagccaacgctttcccgcacacgtcgatgaccgg acgcacagaataaatgatcggactcagagggctgcccgcgttagatcgagtctagtaagtgtttttagcgcgtgaatagtgacca gacacgttaggtcatcggtgacctgacgcggcggtgtgtccggtcgtcctccctcttttctactcgcacccgcgaccattgacttgac atgcaggtcacgctcggccagctacctccgcgctcttcttcttcctccgtccgtccgtcgtgtgcccttccctcgtgccgccccacgac ttccgccccggagcgctgccctcttcctcctcccaatggcgcctcagctgcccccgctcgg

SEQ ID NO: 32
gtttacaccacaacccagcaatgtgt
SEQ ID NO: 33
tgcccccgctcggggctcgccctcga

SEQ ID NO: 34

MDNNPNINECIPYNCLSNPEVEVLGGERIETGYTPIDISLSLTQFLLSEFVPGAGFVLGLVDII WGIFGPSQWDAFLVQIEQLINQRIEEFARNQAISRLEGLSNLYQIYAESFREWEADPTNPALR EEMRIQFNDMNSALTTAIPLFAVQNYQVPLLSVYVQAANLHLSVLRDVSVFGQRWGFDAATI NSRYNDLTRLIGNYTDHAVRWYNTGLERVWGPDSRDWIRYNQFRRELTLTVLDIVSLFPNY DSRTYPIRTVSQLTREIYTNPVLENFDGSFRGSAQGIEGSIRSPHLMDILNSITIYTDAHRGEY YWSGHQIMASPVGFSGPEFTFPLYGTMGNAAPQQRIVAQLGQGVYRTLSSTLYRRPFNIGI NNQQLSVLDGTEFAYGTSSNLPSAVYRKSGTVDSLDEIPPQNNNVPPRQGFSHRLSHVSM FRSGFSNSSVSIIRAPMFSWIHRSAEFNNIIASDSITQIPAVKGNFLFNGSVISGPGFTGGDLV RLNSSGNNIQNRGYIEVPIHFPSTSTRYRVRVRYASVTPIHLNVNWGNSSIFSNTVPATATSL DNLQSSDFGYFESANAFTSSLGNIVGVRNFSGTAGVIIDRFEFIPVTATLEAE

SEQ ID NO: 35

MWIEQDGLHAGSPAAWVERLFGYDWAQQTIGCSDAAVFRLSAQGRPVLFVKTDLSGALNE LQDEAARLSWLATTGVPCAAVLDVVTEAGRDWLLLGEVPGQDLLSSHLAPAEKVSIMADAM RRLHTLDPATCPFDHQAKHRIERARTRMEAGLVDQDDLDEEHQGLAPAELFARLKARMPD GEDLVVTHGDACLPNIMVENGRFSGFIDCGRLGVADRYQDIALATRDIAEELGGEWADRFL VLYGIAAPDSQRIAFYRLLDEFF

SEQ ID NO: 36

ggctcgccctcgagtctagagtcgacgagctcctgcagccatggacgcgtaagcttcgatcgatgcctgcagtgcagcgtgacc cggtcgtgcccctctctagagataatgagcattgcatgtctaagttataaaaaattaccacatatttttttgtcacacttgtttgaagtgc agtttatctatctttatacatatatttaaactttactctacgaataatataatctatagtactacaataatatcagtgttttagagaatcatat aaatgaacagttagacatggtctaaaggacaattgagtattttgacaacaggactctacagttttatctttttagtgtgcatgtgttctcct tttttttgcaaatagcttcacctatataatacttcatccattttattagtacatccatttagggtttagggttaatggtttttatagactaattttt tagtacatctattttattctattttagcctctaaattaagaaaactaaaactctattttagttttttatttaataatttagatataaaatagaata aaataaagtgactaaaaattaaacaaataccctttaagaaattaaaaaaactaaggaaacatttttcttgtttcgagtagataatgc cagcctgttaaacgccgtcgacgagtctaacggacaccaaccagcgaaccagcagcgtcgcgtcgggccaagcgaagcag acggcacggcatctctgtcgctgcctctggacccctctcgagagttccgctccaccgttggacttgctccgctgtcggcatccagaa attgcgtggcggagcggcagacgtgagccggcacggcaggcggcctcctcctcctctcacggcacggcagctacgggggatt

cctttcccaccgctccttcgctttcccttcctcgcccgccgtaataaatagacaccccctccacaccctctttccccaacctcgtgttgtt
cggagcgcacacacacacaaccagatctcccccaaatccacccgtcggcacctccgcttcaaggtacgccgctcgtcctcccc
cccccccctctctaccttctctagatcggcgttccggtccatggttagggcccggtagttctacttctgttcatgtttgtgttagatccgtg
tttgtgttagatccgtgctgctagcgttcgtacacggatgcgacctgtacgtcagacacgttctgattgctaacttgccagtgtttctcttt
ggggaatcctgggatggctctagccgttccgcagacgggatcgatttcatgattttttttgtttcgttgcatagggtttggtttgcccttttcc
tttatttcaatatatgccgtgcacttgtttgtcgggtcatcttttcatgcttttttttgtcttggttgtgatgatgtggtctggttgggcggtcgttct
agatcggagtagaattctgtttcaaactacctggtggatttattaattttggatctgtatgtgtgtgccatacatattcatagttacgaattg
aagatgatggatggaaatatcgatctaggataggtatacatgttgatgcgggttttactgatgcatatacagagatgctttttgttcgctt
ggttgtgatgatgtggtgtggttgggcggtcgttcattcgttctagatcggagtagaatactgtttcaaactacctggtgtatttattaatttt
ggaactgtatgtgtgtgtcatacatcttcatagttacgagtttaagatggatggaaatatcgatctaggataggtatacatgttgatgtg
ggttttactgatgcatatacatgatggcatatgcagcatctattcatatgctctaaccttgagtacctatctattataataaacaagtatgt
tttataattattttgatcttgatatacttggatgatggcatatgcagcacttttgtcgatgctcaccctgttgtttggtgttacttctgcaggtcg
actctagaatggacaacaacccaaacatcaacgagtgcatcccatacaactgcctgagcaacccagaggtggaggtgctggg
tggcgagcgcatcgagaccggttacacccccatcgacatctccctgtccttgacccagttcctgctcagcgagttcgtgccaggtg
ctggcttcgtgctcggcctggtggacatcatctggggtatcttcggtccatcccaatgggacgccttcctggtgcaaatcgagcagct
gatcaaccagaggatcgaagagttcgccaggaaccaggccatctccaggctggagggcctgagcaacctctaccaaatctac
gccgagagcttcagggagtgggaggccgacccgaccaacccagctctccgcgaggaaatgcgcattcaattcaacgacatga
acagcgccctgaccaccgctatcccactgttcgccgtccagaactaccaagtgccgctcctgtccgtgtacgtgcaagccgctaa
cctgcacctcagcgtgctgcgcgacgtgagcgtgttcggccaaaggtggggcttcgatgctgccaccatcaacagccgctacaa
cgacctgaccaggctgattggcaactacaccgaccacgctgtgcgctggtacaacaccggcctggagcgcgtctggggtccgg
actccagggactggatcaggtacaaccagttcaggagggagttgaccctcaccgtgctggacattgtgtccctcttcccgaactac
gactccaggacctacccgatccgcaccgtgtcccaactcaccagggagatctacaccaacccagtgctggagaacttcgacgg
tagcttccgcggttccgcccagggtatcgagggctccatcaggagcccacacctgatggacatcctgaacagcatcaccatctac
accgacgctcacaggggcgagtactactggtccggccaccagatcatggcctccccagtgggcttcagcggccccgagttcac
cttcccgctctacggcaccatgggcaacgccgctccacagcaacgcatcgtggctcaactgggtcagggtgtctacaggaccct
gtcctccaccctgtacaggaggcccttcaacatcggtatcaacaaccagcaactgtccgtgctcgacggcaccgagttcgcctac
ggcacctcctccaacctgccatccgctgtctacaggaagagcggcaccgtggactccctggacgagatcccaccacagaaca
acaacgtgccacccaggcaaggcttctcccacaggctgagccacgtgtccatgttccgctccggcttcagcaacagctccgtga
gcatcatcagggctccgatgttctcctggatccaccgcagcgctgagttcaacaacatcatcgcctccgacagcatcacccaaat
cccggccgtgaagggcaacttcctcttcaacggttccgtcatttccggcccaggcttcaccggtggcgacctcgtgaggctcaaca
gcagcggcaacaacatccagaacaggggctacatcgaggtgccaatccacttcccatccacctccaccaggtacagggtgcg
cgtgaggtacgcttccgtgaccccgatccacctcaacgtgaactggggtaactcctccatcttctccaacaccgtgccagctaccg
ctacctccctggacaacctccaatccagcgacttcggttacttcgagagcgccaacgctttcacctcctccctcggtaacatcgtgg
gcgtgaggaacttcagcggcaccgccggcgtgatcatcgacaggttcgagttcatcccagtgaccgccaccctcgaggctgagt
gagatcgttcaaacatttggcaataaagtttcttaagattgaatcctgttgccggtcttgcgatgattatcatataatttctgttgaattacg
ttaagcatgtaataattaacatgtaatgcatgacgttatttatgagatgggtttttatgattagagtcccgcaattatacatttaatacgcg
atagaaaacaaaatatagcgcgcaaactaggataaattatcgcgcgcggtgtcatctatgttactagatcggcgcgccaagggc
gaattccagcacactggcggccgttactagtggatcacgcgtatgcctgcagtgcagcgtgacccggtcgtgcccctctctagag
ataatgagcattgcatgtctaagttataaaaaattaccacatattttttttgtcacacttgtttgaagtgcagtttatctatctttatacatata
tttaaactttactctacgaataatataatctatagtactacaataatatcagtgttttagagaatcatataaatgaacagttagacatggt
ctaaaggacaattgagtattttgacaacaggactctacagttttatctttttagtgtgcatgtgttctcctttttttttgcaaatagcttcacct
atataatacttcatccattttattagtacatccatttagggtttagggttaatggtttttatagactaattttttttagtacatctattttattctatttt
agcctctaaattaagaaaactaaaactctattttagtttttttatttaataatttagatataaaatagaataaaataaagtgactaaaaat
taaacaaataccctttaagaaattaaaaaaactaaggaaacatttttcttgtttcgagtagataatgccagcctgttaaacgccgtcg
acgagtctaacggacaccaaccagcgaaccagcagcgtcgcgtcgggccaagcgaagcagacggcacggcatctctgtcg
ctgcctctggacccctctcgagagttccgctccaccgttggacttgctccgctgtcggcatccagaaattgcgtggcggagcggca

gacgtgagccggcacggcaggcggcctcctcctcctctcacggcacggcagctacgggggattcctttcccaccgctccttcgctt
tcccttcctcgcccgccgtaataaatagacacccccctccacaccctctttccccaacctcgtgttgttcggagcgcacacacacaca
accagatctcccccaaatccacccgtcggcacctccgcttcaaggtacgccgctcgtcctcccccccccccccctctctaccttctct
agatcggcgttccggtccatggttagggcccggtagttctacttctgttcatgtttgtgttagatccgtgtttgtgttagatccgtgctgcta
gcgttcgtacacggatgcgacctgtacgtcagacacgttctgattgctaacttgccagtgtttctctttggggaatcctgggatggctct
agccgttccgcagacgggatcgatttcatgattttttttgtttcgttgcatagggtttggtttgcccttttcctttatttcaatatatgccgtgca
cttgtttgtcgggtcatcttttcatgcttttttttgtcttggttgtgatgatgtggtctggttgggcggtcgttctagatcggagtagaattctgttt
caaactacctggtggatttattaattttggatctgtatgtgtgtgccatacatattcatagttacgaattgaagatgatggatggaaatat
cgatctaggataggtatacatgttgatgcgggttttactgatgcatatacagagatgctttttgttcgcttggttgtgatgatgtggtgtggt
tgggcggtcgttcattcgttctagatcggagtagaatactgtttcaaactacctggtgtatttattaattttggaactgtatgtgtgtgtcat
acatcttcatagttacgagtttaagatggatggaaatatcgatctaggataggtatacatgttgatgtgggttttactgatgcatatacat
gatggcatatgcagcatctattcatatgctctaaccttgagtacctatctattataataaacaagtatgttttataattattttgatcttgatat
acttggatgatggcatatgcagcagctatatgtggatttttttagccctgccttcatacgctatttatttgcttggtactgtttcttttgtcgatg
ctcaccctgttgtttggtgttacttctgcaggtcgactctagaatgtggattgaacaagatggattgcacgcaggttctccggccgcttg
ggtggagaggctattcggctatgactgggcacaacagacaatcggctgctctgatgccgccgtgttccggctgtcagcgcaggg
gcgcccggttctttttgtcaagaccgacctgtccggtgccctgaatgaactgcaggacgaggcagcgcggctatcgtggctggcc
acgacgggcgttccttgcgcagctgtgctcgacgttgtcactgaagcgggaagggactggctgctattgggcgaagtgccgggg
caggatctcctgtcatctcaccttgctcctgccgagaaagtatccatcatggctgatgcaatgcggcggctgcatacgcttgatccg
gctacctgcccattcgaccaccaagcgaaacatcgcatcgagcgagcacgtactcggatggaagccggtcttgtcgatcaggat
gatctggacgaagagcatcaggggctcgcgccagccgaactgttcgccaggctcaaggcgcgcatgcccgacggcgaggat
ctcgtcgtgacccatggcgatgcctgcttgccgaatatcatggtggaaaatggccgcttttctggattcatcgactgtggccggctgg
gtgtggcggaccgctatcaggacatagcgttggctacccgtgatattgctgaagagcttggcggcgaatgggctgaccgcttcctc
gtgctttacggtatcgccgctcccgattcgcagcgcatcgccttctatcgccttcttgacgagttcttctgagatcgttcaaacatttggc
aataaagtttcttaagattgaatcctgttgccggtcttgcgatgattatcatataatttctgttgaattacgttaagcatgtaataattaaca
tgtaatgcatgacgttatttatgagatgggtttttatgattagagtcccgcaattatacatttaatacgcgatagaaaacaaaatatagc
gcgcaaactaggataaattatcgcgcgcggtgtcatctatgttactagatcggcgcgccaagggcgaattccagcacactggcg
gccgttactagtggatcgagctcgcggccgccccgggtaccgatatcagtactaattcagtacattaaaaacgtccgcaatgtgtt
attaagttgtctaagcgtcaatttgtttacaccacaa

SEQ ID NO: 37

cgtccgtccgtcgtgtgcccttccctcgtgccgccccacgacttccgccccggagcgctgccctcttcctcctcccaatggcgcctca
gctgcccccgctcggggctcgccctcgagtctagagtcgacgagctcctgcagccatggacgcgtaagcttcgatcgatgcctgc
agtgcagcgtgacccggtcgtgcccctctctagagataatgagcattgcatgtctaagttataaaaaattaccacatattttttttgtca
cacttgtttgaagtgcagtttatctatctttatacatatatttaaactttactctacgaataatataatctatagtactacaataatatcagtgt
tttagagaatcatataaatgaacagttagacatggtctaaaggacaattgagtattttgacaacaggactctacagttttatctttttagt
gtgcatgtgttctcctttttttttgcaaatagcttcacctatataatacttcatccattttattagtacatccatttagggtttagggttaatggttt
ttatagactaattttttttagtacatctattttattctattttagcctctaaattaagaaaactaaaactctattttagtttttttatttaataatttaga
tataaaatagaataaaataaagtgactaaaaattaaacaaataccctttaagaaattaaaaaaactaaggaaacatttttcttgtttc
gagtagataatgccagcctgttaaacgccgtcgacgagtctaacggacaccaaccagcgaaccagcagcgtcgcgtcgggcc
aagcgaagcagacggcacggcatctctgtcgctgcctctggacccctctcgagagttccgctccaccgttggacttgctccgctgt
cggcatccagaaattgcgtggcggagcggcagacgtgagccggcacggcaggcggcctcctcctcctctcacggcacggcag
ctacgggggattcctttcccaccgctccttcgctttcccttcctcgcccgccgtaataaatagacacccccctccacaccctctttcccc
aacctcgtgttgttcggagcgcacacacacacaaccagatctcccccaaatccacccgtcggcacctccgcttcaaggtacgcc
gctcgtcctcccccccccccccctctctaccttctctagatcggcgttccggtccatggttagggcccggtagttctacttctgttcatgttt
gtgttagatccgtgtttgtgttagatccgtgctgctagcgttcgtacacggatgcgacctgtacgtcagacacgttctgattgctaacttg
ccagtgtttctctttggggaatcctgggatggctctagccgttccgcagacgggatcgatttcatgattttttttgtttcgttgcatagggttt

ggtttgcccttttcctttatttcaatatatgccgtgcacttgtttgtcgggtcatcttttcatgcttttttttgtcttggttgtgatgatgtggtctggtt gggcggtcgttctagatcggagtagaattctgtttcaaactacctggtggatttattaattttggatctgtatgtgtgtgccatacatattca tagttacgaattgaagatgatggatggaaatatcgatctaggataggtatacatgttgatgcgggttttactgatgcatatacagaga tgctttttgttcgcttggttgtgatgatgtggtgtggttgggcggtcgttcattcgttctagatcggagtagaatactgtttcaaactacctgg tgtatttattaattttggaactgtatgtgtgtgtcatacatcttcatagttacgagtttaagatggatggaaatatcgatctaggataggtat acatgttgatgtgggttttactgatgcatatacatgatggcatatgcagcatctattcatatgctctaaccttgagtacctatctattataat aaacaagtatgttttataattattttgatcttgatatacttggatgatggcatatgcagcacttttgtcgatgctcaccctgttgtttggtgtta cttctgcaggtcgactctagaatggacaacaacccaaacatcaacgagtgcatcccatacaactgcctgagcaacccagaggt ggaggtgctgggtggcgagcgcatcgagaccggttacacccccatcgacatctccctgtccttgacccagttcctgctcagcgagt tcgtgccaggtgctggcttcgtgctcggcctggtggacatcatctggggtatcttcggtccatcccaatgggacgccttcctggtgca aatcgagcagctgatcaaccagaggatcgaagagttcgccaggaaccaggccatctccaggctggagggcctgagcaacctc taccaaatctacgccgagagcttcagggagtgggaggccgacccgaccaacccagctctccgcgaggaaatgcgcattcaatt caacgacatgaacagcgccctgaccaccgctatcccactgttcgccgtccagaactaccaagtgccgctcctgtccgtgtacgtg caagccgctaacctgcacctcagcgtgctgcgcgacgtgagcgtgttcggccaaaggtggggcttcgatgctgccaccatcaac agccgctacaacgacctgaccaggctgattggcaactacaccgaccacgctgtgcgctggtacaacaccggcctggagcgcgt ctggggtccggactccagggactggatcaggtacaaccagttcaggagggagttgaccctcaccgtgctggacattgtgtccctct tcccgaactacgactccaggacctacccgatccgcaccgtgtcccaactcaccagggagatctacaccaacccagtgctggag aacttcgacggtagcttccgcggttccgcccagggtatcgagggctccatcaggagcccacacctgatggacatcctgaacagc atcaccatctacaccgacgctcacaggggcgagtactactggtccggccaccagatcatggcctccccagtgggcttcagcggc cccgagttcaccttcccgctctacggcaccatgggcaacgccgctccacagcaacgcatcgtggctcaactgggtcagggtgtct acaggaccctgtcctccaccctgtacaggaggcccttcaacatcggtatcaacaaccagcaactgtccgtgctcgacggcaccg agttcgcctacggcacctcctccaacctgccatccgctgtctacaggaagagcggcaccgtggactccctggacgagatcccac cacagaacaacaacgtgccacccaggcaaggcttctcccacaggctgagccacgtgtccatgttccgctccggcttcagcaac agctccgtgagcatcatcagggctccgatgttctcctggatccaccgcagcgctgagttcaacaacatcatcgcctccgacagcat cacccaaatcccggccgtgaagggcaacttcctcttcaacggttccgtcatttccggcccaggcttcaccggtggcgacctcgtga ggctcaacagcagcggcaacaacatccagaacaggggctacatcgaggtgccaatccacttcccatccacctccaccaggta cagggtgcgcgtgaggtacgcttccgtgaccccgatccacctcaacgtgaactggggtaactcctccatcttctccaacaccgtgc cagctaccgctacctccctggacaacctccaatccagcgacttcggttacttcgagagcgccaacgctttcacctcctccctcggta acatcgtgggcgtgaggaacttcagcggcaccgccggcgtgatcatcgacaggttcgagttcatcccagtgaccgccaccctcg aggctgagtgagatcgttcaaacatttggcaataaagtttcttaagattgaatcctgttgccggtcttgcgatgattatcatataatttctg ttgaattacgttaagcatgtaataattaacatgtaatgcatgacgttatttatgagatgggtttttatgattagagtcccgcaattatacatt taatacgcgatagaaaacaaaatatagcgcgcaaactaggataaattatcgcgcgcggtgtcatctatgttactagatcggcgcg ccaagggcgaattccagcacactggcggccgttactagtggatcacgcgtatgcctgcagtgcagcgtgacccggtcgtgcccc tctctagagataatgagcattgcatgtctaagttataaaaaattaccacatatttttttgtcacacttgtttgaagtgcagtttatctatcttt atacatatatttaaactttactctacgaataatataatctatagtactacaataatatcagtgttttagagaatcatataaatgaacagtt agacatggtctaaaggacaattgagtattttgacaacaggactctacagttttatctttttagtgtgcatgtgttctcctttttttttgcaaata gcttcacctatataatacttcatccattttattagtacatccatttagggtttagggttaatggtttttatagactaatttttttagtacatctatttt attctattttagcctctaaattaagaaaactaaaactctattttagtttttttatttaataatttagatataaaatagaataaaataaagtga ctaaaaattaaacaaataccctttaagaaattaaaaaaactaaggaaacatttttcttgtttcgagtagataatgccagcctgttaaa cgccgtcgacgagtctaacggacaccaaccagcgaaccagcagcgtcgcgtcgggccaagcgaagcagacggcacggca tctctgtcgctgcctctggacccctctcgagagttccgctccaccgttggacttgctccgctgtcggcatccagaaattgcgtggcgg agcggcagacgtgagccggcacggcaggcggcctcctcctctcacggcacggcagctacgggggattcctttcccaccgc tccttcgctttcccttcctcgcccgccgtaataaatagacacccctccacaccctctttccccaacctcgtgttgttcggagcgcaca cacacacaaccagatctcccccaaatccacccgtcggcacctccgcttcaaggtacgccgctcgtcctcccccccccccctctc taccttctctagatcggcgttccggtccatggttagggcccggtagttctacttctgttcatgtttgtgttagatccgtgtttgtgttagatcc gtgctgctagcgttcgtacacggatgcgacctgtacgtcagacacgttctgattgctaacttgccagtgtttctctttggggaatcctgg

gatggctctagccgttccgcagacgggatcgatttcatgattttttttgtttcgttgcatagggtttggtttgcccttttcctttatttcaatatat gccgtgcacttgtttgtcgggtcatcttttcatgcttttttttgtcttggttgtgatgatgtggtctggttgggcggtcgttctagatcggagtag aattctgtttcaaactacctggtggatttattaattttggatctgtatgtgtgtgccatacatattcatagttacgaattgaagatgatggat ggaaatatcgatctaggataggtatacatgttgatgcgggttttactgatgcatatacagagatgctttttgttcgcttggttgtgatgatg tggtgtggttgggcggtcgttcattcgttctagatcggagtagaatactgtttcaaactacctggtgtatttattaattttggaactgtatgt gtgtgtcatacatcttcatagttacgagtttaagatggatggaaatatcgatctaggataggtatacatgttgatgtgggttttactgatg catatacatgatggcatatgcagcatctattcatatgctctaaccttgagtacctatctattataataaacaagtatgttttataattattttg atcttgatatacttggatgatggcatatgcagcagctatatgtggatttttttagccctgccttcatacgctatttatttgcttggtactgtttct tttgtcgatgctcaccctgttgtttggtgttacttctgcaggtcgactctagaatgtggattgaacaagatggattgcacgcaggttctcc ggccgcttgggtggagaggctattcggctatgactgggcacaacagacaatcggctgctctgatgccgccgtgttccggctgtca gcgcaggggcgcccggttctttttgtcaagaccgacctgtccggtgccctgaatgaactgcaggacgaggcagcgcggctatcgt ggctggccacgacgggcgttccttgcgcagctgtgctcgacgttgtcactgaagcgggaagggactggctgctattgggcgaagt gccggggcaggatctcctgtcatctcaccttgctcctgccgagaaagtatccatcatggctgatgcaatgcggcggctgcatacgc ttgatccggctacctgcccattcgaccaccaagcgaaacatcgcatcgagcgagcacgtactcggatggaagccggtcttgtcg atcaggatgatctggacgaagagcatcaggggctcgcgccagccgaactgttcgccaggctcaaggcgcgcatgcccgacgg cgaggatctcgtcgtgacccatggcgatgcctgcttgccgaatatcatggtggaaaatggccgcttttctggattcatcgactgtggc cggctgggtgtggcggaccgctatcaggacatagcgttggctacccgtgatattgctgaagagcttggcggcgaatgggctgacc gcttcctcgtgctttacggtatcgccgctcccgattcgcagcgcatcgccttctatcgccttcttgacgagttcttctgagatcgttcaaa catttggcaataaagtttcttaagattgaatcctgttgccggtcttgcgatgattatcatataatttctgttgaattacgttaagcatgtaat aattaacatgtaatgcatgacgttatttatgagatgggtttttatgattagagtcccgcaattatacatttaatacgcgatagaaaacaa aatatagcgcgcaaactaggataaattatcgcgcgcggtgtcatctatgttactagatcggcgcgccaagggcgaattccagcac actggcggccgttactagtggatcgagctcgcggccgccccgggtaccgatatcagtactaattcagtacattaaaaacgtccgc aatgtgttattaagttgtctaagcgtcaatttgtttacaccacaacccagcaatgtgttattaggttcagcgcaggcaacccagtcacg tcaacctgggcgaccgatcctggcgtggcgacagcgccggcctagggcttcgcctctcc

SEQ ID NO: 38

ctcgcggccgccccgggtaccgatatcagtactaattcagtacattaaaaacgtccgcaatgtgttattaagttgtctaagcgtcaat ttgtttacaccacaacccagcaatgtgttattaggttcagcgcaggcaacccagtcacgtcaacctgggcgaccgatcctggcgtg gcgacagcgccggcctagggcttcgcctctcc

SEQ ID NO: 39

cgtccgtccgtcgtgtgcccttccctcgtgccgccccacgacttccgccccggagcgctgccctcttcctcctcccaatggcgcctca gctgcccccgctcggggctcgccctcgagtctagagtcgacgagctcctgcagccatggacgcgtaagcttcgatcgatgcctgc agtgcagcgtgacccggtcgtgcccctctctag

SEQ ID NO: 40
gaggtgccaatccacttccc
SEQ ID NO: 41
gagttaccccagttcacgttgag
SEQ ID NO: 42
acctccaccaggtacag
SEQ ID NO: 43
tgccgaatatcatggtggaa
SEQ ID NO: 44
cggccacagtcgatgaatc
SEQ ID NO: 45
tggccgcttttct
SEQ ID NO: 46
gccgcttgtagccttcca

SEQ ID NO: 47
ccgccgacctggtgga
SEQ ID NO: 48
cgtgacctcaatgcg
SEQ ID NO: 49
cacaatcgtcacctcaaccg
SEQ ID NO: 50
atcaacgaccttctggaaacg
SEQ ID NO: 51
cgcaggatttcgctctcg
SEQ ID NO: 52
tttgacaacaggactctac
SEQ ID NO: 53
tgcaatgctcattatctctag
SEQ ID NO: 54
tagcttcacctatataatacttc
SEQ ID NO: 55
tgcactgcaggcatcgatc
SEQ ID NO: 56
tcgtcgactctagactcgag
SEQ ID NO: 57
gataatgccagcctgttaaac
SEQ ID NO: 58
ggtaatccttcaaaggattttaaaaaatac
SEQ ID NO: 59
agatagagagcgggaaattgagatgtaac
SEQ ID NO: 60
tcgtccgatgcaggatcac
SEQ ID NO: 61
ggttgacgtgactgggttg
SEQ ID NO: 62
gttgacgtgactgggttgc
SEQ ID NO: 63
ggccgttactagtggatcg
SEQ ID NO: 64
gccgttactagtggatcgag
SEQ ID NO: 65
caacaacccaaacatcaacg
SEQ ID NO: 66
atggctgcaggagctcgtcgactcta
SEQ ID NO: 67
cgtcggtgtagatggtgatg
SEQ ID NO: 68
tgatgcatatacagagatgcttttt
SEQ ID NO: 69
ttcatccattttattagtacatcca
SEQ ID NO: 70
acggatgcgacctgtacg
SEQ ID NO: 71
tcagtaaaacccacatcaac
SEQ ID NO: 72
gaccacatcatcacaaccaag
SEQ ID NO: 73
gctccgaacaacacgaggttg
SEQ ID NO: 74
atgaagtattatataggtgaag
SEQ ID NO: 75
agaggctattcggctatgac

SEQ ID NO: 76
cagccgaactgttcgccagg
SEQ ID NO: 77
agcacgaggaagcggtcagc
SEQ ID NO: 78
tgagatgacaggagatcctg
SEQ ID NO: 79
caacagctccgtgagcatcatc
SEQ ID NO: 80
ccagcgacttcggttacttc
SEQ ID NO: 81
ctctcggcgtagatttggtag
SEQ ID NO: 82
cagaacaacaacgtgccacc
SEQ ID NO: 83
agcatcaccatctacaccgac
SEQ ID NO: 84
gatccaggagaacatcggagc
SEQ ID NO: 85
gtcactgggatgaactcgaac
SEQ ID NO: 86
gggtcctgtagacaccctga
SEQ ID NO: 87
gctcaccctgttgtttggtgtt
SEQ ID NO: 88
ctgtcggcatccagaaattg
SEQ ID NO: 89
tcccctcgggatcaaagta
SEQ ID NO: 90
tagcgtgtttgtgcttttgc
SEQ ID NO: 91
ggccgctgaaattaaatcaa
SEQ ID NO: 92
gagtcagtgagcgaggaagc
SEQ ID NO: 93
cggtgaaaacctctgacaca
SEQ ID NO: 94
atacaggcagcccatcagtc

## Claims

1. A Polynucleotide comprising at least 14 contiguous nucleotides of SEQ ID NO: 18.

2. The polynucleotide of claim 1, comprising at least 15 contiguous nucleotides of SEQ ID NO: 18, preferably at least 16 contiguous nucleotides of SEQ ID NO: 18, wherein preferably the polynucleotide comprises SEQ ID NO: 18, and wherein more preferably the polynucleotide comprises SEQ ID NO: 13, 5 or 22.

3. A Polynucleotide, comprising at least 14 contiguous nucleotides of SEQ ID NO: 19.

4. The polynucleotide of claim 3 comprising at least 15 contiguous nucleotides of SEQ ID NO: 19, preferably comprising at least 16 contiguous nucleotides of SEQ ID NO: 19, wherein preferably the polynucleotide comprises SEQ ID NO: 19, and more preferably wherein the polynucleotide comprises SEQ ID NO: 12, 5 or 22.

5. Primer pairs comprising forward and reverse primers wherein the forward primer comprises SEQ ID NO: 6 and the reverse primer comprises SEQ ID NO: 7, or the forward primer comprises SEQ ID NO: 8 and the reverse primer comprises SEQ ID NO: 9.

6. A method of detecting plant material from genetically modified sugarcane of event CTC79005-2, comprising the steps of:

   a) obtaining a plant material sample for analysis;
   b) extracting DNA from the sample;
   c) providing primer pairs comprising at least a forward and a reverse primer;
   d) amplifying a region between the primer pair; and
   e) detecting the presence of a product from amplification;
   or comprising the steps of

      (a) obtaining a plant material sample for analysis;

(b) extracting DNA or RNA from the sample;
(c) providing a probe or a combination of probes designed to bind to a polynucleotide comprising contiguous nucleotides of sequences selected from the group consisting of SEQ ID NO 2, SEQ ID NO 3, SEQ ID NO 4, SEQ ID NO 5, SEQ ID NO 12, SEQ ID NO 13, SEQ ID NO: 18, SEQ ID NO: 19, SEQ ID NO 22, SEQ ID NO 23, SEQ ID NO 24, SEQ ID NO 29, SEQ ID NO 30, SEQ ID NO 31, SEQ ID NO: 32, SEQ ID NO: 33, SEQ ID NO 36, SEQ ID NO 37, SEQ ID NO 38 and SEQ ID NO 39;
(d) hybridizing said probe with the sample; and
(e) detecting the actual hybridization of the probe.

**7.** The method of claim 6, wherein the primer pairs in step c) are designed to bind to a polynucleotide comprising contiguous nucleotides of sequences selected from the group consisting of SEQ ID NO: 22 and SEQ ID NO: 29, wherein at least one pair of primers comprises contiguous nucleotides sequences selected from the group consisting of SEQ ID NO: 23, SEQ ID NO: 24, SEQ ID NO: 30 and SEQ ID NO: 31 and optionally at least one primer pair consists of a first primer comprising contiguous nucleotides of sequences selected from the group consisting of SEQ ID NO: 23, SEQ ID NO: 24, SEQ ID NO: 30 and SEQ ID NO: 31 and a second primer comprising contiguous nucleotides of sequences selected from the group consisting of SEQ ID NO: 2 and SEQ ID NO: 36; or wherein the primer pairs in step c) are designed to bind to a polynucleotide comprising contiguous nucleotides of sequences selected from the group consisting of SEQ ID NO: 5 and SEQ ID NO: 37, wherein at least one pair of primers comprises contiguous nucleotides of sequences selected from the group consisting of SEQ ID NO: 3, SEQ ID NO: 4, SEQ ID NO: 38 and SEQ ID NO: 39; and optionally at least one primer pair consists of a first primer comprising contiguous nucleotides of sequences selected from the group consisting of SEQ ID NO: 3, SEQ ID NO: 4, SEQ ID NO: 38 and SEQ ID NO: 39 and a second primer comprising contiguous nucleotides of sequences selected from the group consisting of SEQ ID NO: 2 and SEQ ID NO: 36; wherein more preferably the forward primer comprises SEQ ID NO: 6 and the reverse primer comprises SEQ ID NO: 7, or the forward primer comprises SEQ ID NO: 8 and the reverse primer comprises SEQ ID NO: 9.; and wherein preferably the product from amplification comprises SEQ ID NO: 12 or SEQ ID NO: 13, wherein optionally detection of the product from amplification is performed through hybridization of a probe comprising SEQ ID NO: 10 or SEQ ID NO: 11.

**8.** A kit for detecting material from transgenic sugarcane comprising a Cry1Ac protein (event CTC79005-2) comprising a means to detect the presence of a polynucleotide comprising, at least, 14 contiguous nucleotides of SEQ ID NO: 18 and/or of SEQ ID NO: 19 and/or a pesticidal crystal protein (Cry), wherein preferably the means comprise primer pairs designed to bind to a polynucleotide comprising contiguous nucleotides of sequences selected from the group consisting of SEQ ID NO: 22 and SEQ ID NO: 29, wherein at least one pair of primers comprises contiguous nucleotides sequences selected from the group consisting of SEQ ID NO: 23, SEQ ID NO: 24, SEQ ID NO: 30 and SEQ ID NO: 31, and wherein preferably the means comprise a probe comprising SEQ ID NO: 10 or SEQ ID NO: 11.

**9.** Genetic construct comprising SEQ ID NO: 1 or SEQ ID NO:2, preferably comprising SEQ ID NO: 14.

**10.** A genetically modified sugarcane (Saccharum spp.) plant or a plant part, plant cell, plant tissue, or seed thereof comprising SEQ ID NO: 18 or SEQ ID NO: 19; or comprising SEQ ID NO: 12 or SEQ ID NO: 13; or comprising SEQ ID NO: 5 or SEQ ID NO: 22, wherein the plant is insect-resistant.

**11.** A tissue culture of the genetically modified sugarcane (Saccharum spp.) plant of claim 10; and optionally a genetically modified sugarcane (Saccharum spp.) plant regenerated therefrom, wherein the regenerate plant comprises SEQ ID NO: 18 or SEQ ID NO: 19.

**12.** A commodity product produced from the genetically modified sugarcane (Saccharum spp.) plant of claim 10.

**13.** A method of producing a genetically modified sugarcane (Saccharum spp.) plant of event CTC79005-2, comprising the steps of:

a) introducing a genetic construct comprising SEQ ID NO: 20 and SEQ ID NO: 21 into an Agrobacterium strain;
b) obtaining embryogenic callus from immature leaf rolls or top stalks of sugarcane (Saccharum spp.);
c) co-cultivating embryogenic callus with a culture of Agrobacterium;
d) selecting transformed cells containing the functional fragment in culture medium containing aminoglycoside antibiotics; and
e) regenerating transformed sugarcane plants, wherein the genetically modified sugarcane plants comprise SEQ ID NO: 20 and SEQ ID NO: 21.

14. A method of making a genetically modified sugarcane (Saccharum spp.) plant of event CTC79005-2, comprising introducing a genetic modification to a sugarcane (Saccharum spp.) plant comprising SEQ ID NO: 5 or SEQ ID NO: 22 to produce a genetically modified sugarcane (Saccharum spp.) plant of event CTC79005-2, wherein the genetically modified sugarcane (Saccharum spp.) plant has improved insect resistance as compared to a sugarcane (Saccharum spp.) plant without the genetic modification.

15. A method of cultivating a genetically modified sugarcane (Saccharum spp.) plant of event CTC79005-2, comprising growing a genetically modified sugarcane (Saccharum spp.) plant of event CTC79005-2 comprising SEQ ID NO: 5 or SEQ ID NO: 22 under conditions comprising insect infestation, wherein the genetically modified sugarcane (Saccharum spp.) plant has an increase in insect resistance as compared to a sugarcane (Saccharum spp.) plant without the genetic modification grown under the same conditions.

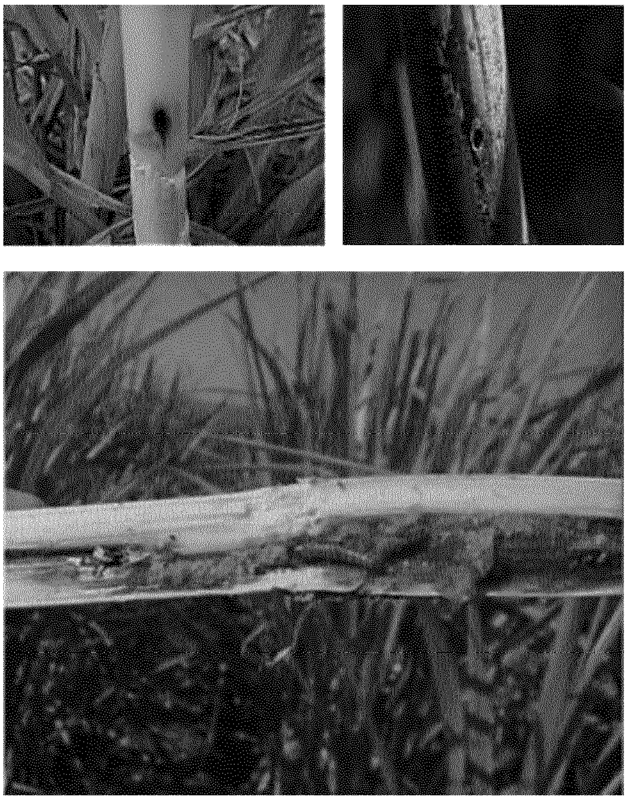

Figure 1

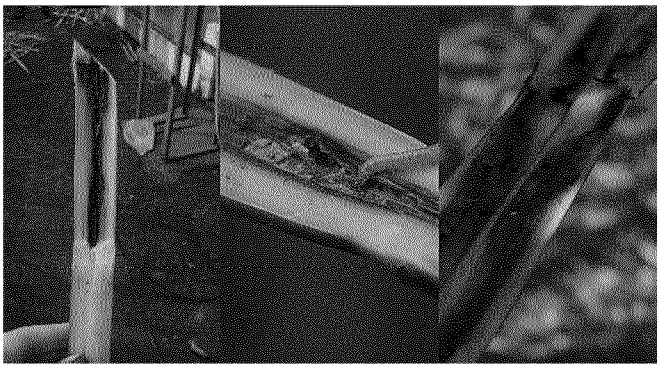

Figure 2

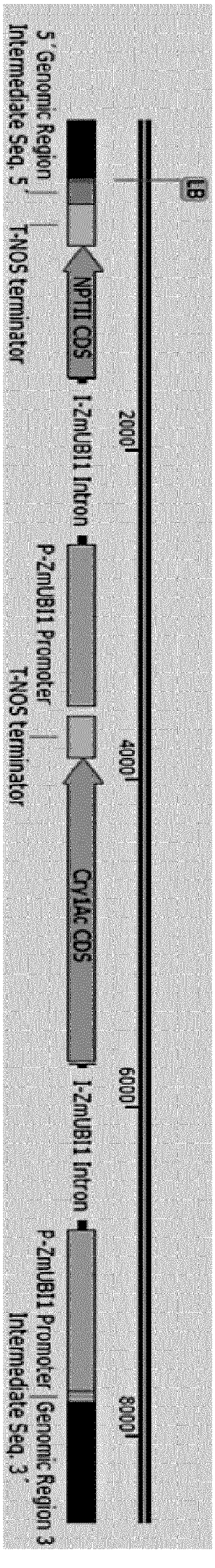
LB
2000
4000
6000
8000
5' Genomic Region
Intermediate Seq. 5'
T-NOS terminator
NPTII CDS
I-ZmUBI1 Intron
P-ZmUBI1 Promoter
T-NOS terminator
Cry1Ac CDS
I-ZmUBI1 Intron
P-ZmUBI1 Promoter
Intermediate Seq. 3'
Genomic Region 3'

Figure 3

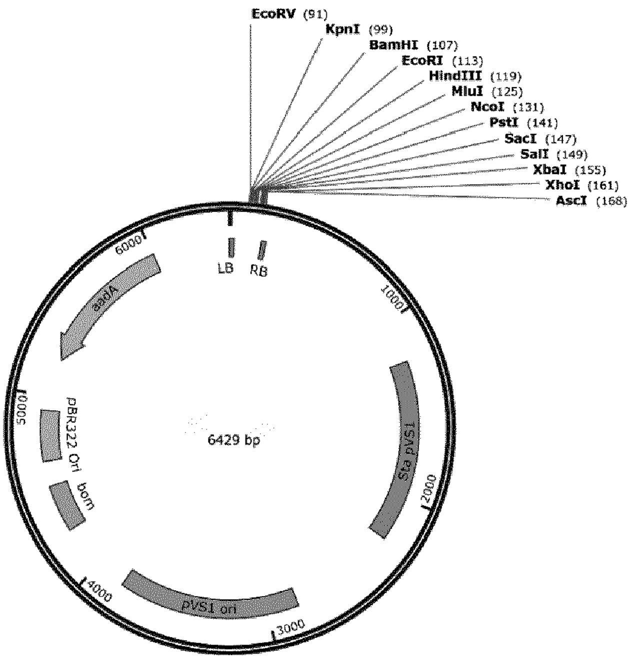


Figure 4

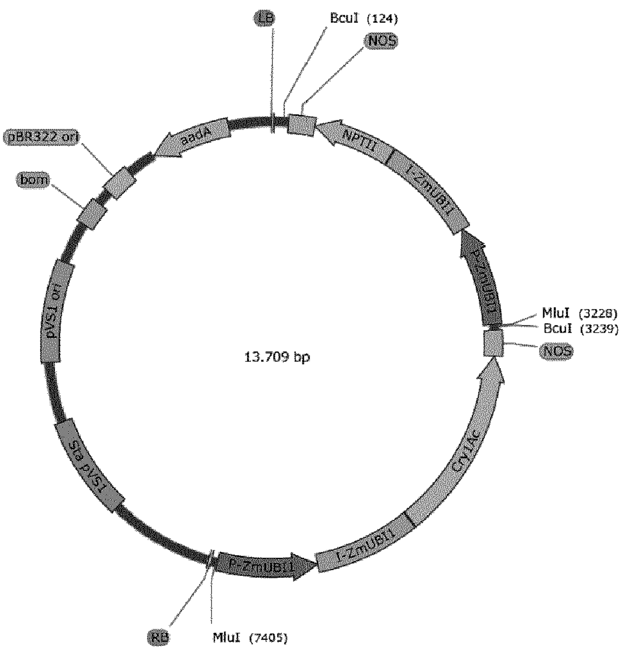


Figure 5

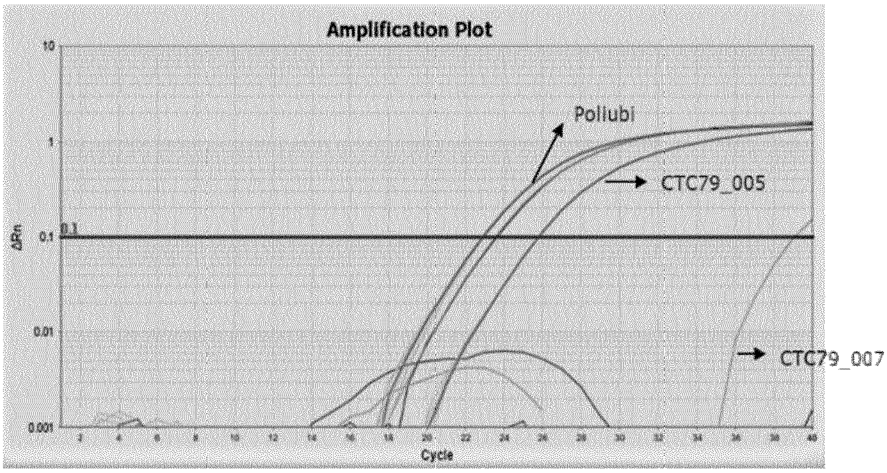


Figure 6

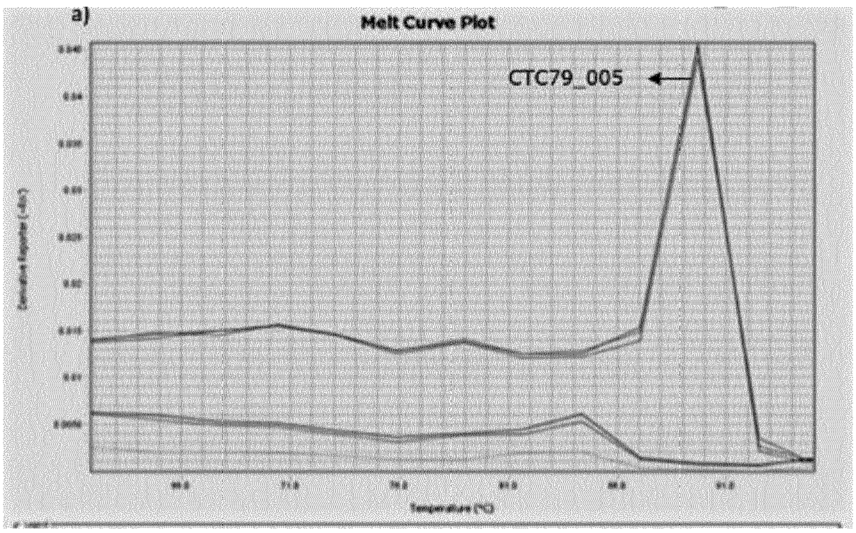


Figure 7

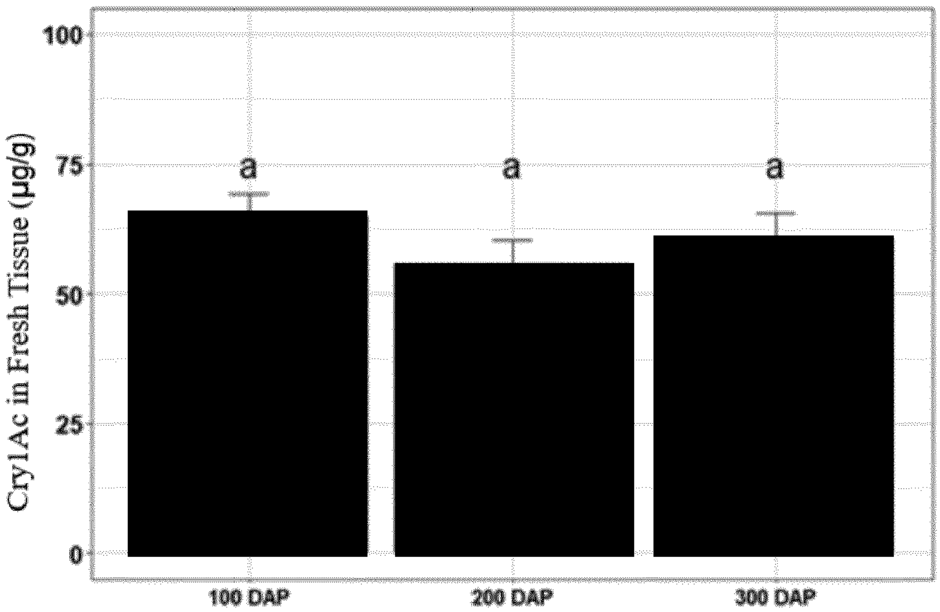


Figure 8

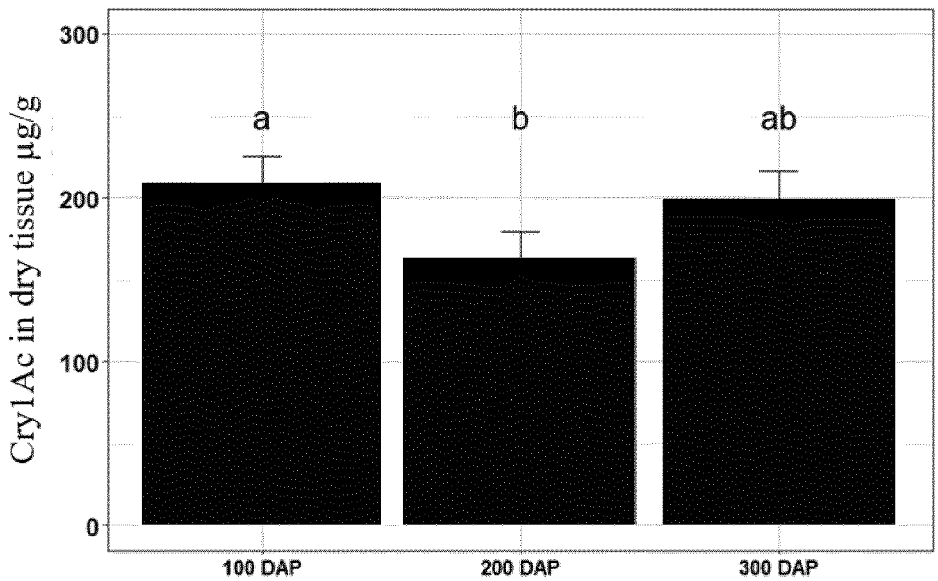


Figure 9

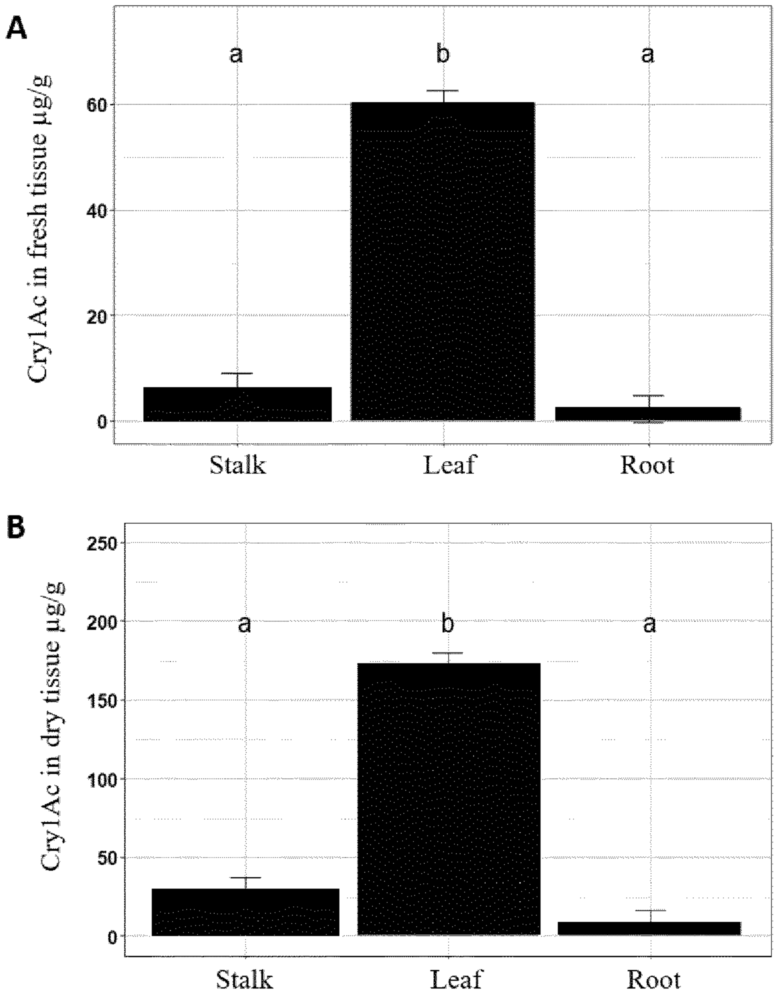
A
Cry1Ac in fresh tissue µg/g
0
20
40
60
a
b
a
Stalk
Leaf
Root
B
Cry1Ac in dry tissue µg/g
0
50
100
150
200
250
a
b
a
Stalk
Leaf
Root

Figure 10

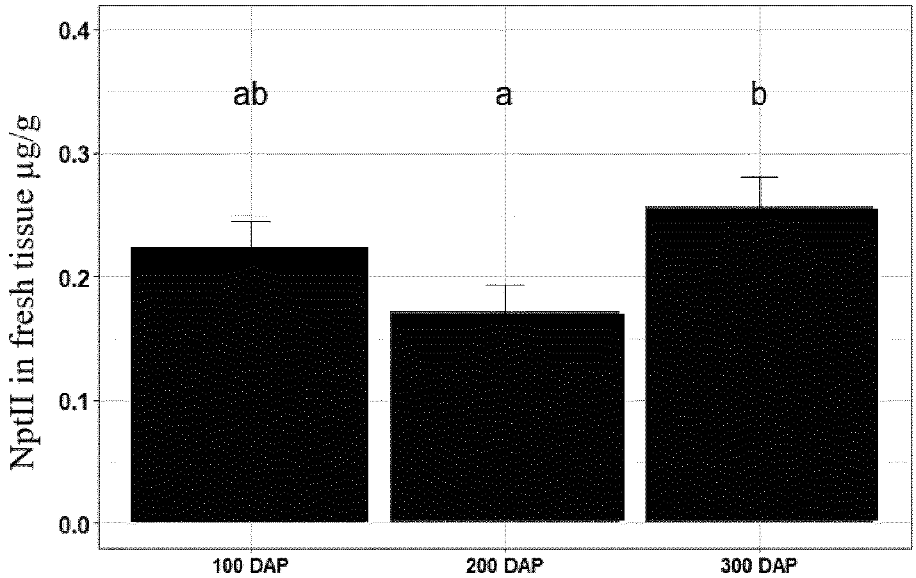


Figure 11

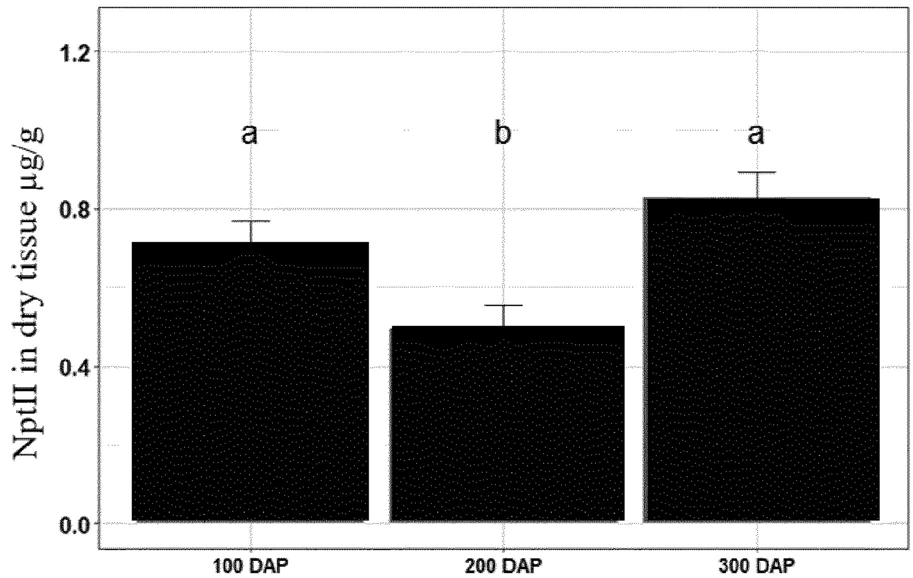


Figure 12

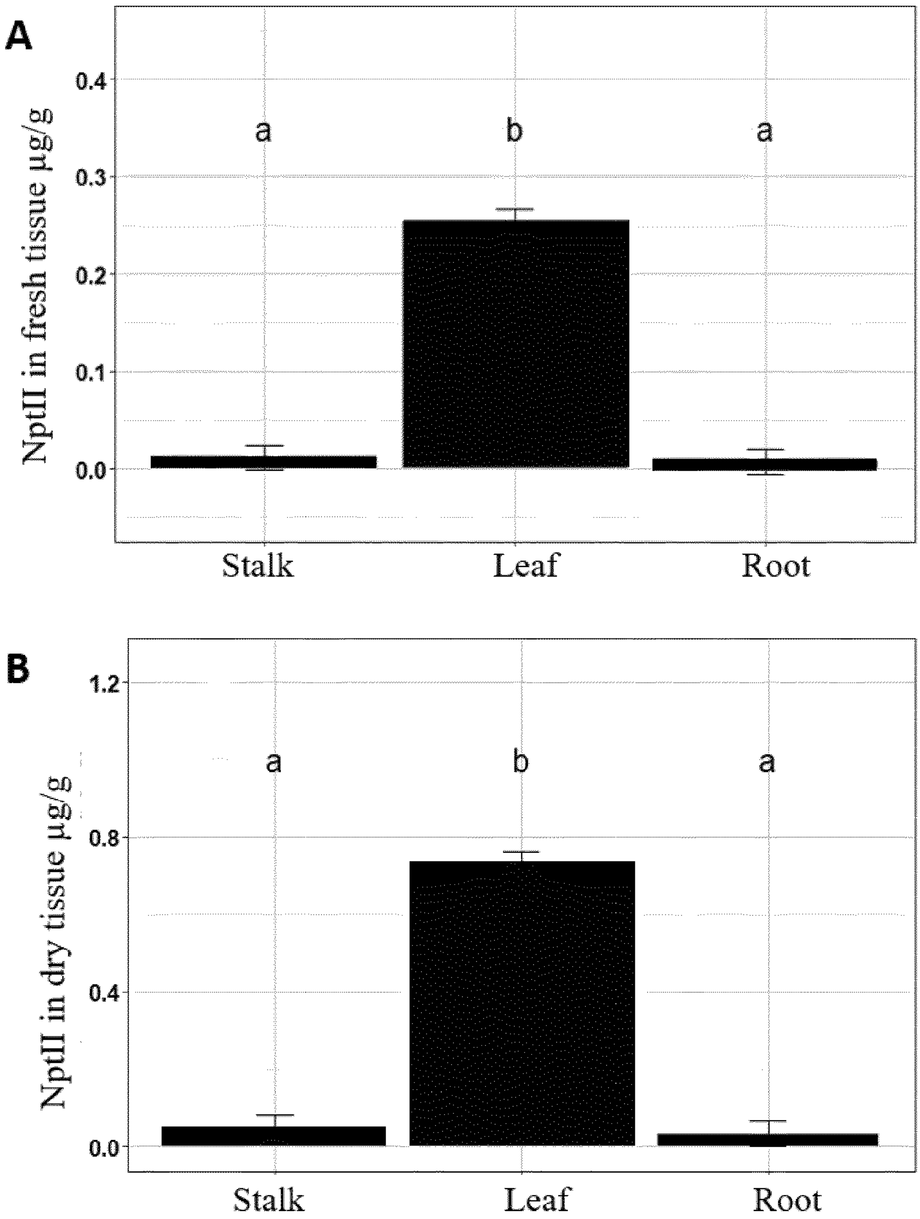
A
NptII in fresh tissue µg/g
0.4
0.3
0.2
0.1
0.0
a
b
a
Stalk
Leaf
Root
B
NptII in dry tissue µg/g
1.2
0.8
0.4
0.0
a
b
a
Stalk
Leaf
Root

Figure 13

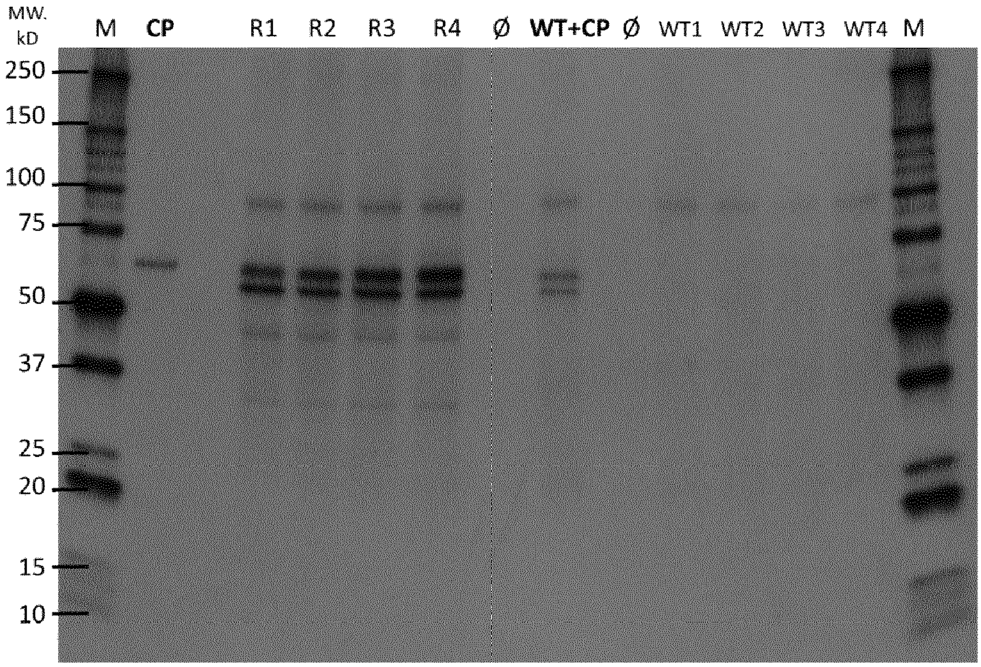


Figure 14

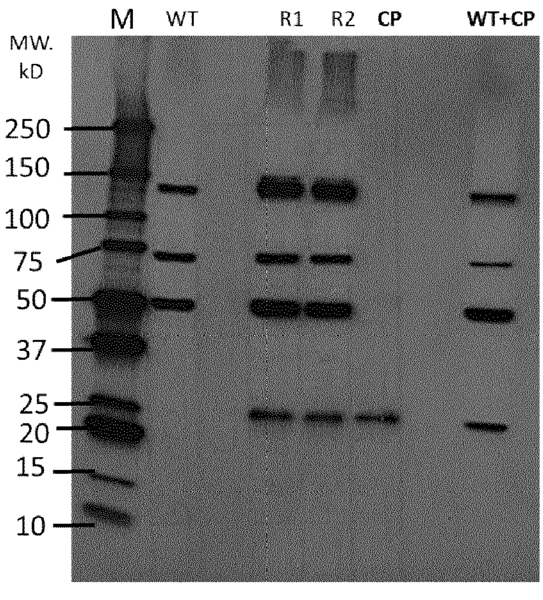


Figure 15

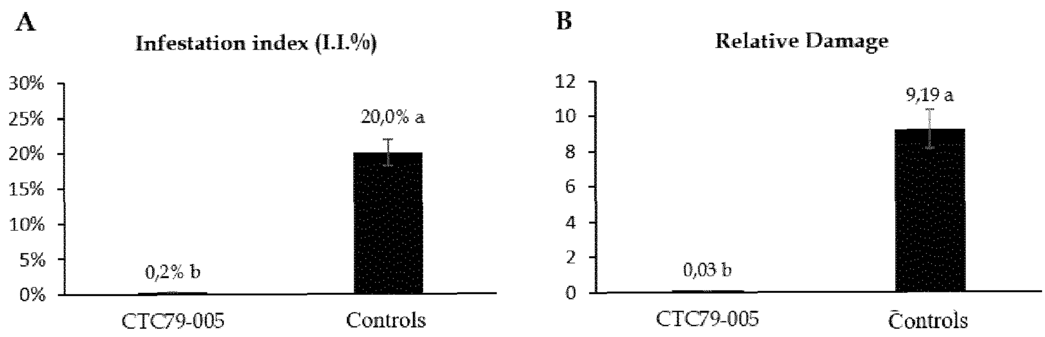


Figure 16

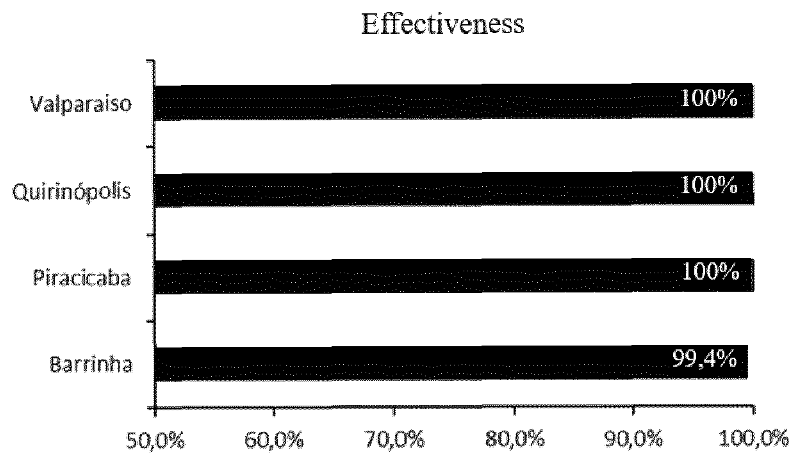


Figure 17

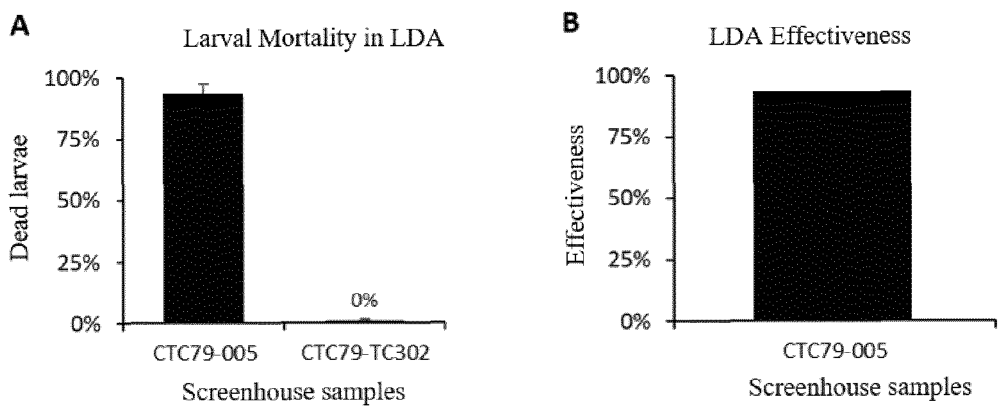

C

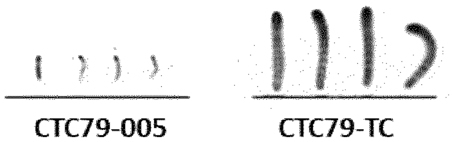


Figure 18

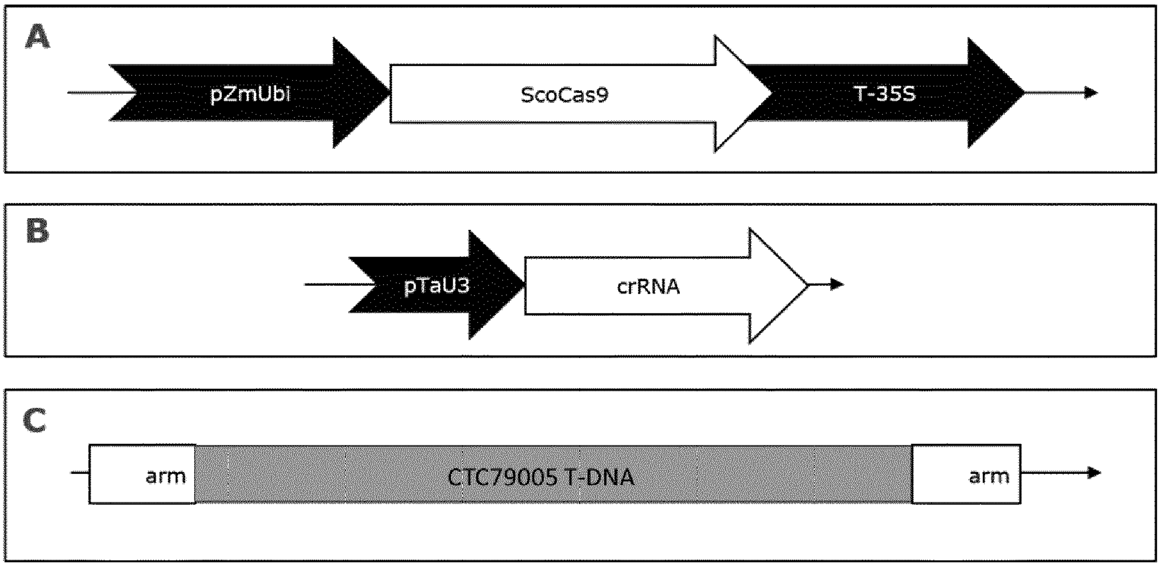


Figure 19

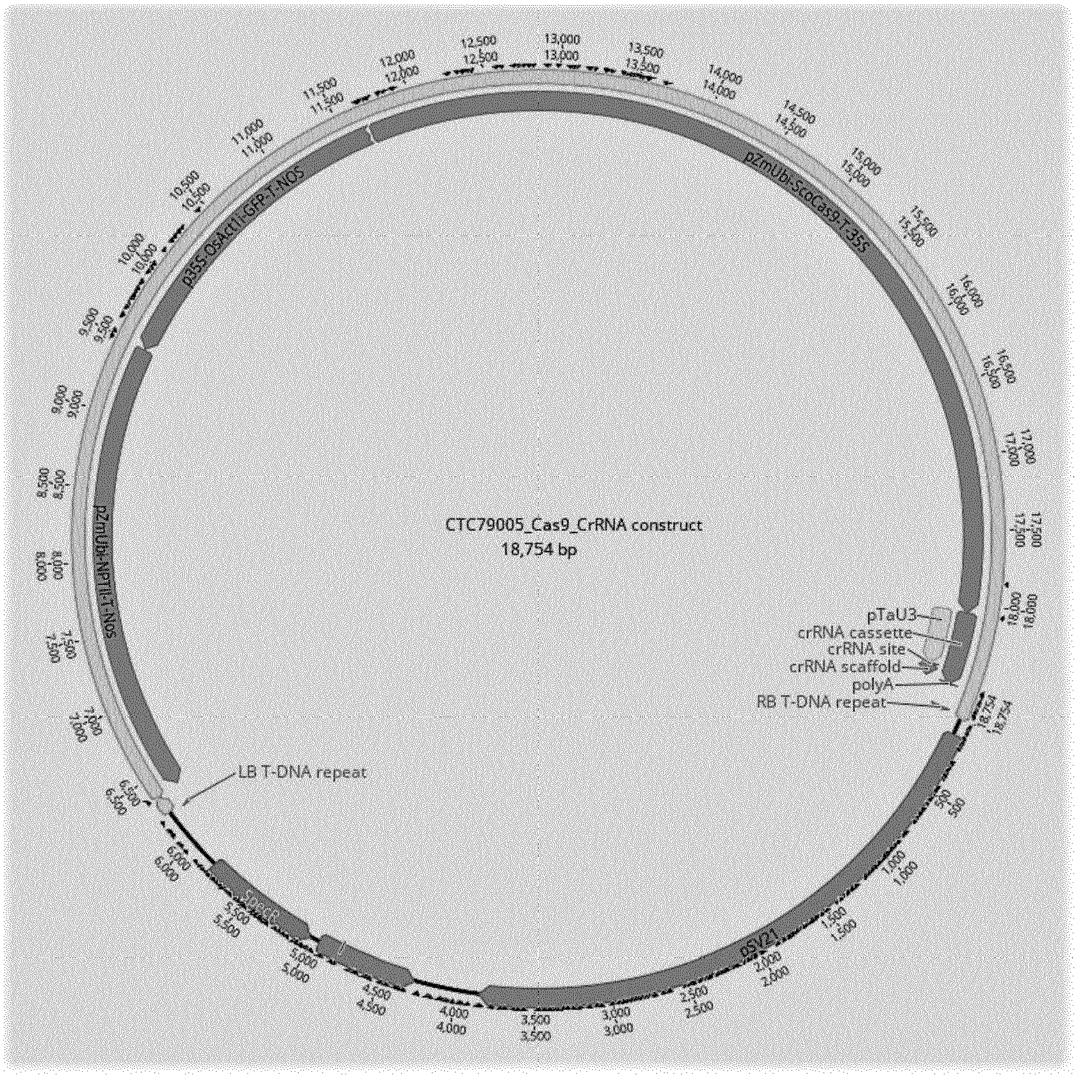
CTC79005_Cas9_CrRNA construct
18,754 bp
pZmUbi-ScoCas9-T-35S
p35S-OsAct1i-GFP-T-NOS
pZmUbi-NPTII-T-Nos
pTaU3
crRNA cassette
crRNA site
crRNA scaffold
polyA
RB T-DNA repeat
LB T-DNA repeat
SpecR
pSV21

Figure 20

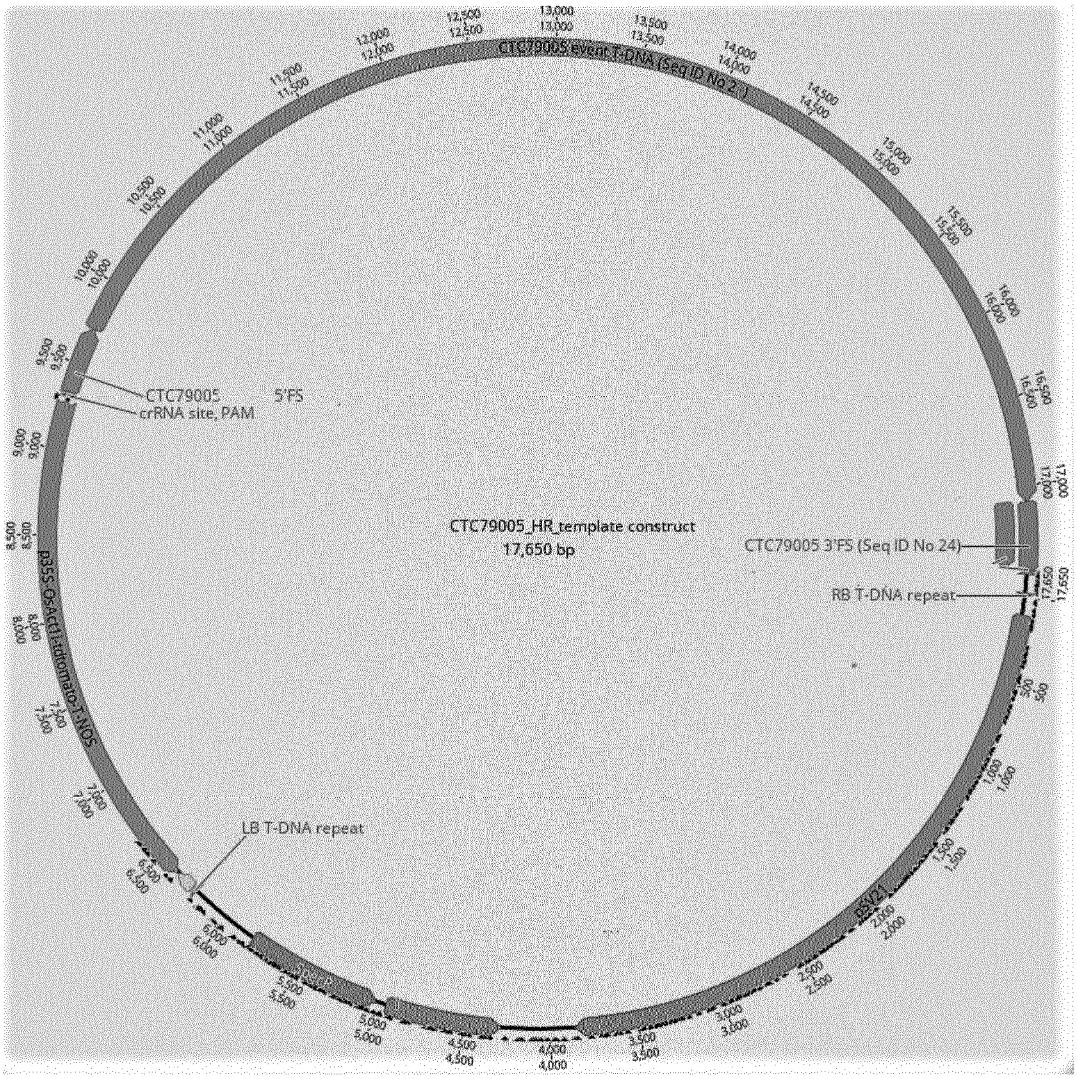
CTC79005 event T-DNA (Seq ID No 2 )
CTC79005 5'FS
crRNA site, PAM
CTC79005_HR_template construct
17,650 bp
CTC79005 3'FS (Seq ID No 24)
RB T-DNA repeat
p35S-OsAct1-tdtomato-T-NOS
LB T-DNA repeat
SpecR
pSV21

Figure 21

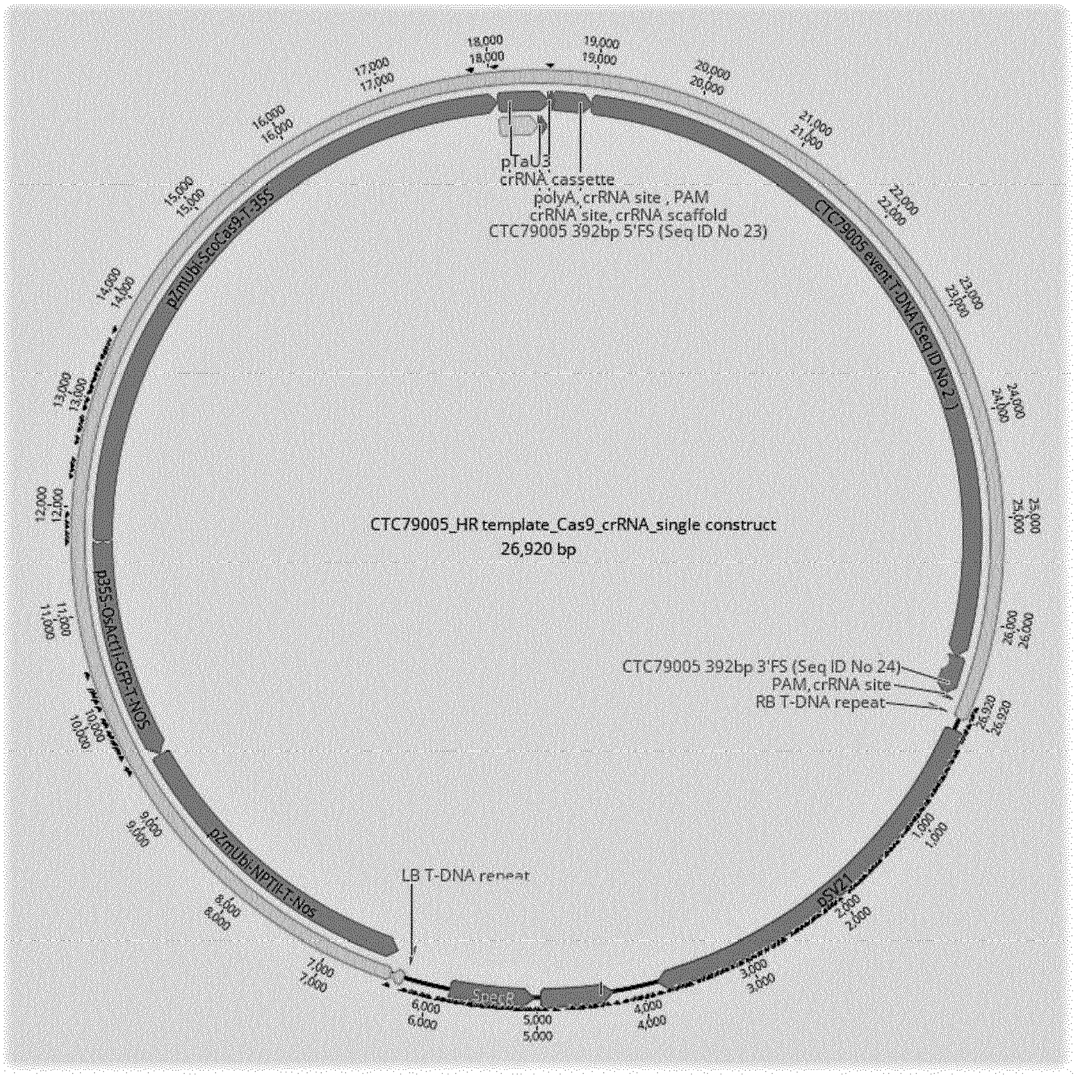


Figure 22

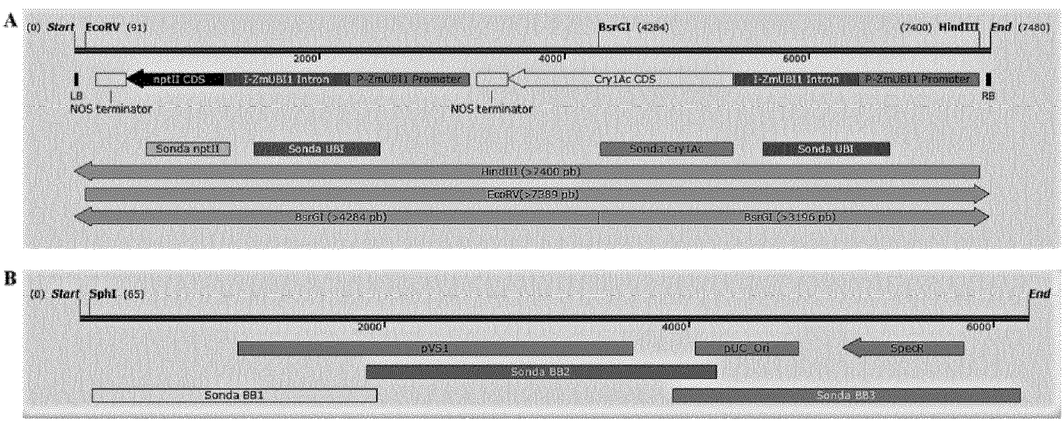


Figure 23

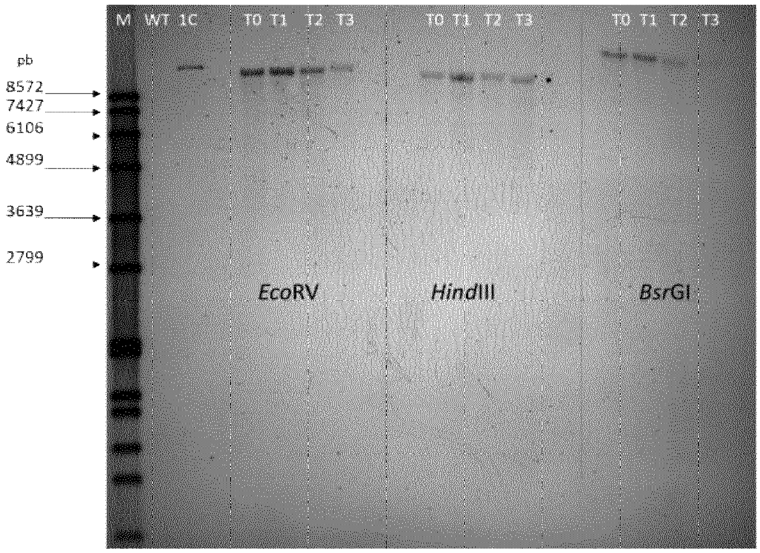


Figure 24

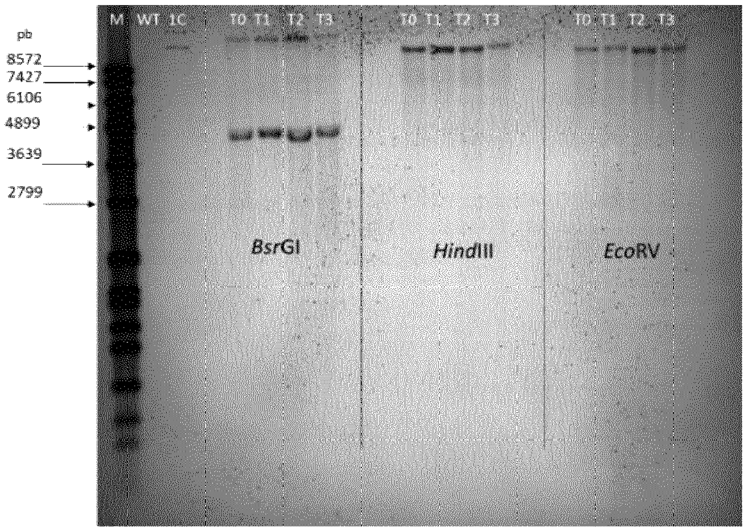


Figure 25

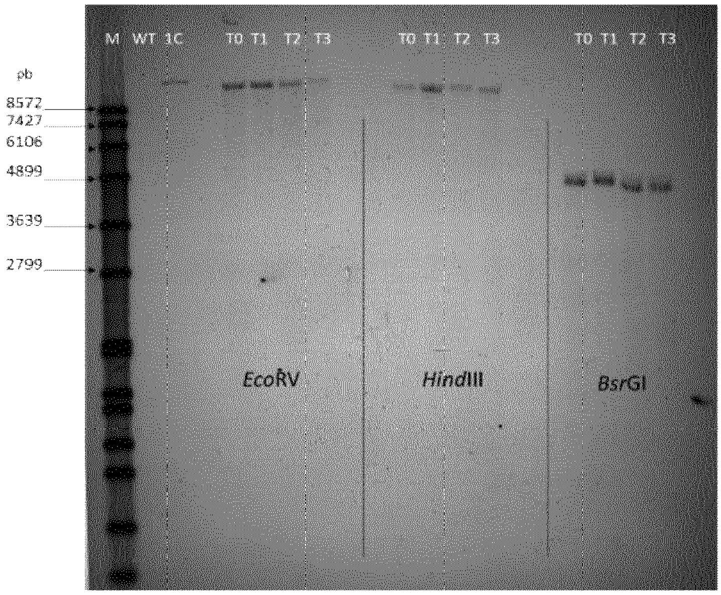


Figure 26

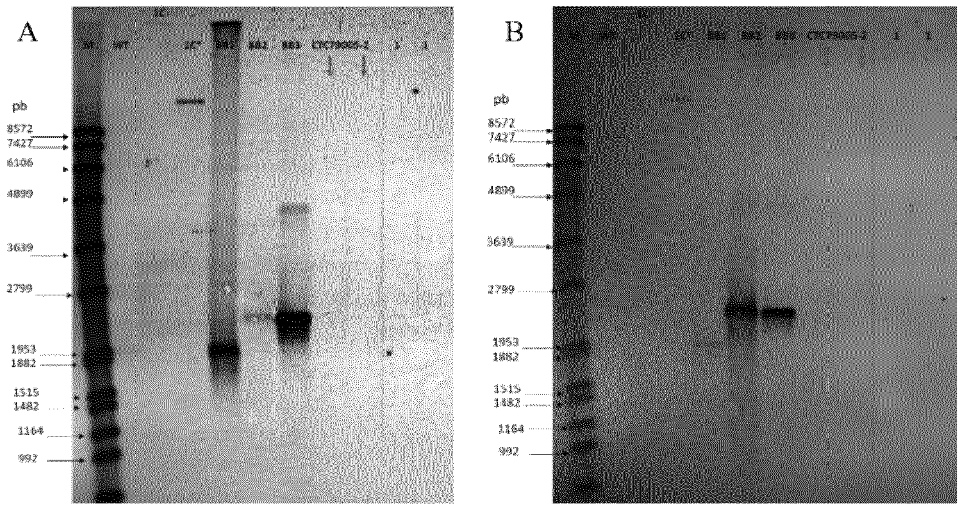


Figure 27

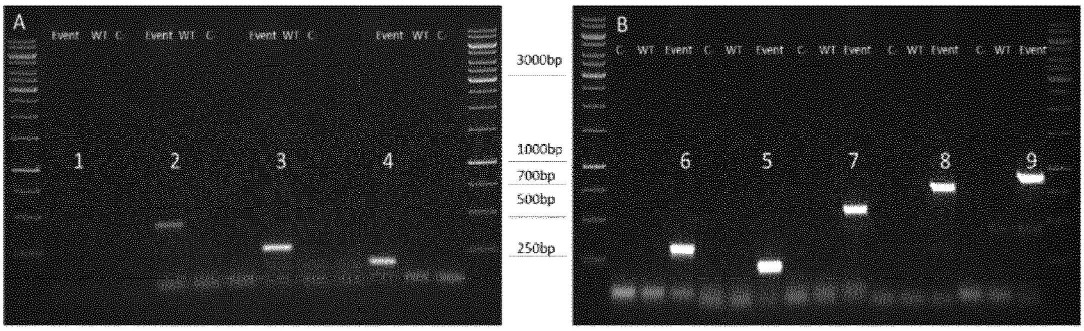
A
Event WT C Event WT C Event WT C Event WT C
1 2 3 4
3000bp
1000bp
700bp
500bp
250bp
B
C WT Event C WT Event C WT Event C WT Event C WT Event
6 5 7 8 9

Figure 28

Europäisches Patentamt
European Patent Office
Office européen des brevets

# EUROPEAN SEARCH REPORT

Application Number
EP 20 20 7634

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br>Y | GAO SHIWU ET AL: "Transgenic Sugarcane with a cry1Ac Gene Exhibited Better Phenotypic Traits and Enhanced Resistance against Sugarcane Borer", PLOS ONE, vol. 11, no. 4, 19 April 2016 (2016-04-19), page e0153929, XP055795869, DOI: 10.1371/journal.pone.0153929<br>* abstract *<br>----- | 1-15<br><br><br><br><br><br><br><br>9,13 | INV.<br>C12N15/82<br>C07K14/325 |
| X<br><br><br><br><br><br><br><br><br><br><br>Y | ZHOU DINGGANG ET AL: "Foreign cry1Ac gene integration and endogenous borer stress-related genes synergistically improve insect resistance in sugarcane", BMC PLANT BIOLOGY, vol. 18, no. 1, 1 December 2018 (2018-12-01), XP055795875, DOI: 10.1186/s12870-018-1536-6 Retrieved from the Internet: URL:http://link.springer.com/content/pdf/10.1186/s12870-018-1536-6.pdf><br>* abstract *<br>----- | 1-15<br><br><br><br><br><br><br><br><br><br><br>9,13 | TECHNICAL FIELDS SEARCHED (IPC) |
| X<br><br><br><br><br><br><br><br><br><br><br><br>Y | ADRIANA C. GIANOTTO ET AL: "The insect-protected CTC91087-6 sugarcane event expresses Cry1Ac protein preferentially in leaves and presents compositional equivalence to conventional sugarcane", GM CROPS & FOOD, vol. 10, no. 4, 20 August 2019 (2019-08-20), pages 208-219, XP055766948, ISSN: 2164-5698, DOI: 10.1080/21645698.2019.1651191<br>* abstract *<br>-----<br>-/-- | 1-15<br><br><br><br><br><br><br><br><br><br><br><br>9,13 | C12N<br>C12R<br>C07K |

The present search report has been drawn up for all claims

1

| Place of search | Date of completion of the search | Examiner |
|---|---|---|
| Munich | 20 April 2021 | Mundel, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 20 20 7634

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | WENG LI-XING ET AL: "Regeneration of sugarcane elite breeding lines and engineering of stem borer resistance", PEST MANAGEMENT SCIENCE, vol. 62, no. 2, 1 January 2006 (2006-01-01), pages 178-187, XP055795879, ISSN: 1526-498X, DOI: 10.1002/ps.1144 Retrieved from the Internet: URL:https://onlinelibrary.wiley.com/doi/pdfdirect/10.1002/ps.1144><br>* abstract *<br>* 2.4. Sugarcane tissue culture and transformation; page 180 *<br>----- | 1-15<br><br>9,13 | |
| X | DATABASE EMBL [Online]<br>23 September 2018 (2018-09-23), Burga et al.: "Nannopterum auritus isolate PW-MI-E2 scaffold36", XP055796806, Database accession no. NFMC01000031<br>* sequence *<br>----- | 3 | TECHNICAL FIELDS SEARCHED (IPC) |
| Y | CN 102 250 923 B (UNIV FUDAN; GUANGZHOU SUGARCANE INDUSTRY RES INST) 16 October 2013 (2013-10-16)<br>* abstract *<br>* sequence 1 *<br>----- | 9,13 | |

1 The present search report has been drawn up for all claims

| Place of search | Date of completion of the search | Examiner |
|---|---|---|
| Munich | 20 April 2021 | Mundel, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 20 20 7634

This annex lists the patent family members relating to the patent documents cited in the above-mentioned European search report. The members are as contained in the European Patent Office EDP file on
The European Patent Office is in no way liable for these particulars which are merely given for the purpose of information.

20-04-2021

| Patent document cited in search report | Publication date | Patent family member(s) | Publication date |
|---|---|---|---|
| CN 102250923 B | 16-10-2013 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SOUZA et al.** *D'Hont & Glaszmann,* 2011, vol. 109 (1-3), 27-33